# EUROPEAN PATENT SPECIFICATION

(11) **EP 0 741 783 B1**
(45) Date of publication and mention of the grant of the patent: **18.03.2009**
(21) Application number: 95906736.4
(22) Date of filing: 30.12.1994
(51) Int. Cl.: C12N 15/12, C07K 14/46, C07K 14/435, A61K 48/00

(54) **VERTEBRATE EMBRYONIC PATTERN-INDUCING HEDGEHOG-LIKE PROTEINS**
"HEDGEHOG-LIKE" PROTEINE VON WIRBELTIEREN, DIE EMBRYONALE STRUKTUREN INDUZIEREN
PROTEINES DE TYPE "HEDGEHOG" INDUISANT UNE STRUCTURE EMBRYONNAIRE CHEZ LES VERTEBRES

(30) Priority: 30.12.1993 US 176427; 14.12.1994 US 356060
(43) Date of publication of application: 13.11.1996
(73) Proprietor: President and Fellows of Harvard College, Cambridge, Massachusetts 02138 (US); IMPERIAL CANCER RESEARCH TECHNOLOGY LIMITED, London WC2A 3NL (GB)
(72) Inventor: INGHAM, Philip W., Summertown, Oxford OX2 7LE (GB); MCMAHON, Andrew, P., Lexington, MA 02173 (US); TABIN, Clifford, J., Cambridge, MA 02138 (US)
(74) Representative: Horner, Martin Grenville
(86) International application number: PCT/US1994/014992
(87) International publication number: WO 1995/018856

(56) References cited:
- DEVELOPMENT, vol.0, no.SUPP, 1994 pages 43 - 51 FIETZ, M. ET AL. 'The hedgehog gene family in Drosophila and vertebrate development'
- CELL., vol.75, no.7, 31 December 1993, CAMBRIDGE, NA US pages 1401 - 1416 RIDDLE, R.D. ET AL. 'Sonic hedgehog mediates the polarizing activity of the ZPA'
- CELL., vol.75, no.7, 31 December 1993, CAMBRIDGE, NA US pages 1417 - 1430 ECHELARD, Y. ET AL. 'Sonic hedgehog a member of a family of putative signaling molecules, is implicated in the regulation of CNS polarity'
- CELL., vol.76, no.4, 25 February 1994, CAMBRIDGE, NA US pages 761 - 775 ROELINK, H. ET AL. 'Floor plate and motor neuron induction by vhh-1, a vertebrate homolog of hedgehog expressed by the notochord'

### SEQUENCE LISTING

(1) GENERAL INFORMATION:
   (i) APPLICANT:
      (A) NAME: President and Fellows of Harvard College
      (B) STREET: 124 Mt. Auburn Street
      (C) CITY: Cambridge
      (D) STATE: MA
      (E) COUNTRY: USA
      (F) POSTAL CODE (ZIP): 02138

      (A) NAME: Imperial Cancer Research Technology Ltd.
      (B) STREET: Sardinia Street
      (C) CITY: London
      (D) STATE: N/A
      (E) COUNTRY: United Kingdom
      (F) POSTAL CODE (ZIP): WC2A3NL
   (ii) TITLE OF INVENTION: Vertebrate Embryonic Pattern-Inducing Proteins and Uses Related Thereto
   (iii) NUMBER OF SEQUENCES: 47
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE:
   (v) CURRENT APPLICATION DATA: APPLICATION NUMBER:
   (vi) PRIOR APPLICATION DATA:
      (A) APPLICATION NUMBER: US 08/176,427
      (B) FILING DATE: 30-DEC-1993
   (vi) PRIOR APPLICATION DATA:
      (A) APPLICATION NUMBER: US 08/356,060
      (B) FILING DATE: 14-DEC-1994
   (viii) ATTORNEY/AGENT INFORMATION:
      (A) NAME: Vincent, Matthew P.
      (B) REGISTRATION NUMBER: 36, 709
      (C) REFERENCE/DOCKET NUMBER: HMI-006PC
   (ix) TELECOMMUNICATION INFORMATION:
      (A) TELEPHONE: (617) 227-7400
      (B) TELEFAX: (617) 227-5941
(2) INFORMATION FOR SEQ ID NO:1:
   (i) SEQUENCE CHARACTERISTICS:
      (A) LENGTH: 1278 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1277
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 1190 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1191
   (xi) SEQUENCE DESCRIPTION: SEQ ID NO:2:
   ATG GCT CTG CCG GCC AGT CTG TTG CCC CTG TGC TGC TTG GCA CTC TTG      48
   GCA CTA TCT GCC CAG AGC TGC GGG CCG GGC CGA GGA CCG GTT GGC CGG      96
   CGG CGT TAT GTG CGC AAG CAA CTT GTG CCT CTG CTA TAC AAG CAG TTT     144
   GTG CCC AGT ATG CCC GAG CGG ACC CTG GGC GCG AGT GGG CCA GCG GAG     192
   GGG AGG GTA ACA AGG GGG TCG GAG CGC TTC CGG GAC CTC GTA CCC AAC     240
   TAC AAC CCC GAC ATA ATC TTC AAG GAT GAG GAG AAC AGC GGC GCA GAC     288
   CGC CTG ATG ACA GAG CGT TGC AAA GAG CGG GTG AAC GCT CTA GCC ATC     336
   GCG GTG ATG AAC ATG TGG CCC GGA GTA CGC CTA CGT GTG ACT GAA GGC     384
   TGG GAC GAG GAC GGC CAC CAC GCA CAG GAT TCA CTC CAC TAC GAA GGC     432
   CGT GCC TTG GAC ATC ACC ACG TCT GAC CGT GAC CGT AAT AAG TAT GGT      480
   TTG TTG GCG CGC CTA GCT GTG GAA GCC GGA TTC GAC TGG GTC TAC TAC      528
   GAG TCC CGC AAC CAC ATC CAC GTA TCG GTC AAA GCT GAT AAC TCA CTG      576
   GCG GTC CGA GCC GGA GGC TGC TTT CCG GGA AAT GCC ACG GTG CGC TTG      624
   CGG AGC GGC GAA CGG AAG GGG CTG AGG GAA CTA CAT CGT GGT GAC TGG      672
   GTA CTG GCC GCT GAT GCA GCG GGC CGA GTG GTA CCC ACG CCA GTG CTG      720
   CTC TTC CTG GAC CGG GAT CTG CAG CGC CGC GCC TCG TTC GTG GCT GTG      768
   GAG ACC GAG CGG CCT CCG CGC AAA CTG TTG CTC ACA CCC TGG CAT CTG      816
   GTG TTC GCT GCT CGC GGG CCA GCG CCT GCT CCA GGT GAC TTT GCA CCG      864
   GTG TTC GCG CGC CGC TTA CGT GCT GGC GAC TCG GTG CTG GCT CCC GGC      912
   GGG GAC GCG CTC CAG CCG GCG CGC GTA GCC CGC GTG GCG CGC GAG GAA      960
   GCC GTG GGC GTG TTC GCA CCG CTC ACT GCG CAC GGG ACG CTG CTG GTC     1008
   AAC GAC GTC CTC GCC TCC TGC TAC GCG GTT CTA GAG AGT CAC CAG TGG     1056
   GCC CAC CGC GCC TTC GCC CCT TTG CGG CTG CTG CAC GCG CTC GGG GCT     1104
   CTG CTC CCT GGG GGT GCA GTC CAG CCG ACT GGC ATG CAT TGG TAC TCT      1152
   CGC CTC CTT TAC CGC TTG GCC GAG GAG TTA ATG GGC TG                  1190
(2) INFORMATION FOR SEQ ID NO:3:
   (i) SEQUENCE CHARACTERISTICS:
      (A) LENGTH: 1056 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1056
   (xi) SEQUENCE DESCRIPTION: SEQ ID NO:3:
   GAG CGC TTC AAA GAG CTC ACC CCC AAC TAC AAT CCC GAC ATC ATC TTC      48
   Glu Arg Phe Lys Glu Leu Thr Pro Asn Tyr Asn Pro Asp Ile Ile Phe
    1               5                  10                  15
   AAG GAC GAG GAG AAC ACG GGT GCC GAC CGC CTC ATG ACC CAG CGC TGC      96
   Lys Asp Glu Glu Asn Thr Gly Ala Asp Arg Leu Met Thr Gln Arg Cys
               20                  25                  30
   AAG GAC CGT CTG AAC TCA CTG GCC ATC TCT GTC ATG AAC CAG TGG CCT     144
   Lys Asp Arg Leu Asn Ser Leu Ala Ile Ser Val Met Asn Gln Trp Pro
           35                  40                  45
   GGT GTG AAA CTG CGG GTG ACC GAA GGC CGG GAT GAA GAT GGC CAT CAC     192
   Gly Val Lys Leu Arg Val Thr Glu Gly Arg Asp Glu Asp Gly His His
       50                  55                  60
   TCA GAG GAG TCT TTA CAC TAT GAG GGC CGC GCG GTG GAT ATC ACC ACC     240
   Ser Glu Glu Ser Leu His Tyr Glu Gly Arg Ala Val Asp Ile Thr Thr
   65                  70                  75                  80
   TCA GAC CGT GAC CGA AAT AAG TAT GGA CTG CTG GCG CGC TTA GCA GTG     288
   Ser Asp Arg Asp Arg Asn Lys Tyr Gly Leu Leu Ala Arg Leu Ala Val
                   85                  90                  95
   GAG GCC GGC TTC GAC TGG GTG TAT TAC GAG TCC AAG GCC CAC GTG CAT     336
   Glu Ala Gly Phe Asp Trp Val Tyr Tyr Glu Ser Lys Ala His Val His
               100                 105                 110
   TGC TCT GTC AAG TCT GAG CAT TCG GCC GCT GCC AAG ACA GGT GGC TGC     384
   Cys Ser Val Lys Ser Glu His Ser Ala Ala Ala Lys Thr Gly Gly Cys
           115                 120                 125
   TTT CCT GCC GGA GCC CAG GTG CGC CTA GAG AAC GGG GAG CGT GTG GCC      432
   Phe Pro Ala Gly Ala Gln Val Arg Leu Glu Asn Gly Glu Arg Val Ala
       130                 135                 140
   CTG TCA GCT GTA AAG CCA GGA GAC CGG GTG CTG GCC ATG GGG GAG GAT      480
   Leu Ser Ala Val Lys Pro Gly Asp Arg Val Leu Ala Met Gly Glu Asp
   145                 150                 155                 160
   GGG ACC CCC ACC TTC AGT GAT GTG CTT ATT TTC CTG GAC CGC GAG CCA      528
   Gly Thr Pro Thr Phe Ser Asp Val Leu Ile Phe Leu Asp Arg Glu Pro
                   165                 170                 175
   AAC CGG CTG AGA GCT TTC CAG GTC ATC GAG ACT CAG GAT CCT CCG CGT      576
   Asn Arg Leu Arg Ala Phe Gln Val Ile Glu Thr Gln Asp Pro Pro Arg
               180                 185                 190
   CGG CTG GCG CTC ACG CCT GCC CAC CTG CTC TTC ATT GCG GAC AAT CAT      624
   Arg Leu Ala Leu Thr Pro Ala His Leu Leu Phe Ile Ala Asp Asn His
           195                 200                 205
   ACA GAA CCA GCA GCC CAC TTC CGG GCC ACA TTT GCC AGC CAT GTG CAA      672
   Thr Glu Pro Ala Ala His Phe Arg Ala Thr Phe Ala Ser His Val Gln
       210                 215                 220
   CCA GGC CAA TAT GTG CTG GTA TCA GGG GTA CCA GGC CTC CAG CCT GCT      720
   Pro Gly Gln Tyr Val Leu Val Ser Gly Val Pro Gly Leu Gln Pro Ala
   225                 230                 235                 240
   CGG GTG GCA GCT GTC TCC ACC CAC GTG GCC CTT GGG TCC TAT GCT CCT      768
   Arg Val Ala Ala Val Ser Thr His Val Ala Leu Gly Ser Tyr Ala Pro
                   245                 250                 255
   CTC ACA AGG CAT GGG ACA CTT GTG GTG GAG GAT GTG GTG GCC TCC TGC      816
   Leu Thr Arg His Gly Thr Leu Val Val Glu Asp Val Val Ala Ser Cys
               260                 265                 270
   TTT GCA GCT GTG GCT GAC CAC CAT CTG GCT CAG TTG GCC TTC TGG CCC      864
   Phe Ala Ala Val Ala Asp His His Leu Ala Gln Leu Ala Phe Trp Pro
           275                 280                 285
   CTG CGA CTG TTT CCC AGT TTG GCA TGG GGC AGC TGG ACC CCA AGT GAG      912
   Leu Arg Leu Phe Pro Ser Leu Ala Trp Gly Ser Trp Thr Pro Ser Glu
       290                 295                 300
   GGT GTT CAC TCC TAC CCT CAG ATG CTC TAC CGC CTG GGG CGT CTC TTG      960
   Gly Val His Ser Tyr Pro Gln Met Leu Tyr Arg Leu Gly Arg Leu Leu
   305                 310                 315                 320
   CTA GAA GAG AGC ACC TTC CAT CCA CTG GGC ATG TCT GGG GCA GGA AGC     1008
   Leu Glu Glu Ser Thr Phe His Pro Leu Gly Met Ser Gly Ala Gly Ser
                   325                 330                 335
   TGAAGGGACT CTAACCACTG CCCTCCTGGA ACTGCTGTGC GTGGATCC                1056
(2) INFORMATION FOR SEQ ID NO:4:
   (i) SEQUENCE CHARACTERISTICS:
      (A) LENGTH: 1313 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1314
   (xi) SEQUENCE DESCRIPTION: SEQ ID NO:4:
   ATG CTG CTG CTG CTG GCC AGA TGT TTT CTG GTG ATC CTT GCT TCC TCG        48
   Met Leu Leu Leu Leu Ala Arg Cys Phe Leu Val Ile Leu Ala Ser Ser
     1               5                  10                  15
   CTG CTG GTG TGC CCC GGG CTG GCC TGT GGG CCC GGC AGG GGG TTT GGA        96
   Leu Leu Val Cys Pro Gly Leu Ala Cys Gly Pro Gly Arg Gly Phe Gly
                20                  25                  30
   AAG AGG CGG CAC CCC AAA AAG CTG ACC CCT TTA GCC TAC AAG CAG TTT       144
   Lys Arg Arg His Pro Lys Lys Leu Thr Pro Leu Ala Tyr Lys Gln Phe
            35                  40                  45
   ATT CCC AAC GTA GCC GAG AAG ACC CTA GGG GCC AGC GGC AGA TAT GAA       192
   Ile Pro Asn Val Ala Glu Lys Thr Leu Gly Ala Ser Gly Arg Tyr Glu
        50                  55                  60
   GGG AAG ATC ACA AGA AAC TCC GAA CGA TTT AAG GAA CTC ACC CCC AAT       240
   Gly Lys Ile Thr Arg Asn Ser Glu Arg Phe Lys Glu Leu Thr Pro Asn
    65                  70                  75                  80
   TAC AAC CCC GAC ATC ATA TTT AAG GAT GAG GAA AAC ACG GGA GCA GAC       288
   Tyr Asn Pro Asp Ile Ile Phe Lys Asp Glu Glu Asn Thr Gly Ala Asp
                    85                  90                  95
   CGG CTG ATG ACT CAG AGG TGC AAA GAC AAG TTA AAT GCC TTG GCC ATC       336
   Arg Leu Met Thr Gln Arg Cys Lys Asp Lys Leu Asn Ala Leu Ala Ile
               100                 105                 110
   TCT GTG ATG AAC CAG TGG CCT GGA GTG AGG CTG CGA GTG ACC GAG GGC       384
   Ser Val Met Asn Gln Trp Pro Gly Val Arg Leu Arg Val Thr Glu Gly
           115                 120                 125
   TGG GAT GAG GAC GGC CAT CAT TCA GAG GAG TCT CTA CAC TAT GAG GGT       432
   Trp Asp Glu Asp Gly His His Ser Glu Glu Ser Leu His Tyr Glu Gly
       130                 135                 140
   CGA GCA GTG GAC ATC ACC ACG TCC GAC CGG GAC CGC AGC AAG TAC GGC       480
   Arg Ala Val Asp Ile Thr Thr Ser Asp Arg Asp Arg Ser Lys Tyr Gly
   145                 150                 155                 160
   ATG CTG GCT CGC CTG GCT GTG GAA GCA GGT TTC GAC TGG GTC TAC TAT 528
   Met Leu Ala Arg Leu Ala Val Glu Ala Gly Phe Asp Trp Val Tyr Tyr
   165 170 175
   GAA TCC AAA GCT CAC ATC CAC TGT TCT GTG AAA GCA GAG AAC TCC GTG 576
   Glu Ser Lys Ala His Ile His Cys Ser Val Lys Ala Glu Asn Ser Val
   180 185 190
   GCG GCC AAA TCC GGC GGC TGT TTC CCG GGA TCC GCC ACC GTG CAC CTG 624
   Ala Ala Lys Ser Gly Gly Cys Phe Pro Gly Ser Ala Thr Val His Leu
   195 200 205
   GAG CAG GGC GGC ACC AAG CTG GTG AAG GAC TTA CGT CCC GGA GAC CGC 672
   Glu Gln Gly Gly Thr Lys Leu Val Lys Asp Leu Arg Pro Gly Asp Arg
   210 215 220
   GTG CTG GCG GCT GAC GAC CAG GGC CGG CTG CTG TAC AGC GAC TTC CTC 720
   Val Leu Ala Ala Asp Asp Gln Gly Arg Leu Leu Tyr Ser Asp Phe Leu
   225 230 235 240
   ACC TTC CTG GAC CGC GAC GAA GGC GCC AAG AAG GTC TTC TAC GTG ATC 768
   Thr Phe Leu Asp Arg Asp Glu Gly Ala Lys Lys Val Phe Tyr Val Ile
   245 250 255
   GAG ACG CTG GAG CCG CGC GAG CGC CTG CTG CTC ACC GCC GCG CAC CTG 816
   Glu Thr Leu Glu Pro Arg Glu Arg Leu Leu Leu Thr Ala Ala His Leu
   260 265 270
   CTC TTC GTG GCG CCG CAC AAC GAC TCG GGG CCC ACG CCC GGG CCA AGC 864
   Leu Phe Val Ala Pro His Asn Asp Ser Gly Pro Thr Pro Gly Pro Ser
   275 280 285
   GCG CTC TTT GCC AGC CGC GTG CGC CCC GGG CAG CGC GTG TAC GTG GTG 912
   Ala Leu Phe Ala Ser Arg Val Arg Pro Gly Gln Arg Val Tyr Val Val
   290 295 300
   GCT GAA CGC GGC GGG GAC CGC CGG CTG CTG CCC GCC GCG GTG CAC AGC 960
   Ala Glu Arg Gly Gly Asp Arg Arg Leu Leu Pro Ala Ala Val His Ser
   305 310 315 320
   GTG ACG CTG CGA GAG GAG GAG GCG GGC GCG TAC GCG CCG CTC ACG GCG 1008
   Val Thr Leu Arg Glu Glu Glu Ala Gly Ala Tyr Ala Pro Leu Thr Ala
   325 330 335
   CAC GGC ACC ATT CTC ATC AAC CGG GTG CTC GCC TCG TGC TAC GCT GTC 1056
   His Gly Thr Ile Leu Ile Asn Arg Val Leu Ala Ser Cys Tyr Ala Val
   340 345 350
   ATC GAG GAG CAC AGC TGG GCA CAC CGG GCC TTC GCG CCT TTC CGC CTG 1104
   Ile Glu Glu His Ser Trp Ala His Arg Ala Phe Ala Pro Phe Arg Leu
   355 360 365
   GCG CAC GCG CTG CTG GCC GCG CTG GCA CCC GCC CGC ACG GAC GGC GGG 1152
   Ala His Ala Leu Leu Ala Ala Leu Ala Pro Ala Arg Thr Asp Gly Gly
   370 375 380
   GGC GGG GGC AGC ATC CCT GCA GCG CAA TCT GCA ACG GAA GCG AGG GGC 1200
   Gly Gly Gly Ser Ile Pro Ala Ala Gln Ser Ala Thr Glu Ala Arg Gly
   385 390 395 400
   GCG GAG CCG ACT GCG GGC ATC CAC TGG TAC TCG CAG CTG CTC TAC CAC 1248
   Ala Glu Pro Thr Ala Gly Ile His Trp Tyr Ser Gln Leu Leu Tyr His
   405 410 415
   ATT GGC ACC TGG CTG TTG GAC AGC GAG ACC ATG CAT CCC TTG GGA ATG 1296
   Ile Gly Thr Trp Leu Leu Asp Ser Glu Thr Met His Pro Leu Gly Met
   420 425 430
   GCG GTC AAG TCC AGC TG 1313
   Ala Val Lys Ser Ser
   435
(2) INFORMATION FOR SEQ ID NO:5:
   (i) SEQUENCE CHARACTERISTICS:
      (A) LENGTH: 1256 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1257
   (xi) SEQUENCE DESCRIPTION: SEQ ID NO:5:
   ATG CGG CTT TTG ACG AGA GTG CTG CTG GTG TCT CTT CTC ACT CTG TCC      48
   Met Arg Leu Leu Thr Arg Val Leu Leu Val Ser Leu Leu Thr Leu Ser
    1               5                  10                  15
   TTG GTG GTG TCC GGA CTG GCC TGC GGT CCT GGC AGA GGC TAC GGC AGA      96
   Leu Val Val Ser Gly Leu Ala Cys Gly Pro Gly Arg Gly Tyr Gly Arg
               20                  25                  30
   AGA AGA CAT CCG AAG AAG CTG ACA CCT CTC GCC TAC AAG CAG TTC ATA     144
   Arg Arg His Pro Lys Lys Leu Thr Pro Leu Ala Tyr Lys Gln Phe Ile
           35                  40                  45
   CCT AAT GTC GCG GAG AAG ACC TTA GGG GCC AGC GGC AGA TAC GAG GGC     192
   Pro Asn Val Ala Glu Lys Thr Leu Gly Ala Ser Gly Arg Tyr Glu Gly
       50                  55                  60
   AAG ATA ACG CGC AAT TCG GAG AGA TTT AAA GAA CTT ACT CCA AAT TAC     240
   Lys Ile Thr Arg Asn Ser Glu Arg Phe Lys Glu Leu Thr Pro Asn Tyr
   65                  70                  75                  80
   AAT CCC GAC ATT ATC TTT AAG GAT GAG GAG AAC ACG GGA GCG GAC AGG     288
   Asn Pro Asp Ile Ile Phe Lys Asp Glu Glu Asn Thr Gly Ala Asp Arg
                   85                  90                  95
   CTC ATG ACA CAG AGA TGC AAA GAC AAG CTG AAC TCG CTG GCC ATC TCT 336
   Leu Met Thr Gln Arg Cys Lys Asp Lys Leu Asn Ser Leu Ala Ile Ser
   100 105 110
   GTA ATG AAC CAC TGG CCA GGG GTT AAG CTG CGT GTG ACA GAG GGC TGG 384
   Val Met Asn His Trp Pro Gly Val Lys Leu Arg Val Thr Glu Gly Trp
   115 120 125
   GAT GAG GAC GGT CAC CAT TTT GAA GAA TCA CTC CAC TAC GAG GGA AGA 432
   Asp Glu Asp Gly His His Phe Glu Glu Ser Leu His Tyr Glu Gly Arg
   130 135 140
   GCT GTT GAT ATT ACC ACC TCT GAC CGA GAC AAG AGC AAA TAC GGG ACA 480
   Ala Val Asp Ile Thr Thr Ser Asp Arg Asp Lys Ser Lys Tyr Gly Thr
   145 150 155 160
   CTG TCT CGC CTA GCT GTG GAG GCT GGA TTT GAC TGG GTC TAT TAC GAG 528
   Leu Ser Arg Leu Ala Val Glu Ala Gly Phe Asp Trp Val Tyr Tyr Glu
   165 170 175
   TCC AAA GCC CAC ATT CAT TGC TCT GTC AAA GCA GAA AAT TCG GTT GCT 576
   Ser Lys Ala His Ile His Cys Ser Val Lys Ala Glu Asn Ser Val Ala
   180 185 190
   GCG AAA TCT GGG GGC TGT TTC CCA GGT TCG GCT CTG GTC TCG CTC CAG 624
   Ala Lys Ser Gly Gly Cys Phe Pro Gly Ser Ala Leu Val Ser Leu Gln
   195 200 205
   GAC GGA GGA CAG AAG GCC GTG AAG GAC CTG AAC CCC GGA GAC AAG GTG 672
   Asp Gly Gly Gln Lys Ala Val Lys Asp Leu Asn Pro Gly Asp Lys Val
   210 215 220
   CTG GCG GCA GAC AGC GCG GGA AAC CTG GTG TTC AGC GAC TTC ATC ATG 720
   Leu Ala Ala Asp Ser Ala Gly Asn Leu Val Phe Ser Asp Phe Ile Met
   225 230 235 240
   TTC ACA GAC CGA GAC TCC ACG ACG CGA CGT GTG TTT TAC GTC ATA GAA 768
   Phe Thr Asp Arg Asp Ser Thr Thr Arg Arg Val Phe Tyr Val Ile Glu
   245 250 255
   ACG CAA GAA CCC GTT GAA AAG ATC ACC CTC ACC GCC GCT CAC CTC CTT 816
   Thr Gln Glu Pro Val Glu Lys Ile Thr Leu Thr Ala Ala His Leu Leu
   260 265 270
   TTT GTC CTC GAC AAC TCA ACG GAA GAT CTC CAC ACC ATG ACC GCC GCG 864
   Phe Val Leu Asp Asn Ser Thr Glu Asp Leu His Thr Met Thr Ala Ala
   275 280 285
   TAT GCC AGC AGT GTC AGA GCC GGA CAA AAG GTG ATG GTT GTT GAT GAT 912
   Tyr Ala Ser Ser Val Arg Ala Gly Gln Lys Val Met Val Val Asp Asp
   290 295 300
   AGC GGT CAG CTT AAA TCT GTC ATC GTG CAG CGG ATA TAC ACG GAG GAG 960
   Ser Gly Gln Leu Lys Ser Val Ile Val Gln Arg Ile Tyr Thr Glu Glu
   305 310 315 320
   CAG CGG GGC TCG TTC GCA CCA GTG ACT GCA CAT GGG ACC ATT GTG GTC     1008
   Gln Arg Gly Ser Phe Ala Pro Val Thr Ala His Gly Thr Ile Val Val
                   325                 330                 335
   GAC AGA ATA CTG GCG TCC TGT TAC GCC GTA ATA GAG GAC CAG GGG CTT     1056
   Asp Arg Ile Leu Ala Ser Cys Tyr Ala Val Ile Glu Asp Gln Gly Leu
               340                 345                 350
   GCG CAT TTG GCC TTC GCG CCC GCC AGG CTC TAT TAT TAC GTG TCA TCA     1104
   Ala His Leu Ala Phe Ala Pro Ala Arg Leu Tyr Tyr Tyr Val Ser Ser
           355                 360                 365
   TTC CTG TCC CCC AAA ACT CCA GCA GTC GGT CCA ATG CGA CTT TAC AAC     1152
   Phe Leu Ser Pro Lys Thr Pro Ala Val Gly Pro Met Arg Leu Tyr Asn
       370                 375                 380
   AGG AGG GGG TCC ACT GGT ACT CCA GGC TCC TGT CAT CAA ATG GGA ACG     1200
   Arg Arg Gly Ser Thr Gly Thr Pro Gly Ser Cys His Gln Met Gly Thr
   385                 390                 395                 400
   TGG CTT TTG GAC AGC AAC ATG CTT CAT CCT TTG GGG ATG TCA GTA AAC     1248
   Trp Leu Leu Asp Ser Asn Met Leu His Pro Leu Gly Met Ser Val Asn
                   405                 410                 415
   TCA AGC TG                                                          1256
   Ser Ser
(2) INFORMATION FOR SEO ID NO:6:
   (i) SEQUENCE CHARACTERISTICS:
      (A) LENGTH: 1425 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1425
   (xi) SEQUENCE DESCRIPTION: SEQ ID NO:6:
   ATG CTG CTG CTG GCG AGA TGT CTG CTG CTA GTC CTC GTC TCC TCG CTG      48
   Met Leu Leu Leu Ala Arg Cys Leu Leu Leu Val Leu Val Ser Ser Leu
    1               5                  10                  15
   CTG GTA TGC TCG GGA CTG GCG TGC GGA CCG GGC AGG GGG TTC GGG AAG      96
   Leu Val Cys Ser Gly Leu Ala Cys Gly Pro Gly Arg Gly Phe Gly Lys
               20                  25                  30
   AGG AGG CAC CCC AAA AAG CTG ACC CCT TTA GCC TAC AAG CAG TTT ATC     144
   Arg Arg His Pro Lys Lys Leu Thr Pro Leu Ala Tyr Lys Gln Phe Ile
           35                  40                  45
   CCC AAT GTG GCC GAG AAG ACC CTA GGC GCC AGC GGA AGG TAT GAA GGG        192
   Pro Asn Val Ala Glu Lys Thr Leu Gly Ala Ser Gly Arg Tyr Glu Gly
        50                  55                  60
   AAG ATC TCC AGA AAC TCC GAG CGA TTT AAG GAA CTC ACC CCC AAT TAC        240
   Lys Ile Ser Arg Asn Ser Glu Arg Phe Lys Glu Leu Thr Pro Asn Tyr
    65                  70                  75                  80
   AAC CCC GAC ATC ATA TTT AAG GAT GAA GAA AAC ACC GGA GCG GAC AGG        288
   Asn Pro Asp Ile Ile Phe Lys Asp Glu Glu Asn Thr Gly Ala Asp Arg
                    85                  90                  95
   CTG ATG ACT CAG AGG TGT AAG GAC AAG TTG AAC GCT TTG GCC ATC TCG        336
   Leu Met Thr Gln Arg Cys Lys Asp Lys Leu Asn Ala Leu Ala Ile Ser
               100                 105                 110
   GTG ATG AAC CAG TGG CCA GGA GTG AAA CTG CGG GTG ACC GAG GGC TGG        384
   Val Met Asn Gln Trp Pro Gly Val Lys Leu Arg Val Thr Glu Gly Trp
           115                 120                 125
   GAC GAA GAT GGC CAC CAC TCA GAG GAG TCT CTG CAC TAC GAG GGC CGC        432
   Asp Glu Asp Gly His His Ser Glu Glu Ser Leu His Tyr Glu Gly Arg
       130                 135                 140
   GCA GTG GAC ATC ACC ACG TCT GAC CGC GAC CGC AGC AAG TAC GGC ATG        480
   Ala Val Asp Ile Thr Thr Ser Asp Arg Asp Arg Ser Lys Tyr Gly Met
   145                 150                 155                 160
   CTG GCC CGC CTG GCG GTG GAG GCC GGC TTC GAC TGG GTG TAC TAC GAG        528
   Leu Ala Arg Leu Ala Val Glu Ala Gly Phe Asp Trp Val Tyr Tyr Glu
                   165                 170                 175
   TCC AAG GCA CAT ATC CAC TGC TCG GTG AAA GCA GAG AAC TCG GTG GCG        576
   Ser Lys Ala His Ile His Cys Ser Val Lys Ala Glu Asn Ser Val Ala
               180                 185                 190
   GCC AAA TCG GGA GGC TGC TTC CCG GGC TCG GCC ACG GTG CAC CTG GAG        624
   Ala Lys Ser Gly Gly Cys Phe Pro Gly Ser Ala Thr Val His Leu Glu
           195                 200                 205
   CAG GGC GGC ACC AAG CTG GTG AAG GAC CTG AGC CCC GGG GAC CGC GTG        672
   Gln Gly Gly Thr Lys Leu Val Lys Asp Leu Ser Pro Gly Asp Arg Val
       210                 215                 220
   CTG GCG GCG GAC GAC CAG GGC CGG CTG CTC TAC AGC GAC TTC CTC ACT        720
   Leu Ala Ala Asp Asp Gln Gly Arg Leu Leu Tyr Ser Asp Phe Leu Thr
   225                 230                 235                 240
   TTC CTG GAC CGC GAC GAC GGC GCC AAG AAG GTC TTC TAC GTG ATC GAG        768
   Phe Leu Asp Arg Asp Asp Gly Ala Lys Lys Val Phe Tyr Val Ile Glu
                   245                 250                 255
   ACG CGG GAG CCG CGC GAG CGC CTG CTG CTC ACC GCC GCG CAC CTG CTC        816
   Thr Arg Glu Pro Arg Glu Arg Leu Leu Leu Thr Ala Ala His Leu Leu
               260                 265                 270
   TTT GTG GCG CCG CAC AAC GAC TCG GCC ACC GGG GAG CCC GAG GCG TCC        864
   Phe Val Ala Pro His Asn Asp Ser Ala Thr Gly Glu Pro Glu Ala Ser
           275                 280                 285
   TCG GGC TCG GGG CCG CCT TCC GGG GGC GCA CTG GGG CCT CGG GCG CTG        912
   Ser Gly Ser Gly Pro Pro Ser Gly Gly Ala Leu Gly Pro Arg Ala Leu
       290                 295                 300
   TTC GCC AGC CGC GTG CGC CCG GGC CAG CGC GTG TAC GTG GTG GCC GAG        960
   Phe Ala Ser Arg Val Arg Pro Gly Gln Arg Val Tyr Val Val Ala Glu
   305                 310                 315                 320
   CGT GAC GGG GAC CGC CGG CTC CTG CCC GCC GCT GTG CAC AGC GTG ACC       1008
   Arg Asp Gly Asp Arg Arg Leu Leu Pro Ala Ala Val His Ser Val Thr
                   325                 330                 335
   CTA AGC GAG GAG GCC GCG GGC GCC TAC GCG CCG CTC ACG GCC CAG GGC       1056
   Leu Ser Glu Glu Ala Ala Gly Ala Tyr Ala Pro Leu Thr Ala Gln Gly
               340                 345                 350
   ACC ATT CTC ATC AAC CGG GTG CTG GCC TCG TGC TAC GCG GTC ATC GAG       1104
   Thr Ile Leu Ile Asn Arg Val Leu Ala Ser Cys Tyr Ala Val Ile Glu
           355                 360                 365
   GAG CAC AGC TGG GCG CAC CGG GCC TTC GCG CCC TTC CGC CTG GCG CAC       1152
   Glu His Ser Trp Ala His Arg Ala Phe Ala Pro Phe Arg Leu Ala His
       370                 375                 380
   GCG CTC CTG GCT GCA CTG GCG CCC GCG CGC ACG GAC CGC GGC GGG GAC       1200
   Ala Leu Leu Ala Ala Leu Ala Pro Ala Arg Thr Asp Arg Gly Gly Asp
   385                 390                 395                 400
   AGC GGC GGC GGG GAC CGC GGG GGC GGC GGC GGC AGA GTA GCC CTA ACC       1248
   Ser Gly Gly Gly Asp Arg Gly Gly Gly Gly Gly Arg Val Ala Leu Thr
                   405                 410                 415
   GCT CCA GGT GCT GCC GAC GCT CCG GGT GCG GGG GCC ACC GCG GGC ATC       1296
   Ala Pro Gly Ala Ala Asp Ala Pro Gly Ala Gly Ala Thr Ala Gly Ile
               420                 425                 430
   CAC TGG TAC TCG CAG CTG CTC TAC CAA ATA GGC ACC TGG CTC CTG GAC       1344
   His Trp Tyr Ser Gln Leu Leu Tyr Gln Ile Gly Thr Trp Leu Leu Asp
           435                 440                 445
   AGC GAG GCC CTG CAC CCG CTG GGC ATG GCG GTC AAG TCC AGC NNN AGC       1392
   Ser Glu Ala Leu His Pro Leu Gly Met Ala Val Lys Ser Ser Xaa Ser
       450                 455                 460
   CGG GGG GCC GGG GGA GGG GCG CGG GAG GGG GCC                           1425
   Arg Gly Ala Gly Gly Gly Ala Arg Glu Gly Ala
   465                 470                 475
(2) INFORMATION FOR SEQ ID NO:7:
   (i) SEQUENCE CHARACTERISTICS:
      (A) LENGTH: 940 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..940
   (xi) SEQUENCE DESCRIPTION: SEQ ID NO:7:
(2) INFORMATION FOR SEQ ID NO:8:
   (i) SEQUENCE CHARACTERISTICS:
      (A) LENGTH: 425 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Val Glu Met Leu Leu Leu Thr Arg Ile Leu Leu Val Gly Phe Ile
   1               5                   10                  15
   Cys Ala Leu Leu Val Ser Ser Gly Leu Thr Cys Gly Pro Gly Arg Gly
               20                  25                  30
   Ile Gly Lys Arg Arg His Pro Lys Lys Leu Thr Pro Leu Ala Tyr Lys
           35                  40                  45
   Gln Phe Ile Pro Asn Val Ala Glu Lys Thr Leu Gly Ala Ser Gly Arg
   50 55 60
   Tyr Glu Gly Lys Ile Thr Arg Asn Ser Glu Arg Phe Lys Glu Leu Thr
   65 70 75 80
   Pro Asn Tyr Asn Pro Asp Ile Ile Phe Lys Asp Glu Glu Asn Thr Gly
   85 90 95
   Ala Asp Arg Leu Met Thr Gln Arg Cys Lys Asp Lys Leu Asn Ala Leu
   100 105 110
   Ala Ile Ser Val Met Asn Gln Trp Pro Gly Val Lys Leu Arg Val Thr
   115 120 125
   Glu Gly Trp Asp Glu Asp Gly His His Ser Glu Glu Ser Leu His Tyr
   130 135 140
   Glu Gly Arg Ala Val Asp Ile Thr Thr Ser Asp Arg Asp Arg Ser Lys
   145 150 155 160
   Tyr Gly Met Leu Ala Arg Leu Ala Val Glu Ala Gly Phe Asp Trp Val
   165 170 175
   Tyr Tyr Glu Ser Lys Ala His Ile His Cys Ser Val Lys Ala Glu Asn
   180 185 190
   Ser Val Ala Ala Lys Ser Gly Gly Cys Phe Pro Gly Ser Ala Thr Val
   195 200 205
   His Leu Glu His Gly Gly Thr Lys Leu Val Lys Asp Leu Ser Pro Gly
   210 215 220
   Asp Arg Val Leu Ala Ala Asp Ala Asp Gly Arg Leu Leu Tyr Ser Asp
   225 230 235 240
   Phe Leu Thr Phe Leu Asp Arg Met Asp Ser Ser Arg Lys Leu Phe Tyr
   245 250 255
   Val Ile Glu Thr Arg Gln Pro Arg Ala Arg Leu Leu Leu Thr Ala Ala
   260 265 270
   His Leu Leu Phe Val Ala Pro Gln His Asn Gln Ser Glu Ala Thr Gly
   275 280 285
   Ser Thr Ser Gly Gln Ala Leu Phe Ala Ser Asn Val Lys Pro Gly Gln
   290 295 300
   Arg Val Tyr Val Leu Gly Glu Gly Gly Gln Gln Leu Leu Pro Ala Ser
   305 310 315 320
   Val His Ser Val Ser Leu Arg Glu Glu Ala Ser Gly Ala Tyr Ala Pro
   325 330 335
   Leu Thr Ala Gln Gly Thr Ile Leu Ile Asn Arg Val Leu Ala Ser Cys
   340 345 350
   Tyr Ala Val Ile Glu Glu His Ser Trp Ala His Trp Ala Phe Ala Pro
   355 360 365
   Phe Arg Leu Ala Gln Gly Leu Leu Ala Ala Leu Cys Pro Asp Gly Ala
   370 375 380
   Ile Pro Thr Ala Ala Thr Thr Thr Thr Gly Ile His Trp Tyr Ser Arg
   385 390 395 400
   Leu Leu Tyr Arg Ile Gly Ser Trp Val Leu Asp Gly Asp Ala Leu His
   405 410 415
   Pro Leu Gly Met Val Ala Pro Ala Ser
   420 425
(2) INFORMATION FOR SEQ ID NO:9:
   (i) SEQUENCE CHARACTERISTICS:
      (A) LENGTH: 396 amino acids
      (B) TYPE: amino acid
      (D) TOPOLOGY: linear
   (ii) MOLECULE TYPE: protein
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 336 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Glu Arg Phe Lys Glu Leu Thr Pro Asn Tyr Asn Pro Asp Ile Ile Phe
   1 5 10 15
   Lys Asp Glu Glu Asn Thr Gly Ala Asp Arg Leu Met Thr Gln Arg Cys
   20 25 30
   Lys Asp Arg Leu Asn Ser Leu Ala Ile Ser Val Met Asn Gln Trp Pro
   35 40 45
   Gly Val Lys Leu Arg Val Thr Glu Gly Arg Asp Glu Asp Gly His His
   50 55 60
   Ser Glu Glu Ser Leu His Tyr Glu Gly Arg Ala Val Asp Ile Thr Thr
   65 70 75 80
   Ser Asp Arg Asp Arg Asn Lys Tyr Gly Leu Leu Ala Arg Leu Ala Val
   85 90 95
   Glu Ala Gly Phe Asp Trp Val Tyr Tyr Glu Ser Lys Ala His Val His
   100 105 110
   Cys Ser Val Lys Ser Glu His Ser Ala Ala Ala Lys Thr Gly Gly Cys
   115 120 125
   Phe Pro Ala Gly Ala Gln Val Arg Leu Glu Asn Gly Glu Arg Val Ala
   130 135 140
   Leu Ser Ala Val Lys Pro Gly Asp Arg Val Leu Ala Met Gly Glu Asp
   145 150 155 160
   Gly Thr Pro Thr Phe Ser Asp Val Leu Ile Phe Leu Asp Arg Glu Pro
   165 170 175
   Asn Arg Leu Arg Ala Phe Gln Val Ile Glu Thr Gln Asp Pro Pro Arg
   180 185 190
   Arg Leu Ala Leu Thr Pro Ala His Leu Leu Phe Ile Ala Asp Asn His
   195 200 205
   Thr Glu Pro Ala Ala His Phe Arg Ala Thr Phe Ala Ser His Val Gln
   210 215 220
   Pro Gly Gln Tyr Val Leu Val Ser Gly Val Pro Gly Leu Gln Pro Ala
   225 230 235 240
   Arg Val Ala Ala Val Ser Thr His Val Ala Leu Gly Ser Tyr Ala Pro
   245 250 255
   Leu Thr Arg His Gly Thr Leu Val Val Glu Asp Val Val Ala Ser Cys
   260 265 270
   Phe Ala Ala Val Ala Asp His His Leu Ala Gln Leu Ala Phe Trp Pro
   275 280 285
   Leu Arg Leu Phe Pro Ser Leu Ala Trp Gly Ser Trp Thr Pro Ser Glu
   290 295 300
   Gly Val His Ser Tyr Pro Gln Met Leu Tyr Arg Leu Gly Arg Leu Leu
   305 310 315 320
   Leu Glu Glu Ser Thr Phe His Pro Leu Gly Met Ser Gly Ala Gly Ser
   325 330 335
(2) INFORMATION FOR SEQ ID NO:11:
   (i) SEQUENCE CHARACTERISTICS:
      (A) LENGTH: 437 amino acids
      (B) TYPE: amino acid
      (D) TOPOLOGY: linear
   (ii) MOLECULE TYPE: protein
   (xi) SEQUENCE DESCRIPTION: SEQ ID NO:11:
   Met Leu Leu Leu Leu Ala Arg Cys Phe Leu Val Ile Leu Ala Ser Ser
   1 5 10 15
   Leu Leu Val Cys Pro Gly Leu Ala Cys Gly Pro Gly Arg Gly Phe Gly
   20 25 30
   Lys Arg Arg His Pro Lys Lys Leu Thr Pro Leu Ala Tyr Lys Gln Phe
   35 40 45
   Ile Pro Asn Val Ala Glu Lys Thr Leu Gly Ala Ser Gly Arg Tyr Glu
   50 55 60
   Gly Lys Ile Thr Arg Asn Ser Glu Arg Phe Lys Glu Leu Thr Pro Asn
   65 70 75 80
   Tyr Asn Pro Asp Ile Ile Phe Lys Asp Glu Glu Asn Thr Gly Ala Asp
   85 90 95
   Arg Leu Met Thr Gln Arg Cys Lys Asp Lys Leu Asn Ala Leu Ala Ile
   100 105 110
   Ser Val Met Asn Gln Trp Pro Gly Val Arg Leu Arg Val Thr Glu Gly
   115 120 125
   Trp Asp Glu Asp Gly His His Ser Glu Glu Ser Leu His Tyr Glu Gly
   130 135 140
   Arg Ala Val Asp Ile Thr Thr Ser Asp Arg Asp Arg Ser Lys Tyr Gly
   145 150 155 160
   Met Leu Ala Arg Leu Ala Val Glu Ala Gly Phe Asp Trp Val Tyr Tyr
   165 170 175
   Glu Ser Lys Ala His Ile His Cys Ser Val Lys Ala Glu Asn Ser Val
   180 185 190
   Ala Ala Lys Ser Gly Gly Cys Phe Pro Gly Ser Ala Thr Val His Leu
   195 200 205
   Glu Gln Gly Gly Thr Lys Leu Val Lys Asp Leu Arg Pro Gly Asp Arg
   210 215 220
   Val Leu Ala Ala Asp Asp Gln Gly Arg Leu Leu Tyr Ser Asp Phe Leu
   225 230 235 240
   Thr Phe Leu Asp Arg Asp Glu Gly Ala Lys Lys Val Phe Tyr Val Ile
   245 250 255
   Glu Thr Leu Glu Pro Arg Glu Arg Leu Leu Leu Thr Ala Ala His Leu
   260 265 270
   Leu Phe Val Ala Pro His Asn Asp Ser Gly Pro Thr Pro Gly Pro Ser
   275 280 285
   Ala Leu Phe Ala Ser Arg Val Arg Pro Gly Gln Arg Val Tyr Val Val
   290 295 300
   Ala Glu Arg Gly Gly Asp Arg Arg Leu Leu Pro Ala Ala Val His Ser
   305 310 315 320
   Val Thr Leu Arg Glu Glu Glu Ala Gly Ala Tyr Ala Pro Leu Thr Ala
   325 330 335
   His Gly Thr Ile Leu Ile Asn Arg Val Leu Ala Ser Cys Tyr Ala Val
   340 345 350
   Ile Glu Glu His Ser Trp Ala His Arg Ala Phe Ala Pro Phe Arg Leu
   355 360 365
   Ala His Ala Leu Leu Ala Ala Leu Ala Pro Ala Arg Thr Asp Gly Gly
   370 375 380
   Gly Gly Gly Ser Ile Pro Ala Ala Gln Ser Ala Thr Glu Ala Arg Gly
   385 390 395 400
   Ala Glu Pro Thr Ala Gly Ile His Trp Tyr Ser Gln Leu Leu Tyr His
   405 410 415
   Ile Gly Thr Trp Leu Leu Asp Ser Glu Thr Met His Pro Leu Gly Met
   420 425 430
   Ala Val Lys Ser Ser
   435
(2) INFORMATION FOR SEQ ID NO:12:
   (i) SEQUENCE CHARACTERISTICS:
      (A) LENGTH: 416 amino acids
      (B) TYPE: amino acid
      (D) TOPOLOGY: linear
   (ii) MOLECULE TYPE: protein
   (xi) SEQUENCE DESCRIPTION: SEQ ID NO:12:
(2) INFORMATION FOR SEQ ID NO:13:
   (i) SEQUENCE CHARACTERISTICS:
      (A) LENGTH: 475 amino acids
      (B) TYPE: amino acid
      (D) TOPOLOGY: linear
   (ii) MOLECULE TYPE: protein
   (xi) SEQUENCE DESCRIPTION: SEQ ID NO:13:
   Met Leu Leu Leu Ala Arg Cys Leu Leu Leu Val Leu Val Ser Ser Leu
   1 5 10 15
   Leu Val Cys Ser Gly Leu Ala Cys Gly Pro Gly Arg Gly Phe Gly Lys
   20 25 30
   Arg Arg His Pro Lys Lys Leu Thr Pro Leu Ala Tyr Lys Gln Phe Ile
   35 40 45
   Pro Asn Val Ala Glu Lys Thr Leu Gly Ala Ser Gly Arg Tyr Glu Gly
   50 55 60
   Lys Ile Ser Arg Asn Ser Glu Arg Phe Lys Glu Leu Thr Pro Asn Tyr
   65 70 75 80
   Asn Pro Asp Ile Ile Phe Lys Asp Glu Glu Asn Thr Gly Ala Asp Arg
   85 90 95
   Leu Met Thr Gln Arg Cys Lys Asp Lys Leu Asn Ala Leu Ala Ile Ser
   100 105 110
   Val Met Asn Gln Trp Pro Gly Val Lys Leu Arg Val Thr Glu Gly Trp
   115 120 125
   Asp Glu Asp Gly His His Ser Glu Glu Ser Leu His Tyr Glu Gly Arg
   130 135 140
   Ala Val Asp Ile Thr Thr Ser Asp Arg Asp Arg Ser Lys Tyr Gly Met
   145 150 155 160
   Leu Ala Arg Leu Ala Val Glu Ala Gly Phe Asp Trp Val Tyr Tyr Glu
   165 170 175
   Ser Lys Ala His Ile His Cys Ser Val Lys Ala Glu Asn Ser Val Ala
   180 185 190
   Ala Lys Ser Gly Gly Cys Phe Pro Gly Ser Ala Thr Val His Leu Glu
   195 200 205
   Gln Gly Gly Thr Lys Leu Val Lys Asp Leu Ser Pro Gly Asp Arg Val
   210 215 220
   Leu Ala Ala Asp Asp Gln Gly Arg Leu Leu Tyr Ser Asp Phe Leu Thr
   225 230 235 240
   Phe Leu Asp Arg Asp Asp Gly Ala Lys Lys Val Phe Tyr Val Ile Glu
   245 250 255
   Thr Arg Glu Pro Arg Glu Arg Leu Leu Leu Thr Ala Ala His Leu Leu
   260 265 270
   Phe Val Ala Pro His Asn Asp Ser Ala Thr Gly Glu Pro Glu Ala Ser
   275 280 285
   Ser Gly Ser Gly Pro Pro Ser Gly Gly Ala Leu Gly Pro Arg Ala Leu
   290 295 300
   Phe Ala Ser Arg Val Arg Pro Gly Gln Arg Val Tyr Val Val Ala Glu
   305 310 315 320
   Arg Asp Gly Asp Arg Arg Leu Leu Pro Ala Ala Val His Ser Val Thr
   325 330 335
   Leu Ser Glu Glu Ala Ala Gly Ala Tyr Ala Pro Leu Thr Ala Gln Gly
   340 345 350
   Thr Ile Leu Ile Asn Arg Val Leu Ala Ser Cys Tyr Ala Val Ile Glu
   355 360 365
   Glu His Ser Trp Ala His Arg Ala Phe Ala Pro Phe Arg Leu Ala His
   370 375 380
   Ala Leu Leu Ala Ala Leu Ala Pro Ala Arg Thr Asp Arg Gly Gly Asp
   385 390 395 400
   Ser Gly Gly Gly Asp Arg Gly Gly Gly Gly Gly Arg Val Ala Leu Thr
   405 410 415
   Ala Pro Gly Ala Ala Asp Ala Pro Gly Ala Gly Ala Thr Ala Gly Ile
   420 425 430
   His Trp Tyr Ser Gln Leu Leu Tyr Gln Ile Gly Thr Trp Leu Leu Asp
   435 440 445
   Ser Glu Ala Leu His Pro Leu Gly Met Ala Val Lys Ser Ser Xaa Ser
   450 455 460
   Arg Gly Ala Gly Gly Gly Ala Arg Glu Gly Ala
   465 470 475
(2) INFORMATION FOR SEQ ID NO:14:
   (i) SEQUENCE CHARACTERISTICS:
      (A) LENGTH: 313 amino acids
      (B) TYPE: amino acid
      (D) TOPOLOGY: linear
   (ii) MOLECULE TYPE: protein
   (xi) SEQUENCE DESCRIPTION: SEQ ID NO:14:
   Arg Arg Leu Met Thr Gln Arg Cys Lys Asp Arg Leu Asn Ser Leu Ala
   1 5 10 15
   Ile Ser Val Met Asn Gln Trp Pro Gly Val Lys Leu Arg Val Thr Glu
   20 25 30
   Gly Trp Asp Glu Asp Gly His His Ser Glu Glu Ser Leu His Tyr Glu
   35 40 45
   Gly Arg Ala Val Asp Ile Thr Thr Ser Asp Arg Asp Arg Asn Lys Tyr
   50 55 60
   Gly Leu Leu Ala Arg Leu Ala Val Glu Ala Gly Phe Asp Trp Val Tyr
   65 70 75 80
   Tyr Glu Ser Lys Ala His Val His Cys Ser Val Lys Ser Glu His Ser
   85 90 95
   Ala Ala Ala Lys Thr Gly Gly Cys Phe Pro Ala Gly Ala Gln Val Arg
   100 105 110
   Leu Glu Ser Gly Ala Arg Val Ala Leu Ser Ala Val Arg Pro Gly Asp
   115 120 125
   Arg Val Leu Ala Met Gly Glu Asp Gly Ser Pro Thr Phe Ser Asp Val
   130 135 140
   Leu Ile Phe Leu Asp Arg Glu Pro His Arg Leu Arg Ala Phe Gln Val
   145 150 155 160
   Ile Glu Thr Gln Asp Pro Pro Arg Arg Leu Ala Leu Thr Pro Ala His
   165 170 175
   Leu Leu Phe Thr Ala Asp Asn His Thr Glu Pro Ala Ala Arg Phe Arg
   180 185 190
   Ala Thr Phe Ala Ser His Val Gln Pro Gly Gln Tyr Val Leu Val Ala
   195 200 205
   Gly Val Pro Gly Leu Gln Pro Ala Arg Val Ala Ala Val Ser Thr His
   210 215 220
   Val Ala Leu Gly Ala Tyr Ala Pro Leu Thr Lys His Gly Thr Leu Val
   225 230 235 240
   Val Glu Asp Val Val Ala Ser Cys Phe Ala Ala Val Ala Asp His His
   245 250 255
   Leu Ala Gln Leu Ala Phe Trp Pro Leu Arg Leu Phe His Ser Leu Ala
   260 265 270
   Trp Gly Ser Trp Thr Pro Gly Glu Gly Val His Trp Tyr Pro Gln Leu
   275 280 285
   Leu Tyr Arg Leu Gly Arg Leu Leu Leu Glu Glu Gly Ser Phe His Pro
   290 295 300
   Leu Gly Met Ser Gly Ala Gly Ser Xaa
   305 310
(2) INFORMATION FOR SEQ ID NO:15:
   (i) SEQUENCE CHARACTERISTICS:
      (A) LENGTH: 64 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:15:
   Gln Arg Cys Lys Asp Lys Leu Asn Ser Leu Ala Ile Ser Val Met Asn
   1 5 10 15
   His Trp Pro Gly Val Lys Leu Arg Val Thr Glu Gly Trp Asp Glu Asp
   20 25 30
   Gly His His Phe Glu Glu Ser Leu His Tyr Glu Gly Arg Ala Val Asp
   35 40 45
   Ile Thr Thr Ser Asp Arg Asp Lys Ser Lys Tyr Gly Thr Leu Ser Arg
   50 55 60
(2) INFORMATION FOR SEQ ID NO:16:
   (i) SEQUENCE CHARACTERISTICS:
      (A) LENGTH: 65 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:16:
   Gln Arg Cys Lys Glu Lys Leu Asn Ser Leu Ala Ile Ser Val Met Asn
   1               5                   10                  15
   Met Trp Pro Gly Val Lys Leu Arg Val Thr Glu Gly Trp Asp Glu Asp
               20                  25                  30
   Gly Asn His Phe Glu Asp Ser Leu His Tyr Glu Gly Arg Ala Val Asp
           35                  40                  45
   Ile Thr Thr Ser Ser Asp Arg Asp Arg Asn Lys Tyr Gly Met Phe Ala
       50                  55                  60
   Arg
   65
(2) INFORMATION FOR SEQ ID NO:17:
   (i) SEQUENCE CHARACTERISTICS:
      (A) LENGTH: 64 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:17:
   Gln Arg Cys Lys Asp Lys Leu Asn Ser Leu Ala Ile Ser Val Met Asn
   1               5                   10                  15
   Leu Trp Pro Gly Val Lys Leu Arg Val Thr Glu Gly Trp Asp Glu Asp
               20                  25                  30
   Gly Leu His Ser Glu Glu Ser Leu His Tyr Glu Gly Arg Ala Val Asp
           35                  40                  45
   Ile Thr Thr Ser Asp Arg Asp Arg Asn Lys Tyr Arg Met Leu Ala Arg
       50                  55                  60
(2) INFORMATION FOR SEQ ID NO:18:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:18:
      GGAATTCCCA GCAGNTGCTA AAGGAAGCAA GNGCTNAA 38
(2) INFORMATION FOR SEQ ID NO:19:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:19:
      TCATCGATGG ACCCAGATCG AAANCCNGCT CTC 33
(2) INFORMATION FOR SEQ ID NO:20:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:20:
      GCTCTAGAGC TCNACNGCNA GANCGTNGC 29
(2) INFORMATION FOR SEQ ID NO:21:
   (i) SEQUENCE CHARACTERISTICS:
      (A) LENGTH:: 50 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
      AGCTGTCGAC GCGGCCGCTA CGTAGGTTAC CGACGTCAAG CTTAGATCTC 50
(2) INFORMATION FOR SEQ ID NO:22:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:22:
      AGCTGAGATC TAAGCTTGAC GTCGGTAACC TACGTAGCGG CCGCGTCGAC 50
(2) INFORMATION FOR SEQ ID NO:23:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:23:
      GATCGGCCAG GCAGGCCTCG CGATATCGTC ACCGCGGTAT TCGAA 45
(2) INFORMATION FOR SEQ ID NO:24:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:24:
      AGTGCCAGTC GGGGCCCCCA GGGCCGCGCC 30
(2) INFORMATION FOR SEQ ID NO:25:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:25:
      TACCACAGCG GATGGTTCGG 20
(2) INFORMATION FOR SEQ ID NO:26:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:26:
      GTGGTGGTTA TGCCGATCGC 20
(2) INFORMATION FOR SEQ ID NO:27:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:27:
      TAAGAGGCCT ATAAGAGGCG G 21
(2) INFORMATION FOR SEQ ID NO:28:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:28:
      AAGTCAGCCC AGAGGAGACT 20
(2) INFORMATION FOR SEQ ID NO:29:
   (i) SEQUENCE CHARACTERISTICS:
      (A) LENGTH: 6 amino acids
      (B) TYPE: amino acid
      (D) TOPOLOGY: linear
   (ii) MOLECULE-TYPE: peptide
   (v) FRAGMENT TYPE: internal
   (xi) SEQUENCE DESCRIPTION: SEQ ID NO:29:
   Cys Gly Pro Gly Arg Gly
   1 5
(2) INFORMATION FOR SEQ ID NO:30:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:30:
      AGCAGNTGCT AAAGGAAGCA AGNGCTNAA 29
(2) INFORMATION FOR SEQ ID NO:31:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:31:
      CTCNACNGCN AGANCKNGTN GCNA 24
(2) INFORMATION FOR SEQ ID NO:32:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:32:
      CTGCAGGGAT CCACCATGCG GCTTTTGACG AG 32
(2) INFORMATION FOR SEQ ID NO:33:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:33:
      CTGCAGGGAT CCTTATTCCA CACGAGGGAT T 31
(2) INFORMATION FOR SEQ ID NO:34:
   (i) SEQUENCE CHARACTERISTICS:
      (A) LENGTH: 471 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:34:
   Met Asp Asn His Ser Ser Val Pro Trp Ala Ser Ala Ala Ser Val Thr
   1 5 10 15
   Cys Leu Ser Leu Asp Ala Lys Cys His Ser Ser Ser Ser Ser Ser Ser
               20                  25                  30
   Ser Lys Ser Ala Ala Ser Ser Ile Ser Ala Ile Pro Gln Glu Glu Thr
           35                  40                  45
   Gln Thr Met Arg His Ile Ala His Thr Gln Arg Cys Leu Ser Arg Leu
       50                  55                  60
   Thr Ser Leu Val Ala Leu Leu Leu Ile Val Leu Pro Met Val Phe Ser
   65                  70                  75                  80
   Pro Ala His Ser Cys Gly Pro Gly Arg Gly Leu Gly Arg His Arg Ala
                   85                  90                  95
   Arg Asn Leu Tyr Pro Leu Val Leu Lys Gln Thr Ile Pro Asn Leu Ser
               100                 105                 110
   Glu Tyr Thr Asn Ser Ala Ser Gly Pro Leu Glu Gly Val Ile Arg Arg
           115                 120                 125
   Asp Ser Pro Lys Phe Lys Asp Leu Val Pro Asn Tyr Asn Arg Asp Ile
       130                 135                 140
   Leu Phe Arg Asp Glu Glu Gly Thr Gly Ala Asp Arg Leu Met Ser Lys
   145                 150                 155                 160
   Arg Cys Lys Glu Lys Leu Asn Val Leu Ala Tyr Ser Val Met Asn Glu
                   165                 170                 175
   Trp Pro Gly Ile Arg Leu Leu Val Thr Glu Ser Trp Asp Glu Asp Tyr
               180                 185                 190
   His His Gly Gln Glu Ser Leu His Tyr Glu Gly Arg Ala Val Thr Ile
           195                 200                 205
   Ala Thr Ser Asp Arg Asp Gln Ser Lys Tyr Gly Met Leu Ala Arg Leu
       210                 215                 220
   Ala Val Glu Ala Gly Phe Asp Trp Val Ser Tyr Val Ser Arg Arg His
   225                 230                 235                 240
   Ile Tyr Cys Ser Val Lys Ser Asp Ser Ser Ile Ser Ser His Val His
                   245                 250                 255
   Gly Cys Phe Thr Pro Glu Ser Thr Ala Leu Leu Glu Ser Gly Val Arg
               260                 265                 270
   Lys Pro Leu Gly Glu Leu Ser Ile Gly Asp Arg Val Leu Ser Met Thr
           275                 280                 285
   Ala Asn Gly Gln Ala Val Tyr Ser Glu Val Ile Leu Phe Met Asp Arg
       290                 295                 300
   Asn Leu Glu Gln Met Gln Asn Phe Val Gln Leu His Thr Asp Gly Gly
   305                 310                 315                 320
   Ala Val Leu Thr Val Thr Pro Ala His Leu Val Ser Val Trp Gln Pro
   325 330 335
   Glu Ser Gln Lys Leu Thr Phe Val Phe Ala Asp Arg Ile Glu Glu Lys
   340 345 350
   Asn Gln Val Leu Val Arg Asp Val Glu Thr Gly Glu Leu Arg Pro Gln
   355 360 365
   Arg Val Val Lys Val Gly Ser Val Arg Ser Lys Gly Val Val Ala Pro
   370 375 380
   Leu Thr Arg Glu Gly Thr Ile Val Val Asn Ser Val Ala Ala Ser Cys
   385 390 395 400
   Tyr Ala Val Ile Asn Ser Gln Ser Leu Ala His Trp Gly Leu Ala Pro
   405 410 415
   Met Arg Leu Leu Ser Thr Leu Glu Ala Trp Leu Pro Ala Lys Glu Gln
   420 425 430
   Leu His Ser Ser Pro Lys Val Val Ser Ser Ala Gln Gln Gln Asn Gly
   435 440 445
   Ile His Trp Tyr Ala Asn Ala Leu Tyr Lys Val Lys Asp Tyr Val Leu
   450 455 460
   Pro Gln Ser Trp Arg His Asp
   465 470
(2) INFORMATION FOR SEQ ID NO:35:
   (i) SEQUENCE CHARACTERISTICS:
      (A) LENGTH: 73 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:35:
   Arg Cys Lys Glu Arg Val Asn Ser Leu Ala Ile Ala Val Met His Met
   1 5 10 15
   Trp Pro Gly Val Arg Leu Arg Val Thr Glu Gly Trp Asp Glu Asp Gly
   20 25 30
   His His Leu Pro Asp Ser Leu His Tyr Glu Gly Arg Ala Leu Asp Ile
   35 40 45
   Thr Thr Ser Asp Arg Asp Arg His Lys Tyr Gly Met Leu Ala Arg Leu
   50 55 60
   Ala Val Glu Ala Gly Phe Asp Trp Val
   65 70
(2) INFORMATION FOR SEQ ID NO:36:
   (i) SEQUENCE CHARACTERISTICS:
      (A) LENGTH: 73 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:36:
   Arg Cys Lys Asp Lys Leu Asn Ala Leu Ala Ile Ser Val Met Asn Gln
   1 5 10 15
   Trp Pro Gly Val Lys Leu Arg Val Thr Glu Gly Trp Asp Glu Asp Gly
   20 25 30
   His His Ser Glu Glu Ser Leu His Tyr Glu Gly Arg Ala Val Asp Ile
   35 40 45
   Thr Thr Ser Asp Arg Asp Arg Ser Lys Tyr Gly Met Leu Ala Arg Leu
   50 55 60
   Ala Val Glu Ala Gly Phe Asp Trp Val
   65 70
(2) INFORMATION FOR SEQ ID NO:37:
   (i) SEQUENCE CHARACTERISTICS:
      (A) LENGTH: 64 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:37:
   Lys Arg Cys Lys Glu Lys Leu Asn Val Leu Ala Tyr Ser Val Met Asn
   1 5 10 15
   Glu Trp Pro Gly Ile Arg Leu Val Val Thr Glu Ser Trp Asp Glu Asp
   20 25 30
   Tyr His His Gly Gln Glu Ser Leu His Tyr Glu Gly Arg Ala Val Thr
   35 40 45
   Ile Ala Thr Ser Asp Arg Asp Gln Ser Lys Tyr Gly Met Leu Ala Arg
   50 55 60
(2) INFORMATION FOR SEQ ID NO:38:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:38:
      AAAAGCTTTA YTGYTAYGTN GGNATHGG 28
(2) INFORMATION FOR SEQ ID NO:39:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:39:
      AAGAATTCTA NGCRTTRTAR TTRTTNGG 28
(2) INFORMATION FOR SEQ ID NO:40:
   (i) SEQUENCE CHARACTERISTICS:
      (A) LENGTH: 165 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:40:
   Cys Gly Pro Gly Arg Gly Xaa Gly Xaa Arg Arg His Pro Lys Lys Leu
   1 5 10 15
   Thr Pro Leu Ala Tyr Lys Gln Phe Ile Pro Asn Val Ala Glu Lys Thr
   20 25 30
   Leu Gly Ala Ser Gly Arg Tyr Glu Gly Lys Ile Xaa Arg Asn Ser Glu
   35 40 45
   Arg Phe Lys Glu Leu Thr Pro Asn Tyr Asn Pro Asp Ile Ile Phe Lys
   50 55 60
   Asp Glu Glu Asn Thr Gly Ala Asp Arg Leu Met Thr Gln Arg Cys Lys
   65 70 75 80
   Asp Lys Leu Asn Xaa Leu Ala Ile Ser Val Met Asn Xaa Trp Pro Gly
   85 90 95
   Val Xaa Leu Arg Val Thr Glu Gly Trp Asp Glu Asp Gly His His Xaa
   100 105 110
   Glu Glu Ser Leu His Tyr Glu Gly Arg Ala Val Asp Ile Thr Thr Ser
   115 120 125
   Asp Arg Asp Xaa Ser Lys Tyr Gly Xaa Leu Xaa Arg Leu Ala Val Glu
   130 135 140
   Ala Gly Phe Asp Trp Val Tyr Tyr Glu Ser Lys Ala His Ile His Cys
   145 150 155 160
   Ser Val Lys Ala Glu
   165
(2) INFORMATION FOR SEQ ID NO:41:
   (i) SEQUENCE CHARACTERISTICS:
      (A) LENGTH: 167 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:41:
   Cys Gly Pro Gly Arg Gly Xaa Xaa Xaa Arg Arg Xaa Xaa Xaa Pro Lys
   1 5 10 15
   Xaa Leu Xaa Pro Leu Xaa Tyr Lys Gln Phe Xaa Pro Xaa Xaa Xaa Glu
   20 25 30
   Xaa Thr Leu Gly Ala Ser Gly Xaa Xaa Glu Gly Xaa Xaa Xaa Arg Xaa
   35 40 45
   Ser Glu Arg Phe Xaa Xaa Leu Thr Pro Asn Tyr Asn Pro Asp Ile Ile
   50 55 60
   Phe Lys Asp Glu Glu Asn Xaa Gly Ala Asp Arg Leu Met Thr Xaa Arg
   65 70 75 80
   Cys Lys Xaa Xaa Xaa Asn Xaa Leu Ala Ile Ser Val Met Asn Xaa Trp
   85 90 95
   Pro Gly Val Xaa Leu Arg Val Thr Glu Gly Xaa Asp Glu Asp Gly His
   100 105 110
   His Xaa Xaa Xaa Ser Leu His Tyr Glu Gly Arg Ala Xaa Asp Ile Thr
   115 120 125
   Thr Ser Asp Arg Asp Xaa Xaa Lys Tyr Gly Xaa Leu Xaa Arg Leu Ala
   130 135 140
   Val Glu Ala Gly Phe Asp Trp Val Tyr Tyr Glu Ser Xaa Xaa His Xaa
   145 150 155 160
   His Xaa Ser Val Lys Xaa Xaa
   165
(2) INFORMATION FOR SEQ ID NO:42:
   (i) SEQUENCE CHARACTERISTICS:
      (A) LENGTH: 3900 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..3897
   (xi) SEQUENCE DESCRIPTION: SEQ ID NO:42:
   ATG GAC CGC GAC AGC CTC CCA CGC GTT CCG GAC ACA CAC GGC GAT GTG 48
   Met Asp Arg Asp Ser Leu Pro Arg Val Pro Asp Thr His Gly Asp Val
   1 5 10 15
   GTC GAT GAG AAA TTA TTC TCG GAT CTT TAC ATA CGC ACC AGC TGG GTG 96
   Val Asp Glu Lys Leu Phe Ser Asp Leu Tyr Ile Arg Thr Ser Trp Val
   20 25 30
   GAC GCC CAA GTG GCG CTC GAT CAG ATA GAT AAG GGC AAA GCG CGT GGC 144
   Asp Ala Gln Val Ala Leu Asp Gln Ile Asp Lys Gly Lys Ala Arg Gly
   35 40 45
   AGC CGC ACG GCG ATC TAT CTG CGA TCA GTA TTC CAG TCC CAC CTC GAA 192
   Ser Arg Thr Ala Ile Tyr Leu Arg Ser Val Phe Gln Ser His Leu Glu
   50 55 60
   ACC CTC GGC AGC TCC GTG CAA AAG CAC GCG GGC AAG GTG CTA TTC GTG 240
   Thr Leu Gly Ser Ser Val Gln Lys His Ala Gly Lys Val Leu Phe Val
   65 70 75 80
   GCT ATC CTG GTG CTG AGC ACC TTC TGC GTC GGC CTG AAG AGC GCC CAG          288
   Ala Ile Leu Val Leu Ser Thr Phe Cys Val Gly Leu Lys Ser Ala Gln
                    85                  90                  95
   ATC CAC TCC AAG GTG CAC CAG CTG TGG ATC CAG GAG GGC GGC GGC CTG          336
   Ile His Ser Lys Val His Gln Leu Trp Ile Gln Glu Gly Gly Gly Leu
               100                 105                 110
   GAG GCG GAA CTG GCC TAC ACA CAG AAG ACG ATC GGC GAG GAC GAG TCG          384
   Glu Ala Glu Leu Ala Tyr Thr Gln Lys Thr Ile Gly Glu Asp Glu Ser
           115                 120                 125
   GCC ACG CAT CAG CTG CTC ATT CAG ACG ACC CAC GAC CCG AAC GCC TCC          432
   Ala Thr His Gln Leu Leu Ile Gln Thr Thr His Asp Pro Asn Ala Ser
       130                 135                 140
   GTC CTG CAT CCG CAG GCG CTG CTT GCC CAC CTG GAG GTC CTG GTC AAG          480
   Val Leu His Pro Gln Ala Leu Leu Ala His Leu Glu Val Leu Val Lys
   145                 150                 155                 160
   GCC ACC GCC GTC AAG GTG CAC CTC TAC GAC ACC GAA TGG GGG CTG CGC          528
   Ala Thr Ala Val Lys Val His Leu Tyr Asp Thr Glu Trp Gly Leu Arg
                   165                 170                 175
   GAC ATG TGC AAC ATG CCG AGC ACG CCC TCC TTC GAG GGC ATC TAC TAC          576
   Asp Met Cys Asn Met Pro Ser Thr Pro Ser Phe Glu Gly Ile Tyr Tyr
               180                 185                 190
   ATC GAG CAG ATC CTG CGC CAC CTC ATT CCG TGC TCG ATC ATC ACG CCG          624
   Ile Glu Gln Ile Leu Arg His Leu Ile Pro Cys Ser Ile Ile Thr Pro
           195                 200                 205
   CTG GAC TGT TTC TGG GAG GGA AGC CAG CTG TTG GGT CCG GAA TCA GCG          672
   Leu Asp Cys Phe Trp Glu Gly Ser Gln Leu Leu Gly Pro Glu Ser Ala
       210                 215                 220
   GTC GTT ATA CCA GGC CTC AAC CAA CGA CTC CTG TGG ACC ACA CTG AAT          720
   Val Val Ile Pro Gly Leu Asn Gln Arg Leu Leu Trp Thr Thr Leu Asn
   225                 230                 235                 240
   CCC GCC TCT GTG ATG CAG TAT ATG AAG CAG AAG ATG TCC GAG GAA AAG          768
   Pro Ala Ser Val Met Gln Tyr Met Lys Gln Lys Met Ser Glu Glu Lys
                   245                 250                 255
   ATC AGC TTC GAC TTC GAG ACC GTG GAG CAG TAC ATG AAG CGT GCG GCC          816
   Ile Ser Phe Asp Phe Glu Thr Val Glu Gln Tyr Met Lys Arg Ala Ala
               260                 265                 270
   ATT GCG AGT GGC TAC ATG GAG AAG CCC TGC CTG AAC CCA CTG AAT CCC          864
   Ile Ala Ser Gly Tyr Met Glu Lys Pro Cys Leu Asn Pro Leu Asn Pro
           275                 280                 285
   AAT TGC CCG GAC ACG GCA CCG AAC AAG AAC AGC ACC CAG CCG CCG GAT          912
   Asn Cys Pro Asp Thr Ala Pro Asn Lys Asn Ser Thr Gln Pro Pro Asp
       290                 295                 300
   GTG GGA GCC ATC CTG TCC GGA GGC TGC TAC GGT TAT GCC GCG AAG CAC        960
   Val Gly Ala Ile Leu Ser Gly Gly Cys Tyr Gly Tyr Ala Ala Lys His
   305                 310                 315                 320
   ATG CAC TGG CCG GAG GAG CTG ATT GTG GGC GGA GCG AAG AGG AAC CGC       1008
   Met His Trp Pro Glu Glu Leu Ile Val Gly Gly Ala Lys Arg Asn Arg
                   325                 330                 335
   AGC GGA CAC TTG AGG AAG GCC CAG GCC CTG CAG TCG GTG GTG CAG CTG       1056
   Ser Gly His Leu Arg Lys Ala Gln Ala Leu Gln Ser Val Val Gln Leu
               340                 345                 350
   ATG ACC GAG AAG GAA ATG TAC GAC CAG TGG CAG GAC AAC TAC AAG GTG       1104
   Met Thr Glu Lys Glu Met Tyr Asp Gln Trp Gln Asp Asn Tyr Lys Val
           355                 360                 365
   CAC CAT CTT GGA TGG ACG CAG GAG AAG GCA GCG GAG GTT TTG AAC GCC       1152
   His His Leu Gly Trp Thr Gln Glu Lys Ala Ala Glu Val Leu Asn Ala
       370                 375                 380
   TGG CAG CGC AAC TTT TCG CGG GAG GTG GAA CAG CTG CTA CGT AAA CAG       1200
   Trp Gln Arg Asn Phe Ser Arg Glu Val Glu Gln Leu Leu Arg Lys Gln
   385                 390                 395                 400
   TCG AGA ATT GCC ACC AAC TAC GAT ATC TAC GTG TTC AGC TCG GCT GCA       1248
   Ser Arg Ile Ala Thr Asn Tyr Asp Ile Tyr Val Phe Ser Ser Ala Ala
                   405                 410                 415
   CTG GAT GAC ATC CTG GCC AAG TTC TCC CAT CCC AGC GCC TTG TCC ATT       1296
   Leu Asp Asp Ile Leu Ala Lys Phe Ser His Pro Ser Ala Leu Ser Ile
               420                 425                 430
   GTC ATC GGC GTG GCC GTC ACC GTT TTG TAT GCC TTC TGC ACG CTC CTC       1344
   Val Ile Gly Val Ala Val Thr Val Leu Tyr Ala Phe Cys Thr Leu Leu
           435                 440                 445
   CGC TGG AGG GAC CCC GTC CGT GGA CAG AGC AGT GTC GGC GTG GCC GGA       1392
   Arg Trp Arg Asp Pro Val Arg Gly Gln Ser Ser Val Gly Val Ala Gly
       450                 455                 460
   GTT CTG CTC ATG TGC TTT AGT ACC GCC GCC GGA TTG GGA TTG TCA GCC       1440
   Val Leu Leu Met Cys Phe Ser Thr Ala Ala Gly Leu Gly Leu Ser Ala
   465                 470                 475                 480
   CTG CTC GGT ATC GTT TTC AAT GCC GCC AGC ACC CAG GTG GTT CCG TTT       1488
   Leu Leu Gly Ile Val Phe Asn Ala Ala Ser Thr Gln Val Val Pro Phe
                   485                 490                 495
   TTG GCC CTT GGT CTG GGC GTC GAT CAC ATC TTC ATG CTG ACC GCT GCC       1536
   Leu Ala Leu Gly Leu Gly Val Asp His Ile Phe Met Leu Thr Ala Ala
               500                 505                 510
   TAT GCG GAG AGC AAT CGG CGG GAG CAG ACC AAG CTG ATT CTC AAG AAA       1584
   Tyr Ala Glu Ser Asn Arg Arg Glu Gln Thr Lys Leu Ile Leu Lys Lys
           515                 520                 525
   GTG GGA CCG AGC ATC CTG TTC AGT GCC TGC AGC ACC GCA GGA TCC TTC     1632
   Val Gly Pro Ser Ile Leu Phe Ser Ala Cys Ser Thr Ala Gly Ser Phe
       530                 535                 540
   TTT GCG GCC GCC TTT ATT CCG GTG CCG GCT TTG AAG GTA TTC TGT CTG     1680
   Phe Ala Ala Ala Phe Ile Pro Val Pro Ala Leu Lys Val Phe Cys Leu
   545                     550                 555                 560
   CAG GCT GCC ATC GTA ATG TGC TCC AAT TTG GCA GCG GCT CTA TTG GTT     1728
   Gln Ala Ala Ile Val Met Cys Ser Asn Leu Ala Ala Ala Leu Leu Val
                   565                 570                 575
   TTT CCG GCC ATG ATT TCG TTG GAT CTA CGG AGA CGT ACC GCC GGC AGG     1776
   Phe Pro Ala Met Ile Ser Leu Asp Leu Arg Arg Arg Thr Ala Gly Arg
               580                 585                 590
   GCG GAC ATC TTC TGC TGC TGT TTT CCG GTG TGG AAG GAA CAG CCG AAG     1824
   Ala Asp Ile Phe Cys Cys Cys Phe Pro Val Trp Lys Glu Gln Pro Lys
           595                 600                 605
   GTG GCA CCA CCG GTG CTG CCG CTG AAC AAC AAC AAC GGG CGC GGG GCC     1872
   Val Ala Pro Pro Val Leu Pro Leu Asn Asn Asn Asn Gly Arg Gly Ala
       610                 615                 620
   CGG CAT CCG AAG AGC TGC AAC AAC AAC AGG GTG GCG CTG CCC GCC CAG     1920
   Arg His Pro Lys Ser Cys Asn Asn Asn Arg Val Ala Leu Pro Ala Gln
   625                     630                 635                 640
   AAT CCT CTG CTG GAA CAG AGG GCA GAC ATC CCT GGG AGC AGT CAC TCA     1968
   Asn Pro Leu Leu Glu Gln Arg Ala Asp Ile Pro Gly Ser Ser His Ser
                   645                 650                 655
   CTG GCG TCC TTC TCT CTG GCA ACA TTC GCC TTT CAG CAC TAC ACT CCC     2016
   Leu Ala Ser Phe Ser Leu Ala Thr Phe Ala Phe Gln His Tyr Thr Pro
               660                 665                 670
   TTC CTC ATG CGC AGC TGG GTG AAG TTC CTG ACC GTT ATG GGT TTC CTG     2064
   Phe Leu Met Arg Ser Trp Val Lys Phe Leu Thr Val Met Gly Phe Leu
           675                 680                 685
   GCG GCC CTC ATA TCC AGC TTG TAT GCC TCC ACG CGC CTT CAG GAT GGC     2112
   Ala Ala Leu Ile Ser Ser Leu Tyr Ala Ser Thr Arg Leu Gln Asp Gly
       690                 695                 700
   CTG GAC ATT ATT GAT CTG GTG CCC AAG GAC AGC AAC GAG CAC AAG TTC     2160
   Leu Asp Ile Ile Asp Leu Val Pro Lys Asp Ser Asn Glu His Lys Phe
   705                     710                 715                 720
   CTG GAT GCT CAA ACT CGG CTC TTT GGC TTC TAC AGC ATG TAT GCG GTT     2208
   Leu Asp Ala Gln Thr Arg Leu Phe Gly Phe Tyr Ser Met Tyr Ala Val
                   725                 730                 735
   ACC CAG GGC AAC TTT GAA TAT CCC ACC CAG CAG CAG TTG CTC AGG GAC     2256
   Thr Gln Gly Asn Phe Glu Tyr Pro Thr Gln Gln Gln Leu Leu Arg Asp
               740                 745                 750
   TAC CAT GAT TCC TTT GTG CGG GTG CCA CAT GTG ATC AAG AAT GAT AAT      2304
   Tyr His Asp Ser Phe Val Arg Val Pro His Val Ile Lys Asn Asp Asn
           755                 760                 765
   GGT GGA CTG CCG GAC TTC TGG CTG CTG CTC TTC AGC GAG TGG CTG GGT      2352
   Gly Gly Leu Pro Asp Phe Trp Leu Leu Leu Phe Ser Glu Trp Leu Gly
       770                 775                 780
   AAT CTG CAA AAG ATA TTC GAC GAG GAA TAC CGC GAC GGA CGG CTG ACC      2400
   Asn Leu Gln Lys Ile Phe Asp Glu Glu Tyr Arg Asp Gly Arg Leu Thr
   785                 790                 795                 800
   AAG GAG TGC TGG TTC CCA AAC GCC AGC AGC GAT GCC ATC CTG GCC TAC      2448
   Lys Glu Cys Trp Phe Pro Asn Ala Ser Ser Asp Ala Ile Leu Ala Tyr
                   805                 810                 815
   AAG CTA ATC GTG CAA ACC GGC CAT GTG GAC AAC CCC GTG GAC AAG GAA      2496
   Lys Leu Ile Val Gln Thr Gly His Val Asp Asn Pro Val Asp Lys Glu
               820                 825                 830
   CTG GTG CTC ACC AAT CGC CTG GTC AAC AGC GAT GGC ATC ATC AAC CAA      2544
   Leu Val Leu Thr Asn Arg Leu Val Asn Ser Asp Gly Ile Ile Asn Gln
           835                 840                 845
   CGC GCC TTC TAC AAC TAT CTG TCG GCA TGG GCC ACC AAC GCG TCT TCG      2592
   Arg Ala Phe Tyr Asn Tyr Leu Ser Ala Trp Ala Thr Asn Ala Ser Ser
       850                 855                 860
   CCT ACG GAG CTT CTC AGG GCA AAT TGT ATC CGG AAC CGC GCC AAC GGA      2640
   Pro Thr Glu Leu Leu Arg Ala Asn Cys Ile Arg Asn Arg Ala Asn Gly
   865                 870                 875                 880
   GCT TCT CAG GGC AAA TTG TAT CCG GAA CCG CGC CAG TAT TTT CAC CAA      2688
   Ala Ser Gln Gly Lys Leu Tyr Pro Glu Pro Arg Gln Tyr Phe His Gln
                   885                 890                 895
   CCC AAC GAG TAC GAT CTT AAG ATA CCC AAG AGT CTG CCA TTG GTC TAC      2736
   Pro Asn Glu Tyr Asp Leu Lys Ile Pro Lys Ser Leu Pro Leu Val Tyr
               900                 905                 910
   GCT CAG ATG CCC TTT TAC CTC CAC GGA CTA ACA GAT ACC TCG CAG ATC      2784
   Ala Gln Met Pro Phe Tyr Leu His Gly Leu Thr Asp Thr Ser Gln Ile
           915                 920                 925
   AAG ACC CTG ATA GGT CAT ATT CGC GAC CTG AGC GTC AAG TAC GAG GGC      2832
   Lys Thr Leu Ile Gly His Ile Arg Asp Leu Ser Val Lys Tyr Glu Gly
       930                 935                 940
   TTC GGC CTG CCC AAC TAT CCA TCG GGC ATT CCC TTC ATC TTC TGG GAG      2880
   Phe Gly Leu Pro Asn Tyr Pro Ser Gly Ile Pro Phe Ile Phe Trp Glu
   945                 950                 955                 960
   CAG TAC ATG ACC CTG CGC TCC TCA CTG GCC ATG ATC CTG GCC TGC GTG      2928
   Gln Tyr Met Thr Leu Arg Ser Ser Leu Ala Met Ile Leu Ala Cys Val
                   965                 970                 975
   CTA CTC GCC GCC CTG GTG CTG GTC TCC CTG CTC CTG CTC TCC GTT TGG      2976
   Leu Leu Ala Ala Leu Val Leu Val Ser Leu Leu Leu Leu Ser Val Trp
               980                 985                 990
   GCC GCC GTT CTC GTG ATC CTC AGC GTT CTG GCC TCG CTG GCC CAG ATC      3024
   Ala Ala Val Leu Val Ile Leu Ser Val Leu Ala Ser Leu Ala Gln Ile
           995                 1000                1005
   TTT GGG GCC ATG ACT CTG CTG GGC ATC AAA CTC TCG GCC ATT CCG GCA      3072
   Phe Gly Ala Met Thr Leu Leu Gly Ile Lys Leu Ser Ala Ile Pro Ala
       1010                1015                1020
   GTC ATA CTC ATC CTC AGC GTG GGC ATG ATG CTG TGC TTC AAT GTG CTG      3120
   Val Ile Leu Ile Leu Ser Val Gly Met Met Leu Cys Phe Asn Val Leu
   1025                1030                1035                1040
   ATA TCA CTG GGC TTC ATG ACA TCC GTT GGC AAC CGA CAG CGC CGC GTC      3168
   Ile Ser Leu Gly Phe Met Thr Ser Val Gly Asn Arg Gln Arg Arg Val
                   1045                1050                1055
   CAG CTG AGC ATG CAG ATG TCC CTG GGA CCA CTT GTC CAC GGC ATG CTG      3216
   Gln Leu Ser Met Gln Met Ser Leu Gly Pro Leu Val His Gly Met Leu
               1060                1065                1070
   ACC TCC GGA GTG GCC GTG TTC ATG CTC TCC ACG TCG CCC TTT GAG TTT      3264
   Thr Ser Gly Val Ala Val Phe Met Leu Ser Thr Ser Pro Phe Glu Phe
           1075                1080                1085
   GTG ATC CGG CAC TTC TGC TGG CTT CTG CTG GTG GTC TTA TGC GTT GGC      3312
   Val Ile Arg His Phe Cys Trp Leu Leu Leu Val Val Leu Cys Val Gly
       1090                1095                1100
   GCC TGC AAC AGC CTT TTG GTG TTC CCC ATC CTA CTG AGC ATG GTG GGA      3360
   Ala Cys Asn Ser Leu Leu Val Phe Pro Ile Leu Leu Ser Met Val Gly
   1105                1110                1115                1120
   CCG GAG GCG GAG CTG GTG CCG CTG GAG CAT CCA GAC CGC ATA TCC ACG      3408
   Pro Glu Ala Glu Leu Val Pro Leu Glu His Pro Asp Arg Ile Ser Thr
                   1125                1130                1135
   CCC TCT CCG CTG CCC GTG CGC AGC AGC AAG AGA TCG GGC AAA TCC TAT      3456
   Pro Ser Pro Leu Pro Val Arg Ser Ser Lys Arg Ser Gly Lys Ser Tyr
               1140                1145                1150
   GTG GTG CAG GGA TCG CGA TCC TCG CGA GGC AGC TGC CAG AAG TCG CAT      3504
   Val Val Gln Gly Ser Arg Ser Ser Arg Gly Ser Cys Gln Lys Ser His
           1155                1160                1165
   CAC CAC CAC CAC AAA GAC CTT AAT GAT CCA TCG CTG ACG ACG ATC ACC      3552
   His His His His Lys Asp Leu Asn Asp Pro Ser Leu Thr Thr Ile Thr
       1170                1175                1180
   GAG GAG CCG CAG TCG TGG AAG TCC AGC AAC TCG TCC ATC CAG ATG CCC      3600
   Glu Glu Pro Gln Ser Trp Lys Ser Ser Asn Ser Ser Ile Gln Met Pro
   1185                1190                1195                1200
   AAT GAT TGG ACC TAC CAG CCG CGG GAA CAG CGA CCC GCC TCC TAC GCG      3648
   Asn Asp Trp Thr Tyr Gln Pro Arg Glu Gln Arg Pro Ala Ser Tyr Ala
                   1205                1210                1215
   GCC CCG CCC CCC GCC TAT CAC AAG GCC GCC GCC CAG CAG CAC CAC CAG      3696
   Ala Pro Pro Pro Ala Tyr His Lys Ala Ala Ala Gln Gln His His Gln
               1220                1225                1230
   CAT CAG GGC CCG CCC ACA ACG CCC CCG CCG CCC TTC CCG ACG GCC TAT      3744
   His Gln Gly Pro Pro Thr Thr Pro Pro Pro Pro Phe Pro Thr Ala Tyr
           1235                1240                1245
   CCG CCG GAG CTG CAG AGC ATC GTG GTG CAG CCG GAG GTG ACG GTG GAG      3792
   Pro Pro Glu Leu Gln Ser Ile Val Val Gln Pro Glu Val Thr Val Glu
       1250                1255                1260
   ACG ACG CAC TCG GAC AGC AAC ACC ACC AAG GTG ACG GCC ACG GCC AAC      3840
   Thr Thr His Ser Asp Ser Asn Thr Thr Lys Val Thr Ala Thr Ala Asn
   1265                1270                1275                1280
   ATC AAG GTG GAG CTG GCC ATG CCC GGC AGG GCG GTG CGC AGC TAT AAC      3888
   Ile Lys Val Glu Leu Ala Met Pro Gly Arg Ala Val Arg Ser Tyr Asn
                   1285                1290                1295
   TTT ACG AGT TAG                                                      3900
   Phe Thr Ser
(2) INFORMATION FOR SEQ ID NO:43:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:43:
      ACCGAGGGCT GGGACGAAGA TGGC 24
(2) INFORMATION FOR SEQ ID NO:44:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:44:
      CGCTCGGTCG TACGGCATGA ACGAC 25
(2) INFORMATION FOR SEQ ID NO:45:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:45:
      ATGGGGATGT GTGTGTGGTC AAGTGTA 27
(2) INFORMATION FOR SEQ ID NO:46:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:46:
      TTCACAGACT CTCAAAGTGT ATTTT 25
(2) INFORMATION FOR SEQ ID NO:47:
   (i) SEQUENCE CHARACTERISTICS:
      (A) LENGTH: 18 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:47:
   Met Gly Ser Ser His His His His His His Leu Val Pro Arg Gly Ser
   1               5                   10                  15
   His Met

## Description

### Background of the Invention

Pattern formation is the activity by which embryonic cells form ordered spatial arrangements of differentiated tissues. The physical complexity of higher organisms arises during embryogenesis through the interplay of cell-intrinsic lineage and cell-extrinsic signaling. Inductive interactions are essential to embryonic patterning in vertebrate development from the earliest establishment of the body plan, to the patterning of the organ systems, to the generation of diverse cell types during tissue differentiation (Davidson, E., (1990) Development 108: 365-389; Gurdon, J. B., (1992) Cell 68: 185-199; Jessell, T. M. et al., (1992) Cell 68: 257-270). The effects of developmental cell interactions are varied. Typically, responding cells are diverted from one route of cell differentiation to another by inducing cells that differ from both the uninduced and induced states of the responding cells (inductions). Sometimes cells induce their neighbors to differentiate like themselves (homoiogenetic induction); in other cases a cell inhibits its neighbors from differentiating like itself. Cell interactions in early development may be sequential, such that an initial induction between two cell types leads to a progressive amplification of diversity. Moreover, inductive interactions occur not only in embryos, but in adult cells as well, and can act to establish and maintain morphogenetic patterns as well as induce differentiation (J.B. Gurdon (1992) Cell 68:185-199).

The origin of the nervous system in all vertebrates can be traced to the end of gastrulation. At this time, the ectoderm in the dorsal side of the embryo changes its fate from epidermal to neural. The newly formed neuroectoderm thickens to form a flattened structure called the neural plate which is characterized, in some vertebrates, by a central groove (neural groove) and thickened lateral edges (neural folds). At its early stages of differentiation, the neural plate already exhibits signs of regional differentiation along its anterior posterior (A-P) and mediolateral axis (M-L). The neural folds eventually fuse at the dorsal midline to form the neural tube which will differentiate into brain at its anterior end and spinal cord at its posterior end. Closure of the neural tube creates dorsal/ventral differences by virtue of precious mediolateral differentiation. Thus, at the end of neurulation, the neural tube has a clear anterior-posterior (A-P), dorsal ventral (D-V) and mediolateral (M-L) polarities (see, for example, Principles in Neural Science (3rd), eds. Kandel, Schwartz and Jessell, Elsevier Science Publishing Company: NY, 1991; and Developmental Biology (3rd), ed. S.F. Gilbert, Sinauer Associates: Sunderland MA, 1991). Inductive interactions that define the fate of cells within the neural tube establish the initial pattern of the embryonic vertebrate nervous system. In the spinal cord, the identify of cell types is controlled, in part, by signals from two midline cell groups, the notochord and floor plate, that induce neural plate cells to differentiate into floor plate, motor neurons, and other ventral neuronal types (van Straaten et al. (1988) Anat. Embryol. 177:317-324; Placzek et al. (1993) Development 117:205-218; Yamada et al. (1991) Cell 64:035-647; and Hatta et al. (1991) Nature 350:339-341). In addition, signals from the floor plate are responsible for the orientation and direction of commissural neuron outgrowth (Placzek, M. et al., (1990) Development 110: 19-30). Besides patterning the neural tube, the notochord and floorplate are also responsible for producing signals which control the patterning of the somites by inhibiting differentiation of dorsal somite derivatives in the ventral regions (Brand-Saberi, B. et al., (1993) Anat. Embryol. 188: 239-245; Porquie, O. et al., (1993) Proc. Natl. Acad Sci. USA 90: 5242-5246).

Another important signaling center exists in the posterior mesenchyme of developing limb buds, called the Zone of Polarizing Activity, or "ZPA". When tissue from the posterior region of the limb bud is grafted to the anterior border of a second limb bud, the resultant limb will develop with additional digits in a mirror-image sequence along the anteroposterior axis (Saunders and Gasseling, (1968) Epithelial-Mesenchymal Interaction, pp. 78-97). This finding has led to the model that the ZPA is responsible for normal anteroposterior patterning in the limb. The ZPA has been hypothesized to function by releasing a signal, termed a "morphogen", which forms a gradient across the early embryonic bud. According to this model, the fate of cells at different distances from the ZPA is determined by the local concentration of the morphogen, with specific thresholds of the morphogen inducing successive structures (Wolpert, (1969) Theor. Biol. 25:1-47). This is supported by the finding that the extent of digit duplication is proportional to the number of implanted ZPA cells (Tickle, (1981) Nature 254:199-202).

A candidate for the putative ZPA morphogen was identified by the discovery that a source of retinoic acid can result in the same type of mirror-image digit duplications when placed in the anterior of a limb bud (Tickle et al., (1982) Nature 296:564-565; Summerbell, (1983) J. Embryol 78:269-289). The response to exogenous retinoic acid is concentration dependent as the morphogen model demands (Tickle et al., (1985) Dev. Biol. 109:82-95). Moreover, a differential distribution of retinoic acid exists across the limb bud, with a higher concentration in the ZPA region (Thaller and Eichele, (1987) Nature 327:625-628).

Recent evidence, however, has indicated that retinoic acid is unlikely to be the endogenous factor responsible for ZPA activity (reviewed in Brockes, (1991) Nature 350:15; Tabin, (1991) Cell 66:199-217). It is now believed that rather than directly mimicking an endogenous signal, retinoic acid implants act by inducing an ectopic ZPA. The anterior limb tissue just distal to a retinoic acid implant and directly under the ectoderm has been demonstrated to acquire ZPA activity by serially transplanting that tissue to another limb bud (Summerbell and Harvey, (1983) Limb Development and Regeneration pp. 109-118; Wanek et al., (1991) Nature 350:81-83). Conversely; the tissue next to a ZPA graft does not gain ZPA activity (Smith, (1979) J. Embryol 52:105-113). Exogenous retinoic acid would thus appear to act upstream of the ZPA in limb patterning,

The immediate downstream targets of ZPA action are not known. However, one important set of genes which are ectopically activated during ZPA-induced pattern duplications are the 5' genes of the Hoxd cluster. These genes are normally expressed in a nested pattern emanating from the posterior margin of the limb bud (Dolle et al., (1989) Nature 342:767-772; Izpisua-Belmonte et al., (1991) Nature 350:585-599). This nested pattern of Hox gene expression has been directly demonstrated to determine the identity of the structures produced along the anteroposterior axis of the limb (Morgan et al., (1993) Nature 358:236-239). As this would predict, ZPA grafts which produce mirror-image duplication of structures at an anatomical level first lead to the ectopic activation of the Hoxd genes in a mirror-image duplication at the molecular level. (Nohno et al., (1991) Cell 64:1197-1205; Izpisua-Belmonte et al., (1991) Nature 350:585-589). The molecular signals which regulate the expression of these important genes are currently not understood.

Fietz, M. et al., Development (1994, vol. (0), p43-51) describes the hedgehog gene family in Drosophila and vertebrate development. Riddle, R. D. et al., Cell (1993, vol. 75(7), p1401-1416), discusses the role of shh in mediating the polarizing activity of the ZPA. Echelard, Y. et al., Cell (1993, voL 75(7), p1417-1430), describes shh as being a member of a family of putative signalling molecules and its role in the regulation of CNS polarity. Roelink, H. et al., Cell (1994, vol. 76(4), p761-775) concerns floor plate and motor neuron induction by vhh-1. a vertebrate homolog of hedgehog expressed by the notochord.

### Summary of the Invention

The present invention relates to the discovery of a novel family of proteins present in vertebrate organisms, referred to hereinafter as "*hedgehog*" proteins, which proteins have apparent broad involvement in the formation and maintenance of ordered spatial arrangements of differentiated tissues in vertebrates, and can be used to generate and/or maintain an array of different vertebrate tissue both *in vitro* and *in vivo*.

In general, the invention features *hedgehog* polypeptides, preferably substantially pure preparations of one or more of the subject *hedgehog* polypeptides. The invention also provides recombinantly produced *hedgehog* polypeptides. In preferred embodiments the polypeptide has a biological activity including: an ability to modulate proliferation, survival and/or differentiation of mesodennally-derived tissue, such as tissue derived from dorsal mesoderm; the ability to modulate proliferation, survival and/or differentiation of ectodermally-derived tissue, such as tissue derived from the neural tube, neural crest, or head mesenchyme. Moreover, the subject *hedgehog* proteins have the ability to induce expression of secondary signalling molecules, such as members of the Transforming Growth Factor β family, as well as members of the fibroblast growth factor (FGF) family.

In a certain embodiments, the polypeptide is identical with or homologous to a mammalian *Sonic hedgehog* (*Shh*) polypeptide, represented by SEQ ID No. 11. For instance, the *Shh* polypeptide preferably has an amino acid sequence at least 90% homologous to a polypeptide represented by any of SEQ ID No: 11, though polypeptides with higher sequence homologies of, for example 95% are also contemplated. Exemplary *Shh* proteins are represented by SEQ ID No. 40. The *Shh* polypeptide can comprise a full length protein, such as represented in the sequence listings, or it can comprise a fragment of, for instance, at least 50, 100 of 150 amino acids in length. Preferred *hedgehog* polypeptides include *Shh* sequences corresponding Approximately to the natural proteolytic fragments of the *hedgehog* proteins, such as from about Cys-24 through Glu-188, or from about Asn-189 through Ala-475 of the human *Shh* protein, or analogous fragments thereto.

Moreover, as described below, the *hedgehog* polypeptide can be either an agonist (e.g. mimics), or alternatively, an antagonist of a biological activity of a naturally occurring form of the protein, e.g., the polypeptide is able to modulate differentiation and/or growth and/or survival of a cell responsive to authentic *hedgehog* proteins. Homologs of the subject *hedgehog* proteins include versions of the protein which are resistant to proteolytic cleavage, as for example, due to mutations which alter potential cleavage sequences or which inactivate an enzymatic activity associated with the protein.

The *hedgehog* polypeptides of the present invention can be glycosylated, or conversely, by choice of the expression system or by modification of the protein sequence to preclude glycosylation, reduced carbohydrate analogs can also be provided. Glycosylated forms include derivatization with glycosaminoglycan chains. Likewise, *hedgehog* polypeptides can be generated which lack an endogenous signal sequence (though this is typically cleaved off even if present in the pro-form of the protein).

The subject proteins can also be provided as chimeric molecules, such as in the form of fusion proteins. For instance, the *hedgehog* protein can be provided as a recombinant fusion protein which includes a second polypeptide portion, e.g., a second polypeptide having an amino acid sequence unrelated to *hedgehog,* e.g. the second polypeptide portion is glutathione-S-transferase, e.g. the second polypeptide portion is an enzymatic activity such as alkaline phosphatase. e.g, the second polypeptide portion is an epitope tag.

Yet another aspects of the present invention concerns an immunogen comprising a *hedgehog* polypeptide in an immunogenic preparation, the immunogen being capable of eliciting an immune response specific for a *hedgehog* polypeptide; e.g. a humoral response, e.g. an antibody response; e.g. a cellular response. In preferred embodiments, the immunogen comprising an antigenic determinant, e.g. a unique determinant, from a protein represented by one of SEQ ID No 11.

A still further aspect of the present invention features antibodies and antibody preparations specifically reactive with an epitope of the *hedgehog* immunogen.

Another aspect of the present invention provides a substantially isolated nucleic acid having a nucleotide sequence which encodes a *hedgehog* polypeptide. In preferred embodiment, the encoded polypeptide specifically agonizes or antagonizes inductive events mediated by wild-type *hedgehog* proteins. The coding sequence of the nucleic acid can comprise a sequence which is identical to a coding sequence represented in one of SEQ ID Nos: 4, or it can merely be homologous to one or more of those sequences. The polypeptide encoded by the nucleic acid can comprise an amino acid sequence represented in SEQ ID No 11 such as one of those full length proteins, or it can comprise a fragment of that nucleic acid, which fragment may, for instance, encode a fragment which is, for example, at least 50 or 100 amino acids in length. The polypeptide encoded by the nucleic acid can be either an agonist (e.g. mimics), or alternatively, an antagonist of a biological activity of a naturally occurring form of a *hedgehog* protein.

Furthermore, in certain preferred embodiments, the subject *hedgehog* nucleic acid will include a transcriptional regulatory sequence. e.g. at least one of a transcriptional promoter or transcriptional enhancer sequence which regulatory sequence is operably linked to the *hedgehog* gene sequence Such regulatory sequences can be used in to render the *hedgehog* gene sequence suitable for use as an expression vector.

In yet a further preferred embodiment, the nucleic acid hybridizes under stringent conditions to a nucleic acid probe corresponding to at least 12 consecutive nucleotides of SEQ ID Nos: 4; though preferably corresponding to at least 20 consecutive nucleotides; and more preferably corresponding to at least 40, 50 or 75 consecutive nucleotides of SEQ ID Nos: 4.

Nucleic acid probes which are specific for *shh hedgehog* proteins are contemplated by the present invention, e.g. probes which can discern between nucleic acid encoding an *Shh* versus an *Ihh* versus a *Dhh* versus an *Mhh*. In preferred embodiment, the probe/primer further includes a label group attached thereto and able to be detected. The label group can be selected, e.g., from a group consisting of radioisotopes, fluorescent compounds, enzymes, and enzyme co-factors. Probes of the invention can be used as a part of a diagnostic test kit for identifying dysfunctions associated with mis-expression of a *hedgehog* protein, such as for detecting in a sample of cells isolated from a patient, a level of a nucleic acid encoding a subject *hedgehog* protein; e.g. measuring a *hedgehog* mRNA level in a cell, or determining whether a genomic *hedgehog* gene has been mutated or deleted. Preferably, the oligonucleotide is at least 50, 100, or 150 nucleotide in length.

In yet another aspect, the invention provides an assay for screening test compounds for inhibitors, or alternatively, potentiators, of an interaction between a *hedgehog* protein and a *hedgehog* receptor. An exemplary method includes the steps of (i) combining a *hedgehog* receptor, either soluble or membrane bound (including whole cells), a *hedgehog* polypeptide, and a test compound, e.g., under conditions wherein, but for the test compound, the *hedgehog* protein and the *hedgehog* receptor are able to interact; and (ii) detecting the formation of a complex which includes the *hedgehog* protein and the receptor either by directly quantitating the complex or by measuring inductive effects of the *hedgehog* protein. A statistically significant change, such as a decrease, in the formation of the complex in the presence of a test compound (relative to what is seen in the absence of the test compound) is indicative of a modulation, e.g., inhibition, of the interaction between the *hedgehog* protein and the receptor.

Another aspect of the present invention relates to the use of *hh* proteins for inducing and/or maintaining a differentiated state, causing proliferation, and/or enhancing survival of a cell (from a vertebrate organism) responsive to a *hedgehog* protein, by contacting the cells with a *hedgehog* agonist. For example, the present use is applicable to cell cultures technique, such as in the culturing of neuronal and other cells whose survival or differentiative state is dependent on *hedgehog* function. Moreover, *hedgehog* agonists and antagonists can be used for therapeutic intervention, such has to enhance survival and maintenance of neurons and other neural cells in both the central nervous system and the peripheral nervous system, as well as to influence other vertebrate organogenic pathways, such as other ectodermal patterning, as well as certain mesodermal and endodermal differentiation processes.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed, 1985); Oligonucleotide Synthesis (M. J. Gait ed., 1984); Mullis et al. U.S. Patent No: 4,683,195; Nucleic Acid Hybridization (B. D. Harnes & S. J. Higgins eds. 1984); Transcription And Translation (B. D. Hames & S. J. Higgins eds. 1984); Culture Of Animal Cells (R. I. Freshney, Alan R. Liss. Inc., 1997); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds, 1987, Cold Spring Harbor Laboratory); Methods In Enzymology, Vols. 154 and 155 (Wu et al. eds.). Immunochemical Methods In Call And Molecular Biology (Mayer and Walker, eds., Academic Press. London, 1987); Handbook Of Experimental Immunology, Volumes I-IV (D. M. Weir and C., C. Blackwell, eds., 1986); Manipulating the Mouse Embryo, (Cold Spring Harbor Laboratory Press, Cold Spring Harbor, N.Y., 1986).

Other features and advantages of the invention will be apparent from the following detailed description, and from the claims. The terms "embryonic cells/tissues", embryo(s) and embryonic stem cells (ES) in the following description do not embrace material derived from humans.

### Brief Description of the Drawings

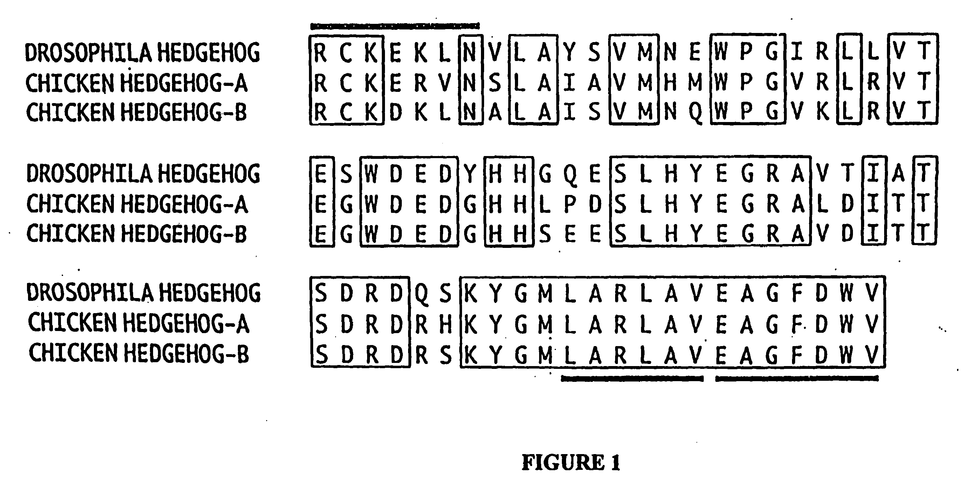

Figures 1 represents the amino acid sequences of two chick *hh* clones, chicken *hedgehog*-A (pCHA; SEQ ID No:35) and chicken *hodgehog-*B (pCHB; SEQ ID No:36). These clones were obtained using degenerate primers corresponding to the underlined amino acid residues of the Drosophila sequence (corresponding to residues 161-232 of SEQ ID No:34) also shown in Figure 1, followed by nested PCR using chicken genomic DNA.

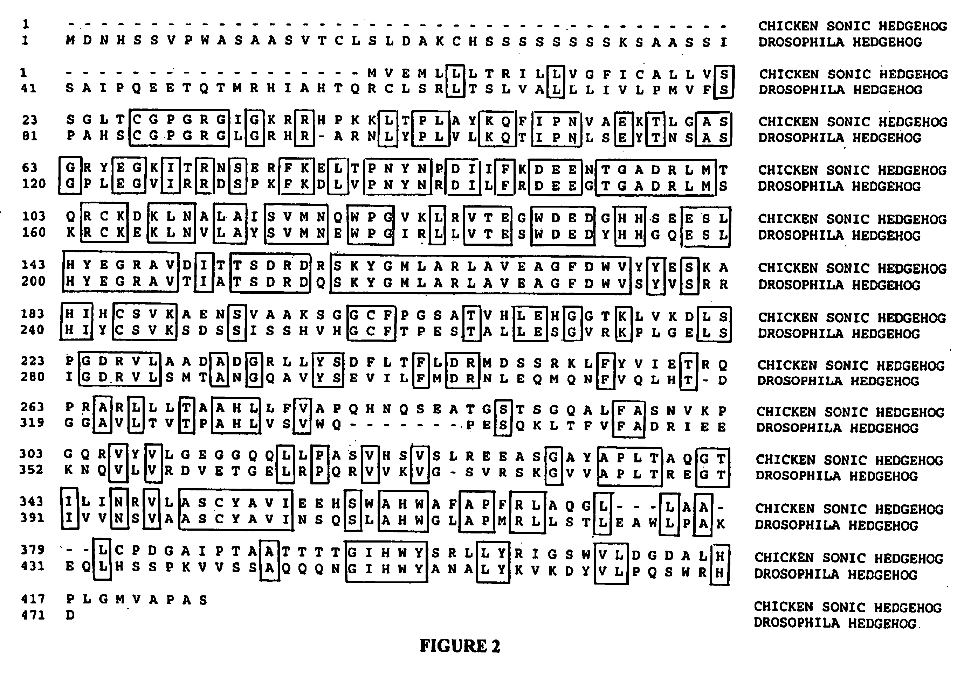

Figure 2 is an alignment comparing the amino acid sequences of chick *Shh* (SEQ ID No:8) with its Drosophila homolog (SEQ ID No:34). *Shh* residues 1-26 correspond to the proposed signal peptide. Identical residues are enclosed by boxes and gaps in order to highlight similarity. The nucleotide sequence of *Shh* has been submitted to Genbank.

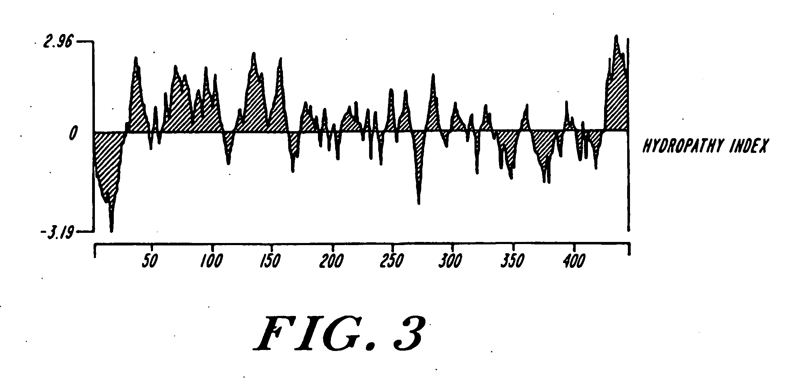

Figure 3 is a hydropathy plot for the predicted chick *Shh* protein, generated by the methods of Kyte and Doolittle (1982). The values of hydrophobicity are plotted against the amino acid positions. Negative values predict a hydrophobic domain of the protein.

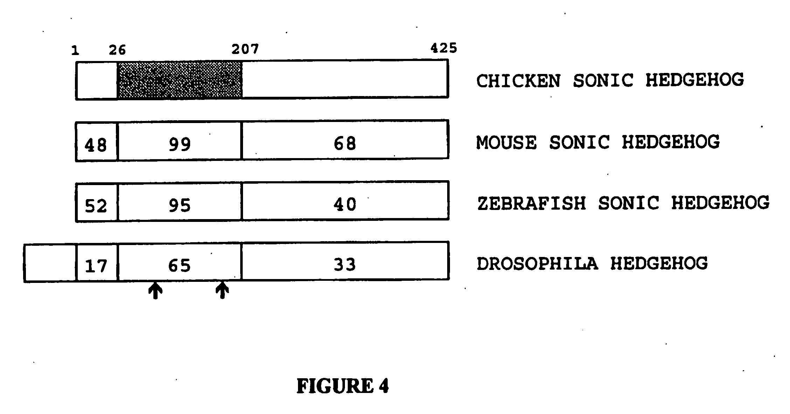

Figure 4 is an alignment comparing the amino acid sequences of various *hh* proteins. The white region on the amino terminus of chicken *Shh* corresponds to the putative signal peptide. The black box refers to a highly conserved region from aa residues 26-207 of SEQ ID No:8). The arrows point to exon boundaries in the Drosophila gene (Lee et al. (1992) Cell 71: 33-50). In each case, the proteins are compared to chicken *Shh* (SEQ ID No:8) and the percent amino acid identity is indicated in each region's box.

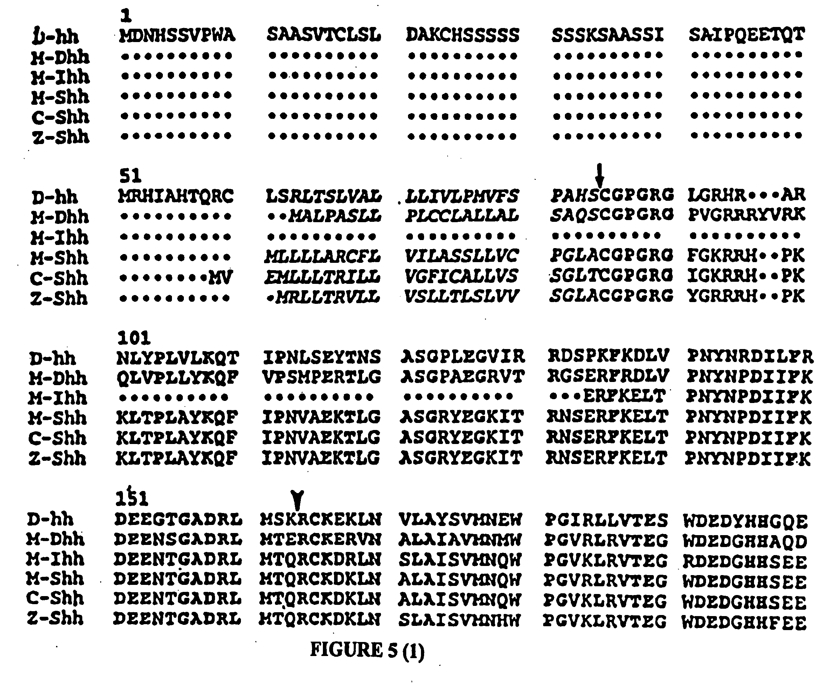

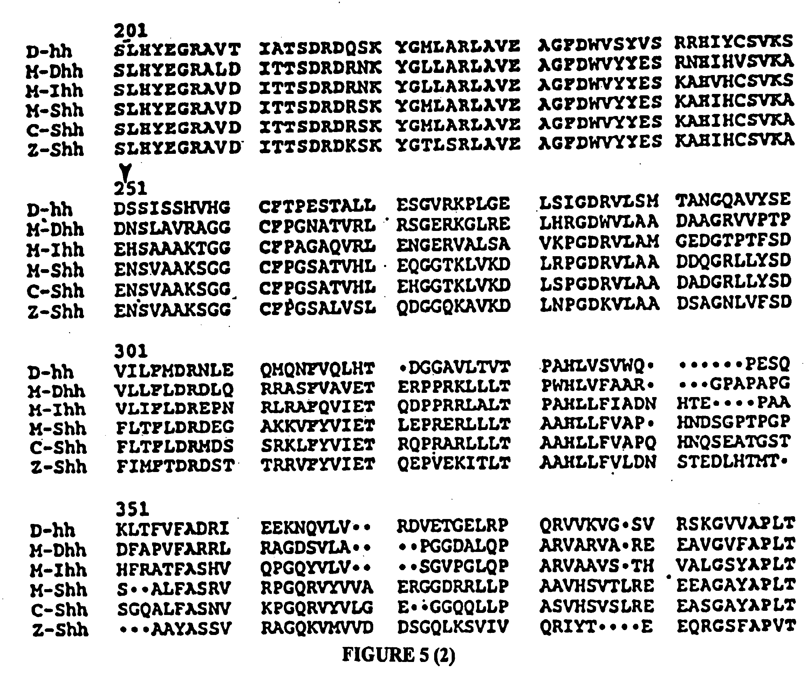

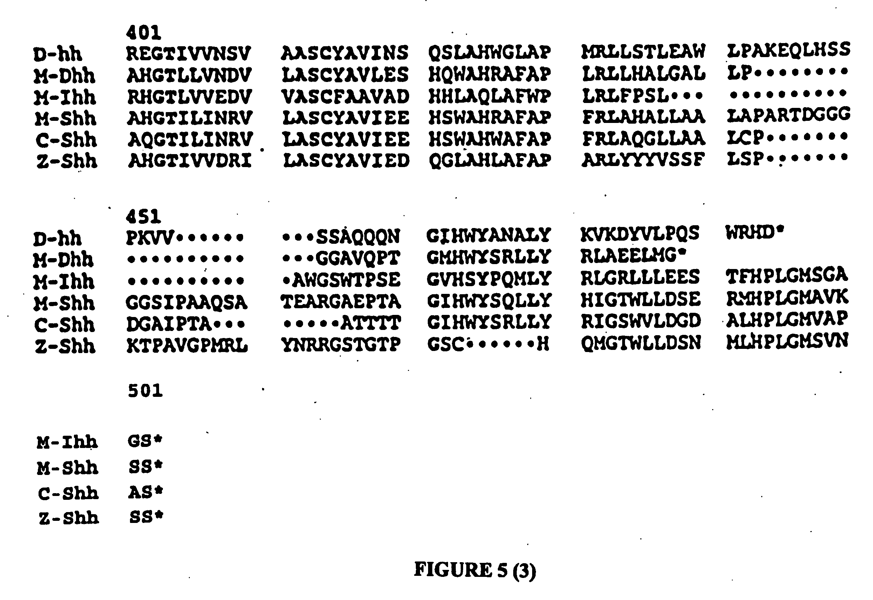

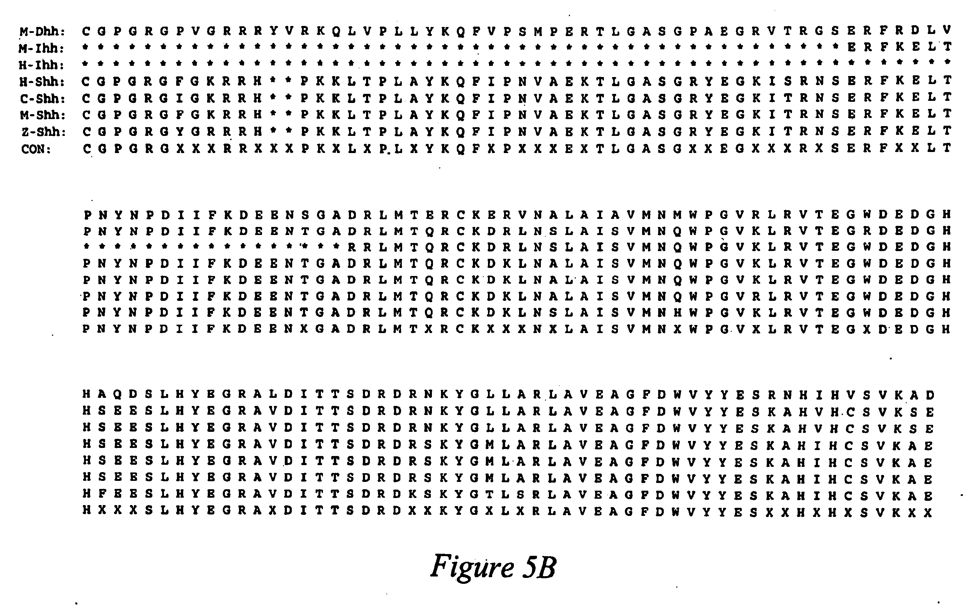

Figure 5A is a "pileup" alignment of predicted amino acid sequences which compares Drosophila *hh* (D-hh; SEQ ID No:34), mouse *hh* (M-*Dhh*; SEQ ID No:9; M-*Ihh*; SEQ ID No:10; M-*Shh*; SEQ ID No:11), chicken *hh* (C*-Shh*; SEQ ID No:8), and zebrafish *hh* (Z-*Shh*; SEQ ID No:12). The predicted hydrophobic transmembrane/signal sequences are indicated in italics and the predicted signal sequence processing site is arrowed. The positions of introns interrupting the Drosophila *hh* and M-*Dhh* open reading frames are indicated by arrowheads. All amino acids shared among the six predicted *hh* proteins are indicated in bold. Figure 5B is a sequence alignment of the N-terminal portion of vertebrate *hedgehog* proteins, and the predicted degenerate sequence "CON" (SEQ ID No: 41).

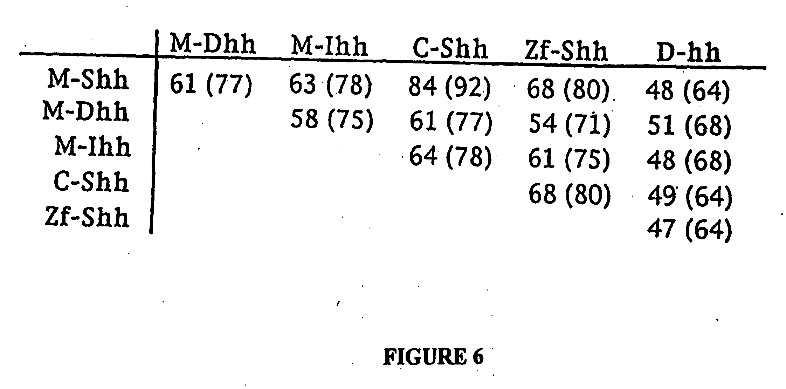

Figure 6 is an inter- and cross-species comparison of amino acid identities among the predicted processed *hh* proteins shown in Figure 5A. All values are percentages. Figures in parentheses represent similarities allowing for conservative amino acid substitutions.

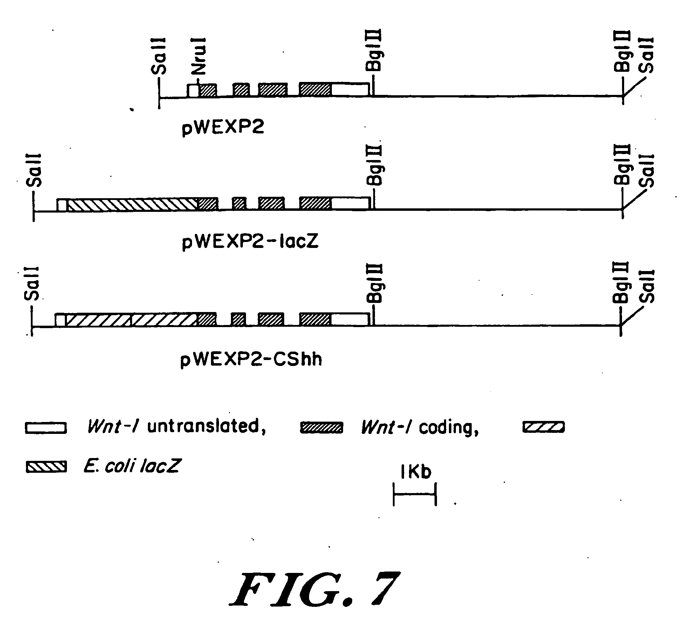

Figure 7 is a representation of the DNA constructs used in transgenic studies to study ectopic expression of chick *Shh* in mouse embryos. Constructs were generated for ectopic expression of cDNA clones in the *Wnt-l* expression domain and tested in transgenic mice embryos using a lac-Z reporter (pWEXP-*lacZ* (used as a control)) and a chick *Shh* reporter (pWEXP-CShh). The pWEXP-CShh construct contained two tandem head to tail copies of a chick *Shh* cDNA. The results of WEXP2-CShh transgenic studies are shown in Table 1.

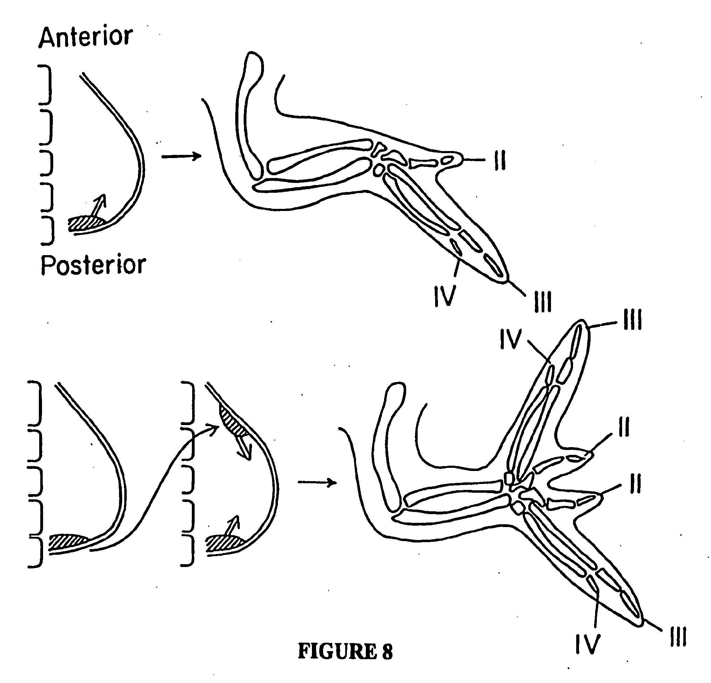

Figure 8 is a model for anterioposterior limb patterning and the Zone of Polarizing Activity (ZPA), based on Saunders and Gasseling (1968). The left portion of the diagram schematizes a stage 20 limb bud. The somites are illustrated as blocks along the left margin of the limb bud; right portion of the same panel illustrates the mature wing. The hatched region on the posterior limb is the ZPA. Normally, the developed wing contains three digits II, III, and IV. The figure further shows the result of transplanting a ZPA from one limb bud to the anterior margin of another. The mature limb now contains six digits IV, III, II, II, III, and IV in a mirror-image duplication of the normal pattern. The large arrows in both panels represent the signal produced by the ZPA which acts to specify digit identity.

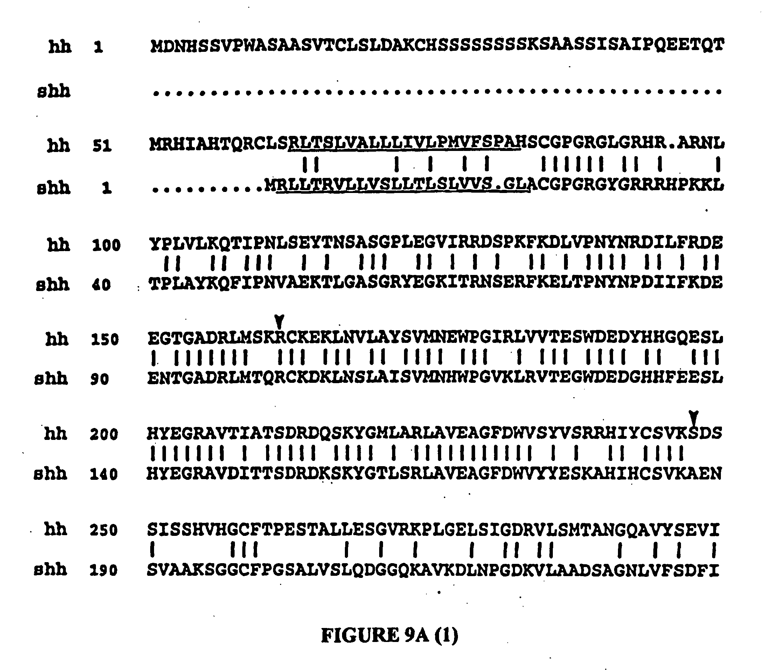

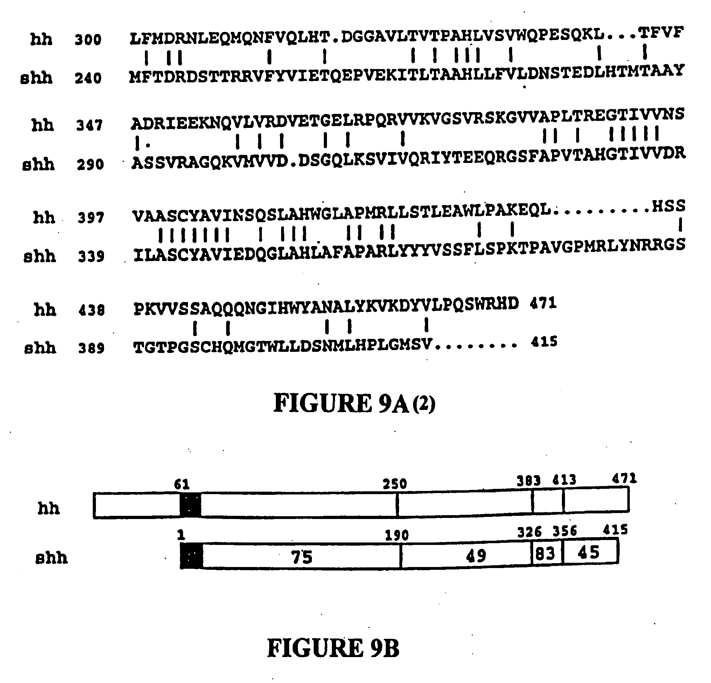

Figures 9A and 9B illustrate the comparison of zebrafish *Shh* (Z*-Shh*) and Drosophila *hh* (hh) amino acid sequences. Figure 9A is an alignment of zebrafish *Shh* and Drosophila *hh* amino acid sequences. Identical amino acids are linked by vertical bars. Dots indicate gaps introduced for optimal alignment. Putative transmembrane/signal peptide sequences are underlined (Kyte and Doolittle (1982) J Mol Biol 157:133-148). The position of exon boundaries in the Drosophila gene are indicated by arrowheads. The region of highest similarity between Z-*Shh* and *hh* overlaps exon 2. Figure 9B is a schematic comparison of Z-*Shh* and Drosophila *hh*. Black boxes indicate the position of the putative transmembrane/signal peptide sequences. relative to the amino-terminus. Sequence homologies were scored by taking into account the alignment of chemically similar amino acids and percentage of homology in the boxed regions is indicated.

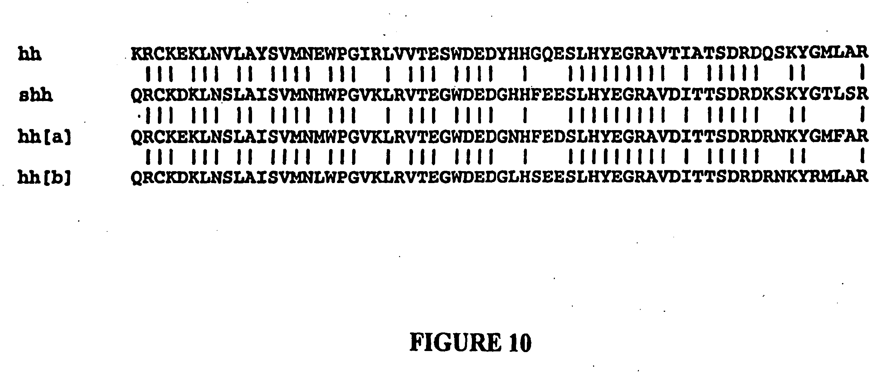

Figure 10 is an alignment of partial predicted amino acid sequences from three different zebrafish *hh* homologs. One of these sequences corresponds to *Shh*, while the other two define additional *hh* homologs in zebrafish, named *hh*(a) and *hh*(b). Amino acid identities among the three partial homologs are indicated by vertical bars.

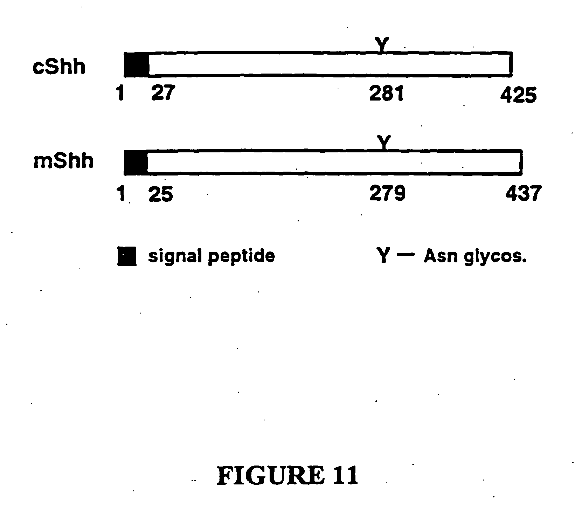

Figure 11 is a schematic representations of chick and mouse *Shh* proteins. The putative signal peptides and Asn-linked glycosylation sites are shown. The numbers refer to amino acid positions.

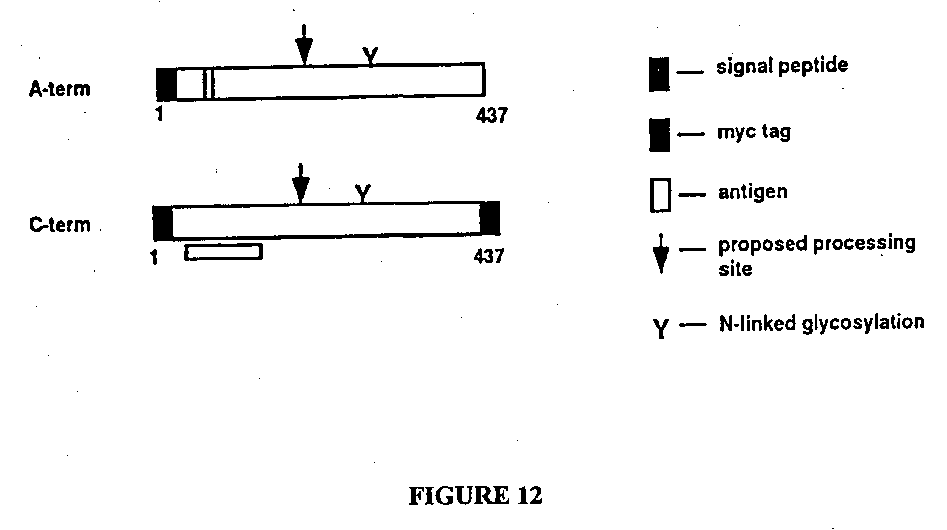

Figure 12 is a schematic representation of myc-tagged *Shh* constructs. The positions of the c-myc epitope tags are shown, as is the predicted position of the proteolytic cleavage site. The shaded area following the signal peptide of the carboxy terminal tagged construct represents the region included in the Glutathione-S-transferase fusion protein used to generate antisera in rabbits.

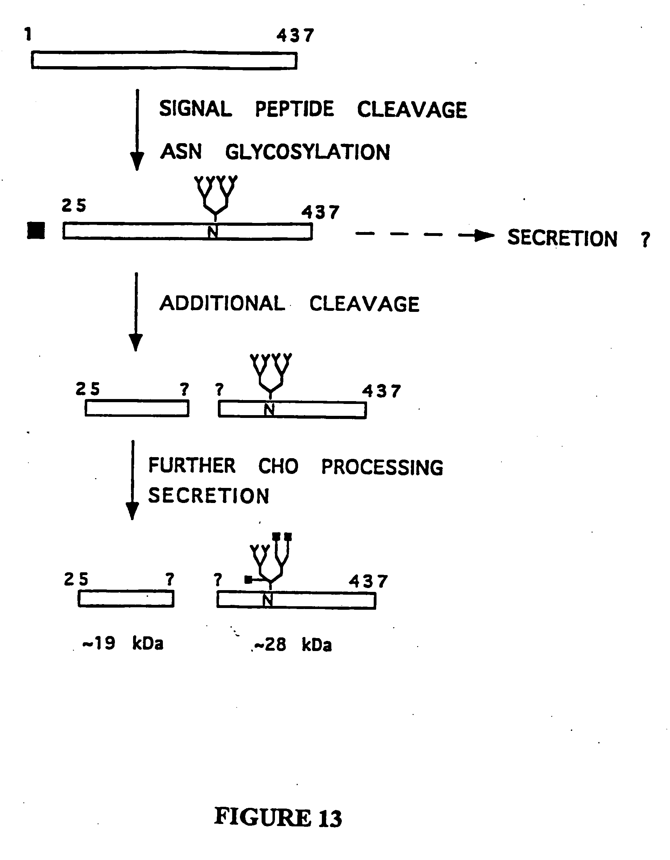

Figure 13 is a schematic diagram of *Shh* processing. Illustrated are cleavage of the signal peptide (black box), glycosylation at the predicted Asn residue (N), and the secondary proteolytic cleavage. The question marks indicate that the precise site of proteolytic cleavage has not been determined. The different symbols representing the carbohydrate moiety indicated maturation of this structure in the Golgi apparatus. The dashed arrow leading from the signal peptide cleaved protein indicates that secretion of this species may be an artifact of the incomplete proteolytic processing of *Shh* seen in *Xenopus* oocytes and cos cells.

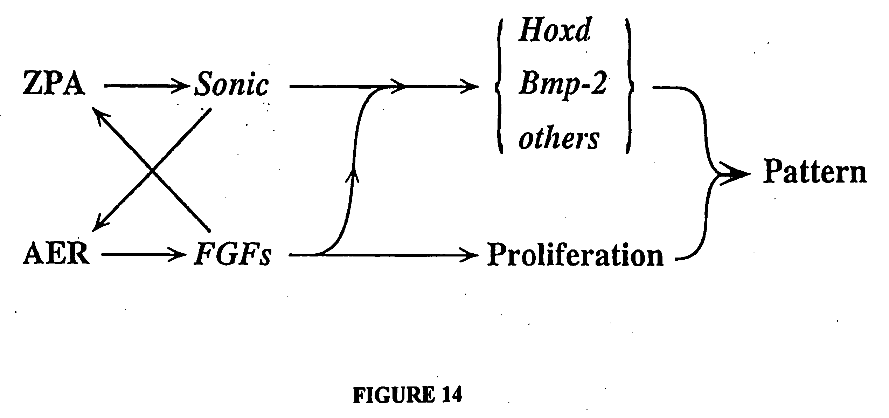

Figure 14 is a schematic diagram of a model for the coordinated growth and patterning of the limb. *Sonic* is proposed to signal directly to the mesoderm to induce expression of the *Hoxd* and *Bmp-2* genes. The induction of these mesodermal genes requires competence signals from the overlying AER. One such signal is apparently *Fgf-4.* Expression of *Fgf-4* in the AER can be induced by *Sonic* providing an indirect signaling pathway from *Sonic* to the mesoderm. FGFs also maintain expression of *Sonic* in the ZPA, thereby completing a positive feedback loop which controls the relative positions of the signaling centers. While *Fgf-4* provides competence signals to the mesoderm, it also promotes mesodermal proliferation. Thus patterning of the mesoderm is dependent on the same signals which promote its proliferation. This mechanism inextricably integrates limb patterning with outgrowth.

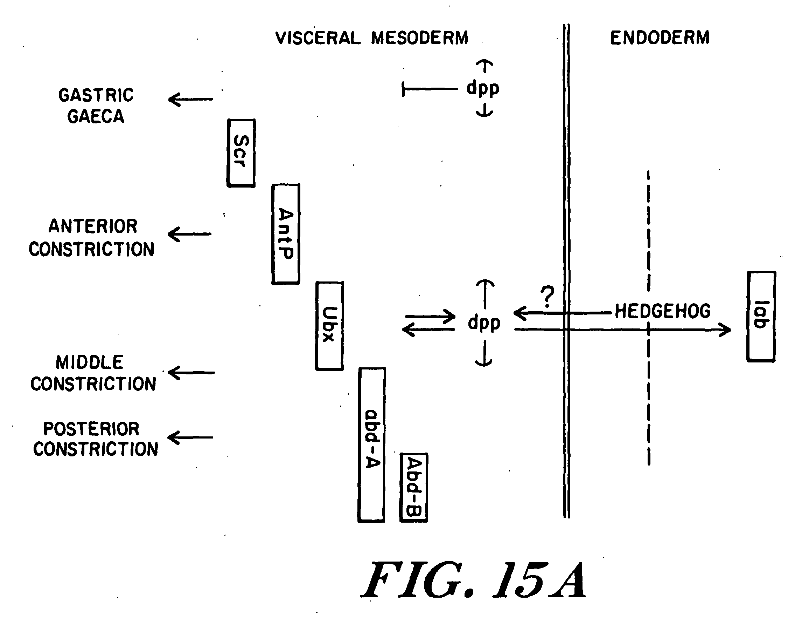

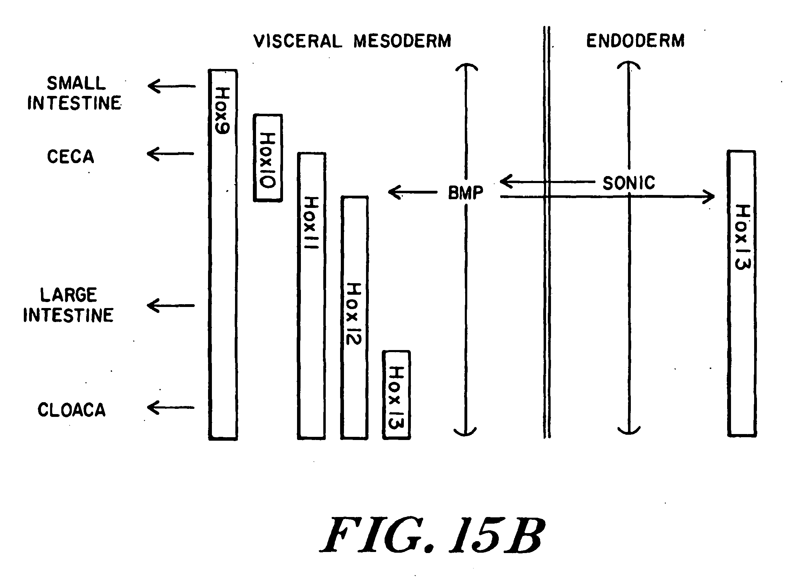

Figure 15 is a schematic diagram of patterning of the *Drosophila* and vertebrate gut. Regulatory interactions responsible for patterning of *Drosophila* midgut (A) are compared to a model for patterning of the vertebrate hindgut (B) based on expression data Morphologic regional distinctions are indicated to the left (A and B), genes expressed in the visceral mesoderm are in the center panel, those in the gut lumenal endoderm are on the right. *HOM*/*Hox* gene expression domains are boxed. Regionally expressing secreted gene products are indicated by lines. Arrows indicate activating interactions, barred lines, inhibiting interactions. Regulatory interactions in *Drosophila* gut (A) have been established by genetic studies except for the relationship between *dpp* and *hedgehog,* which is hypothesized based on their interactions in the *Drosophila* imaginal discs, *hedgehog* appears to be a signal from the endoderm to the mesoderm, and that *dpp* is expressed in the mesoderm.

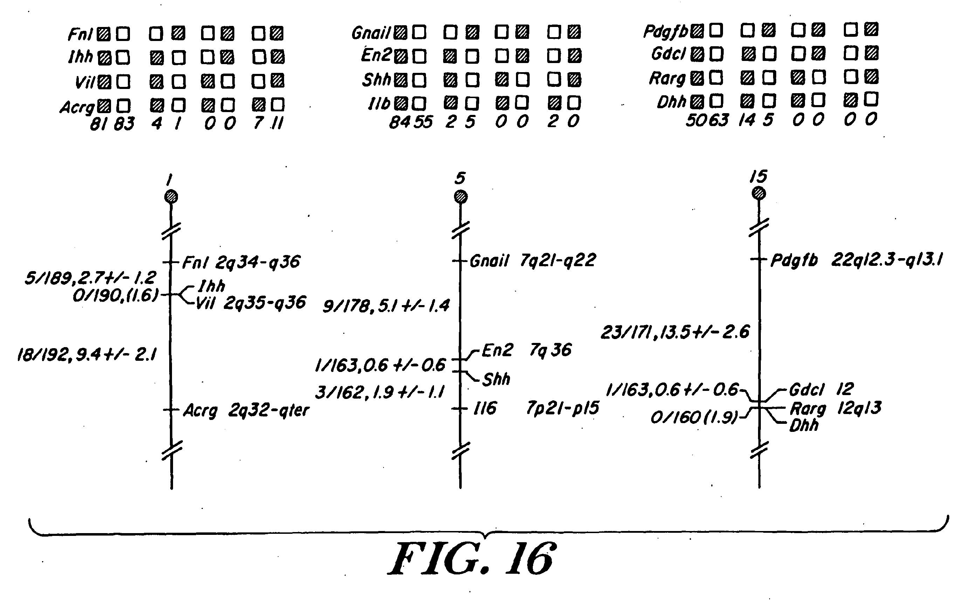

Figure 16 is a schematic diagram of chromosomal locations of *Ihh*, *Shh* and *Dhh* in the mouse genome. The loci were mapped by interspecific backcross analysis. The segregation patterns of the loci and flanking genes in backcross animals that were typed for all loci are shown above the chromosome maps. For individual pairs of loci more animals were typed. Each column represents the chromosome identified in the backcross progeny that was inherited from the (C57BL/6J x *M. spretus*) F1 parent. The shaded boxes represent the presence of a C57BL/6J allele and white boxes represent the presence of a *M. spretus* allele. The number of the offsprings inheriting each type of chromosome is listed at the bottom of each column. Partial chromosome linkage maps showing location of *Ihh, Shh* and *Dhh* in relation too linked genes is shown. The number of recombinant N₂ animals is presented over total number of N₂ animals typed to the left of the chromosome maps between each pair of loci. The recombinant frequencies, expressed as genetic distance in centimorgans (± one standard error) are also shown. When no recombination between loci was detected, the upper 95% confidence limit of the recombination distance is indicated in parentheses. Gene order was determined by minimizing the number of recombinant events required to explain the allele distribution patterns. The position of loci in human, chromosomes can be obtained from GDB (Genome Data Base), a computerized database of human linkage information maintained by the William H. Welch Medical Library of the John Hopkins University (Baltimore, MD).

### Detailed Description of the Invention

Embryonic inductive signals are key regulatory proteins that function in vertebrate pattern formation, and are present in important signaling centers known to operate embryonically to define the organization of the vertebrate embryo. For example, these signaling structures include the notochord, a transient structure which initiates the formation of the nervous system and helps to define the different types of neurons within it. The notochord also regulates mesodermal patterning along the body axis. Another distinct groups of cells having apparent signaling activity is the floorplate of the neural tube (the precursor of the spinal cord and brain) which also signal the differentiation of different nerve cell types. It is also generally believed that the region of mesoderm at the bottom of the buds which form the limbs (called the Zone of Polarizing Activity or ZPA) operates as a signaling center by secreting a morphogen which ultimately produces the correct patterning of the developing limbs.

The present invention concerns the discovery that proteins encoded by a family of vertebrate genes, termed here *hedgehog*-related genes, comprise the signals produced by these embryonic patterning centers. As described herein, each of the disclosed vertebrate *hedgehog* (*hh*) homologs exhibits spatially and temporally restricted expression domains indicative of important roles in embryonic patterning. For instance, the results provided below indicate that vertebrate *hh* genes are expressed in the posterior limb bud, Hensen's node, the early notochord, the floor plate of the neural tube, the fore- and hindgut and their derivatives. These are all important signaling centers known to be required for proper patterning of surrounding embryonic tissues.

The *Hedgehog* family of vertebrate inter-cellular signaling molecules consists of at least four members. Three of these members, herein referred to as Desert *hedgehog* (*Dhh*), Sonic *hedgehog* (*Shh*) and Indian *hedgehog* (*Ihh*), exist in all vertebrates, including fish, birds, and mammals. A fourth member, herein referred to as Moonrat *hedgehog* (*Mhh*), appears specific to fish. According to the appended sequence listing, (see also Table 1) a chicken *Shh* polypeptide is encoded by SEQ ID No:1; a mouse *Dhh* polypeptide is encoded by SEQ ID No:2; a mouse *Ihh* polypeptide is encoded by SEQ ID No:3: a mouse *Shh* polypeptide is encoded by SEQ ID No:4 a zebrafish *Shh* polypeptide is encoded by SEQ ID No:5; a human *Shh* polypeptide is encoded by SEQ ID No:6; and a human *Ihh* polypeptide is encoded by SEQ ID No:7.

**Table 1**

| Guide to vertebrate *hedgehog* sequences | | |
|---|---|---|
| | | |
| | Nucleotide | Amino Acid |
| Chicken *Shh* | SEQ ID No.1 | SEQ ID No. 8 |
| Mouse *Dhh* | SEQ ID No. 2 | SEQ ID No. 9 |
| Mouse *Ihh* | SEQ ID No. 3 | SEQ ID No.10 |
| Mouse *Shh* | SEQ ID No. 4 | SEQ ID No. 11 |
| Zebrafish *Shh* | SEQ ID No. 5 | SEQ ID No. 12 |
| Human *Shh* | SEQ ID No. 6 | SEQ ID No. 13 |
| Human *Ihh* | SEQ ID No. 7 | SEQ ID No. 14 |

The vertebrate *Hedgehog* protein (*hh*) of the present invention is defined by SEQ ID No:11 and can be cloned from mammalian sources. This protein is distinct from the *Drosophila* protein referred to in the literature as a hedgehog protein which, for clarity, will be referred to hereinafter as "Dros-HH". In addition to the sequence variation between the various *hh* homologs, the vertebrate *hedgehog* proteins are apparently present naturally in a number of different forms, including a pro-form, a full-length mature form, and several processed fragments thereof. The pro-form includes an N-terminal signal peptide for directed secretion of the extracellular domain, while the full-length mature form lacks this signal sequence. Further processing of the mature form apparently occurs in some instances to yield biologically active fragments of the protein. For instance, *sonic hedgehog* undergoes additional proteolytic processing to yield two peptides of approximately 19 kDa and 27 kDa, both of which are secreted. In addition to proteolytic fragmentation, the vertebrate *hedgehog* proteins can also be modified post-translationally, such as by glycosylation, through bacterially produce (e.g. unglycosylated) forms of the proteins apparently still maintain at least some of the activity of the native protein.

As described in the following examples, the cDNA clones provided by the present invention were first obtained by screening a mouse genomic library with a partial Drosophila *hh* cDMA clone (.7kb). Positive plaques were identified and one mouse clone was selected. This clone was then used as a probe to obtain a genomic clone containing the full coding sequence of the Mouse *Dhh* gene. As described in the attached Examples, Northern blots and *in situ* hybridization demonstrated that Mouse *Dhh* is expressed in the testes, and potentially the ovaries, and is also associated with sensory neurons of the head and trunk. *Dhh* is clearly a secreted factor expressed by Sertoli cells in the male testes, which is required for maintenance of the male germ line as probably a mitotic and survival factor. *Dhh* mutants are male sterile. Furthermore, *Dhh* is expressed as one of the first signs of differentiation of _ the gonad, thus *Dhh* may be a target of the sex determining gene, Sry. Interestingly, no expression was detected on the nerve cell bodies themselves (only the axons), indicating that *Dhh* is likely produced by the Shwann cells.

In order to obtain cDNA clones encoding chicken *hh* genes, degenerate oligonucleotides were designed corresponding to the amino and carboxy ends of Drosophila *hh* exon 2. As described in the Examples below, these oligonucleotides were used to isolate PCR fragments from chicken genomic DNA. These fragments were then cloned and sequenced. Ten clones yielded two different *hh* homologs, chicken *Dhh* and chicken *Shh.* The chicken *Shh* clone was then used to screen a stage 21/22 limb bud cDNA library which yielded a full length *Shh* clone.

In order to identify other vertebrate *hedgehog* homologs, the chicken clones (*Dhh* and *Shh*) were used to probe a genomic southern blot containing chicken DNA. As described below, genomic DNA was cut with various enzymes which do not cleave within the probe sequences. The DNA was run on a gel and transferred to a nylon filter. Probes were derived by ligating each 220 bp clone into a concatomer and then labeling with a random primer kit. The blots were hybridized and washed at low stringency. In each case, three hybridizing bands were observed following autoradiography, one of which was significantly more intense (a different band with each probes), indicating that there are at least three vertebrate *hh* genes. Additional cDNA and genomic screens carried out have yielded clones of three *hh* homologs from chickens and mice (*Shh, Dhh* and *Ihh*), and four *hh* homologs from zebrafish (*Shh, Dhh, Ihh* and *Mhh*). Weaker hybridization signals suggested that the gene family may be even larger. Moreover, a number of weakly hybridizing genomic clones have been isolated. Subsequently, the same probes derived from chicken *hedgehog* homologs have been utilized to screen a human genomic library. PCR fragments derived from the human genomic library were then sequenced, and PCR probes derived from the human sequences were used to screen human fetal cDNA libraries. Full-length cDNA encoding human sonic *hedgehog* protein (*Shh*) and partial cDNA encoding human Indian *hedgehog* protein (*Ihh*) were isolated from the fetal library, and represent a source of recombinant human *hedgehog* proteins.

To order to determine the expression patterns of the various vertebrate *hh* homologs, *in situ* hybridizations were performed in developing embryos of chicken, mice and fish. As described in the Examples below, the resulting expression patterns of each *hh* homolog were similar across each species and revealed that *hh* genes are expressed in a number of important embryonic signaling centers. For example, *Shh* is expressed in Hensen's node, the notochord, the ventral floorplate of the developing neural tube, and the ZPA at the base of the limb buds. *Shh* is also expressed in differntiated motor neurons in the embryonic mouse (at 11.5 days post fertilization), therefore, *Shh* may play a role in later stages of motor neuron development, perhaps in proliferation, but more likely in survival of this cell population. *Ihh* is expressed in the embryonic yolksac and hindgut, and appear also to be involved in chondrogenesis; *Dhh* is expressed in the testes; and *Mhh* (only in zebrafish) is expressed in the notochord and in certain cranial nerves.

Furthermore, experimental evidence indicates that certain *hedgehog* proteins initiate expression of secondary signaling molecules, including *Bmp-2* (a TGF-β relative) in the mesoderm and *Fgf-4* in the ectoderm. The mesoderm requires ectodermally-derived competence factor(s), which include *Fgf-4*, to activate target gene expression in response to *hedgehog* signaling. The expression of, for example, Sonic and Fgf-4 is coordinately regulated by a positive feedback loop operating between the posterior mesoderm and the overlying AER, which is the ridge of pseudostratified epithelium extending antero-posteriorly along the distal margin of the bud. These data provide a basis for understanding the integration of growth and patterning in the developing limb which can have important implication in the treatment of bone disorders described in sweater detail herein.

To determine the role *hedgehog* proteins plays in inductive interactions between the endoderm and mesoderm, which are critical to gut morphogenesis, in situ hybridizations and recombinant retroviral injections were performed in developing chick embryos. The ventral mesoderm is induced to undergo gut-specific differentiation by the adjacent endoderm. As described in Examples below, at the earliest stages of chick gut formation *Shh* is expressed by the endoderm, and *BMP-4* (a TGF-β relative) is expressed in the adjacent visceral mesoderm. Ectopic expression of *Sonic* is sufficient to induce expression of BMP-4 in visceral mesoderm, suggesting that *Sonic* serves as an inductive signal from the endoderm to the mesoderm, Subsequent organ-specific endodermal differentiation depends on regional inductive signal from the visceral mesoderm. Hox genes are expressed in the undifferentiated chick hind gut mesoderm with boundaries corresponding to morphologic borders, suggesting a role in regulating gut morphogenesis.

Accordingly, certain aspects of the present invention relate to nucleic acids encoding vertebrate *hedgehog* proteins, the *hedgehog* proteins themselves, antibodies immunoreactive with *hh* proteins, and preparations of such compositions. Moreover, the present invention provides reagents for treating disorders involving, for example, aberrant expression of vertebrate *hedgehog* homologs. In addition, drug discovery assays are provided for identifying agents which can modulate the binding of vertebrate *hedgehog* homologues to *hedgehog*-binding moieties (such as *hedgehog* receptors, ligands, or other extracellular matrix components). Such agents can be useful therapeutically to after the growth and/or differentiation of a cell. Other aspects of the invention are described below or will be apparent to those skilled in the art in light of the present disclosure.

For convenience, certain terms employed in the specification, examples, and appended claims are collected here.

As used herein, the term "nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides.

As used herein, the term "genes" or "recombinant gene" refers to a nucleic acid comprising an open reading frame encoding one of the vertebrate *hh* polypeptides of the present invention, including both exon and (optionally) intron sequences. A "recombinant gene" refers to nucleic acid encoding a vertebrate *hh* polypeptide and comprising vertebrate *hh*-encoding exon sequences, though it may optionally include intron sequences which are either derived from a chromosomal vertebrate *hh* gene or from an unrelated chromosomal gene. Exemplary recombinant genes encoding vertebrate *hh* polypeptides are represented by SEQ ID No:1, SEQ ID No:2. SEQ ID No:3. SEQ ID No:4, SEQ ID No:5, SEQ ID No:6 or SEQ ID No:7. The term "intron" refers to a DNA sequence present in a given vertebrate *hh* gene which is not translated into protein and is generally found between exons.

As used herein, the term "transfection" means the introduction of a nucleic acid. e.g., an expression vector, into a recipient cell by nucleic acid-mediated gene transfer. "Transformation", as used herein, refers to a process in which a cell's genotype is chanted as a result of the cellular uptake of exogenous DNA or RNA, and, for example, the transformed cell expresses a recombinant form of a vertebrate *hh* polypeptide or, where anti-sense expression occurs from the transferred gene, the expression of a naturally-occurring form of the vertebrate *hh* proteins is disrupted.

As used herein, the term "vector" refers to a nucleic acid molecule capable of transporting another nucleic acid to which it has been linked. One type of preferred vector is an episome, i.e., a nucleic acid capable of extra-chromosomal replication. Preferred vectors are those capable of autonomous replication and/expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

"Transcriptional regulatory sequence" is a generic term used throughout the specification to refer to DNA sequences, such as initiation signals, enhancers, and promoters, which induce or control transcription of protein coding sequences with which they are operably linked. In preferred embodiments, transcription of one of the recombinant vertebrate *hedgehog* genes is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally-occurring forms of *hedgehog* proteins.

As used herein, the term "tissue-specific promoter" means a DNA sequence that serves as a promoter, i.e., regulates expression of a selected DNA sequence operably linked to the promoter, and which effects expression of the selected DNA sequence in specific cells of a tissue, such as cells of neural origin, e.g. neuronal cells. The term also covers so-called "leaky" promoters, which regulate expression of a selected DNA primarily in one tissue, but cause expression in other tissues as well.

As used herein, a "non-human transgenic animal" embraces a non-human mammal, bird or an amphibian, in which one or more of the cells of the non-human animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or *in vitro* fertilization, but rather is directed to the introduction of a recombinant DNA molecule. This molecule may be integrated within a chromosome, or it may be extrachromosomally replicating DNA. In the typical non-human transgenic animals described herein, the transgene causes cells to express a recombinant form of one of the vertebrate *hh* proteins, e.g. either agonistic or antagonistic forms. However, non-human transgenic animals in which the recombinant vertebrate *hh* gene is silent are also contemplated, as for example, the FLP or CRE recombinase dependent constructs described below. The "non-human animals" of the invention include vertebrates such as rodents, non-human primates, sheep, dog, cow, chickens, amphibians, reptiles, etc. Preferred non-human animals are selected from the rodent family including rat and mouse, most preferable mouse, though transgenic amphibians, such as members of the *Xenopus* genus, and transgenic chickens can also provide important tools for understanding and identifying agents which can affect, for example, embryogenesis and tissue formation. The term "chimeric animal" is used herein to refer to non-human animals in which the recombinant gene is found, or in which the recombinant is expressed in some but not all cells of the non-human animal. The term "tissue-specific chimeric animal" indicates that one of the recombinant vertebrate *hh* genes is present and/or expressed in some tissues but not others.

As used herein, the term "transgene" means a nucleic acid sequence (encoding, e.g., one of the vertebrate *hh* polypeptides), which is partly or entirely heterologous, i.e., foreign, to the transgenic non-human animal or cell into which it is introduced, or, is homologous to an endogenous gene of the transgenic non-human animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can include one or more transcriptional regulatory sequences and any other nucleic acid, such as introns, that may be necessary for optimal expression of a selected nucleic acid.

As is well known, genes for a particular polypeptide may exist in single or multiple copies within the genome of an individual. Such duplicate genes may be identical or may have certain modifications, including nucleotide substitutions, additions or deletions, which all still code for polypeptides having substantially the same activity. The term "DNA sequence encoding a vertebrate *hh* polypeptide" may thus refer to one or more genes within a particular individual. Moreover, certain differences in nucleotide sequences may exist between individual organisms, which are called alleles. Such allelic differences may or may not result in differences in amino acid sequence of the encoded polypeptide yet still encode a protein with the same biological activity.

"Homology" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40 percent identity, though preferably less than 25 percent identity, with one of the vertebrate *hh* sequences of the present invention.

"Cells," "host cells" or "recombinant host cells" are terms used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A "chimeric protein" or "fusion protein" is a fusion of a first amino acid sequence encoding one of the subject vertebrate *hh* polypeptides with a second amino acid sequence defining a domain foreign to and not substantially homologous with any domain of one of the vertebrate *hh* proteins. A chimeric protein may present a foreign domain which is found (albeit in a different protein) in an organism which also expresses the first protein, or it may be an "interspecies", "intergenic", etc. fusion of protein structures expressed by different kinds of organisms. In general, a fusion protein can be represented by the general formula X-*hh-*Y, wherein *hh* represents a portion of the protein which is derived from one of the vertebrate *hh* proteins, and X and Y are independently absent or represent amino acid sequences which are not related to one of the vertebrate *hh* sequences in an organism, including naturally occurring mutants.

As used herein, the terms "transforming growth factor-beta" and "TGF-β" denote a family of structurally related paracrine polypeptides found ubiquitously in vertebrates, and prototypic of a large family of metazoan growth, differentiation, and morphogenesis factors (see, for review, Massaque et al. (1990) Ann Rev Cell Biol 6:597-641; and Sporn et al. (1992) J Cell Biol 119:1017-1021). Included in this family are the "bone morphogenetic proteins" or "BMPs" which refers to proteins isolated from bone, and fragments thereof and synthetic peptides which are capable of inducing bone deposition alone or when combined with appropriate cofactors. Preparation of BMPs, such as BMP-1, -2, -3, and -4, is described in, for example, PCT publication WO 88/00205. Womey (1989) Growth Fact Res 1:267-280 describes additional BMP proteins closely related to BMP-2, and which have been designated BMP-5, -6, and -7. PCT publications WO89/09787 and WO89/09788 describe a protein called "OP-1," now known to be BMP-7. Other BMPs are known in the art.

The term "isolated" as also used herein with respect to nucleic acids, such as DNA or RNA, refers to molecules separated from other DNAs, or RNAs, respectively, that are present in the natural source of the macromolecule. For example, an isolated nucleic acid encoding one of the subject vertebrate *hh* polypeptides preferably includes no more than 10 kilobases (kb) of nucleic acid sequence which naturally immediately flanks the vertebrate *hh* gene in genomic DNA, more preferably no more than 5kb of such naturally occurring flanking sequences, and most preferably less than 1.5kb of such naturally occurring flanking sequence. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state.

As described below, one aspect of the invention pertains to isolated nucleic acids comprising the nucleotide sequences encoding vertebrate *hh* homologues and/or equivalents of such nucleic acids. The term nucleic acid as used herein is intended to include fragments as equivalents. The term equivalent is understood to include nucleotides sequences encoding functionally equivalent *hh* polypeptides or functionally equivalent peptides having an activity of a vertebrate *hh* protein such as described herein. Equivalent nucleotide sequences will include sequences that differ by one or more nucleotide substitutions, additions or deletions, such as allelic variants; and will therefore, include sequences that differ from the nucleotide sequence of the vertebrate *hh* cDNAs shown in SEQ ID No:4 due to the degeneracy of the genetic code. Equivalents will also include nucleotide sequences that hybridize under stringent conditions (i.e., equivalent to about 20-27°C below the melting temperature (Tₘ) of the DNA duplex formed in about 1M salt) to the nucleotide sequences represented in SEQ ID Nos:1-7. In one embodiment, equivalents will further include nucleic acid sequences derived from and evolutionarily related to, a nucleotide sequences shown in any of SEQ ID No:4.

Moreover, it will be generally appreciated that, under certain circumstances, it may be advantageous to provide homologs of one of the subject *hedgehog* polypeptides which function in a limited capacity as one of cither an *hh* agonist or an *hh* antagonist, in order to promote or inhibit only a subset of the biological activities of the naturally-occurring form of the protein. Thus, specific biological affects can be elicited by treatment with a homolog of limited function, and with fewer side effect relative to treatment with agonists or antagonists which are directed to all of the biological activities of naturally occurring forms of *hedgehog* proteins.

Homologs of one of the subject *hedgehog* proteins can be generated by mutagenesis, such as by discrete point mutation(s), or by truncation. For instance, mutation can give rise to homolog which retain substantially the same, or merely a subset, of the biological activity of the *hh* polypeptide from which it was derived. Alternatively, antagonistic forms of the protein can be generated which are able to inhibit the function of the naturally occurring form of the protein, such as by competitively binding to an *hh* receptor.

Polypeptides referred to herein as having an activity of a vertebrate *hh* protein are defined as peptides that have an amino acid sequence corresponding to all or a portion of the amino acid sequences of a vertebrate *hh* protein shown in SEQ ID No:11 and which have at least one biological activity of a vertebrate *hh* protein. Examples of such biological activity of a vertebrate *hh* protein include the ability co induce (or otherwise modulate) formation and differentiation of the head, limbs, lungs, central nervous system (CNS), or mesodermal patterning of developing vertebrate embryos, In preferred embodiments" the biological activity can comprise an ability to regulate neurogenesis, such as a motor neuron inducing activity, a neuronal differentiation inducing activity, or a neuronal survival promoting activity. *Hedgehog* proteins of the present invention can also have biological activities which include an ability to regulate organogensis, such as through the ability to influence limb patterning, by, for example, skeletogenic activity. The biological activity associated with the *hedgehog* proteins of the present invention can also include the ability to induce stem cell (not human embryonic stem cells) or germ cell differentiation, including the ability to induce differentiation of chondrocytes or an involvement in spermatogenesis. *Hedgehog* proteins of the present invention can also be characterized in terms of biological activities which include: an ability to modulate proliferation, survival and/or differentiation of mesodermally-derived tissue, such as tissue derived from dorsal mesoderm the ability to modulate proliferation, survival and/or differentiation of ectodermally-derived tissue, such as tissue derived from the neural tube, neural crest, or head mesenchyme. Moreover, as described in the Examples below, the subject *hedgehog* proteins have the ability to induce expression of secondary signaling molecules, such as members of the Transforming Growth Factor β (TGFβ) family, including bone morphogenic proteins, e.g. *BMP-2* and *BMP-4,* as wall as members of the fibroblast growth factor (FGF) family, such as *Fgf-4.* Other biological activities of the subject *hedgehog* proteins are described herein or will be reasonably apparent to those skilled in the art. According to the present invention, a polypeptide has biological activity if it is a specific agonist or antagonist of a naturally-occurring form of a vertebrate *hedgehog* protein.

Another aspect of the invention provides a nucleic acid which hybridizes under high or low suringency conditions to a nucleic acid represented by one of SEQ ID No:4. Appropriate stringency conditions which promote DNA hybridization, for example sodium chloride/sodium citrate (SSC) at about 45°C, followed by a wash of 0.2 x SSC at 65°C, are known to those skilled in the art or can be found in Current Protocols in Molecular Biology, John Wiley & Sons. N.Y. (1989), 6.3.1-6.3.6.

Nucleic acids, having a sequence that differs from the nucleotide sequence shown in SEQ ID No:4, due to degeneracy in the genetic code are also within the scope of the invention. Such nucleic acids encode functionally equivalent peptides (i.e., a peptide having a biological activity of a vertebrate *hh* polypeptide) but differ in sequence from the sequence shown in the sequences listing due to degeneracy in the genetic code. For example, a number of amino acids are designated by more than one triplet. Codons that specify the same amino acid, or synonyms (for example, CAU and CAC each encode histidine) may result in "silent" mutations which do not affect the amino acid sequence of a vertebrates *hh* polypeptide. However, it is expected that DNA sequence polymorphisms that do lead to changes in the amino acid sequences of the subject *hh* polypeptides will exist among vertebrates. One skilled in the art will appreciate that these variations in one or more nucleotides (up to about 3-5% of the nucleotides) of the nucleic acids encoding polypeptides having an activity of a vertebrate *hh* polypeptide may exist among individuals of a given species due to natural allelic variation.

Fragments of the nucleic acids encoding an active portion of the vertebrate *hedgehog* proteins are also within the scope of the invention. As used herein, a *hedgehog* gene fragment refers to a nucleic acid having fewer nucleotides than the nucleotide sequence encoding the entire amino acid sequence of a vertebrate *hh* protein represented in SEQ ID No:11, yet which (preferably) encodes a peptide which retains some biological activity of the full length protein, e.g. the fragments retains the ability to induce formation and differentiation of the head, limbs, lungs, central nervous system (CNS), or mesodermal patterning of developing vertebrate embryo. Nucleic acids within the scope of the invention may also contain linker sequences, modified restriction endonuclease sites and other sequences useful for molecular cloning, expression or purification of recombinant forms of the subject *hh* polypeptides.

As indicated by the examples set out below, *hedgehog* protein-encoding nucleic acids can be obtained from mRNA present in any of a number of eukaryotic cells. It should also be possible to obtain nucleic acids encoding vertebrate *hh* polypeptides of the present invention from genomic DNA obtained from both adults and embryos. For example, a gene encoding a *hh* protein can be cloned from either a cDNA or a genomic library in accordance with protocols described herein, as well as those generally known to persons skilled in the art A cDNA encoding a *hedgehog* protein can be obtained by isolating total mRNA from a cell, e.g. a mammalian cell, e.g. a human cell, including embryonic cells. Double stranded cDNAs can then be prepared from the total mRNA, and subsequently inserted into a suitable: plasmid or bacteriophage vector using any one of a number of known techniques. The gene encoding a vertebrate *hh* protein can also be cloned using established polymerase chain reaction techniques in accordance with the nucleotide sequence information provided by the invention. The nucleic acid of the invention can be DNA or RNA. A preferred nucleic acid is a cDNA represented by a sequence selected from the group consisting ofSEQ ID Nos:1-7.

This invention also provides expression vectors containing a nucleic acid encoding a vertebrate *hh* polypeptide, operably linked to at least one transcriptional regulatory sequence. Operably linked is intended to mean that the nucleotide sequence is linked to a regulatory sequence in a manner which allows expression of the nucleotide sequence. Regulatory sequences are art-recognized and are selected to direct expression of the subject vertebrate *hh* proteins. Accordingly, the term transcriptional regulatory sequence includes promoters, enhancers and other expression control elements. Such regulatory sequences are described in Goeddel; Gene Expression Technology: Methods in Enzymology 185, Academic Press, San Diego, CA (1990). For instance, any of a wide variety of expression control sequences, sequences that control the expression of a DNA sequence when operatively linked to it, may be used in these vectors to express DNA sequences encoding vertebrate *hh* polypeptides of this invention. Such useful expression control sequences, include, for example, a viral LTR, such as the LTR of the Moloney murine leukemia virus, the early and late promoters of SV40, adenovirus or cytomegalovirus immediate early promoter, the lac system, the trp system, the TAC or TRC system, T7 promoter whose expression is directed by T7 RNA polymerase, the major operator and promoter regions of phage λ, the control regions for fd coat protein, the promoter for 3-phosphoglycerate kinase or other glycolytic enzymes, the promoters of acid phosphatase, e.g., Pho5, the promoters of the yeast α-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other proteins encoded by the vector, such as antibiotic markers, should also be considered. In one embodiment, the expression vector includes a recombinant gene encoding a peptide having an agonistic activity of a subject *hedgehog* polypeptide, or alternatively, encoding a peptide which is an antagonistic form of the *hh* protein. Such expression vectors can be used to transfect cells and thereby produce polypeptides, including fusion proteins, encoded by nucleic acids as described herein.

Moreover, the gene constructs of the present invention can also be used as a part of a gene therapy protocol to deliver nucleic acids encoding either an agonistic or antagonistic form of one of the subject vertebrate *hedgehog* proteins. Thus, another aspect of the invention features expression vectors for *in vivo* or *in vitro* transfection and expression of a vertebrate *hh* polypeptide in particular cell types so as to reconstitute the function of, or alternatively, abrogate the function of *hedgehog-*induced signaling in a tissue in which the naturally-occurring form of the protein is misexpressed; or to deliver a form of the protein which alters differentiation of tissue, or which inhibits neoplastic transformation.

Expression constructs of the subject vertebrate *hh* polypeptide, and mutants thereof, may be administered in any biologically effective carrier, e.g. any formulation or composition capable of effectively delivering the recombinant gene to cells *in vivo*. Approaches include insertion of the subject gene in viral vectors including recombinant retroviruses, adenovirus, adeno-associated virus, and herpes simplex virus-1, or recombinant bacterial or eukaryotic plasmids. Viral vectors transfect cells directly; plasmid. DNA can be delivered with the help of, for example, cationic liposomes (lipofectin) or derivatized (e.g. antibody conjugated), polylysine conjugates, gramacidin S, artificial viral envelopes or other such intracellular carriers, as well as direct injection of the gene construct or CaPO₄ precipitation carried out *in vivo.* It will be appreciated that because transduction of appropriate target cells represents the critical first step in gene therapy, choice of the particular gene delivery system will depend on such factors as the phenotype of the intended target and the route of administration, e.g. locally or systemically. Furthermore, it will be recognized that the particular gene construct provided for *in vivo* transduction of *hedgehog* expression are also useful for *in vitro* transduction of cells, such as for use in the *ex vivo* tissue culture systems described below.

A preferred approach for *in vivo* introduction of nucleic acid into a cell is by use of a viral vector containing nucleic acid, e.g. a cDNA, encoding the particular form of the *hedgehog* polypeptide desired. Infection of cells with a viral vector has the advantage that a large proportion of the targeted cells can receive the nucleic acid. Additionally, molecules encoded within the viral vector, e.g., by a cDNA contained in the viral vector, are expressed efficiently in cells which have taken up viral vector nucleic acid.

Retrovirus vectors and adeno-associated virus vectors are generally understood to be the recombinant gene delivery system of choice for the transfer of exogenous genes *in vivo,* particularly into humans. These vectors provide efficient delivery of genes into cells, and the transferred nucleic acids are stably integrated into the chromosomal DNA of the host. A major prerequisite for the use of retroviruses is to ensure the safety of their use, particularly with regard to the possibility of the spread of wild-type virus in the cell population. The development of specialized cell lines (termed "packaging cells") which produce only replication-defective retroviruses has increased the utility of retroviruses for gene therapy, and defective retroviruses are well characterized for use in gene transfer for gene therapy purposes (for a review see Miller, A.D. (1990) Blood 76:271).. Thus, recombinant retrovirus can be constructed in which part of the retroviral coding sequence (*gag, pol, env*) has been replaced by nucleic acid encoding one of the subject proteins rendering the retrovirus replication defective. The replication defective retrovirus is then packaged into virions which can be used to infect a target cell through the use of a helper virus by standard techniques. Protocols for producing recombinant retroviruses and for infecting cells *in vitro* or *in vivo* with such viruses can be found in Current Protocols in Molecular Biology, Ausubel, F.M. et al. (eds.) Greene Publishing Associates, (1989), Sections 9.10-9.14 and other standard laboratory manuals. Examples of suitable retroviruses include pLJ, pZIP, pWE and pEM which are well known to those skilled in the art. Examples of suitable packaging virus lines for preparing both ecotropic and amphotropic retroviral systems include ψCrip, ψCre, ψ2 and ψAm. Retroviruses have been used to introduce a variety of genes into many different cell types, including neuronal cells, *in vitro* and/or *in vivo* (see for example Eglitis, et al. (1985) Science 230:1395-1398; Danos and Mulligan (1988) Proc. Natl. Acad. Sci. USA 85:6460-6464; Wilson et al. (1988) Proc. Natl. Acad Sci. USA 85:3014-3018; Armentano et al. (1990) Proc. Natl. Acad Sci. USA 87:6141-6145; Huber et al. (1991) Proc. Natl. Acad. Sci. USA 88:8039-8043; Ferry et al. (1991) Proc. Natl. Acad Sci. USA 88:8377-8381; Chowdhury ct al. (1991) Science 254:1802-1805; van Beusechem et al. (1992) Proc. Natl. Acad. Sci. USA 89:7640-7644; Kay et al. (1992) Human Gene Therapy 3:641-647; Dai et al. (1992) Proc. Natl. Acad Sci. USA 89:10892-10895; Hwu et al. (1993) J. Immunol. 150:4104-4.115; U.S. Patent No. 4,868,116; U.S. Patent No. 4,980,286; PCT Application WO 89/07136; PCT Application WO 89/02468; PCT Application WO 89/05345; and PCT Application WO 92/07573).

Furthermore, it has been shown that it is possible to limit the infection spectrum of retroviruses and consequently of retroviral-based vectors, by modifying the viral packaging proteins on the surface of the viral particle (see, for example PCT publications WO93/25234 and WO94/06920). For instance, strategies for the modification of the infection spectrum of retroviral vectors include: coupling antibodies specific for cell surface antigens to the viral *env* protein (Roux et al. (1989) PNAS 86:9079.9083; Julan et al. (1992) J. Gen Virol 73:3251-3255; and Goud et al. (1983) Virology 163:251-254); or coupling cell surface receptor ligands to the viral *env* proteins (Neda et al. (1991) J Biol Chem 266:14143-14146). Coupling can be in the form of the chemical cross-linking with a protein or other variety (e.g. lactose to convert the env protein to an asialoglycoprotein), as well as by generating fusion proteins (e.g. single-chain antibody/env fusion proteins). This technique, while useful to limit or otherwise direct the infection to certain tissue types, can also be used to convert an ecotropic vector in to an amphotropic vector.

Moreover, use of retroviral gene delivery can be further enhanced by the use of tissue- or cell-specific transcriptional regulatory sequences which control expression of the *hh* gene of the retroviral vector.

Another viral gene delivery system useful in the present invention utilizes adenovirus-derived vectors. The genome of an adenovirus can be manipulated such that it encodes and expresses a gene product of interest but is inactivated in terms of its ability to replicate in a normal lytic viral life cycle. See for example Berkner et al. (1988) BioTechniques 6:616; Rosenfeld et al. (1991) Science 252:431-434; and Rosenfeld et al. (1992) Cell 68:143-155. Suitable adenoviral vectors derived from the adenovirus strain Ad type 5 dl324 or other strains of adenovirus (e.g., Ad2, Ad3, Ad7 etc.) are well known to those skilled in the art. Recombinant adenoviruses can be advantageous in certain circumstances in that they can be used to infect a wide variety of cell types, including airway epithelium (Rosenfeld et al. (1992) cited *supra*), endothelial cells (Lemarchand et al. (1992) Proc. Natl. Acad Sci. USA 89:6482-6486), hepatocytes (Herz and Gerard (1993) Proc. Natl. Acad Sci. USA 90:2812-2816) and muscle cells (Quantin et al. (1992) Proc. Natl. Acad Sci. USA 89:2581-2584). Furthermore, the virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (e.g., retroviral DNA). Moreover, the carrying capacity of the adenoviral genome for foreign DNA is large (up to 8 kilobases) relative to other gene delivery vectors (Berkner et al. cited *supra*; Haj-Ahmand and Graham (1986) J. Virol. 57:267). Most replication-defective adenoviral vectors currently in use and therefore favored by the present invention are deleted for all or parts of the viral E1 and E3 genes but retain as much as 80% of the adenoviral genetic material (see, e.g., Jones et al. (1979) Cell 16:683; Berkner et al., *supra*; and Graham et al. in Methods in Molecular Biology, E.J. Murray, Ed. (Humana, Clifton, NJ, 1991) vol. 7. pp. 109-127). Expression of the inserted *hedgehog* gene can be under control of, for example, the E1A promoter, the major late promoter (MLP) and associated leader sequences, the E3 promoter, or exogenously added promoter sequences.

Yet another viral vector system useful for delivery of one of the subject vertebrate *hh* genes is the adeno-associated virus (AAV). Adeno-associated virus is a naturally occurring defective virus that requires another virus, such as an adenovirus or a herpes virus, as a helper virus for efficient replication and a productive life cycle. (For a review see Muzyczka et al. Curr. Topics in Micro. and Immunol. (1992) 158:97-129). It is also one of the few viruses that may integrate its DNA into non-dividing cells, and exhibits a high frequency of stable integration (see for example Flotte et al. (1992) Am. J. Respir. Cell. Mol. Biol. 7:349-356; Samulski et al. (1989) J. Virol. 63:3822-3828; and McLaughlin et al. (1989) J. Virol. 62:1963-1973). Vectors containing as little as 300 base pairs of AAV can be packaged and can integrate. Space for exogenous DNA is limited to about 4.5 kb. An AAV vector such as that described in Tratschin et al. (1985) Mol. Cell. Biol. 5:3251-3260 can be used to introduce DNA into cells. A variety of nucleic acids have been introduced into different cell types using AAV vectors (see for example Hermonat et al. (1984) Proc. Natl. Acad Sci. USA 81:6466-6470; Tratschin et al. (1985) Mol. Cell. Biol. 4:2072-2081; Wondisford et al. (1988) Mol. Endocrinol. 2:32-39; Tratschin et al. (1984) J. Virol. 51:611-619. and Flotte et al. (1993) J. Biol. Chem. 268:3781-3790).

In addition to viral transfer methods, such as those illustrated above, non-viral methods can also be employed to cause expression of a subject *hedgehog* polypeptide in the tissue of a non-human animal. Most nonviral methods of gene transfer rely on normal mechanism used by mammalian cells for the uptake and intracellular transport of macromolecules. In prefered embodiments, non-viral gene delivery systems of the present invention rely on endocytic pathways for the uptake of the subject *hh* polypeptide gene by the targeted cell Exemplary gene delivery systems of this type include liposomal derived systems, poly-lysine conjugates, and artificial viral envelopes.

In clinical settings, the gene delivery systems for the therapeutic *hedgehog* gene can be introduced into a patient by any of a number of methods, each of which is familiar in the art. For instance, a pharmaceutical preparation of the gene delivery system can be introduced systemically, e.g. by intravenous injection, and specific transduction of the protein in the target cells occurs predominantly from specificity of transfection provided by the gene delivery vehicle, cell-type or tissue-type expression due to the transcriptional regulatory sequences controlling expression of the receptor gene, or a combination thereof. In other embodiments, initial delivery of the recombinant gene is more limited with introduction into the non-human animal being quite localized. For example, the gene delivery vehicle can be introduced by catheter (see U.S. Patent 5.328.470) or by stereotactic injection (e.g. Chen et al. (1994) PNAS 91: 3054-3057). A vertebrale *hh* gene, such as any one of the clones represented In the group consisting of SEQ ID NO:1.7, can be delivered in a gene therapy construct by electroporation using technique described, for example, by Dev et al. ((1994) Cancer Treat Rev 20:105-115).

The pharmaceutical preparation of the gene therapy construct can consist essentially of the gene delivery system in an acceptable diluent, or can comprise a slow release matrix in which the gene delivery vehicle is imbedded. Alternatively, where the complete gene delivery system can be produced intact from recombinant cells, e.g. retroviral vectors, the pharmaceutical preparation can comprise one or more cells which produce the gene delivery system.

Another aspect of the present invention concerns recombinant forms of the *hedgehog* proteins. Recombinant polypeptides preferred by the present invention, possess an activity of a *hedgehog* protein (i.e. either agonistic or antagonistic), and which are at least 90%, more preferably at least 95%, and most preferably at least about 98-99% homologous with a sequence selected from the group consisting of SEQ ID No:11 are within the scope of the invention.

The term "recombinant proteins" refers to a polypeptide of the present invention which is produced by recombinant DNA techniques, wherein generally. DNA encoding a vertebrate *hh* polypeptide is inserted into a suitable expression vector, which is in turn used to transform a host cell to produce the heterologous protein. Moreover, the phrase "derived from", with respect to a recombinant *hedgehog* gene, is meant to include within the meaning of "recombinant protein" those proteins having an amino acid sequence of a native *hedgehog* protein or an amino acid sequence similar thereto which is generated by mutations including substitutions and deletions (including truncation) of a naturally occuring form of the protein.

The present invention further pertains to recombinant forms of one of the subject *hedgehog* polypeptides which are encoded by genes derived from a vertebrate organism, particularly a mammal (e.g. a human), and which have amino acid sequences evolutionarily related to the *hedgehog* proteins represented in SEQ ID No:11. Such recombinant *hh* polypeptides preferably are capable of functioning in one of either role of an agonist or antagonist of at least one biological activity of a wild-type ("authentic") *hedgehog* protein of the appended sequence listing. The present invention further pertains to methods of producing the subject *hedgehog* polypeptides. For examples, a host cell transfected with a nucleic acid vector directing expression of a nucleotide sequence encoding the subject polypeptides can be cultured under appropriate conditions to allow expression of the peptide to occur. The polypeptide *hedgehog* may be secreted and isolated from a mixture of cells and medium containing the recombinant vertebrate *hh* polypeptide. Alternatively, the peptide may be retained cytoplasmically by removing the signal peptide sequence from the recombinant *hh* gene and the ceUs harvested, lysed and the protein isolated. A cell culture includes host cells, media and other by products. Suitable media for cell culture are well known in the art. The recombinant *hh* polypeptide can be isolated from cell culture medium, host cells, or both using techniques known in the art for purifying proteins including ion-exchange chromatography, gel filtration chromatography, ultrafiltration, electrophoresis, and immunoaffinity purification with antibodies specific for such peptide. In a preferred embodiment, the recombinant *hh* polypeptide is a fusion protein containing a domain which facilitates its purification, such as an *hh*/GST fusion protein.

This invention also pertains to a host cell transfected to express a recombinant form of the subject *hedgehog* polypeptides. The host cell may be any prokaryotic or eukaryotic cell. Thus, a nucleotide sequence derived from the cloning of vertebrate *hedgehog* proteins, encoding all or a selected portion of the full-length protein, can be used to produce a recombinant form of a vertebrate *hh* polypeptide via microbial or eukaryotic cellular processes. Ligating the polynucleotide sequence into a gene construct, such as an expression victor, and transforming or transfecting into hosts, either eukaryotic (yeast, avian, insect or mammalian) or prokaryotic (bacterial cells), are standard procedures used in producing other well-known proteins, e.g. insulin, interferons, human growth hormone. IL-1, IL-2, and the like. Similar procedures, or modifications thereof, can be employed to prepare recombinant *hedgehog* polypeptides by microbial means or tissue-culture technology in accord with the subject invention.

The recombinant *hedgehog* genes can be produced by ligating nucleic acid encoding an *hh* protein, or a portion thereof into a vector suitable for expression in either prokaryotic cells, eukaryotic cells, or both. Expression vectors for production of recombinant forms of the subject *hh* polypeptides include plasmids and other vector For instance, suitable vectors for the expression of a *hedgehog* polypeptide include plasmids of the types: pBR322-derived plasmids, pEMBL-derived plasmids, pEX-derived plasmids, pBTac-derived plasmids and pUC-derived plasmids for expression in prokaryotic cells, such as *E. coli.*

A number of vectors exist for the expression of recombinant proteins in yeast. For instance, YEP24, YIPS, YEP51, YEP52, pYES2. and YRP17 are cloning and expression vehicles useful in the introduction of genetic constructs into *S. cerevisiae* (see, for example, Broach et al. (1983) in Experimental Manipulation of Gene Expression, ed. M. Inouye Academic Press. p. 83). These vectors can replicate in *E. coli* due the presence of the pBR322 ori, and in *S. cerevisiae* due to the replication determinant of the yeast 2 micron plasmid. In addition, drug resistance markers such as ampicillin can be used. In an illustrative embodiment, an *hh* polypeptide is produced recombinantly utilizing an expression vector generated by sub-cloning the coding sequence of one of the *hedgehog* genes represented in SEQ ID NO:4.

The preferred mammalian expression vectors contain both prokaryotic sequences, to facilitate the propagation of the vector in bacteria, and one or more eukaryotic transcription units that are expressed in eukaryotic cells. The pcDNAI/amp, pcDNAI/nco, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papillomavirus (BPV-1), or Epstein-Barr virus (pHEBo, pREP-derived and p205) can be used for transient expression of proteins in eukaryotic cells. The various methods employed in the preparation of the plasmids and transformation of host organisms are well known in the art. For other suitable expression systems for both prokaryotic and eukaryotic cells, as well as general recombinant procedures, see Molecular Cloning A Laboratory Manual, 2nd Ed., ed. by Sambrook, Fritsch and Maniatis (Cold Spring Harbor Laboratory Press: 1989) Chapters 16 and 17.

In some instances, it may be desirable to express the recombinant *hedgehog* polypeptide by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the β-gal containing pBlueBac III).

When it is desirable to express only a portion of an *hh* protein, such as a form lacking a portion of the N-terminus, i.e. a truncation mutant which lacks the signal peptide, it may be necessary to add a start codon (ATG) to the oligonucleotide fragment containing the desired sequence to be expressed. It is well known in the art that a methionine at the N-terminal position can be enzymatically cleaved by the use of the enzyme methionine aminopeptidase (MAP). MAP has been cloned from *E. coli* (Ben-Bassat et al. (1987) J. Bacteriol. 169:751-757) and *Salmonella typhimurium* and its *in vitro* activity has been demonstrated on recombinant proteins (Miller et al. (1987) PNAS 84:2718-1722). Therefore, removal of an N-terminal methionine, if desired, can be achieved either *in vivo* by expressing *hedgehog-*derived polypeptides in a host which produces MAP (e.g., *E. coli* or CM89 or *S. cerevisiae*), or *in vitro* by use of purified MAP (e.g., procedure of Miller et al., *supra*).

Alternatively, the coding sequences for the polypeptide can be incorporated as a part of a fusion gene including a nucleotide sequence encoding a different polypeptide. This type of expression system can be useful under conditions where it is desirable to produce an immunogenic fragment of a *hedgehog* protein. For example, the VP6 capsid protein of rotavirus can be used as an immunologic carrier protein for portions of the *hh* polypeptide, either in the monomeric form or in the form of a viral particle. The nucleic acid sequences corresponding to the portion of a subject *hedgehog* protein to which antibodies are to be raised can be incorporated into a fusion gene construct which includes coding sequences for a late vaccinia virus structural protein to produce a set of recombinant viruses expressing fusion proteins comprising *hh* epitopes as part of the virion. It has been demonstrated with the use of immunogenic fusion proteins utilizing the Hepatitis B surface antigen fusion proteins that recombinant Hepatitis B virions can be utilized in this role as well. Similarly, chimeric constructs coding for fusion proteins containing a portion of an *hh* protein and the poliovirus capsid protein can be created to enhance immunogenicity of the set of polypeptide antigens (see, for example, EP Publication No: 0259149; and Evans et al. (1989) Nature 339:385; Huang et al. (1988) J. Virol. 62:3855; and Schlienger et al. (1992) J. Virol. 66:2).

The Multiple Antigen Peptide system for peptide-based immunization can also be utilized to generate an immunogen, wherein a desired portion of an *hh* polypeptide is obtained directly from organo-chemical synthesis of the peptide onto an oligomeric branching lysine core (see, for example, Posnett et al. (1988) JBC 263:1719 and Nardelli et al. (1992) J. Immunol. 148:914). Antigenic determinants of *hh* proteins can also be expressed and presented by bacterial cells.

In addition to utilizing fusion proteins to enhance immunogenicity, it is widely appreciated that fusion proteins can also facilitate the expression of proteins, and accordingly, can be used in the expression of the vertebrate *hh* polypeptides of the present invention. For example, *hedgehog* polypeptides can be generated as glutathione-S-transferase (GST-fusion) proteins. Such GST-fusion proteins can enable easy purification of the *hedgehog* polypeptide, as for example by the use of glutathione-derivatized matrices (see, for example, Current Protocols in Molecular Biology, eds. Ausubel et al. (N.Y.: John Wiley & Sons, 1991)). In another embodiment, a fusion gene coding for a purification leader sequence, such as a poly-(His)/enterokinase cleavage site sequence, can be used to replace the signal sequence which naturally occurs at the N-terminus of the *hh* protein (e.g., of the pro-form, in order to permit purification of the poly(His)-*hh* protein by affinity chromatography using a Ni²⁺ metal resin. The purification leader sequence can then be subsequently removed by treatment with enterokinase (e.g., see Hochuli et al. (1987) J. Chromatography 411:177; and Janknecht et al. PNAS 88:8972).

Techniques for making fusion genes are known to those skilled in the art. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence (see, for example, Current Protocols in Molecular Biology, eds. Ausubel et al. John Wiley & Sons: 1992).

*Hedgehog* polypeptides may also be chemically modified to create *hh* derivatives by forming covalent or aggregate conjugates with other chemical moieties, such as glycosyl groups, lipids, phosphate, acetyl groups and the like. Covalent derivatives of *hedgehog* proteins can be prepared by linking the chemical moieties to functional groups on amino acid sidechains of the protein or at the N-terminus or at the C-terminus of the polypeptide.

For instance, *hedgehog* proteins can be generated to include a moiety, other than sequence naturally associated with the protein, that binds a component of the extracellular matrix and enhances localization of the analog to cell surfaces. For example, sequences derived from the fibronectin "type-III repeat", such as a tetrapeptide sequence R-G-D-S (Pierschbacher et al. (1984) Nature 309:30-3; and Kornblihtt et al. (1985) EMBO 4:1755-9) can be added to the *hh* polypeptide to support attachment of the chimeric molecule to a cell through binding ECM components (Ruoslahti et al. (1987) Science 238:491-497; Pierschbacheret al. (1987) J. Biol. Chem. 262:17294-8.; Hynes (1987) Cell 48:549-54; and Hynes (1992) Cell 69:11-25).

The present invention also makes available isolated *hedgehog* polypeptides which are isolated from, or otherwise substantially free of other cellular and extracellular proteins, especially morphogenic proteins or other extracellular or cell surface associated proteins which may normally be associated with the *hedgehog* polypeptide. The term "substantially free of other cellular or extracellular proteins" (also referred to herein as "contaminating proteins") or "substantially pure or purified preparations" are defined as encompassing preparations of *hh* polypeptides having less than 20% (by dry weight) contaminating protein, and preferably having less than 5% contaminating protein. Functional forms of the subject polypeptides can be prepared, for the first time, as purified preparations by using a cloned gene as described herein. By "purified", it is meant, when referring to a peptide or DNA or RNA sequence, that the indicated molecule is present in the substantial absence of other biological macromolecules, such as other proteins. The term "purified" as used herein preferably means at least 80% by dry weight, more preferably in the range of 95-99% by weight, and most preferably at least 99.8% by weight, of biological macromolecules of the same type present (but water, buffers, and other small molecules, especially molecules having a molecular weight of less than 5000, can be present). The term "pure" as used herein preferably has the same numerical limits as "purified" immediately above. "Isolated" and "purified" do not encompass either natural materials in their native state or natural materials that have been separated into components (e.g., in an acrylamide gel) but not obtained either as pure (e.g. lacking contaminating proteins, or chromatography reagents such as denaturing agents and polymers, e.g. acrylamide or agarose) substances or solutions. In preferred embodiments, purified *hedgehog* preparations will lack any contaminating proteins from the same animal from that *hedgehog* is normally produced, as can be accomplished by recombinant expression of, for example, a human *hedgehog* protein in a non-human cell.

As described above for recombinant polypeptides, isolated *hh* polypeptides can include all or a portion of the amino acid sequences represented in SEQ ID No:11, or a homologous sequence thereto. Preferred fragments of the subject *hedgehog* proteins correspond to the N-terminal and C-terminal proteolytic fragments of the mature protein (see, for instance, Examples 6 and 9).

Isolated peptidyl portions of *hedgehog* proteins can be obtained by screening peptides recombinantly produce from the corresponding fragment of the nucleic add encoding such peptides. In addition, fragments can be chemically synthesized using technique known in the art such as conventional Merrifield solid phase f-Moc or t-Boc chemistry. For example, a *hedgehog* polypeptide of the present invention may be arbitrarily divided into fragments of desired length with no overlap of the fragments, or preferably divided into overlapping fragments of a desired length. The fragments can be produced (recombinantly or by chemical synthesis) and tested to identify those peptidyl fragments which can function as either agonists or antagonists of a wild-type (e.g.. "authentic"*) hedgehog* protein.

The recombinant *hedgehog* polypeptides of the present invention also include homologs of the authentic *hedgehog* proteins, such as versions of those protein which are resistant to proteolytic cleavage, as for example, due to mutations which alter potential cleavage sequences or which inactivate an enzymatic activity associated with the protein. *Hedgehog* homologs of the present invention also include proteins which have been post-translationally modified in a manner different than the authentic protein. Exemplary derivatives of vertebrate *hedgehog* proteins include polypeptides which lack N-glycosylation sites (e.g. to produce an unglycosylated protein), or which lack N-terminal and/or C-terminal sequences.

Modification of the structure of the subject vertebrate *hh* polypeptides can be for such purposes as enhancing therapeutic or prophylactic efficacy, or stability (e.g., *ex vivo* shelf life and resistance to proteolytic degradation *in vivo*). Such modified peptides, when designed to retain at least one activity of the naturally-occurring form of the protein, are considered functional equivalents of the *hedgehog* polypeptides described in more detail herein. Such modified peptides can be produced, for instance, by amino acid substitution, deletions, or addition.

For example, it is reasonable to expect that an isolated replacement of a leucine with an isoleucine or valine, an aspartate with a glutamate, a threonine with a serine, or a similar replacement of an amino acid with a structurally related amino acid (i.e. isosteric and/or isoelectric mutations) will not have a major effect on the biological activity of the resulting molecule. Conservative replacements are those that take place within a family of amino acids that are related in their side chains. Genetically encoded amino acids are can be divided into four families: (1) acidic = aspartate, glutamate; (2) basic = lysine, arginine, histidine; (3) nonpolar = alanine, valine, leucine, isoleucine, proline, phenylalanine, methionine, tryptophan; and (4) uncharged polar = glycine, asparagine, glutamine, cysteine, serine, threonine, tyrosine. Phenylalanine, tryptophan, and tyrosine are sometimes classified jointly as aromatic amino acids. In similar fashion, the amino acid repertoire can be grouped as (1) acidic = aspartate, glutamate; (2) basic = lysine, arginine histidine, (3) aliphatic = glycine, alanine, valine, leucine, isoleucine, serine, threonine, with serine and threonine optionally be grouped separately as aliphatic-hydroxyl; (4) aromatic = phenylalanine, tyrosine, tryptophan; (5) amide = asparagine, glutamine; and (6) sulfur -containing = cysteine and methionine. (see, for example, Biochemistry, 2nd ed., Ed. by L. Stryer, WH Freeman and Co.: 1981). Whether a change in the amino acid sequence of a peptide results in a functional *hedgehog* homolog (e.g. functional in the sense that it acts to mimic or antagonize the wild-type form) can be readily determined by assessing the ability of the variant peptide to produce a response in cells in a fashion similar to the wild-type protein, or competitively inhibit such a response. Polypeptides in which more than one replacement has taken place can readily be tested in the same manner.

This invention further contemplates a method for generating sets of combinatorial mutants of the subject *hedgehog* proteins as well as truncation mutants, and is especially useful for identifying potential variant sequences (e.g. homologs) that are functional in binding to a receptor for *hedgehog* proteins. The purpose of screening such combinatorial libraries is to generate, for example, novel *hh* homologs which can act as either agonists or antagonist, or alternatively, possess novel activities all together. To illustrate, *hedgehog* homologs can be engineered by the present method to provide more efficient binding to a cognate receptor, yet still retain at least a portion of an activity associated with *hh*. Thus, combinatorially-derived homologs can be generated to have an increased potency relative to a naturally occurring form of the protein. Likewise, *hedgehog* homologs can be generated by the present combinatorial approach to act as antagonists, in that they are able to mimic, for example, binding to other extracellular matrix components (such as receptors), yet not induce any biological response, thereby inhibiting the action of authentic *hedgehog* or *hedgehog* agonists. Moreover, manipulation of certain domains of *hh* by the present method can provide domains more suitable for use in fusion proteins, such as one that incorporates portions of other proteins which are derived from the extracellular matrix and/or which bind extracellular matrix components.

In one aspect of this method, the amino acid sequences for a population of *hedgehog* homologs or other related proteins are aligned, preferably to promote the highest homology possible. Such a population of variants can include, for example, *hh* homologs from one or more species. Amino acids which appear at each position of the aligned sequences are selected to create a degenerate set of combinatorial sequences. In a preferred embodiment, the variegated library of *hedgehog* variants is generated by combinatorial mutagenesis at the nucleic acid level, and is encoded by a variegated gene library. For instance, a mixture of synthetic oligonucleotides can be enzymatically ligated into gene sequences such that the degenerate set of potential *hh* sequences are expressible as individual polypeptides, or alternatively, as a set of larger fusion proteins (e.g. for phage display) containing the set of *hh* sequences therein.

As illustrated in Figure 5A, to analyze the sequences of a population of variants, the amino acid sequences of interest can be aligned relative to sequence homology. The presence or absence of amino acids from an aligned sequence of a particular variant is relative to a chosen consensus length of a reference sequence, which can be real or artificial. In order to maintain the highest homology in alignment or sequences, deletions in the sequence of a variant relative to the reference sequence can be represented by an amino acid space (• or *), while insertional mutations in the variant relative to the reference sequence can be disregarded and left out of the sequence of the variant when aligned. For instance, Figure 5A includes the alignment of several cloned forms of *hh* from different species. Analysis of the alignment of the *hh* clones shown in Figure 5A can give rise to the generation of a degenerate library of polypeptides comprising potential *hh* sequences.

In an illustrative embodiment, alignment of exon 1/2 encoded sequences (e.g. the N-terminal approximately 165 residues of the mature protein) of each of the *Shh* clones produces a degenerate set of *Shh* polypeptides represented by the general formula: wherein each of the degenerate positions "X" can be an amino acid which occurs in that position in one of the human, mouse, chicken or zebrafish *Shh* clones, or, to expand the library, each X can also be selected from amongst amino acid residue which would be conservative substitutions for the amino acids which appear naturally in each of those positions. For instance, Xaa(1) represents Gly, Ala, Val, Leu, Ile, Phe, Tyr or Trp ; Xaa(2) represents Arg, His or Lys; Xaa(3) represents Gly, Ala, Val, Leu, Ile, Ser or Thr; Xaa(4) represents Gly, Ala, Val, Leu, Ile, Ser or Thr; Xaa(5) represents Lys, Arg, His, Asn or Gln; Xaa(6) represents Lys, Arg or His; Xaa(7) represents Ser, Thr, Tyr, Trp or Phe; Xaa(8) represents Lys, Arg or His; Xaa(9) represents Met, Cys, Ser or Thr; and Xaa(10) represents Gly, Ala, Val, Leu, Ile, Ser or Thr. In an even more expansive library, each X can be selected from any amino acid.

In similar fashion, alignment of each of the human, mouse, chicken and zebrafish *hedgehog* clones (Figure 5B), can provide a degenerate polypeptide sequence represented by the general formula: wherein, as above, each of the degenerate positions "X" can be an amino acid which occurs in a corresponding position in one of the wild-type clones, and may also include amino acid residue which would be conservative substitutions, or each X can be any amino acid residue. In an exemplary embodiment, Xaa(1) represents Gly, Ala, Val, Leu, Ile, Pro, Phe or Tyr; Xaa(2) represents Gly, Ala, Val, Leu or Ile; Xaa(3) represents Gly, Ala, Val, Leu, Ile, Lys, His or Arg; Xaa(4) represents Lys, Arg or His; Xaa(5) represents Phe, Trp, Tyr or an amino acid gap; Xaa(6) represents Gly, Ala, Val, Leu, Ile or an amino acid gap; Xaa(7) represents Asn, Gin, His, Arg or Lys; Xaa(8) represents Gly, Ala, Val, Leu, Ile, Ser or Thr; Xaa(9) represents Gly, Ala, Val, Leu, Ile, Ser or Thr; Xaa(10) represents Gly, Ala, Val, Leu, Ile, Ser or Thr; Xaa(11) represents Ser, Thr, Gln or Asn; Xaa(12) represents Met, Cys, Gly, Ala, Val, Leu, Ile, Ser or Thr; Xaa(13) represents Gly, Ala, Val, Leu, Ile or Pro; Xaa(14) represents Arg, His or Lys; Xaa(15) represents Gly, Ala, Val, Leu, Ile, Pro, Arg, His or Lys; Xaa(16) represents Gly, Ala, Val, Leu, Ile, Phe or Tyr; Xaa(17) represents Arg, His or Lys; Xaa(18) represents Gly, Ala, Val, Leu, Ile, Ser or Thr; Xaa(19) represents Thr or Ser; Xaa(20) represents Gly, Ala, Val, Leu, Ile, Asn or Gln; Xaa(21) represents Arg, His or Lys; Xaa(22) represents Asp or Glu; Xaa(23) represents Ser or Thr; Xaa(24) represents Glu, Asp, Gln or Asn; Xaa(25) represents Glu or Asp; Xaa(26) represents Arg, His or Lys; Xaa(27) represents Gly, Ala, Val, Leu or Ile; Xaa(28) represents Gly, Ala, Val, Leu, Ile, Thr or Ser; Xaa(29) represents Met, Cys, Gln, Asn, Arg, Lys or His; Xaa(30) represents Arg, His or Lys; Xaa(3 1) represents Trp, Phe, Tyr, Arg, His or Lys; Xaa(32) represents Gly, Ala, Val, Leu, Ile, Ser, Thr, Tyr or Phe; Xaa(33) represents Gin, Asn, Asp or Glu; Xaa(34) represents Asp or Glu; Xaa(35) represents Gly, Ala, Val, Leu, or Ile; Xaa(36) represents Arg, His or Lys; Xaa(37) represents Asn, Gln, Thr or Ser; Xaa(38) represents Gly, Ala, Val, Leu, Ile, Ser, Thr, Met or Cys; Xaa(39) represents Gly, Ala, Val, Leu, Ile, Thr or Ser; Xaa(40) represents Arg, His or Lys; Xaa(41) represents Asn, Gln, Gly, Ala, Val. Leu or Ile; Xaa(42) represents Gly, Ala, Val, Leu or Ile; Xaa(43) represents Gly, Ala, Val, Leu, Ile, Ser, Thr or Cys; Xaa(44) represents Gly, Ala, Val, Leu, Ile, Thr or Ser; and Xaa(45) represents Asp or Glu.

There are many ways by which the library of potential *hh* homologs can be generated from a degenerate oligonucleotide sequence. Chemical synthesis of a degenerate gene sequence can be carried out in an automatic DNA synthesizer, and the synthetic genes then ligated into an appropriate expression vector. The purpose of a degenerate set of genes is to provide, in one mixture, all of the sequences encoding the desired set of potential *hh* sequences. The synthesis of degenerate oligonucleotides is well known in the art (see for example, Narang, SA (1983) Tetrahedron 39:3; Itakura et al. (1981) Recombinant DNA, Proc 3rd Cleveland Sympos. Macromolecules, ed. AG Walton, Amsterdam: Elsevier pp273-289; Itakura et al. (1984) Annu. Rev. Biochem. 53:323; Itakura et al. (1984) Science 198:1056; Ike et al. (1983) Nucleic Acid Res. 11:477. Such techniques have been employed in the directed evolution of other proteins (see, for example, Scott et al. (1990) Science 249:386-390; Roberts et al. (1992) PNAS 89:2429-2433; Devlin et al. (1990) Science 249: 404-406; Cwirla et al. (1990) PNAS 87: 6378-6382; as well as U.S. Patents Nos. 5,223,409, 5,198,346, and 5,096,815).

A wide range of techniques are known in the art for screening gene products of combinatorial libraries made by point mutations, and for screening cDNA libraries for gene products having a certain property. Such techniques will be generally adaptable for rapid screening of the gene libraries generated by the combinatorial mutagenesis of *hedgehog* homologs. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Each of the illustrative assays described below are amenable to high through-put analysis as necessary to screen large numbers of degenerate *hedgehog* sequences created by combinatorial mutagenesis techniques.

In one embodiment, the combinatorial library is designed to be secreted (e.g. the polypeptides of the library all include a signal sequence but no transmembrane or cytoplasmic domains), and is used to transfect a eukaryotic cell that can be co-cultured with embryonic cells. A functional *hedgehog* protein secreted by the cells expressing the combinatorial library will diffuse to neighboring embryonic cells and induce a particular biological response, such as to illustrate, neuronal differentiation. Using antibodies directed to epitopes of particular neuronal cells (e.g. Islet-1 or Pax-1), the pattern of detection of neuronal induction will resemble a gradient function, and will allow the isolation (generally after several repetitive rounds of selection) of cells producing active *hedgehog* homologs. Likewise, *hh* antagonists can be selected in similar fashion by the ability of the cell producing a functional antagonist to protect neighboring cells from the effect of wild-type *hedgehog* added to the culture media.

To illustrate, target cells are cultured in 24-well microtitre plates. Other eukaryotic cells are transfected with the combinatorial *hh* gene library and cultured in cell culture inserts (e.g. Collaborative Biomedical Products, Catalog #40446) that are able to fit into the wells of the microtitre plate. The cell culture inserts are placed in the wells such that recombinant *hh* homologs secreted by the cells in the insert can diffuse through the porous bottom of the insert and contact the target cells in the microtitre plate wells. After a period of time sufficient for functional forms of a *hedgehog* protein to produce a measurable response in the target cells, the inserts are removed and the effect of the variant *hedgehog* proteins on the target cells determined. For example, where the target cell is a neural crest cell and the activity desired from the *hh* homolog is the induction of neuronal differentiation, then fluorescently-labeled antibodies specific for Islet-1 or other neuronal markers can be used to score for induction in the target cells as indicative of a functional *hh* in that well. Cells from the inserts corresponding to wells which score positive for activity can be split and re-cultured on several inserts, the process being repeated until the active clones are identified.

In yet another screening assay, the candidate *hedgehog* gene products are displayed on the surface of a cell or viral particle, and the ability of particular cells or viral particles to associate with a *hedgehog*-binding moiety (such as an *hedgehog* receptor or a ligand which binds the *hedgehog* protein) via this gene product is detected in a "panning assay". Such panning steps can be carried out on cells cultured from embryos. For instance, the gene library can be cloned into the gene for a surface membrane protein of a bacterial cell, and the resulting fusion protein detected by panning (Ladner et al., WO 88/06630; Fuchs et al. (1991) BiolTechnology 9:1370-1371; and Goward et al. (1992) TIBS 18:136-140). In a similar fashion, fluorescently labeled molecules which bind *hh* can be used to score for potentially functional *hh* homologs. Cells can be visually inspected and separated under a fluorescence microscope, or, where the morphology of the cell permits, separated by a fluorescence-activated cell sorter.

In an alternate embodiment, the gene library is expressed as a fusion protein on the surface of a viral particle. For instance, in the filamentous phage system, foreign peptide sequences can be expressed on the surface of infectious phage, thereby conferring two significant benefits. First, since these phage can be applied to affinity matrices at very high concentrations, large number of phage can be screened at one time. Second, since each infectious phage displays the combinatorial gene product on its surface, if a particular phage is recovered from an affinity matrix in low yield, the phage can be amplified by another round of infection. The group of almost identical *E.coli* filamentous phages M13, fd, and fl are most often used in phage display libraries, as either of the phage gIII or gVIII coat proteins can be used to generate fusion proteins without disrupting the ultimate packaging of the viral particle (Ladner et al. PCT publication WO 90/02909; Garrard et al., PCT publication WO 92/09G90; Marks et al. (1992) J. Biol. Chem. 267:16007-16010; Griffths et al. (1993) EMBO J 12:725-734; Clackson et al. (1991) Nature 352:624-628: and Barbas et al. (1992) PNAS 89:4457-4461).

In an illustrative embodiment, the recombinant phage antibody system (RAS, Pharamacia Catalog number 27-9400-01) can be easily modified for use in expressing and screening *hh* combinatorial libraries. For instance, the pCANTAB 5 phagemid of the RPAS kit contains the gene which encodes the phage gIII coat protein. The *hh* combinatorial gene library can be cloned into the phagemid adjacent to the gIII signal sequence such that it will be expressed as a gI**II** fusion protein. After ligation, the phagernid is used to transform competent *E. coli* TG1 cells. Transformed cells are subsequently infected with M13K07 helper phage to rescue the phagemid and its candidate *hh* gene insert. The resulting recombinant phage contain phagemid DNA encoding a specific candidate *hh*, and display one or more copies of the corresponding fusion coat protein. The phage-displayed candidate *hedgehog* proteins which are capable of binding an *hh* receptor are selected or enriched by panning. For instance, the phage library can be applied to cultured embryonic cells and unbound phage washed away from the cells. The bound phage is then isolated, and if the recombinant phage express at least one copy of the wild type gIII coat protein, they will retain their ability to infect *E. coli*. Thus, successive rounds of reinfection of *E. coli*, and panning will greatly enrich for *hh* homologs, which can then be screened for Further biological activities in order to differentiate agonist and antagonists. Moreover, differential panning, e.g., with two or more different *hh*-responsive cells, can facilitate isolation of *hedgehog* homologs of selectively arrower biological activity relative to the wild-type protein.

Another aspect of the invention pertains to an antiboby specifically reactive with a vertebrate *hedgehog* protein. For example, by using immunogens derived from *hedgehog* Protein, e.g. based on the cDNA sequences, anti-protein/anti-peptide antisera or monoclonal antibodies can be made by standard protocols (See, for example, Antibodies: A laboratory Manual ed. by Harlow and Lane (Cold Spring Harbor Press: 1988)). A mammal, such as a mouse, a hamster or rabbit can be immunized with an immunogenic form of the peptide (e.g., a vertebrate *hh* polypeptide or an antigenic fragment which is capable of eliciting an antibody response). Techniques for conferring immunogenicity on a protein or peptide include conjugation to carriers or other techniques well known in the art. An immunogenic portion of a *hedgehog* protein can be administered in the presence of adjuvant. The progress of immunization can be monitored by detection of antibody titers in plasma or serum. Standard ELISA or other immunoassays can be used with the immunogen as antigen to assess the levels of antibodies. In a preferred embodiment, the subject antibodies are immunospecific for antigenic determinants of a *hedgehog* protein of a vertebrale organism, such as a mammal, e.g. antigenic determinants of a protein represented by SEQ ID No.11 or a closely related homolog (e.g., at least 90% homologous, and more preferably at least 95% homologous). In yet a further preferred embodiment of the present invention, in order to provide, for example, antibodies which are immuno-selective for discrete *hedgehog* homologs, e.g. *Shh* versus *Dhh* versus *Ihh,* the anti-*hh* polypeptide antibodies do not substantially cross react (i.e. does not mm specifically with a proteins which is, for example, less than 85% homologous to SEQ ID No 11; e.g., less that 95% homologous with SEQ ID No 11: e.g., less than 98-99% homologous with SEQ ID No:11. By "not substantially cross react", it is meant that the antibody has a binding affinity for a non-homologous protein which is at least one order of magnitude, more preferably at least 2 orders of magnitude, and even more preferably at least 3 orders of magnitude less than the binding affinity of the antibody for the protein SEQ ID No 11.

Following immunization of an animal with an antigenic preparation of a hedgehog protein, anti-*hh* antisera can be obtained and, if desired, polyclonal anti-*hh* antibodies isolated from the serum. To produce monoclonal antibodies, antibody-producing cells (lymphocyte) can be harvested from an immunized animal and fused by standard somatic cell fusion procedures with immortalizing cells such as myeloma cells to yield hybridoma cells. Such techniques are well known in the art, an include, for example, the hybridoma technique (originally developed by Kohler and Milstein, (1975) Nature, 256: 495-497), the human B cell hybridoma technique (Kozbar et al., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with a vertebrate *hh* polypeptide of the present invention and monoclonal antibodies isolated from a culture comprising such hybridoma cells.

The term antibody as used herein is intended to include fragments thereof which are also specifically reactive with one of the subject vertebrate *hh* polypeptides. Antibodies can be fragmented using conventional techniques and the fragments screened for utility in the same manner as described above for whole antibodies. For example, F(ab)₂ fragments can be generated by treating antibody with pepsin. The resulting F(ab)₂ fragment can be treated to reduce disulfide bridges to produce Fab fragments. The antibody of the present invention is further intended to include bispecific and chimeric molecules having affinity for a *hedgehog* protein conferred by at least one CDR region of the antibody.

Both monoclonal and polyclonal antibodies (Ab) directed against authentic *hedgehog* polypeptides, or *hedgehog* variants, and antibody fragments such as Fab and F(ab)₂, can be used to block the action of one or more *hedgehog* proteins and allow the study of the role of these proteins in, for example, embryogenesis and/or maintenance of differential tissue. For example, purified monoclonal Abs can be injected directly into the limb buds of chick or mouse embryos. It is demonstrated in the examples below that *hh* is expressed in the limb buds of, for example, day 105 embryos. Thus, the use of anti-*hh* Abs during this developmental stage can allow assessment of the effect of *hh* on the formation of limbs *in vivo.* In a similar approach, hybridomas producing anti-*hh* monoclonal Abs, or biodegradable gels in which anti-*hh* Abs are suspended, can be implanted at a site proximal or within the area at which *hh* action is intended to be blocked. Experiments of this nature can aid in deciphering the role of this and other factors that may be involved in limb patterning and tissue formation.

Antibodies which specifically bind *hedgehog* epitopes can also be used in immunohistochemical staining of tissue samples in order to evaluate the abundance and pattern of expression of each of the subject *hh* polypeptides. Anti*-hedgehog* antibodies can be used diagnostically in immuno-precipitation and immuno-blotting to detect and evaluate *hedgehog* protein levels in tissue as part of a clinical testing procedure. For instance, such measurement can be useful in predictive valuations of the onset or progression of neurological disorders, such as those marked by denervation-like or disuse-like symptoms. Likewise, the ability to monitor *hh* levels in an individual can allow determination of the efficacy of a given treatment regimen for an individual afflicted with such a disorder. The level of *hh* polypeptides may be measured in bodily fluid, such as in samples of cerebral spinal fluid or amniotic fluid, or can be measured in tissue, such as produced by biopsy. Diagnostic assays using anti-*hh* antibodies can include, for examples, immunoassays designed to aid in early diagnosis of a neurodegenerative disorder, particularly ones which are manifest at birth. Diagnostic assays using anti-*hh* polypeptide antibodies can also include immunoassays designed so aid in early diagnosis and phenotyping of a differentiative disorder, as well as neoplastic or hyperplastic disorders.

Another application of anti-*hh* antibodies of the present invention is in the immunological screening of cDNA libraries constructed in expression vectors such as λgt11, λgt18-23, λZAP, and λORF8. Messenger libraries of this type, having coding sequences inserted in the correct reading frame and orientation, can product fusion proteins. For instance, λgt11 will produce fusion proteins whose amino termini consist of β-galactosidase amino acid sequences and whose carboxy termini consist of a foreign polypeptide. Antigenic epitopes of an *hh* protein, e.g. other orthologs of a particular *hedgehog* protein or other homologs from the same species, can then be detected with antibodies, as, for example, reacting nitrocellulose filters lifted from infected plates with anti-*hh* antibodies. Positive phage detected by this assay can then be isolated from the infected plate. Thus, the presence of *hedgehog* homologs can be detected and cloned from other animals, as can alternate isoforms (including splicing variants) from humans.

Moreover, the nucleotide sequences determined from the cloning of *hh* genes from vertebrate organisms will further allow for the generation of probes and primers designed for use in identifying and/or cloning *hedgehog* homologs in other cell types, e.g. from other tissues, as well as *hh* homologs from other vertebrate organisms. For instance, the present invention also provides a probe/primer comprising a substantially purified oligonucleotide, which oligonucleotide comprises a region of nucleotide sequence that hybridizes under stringent conditions to at least 12 consecutive nucleotides of sense or anti-sense sequence of SEQ ID No:4, or naturally occurring mutants thereof. For instances, primers based on the nucleic acid represented in SEQ ID No:4 can be used in PCR reactions to clone *hedgehog* homologs. Likewise, probes based on the subject *hedgehog* sequences can be used to detect transcripts or genomic sequences encoding the same or homologous proteins. In preferred embodiments, the probe further comprises a label group attached thereto and able to be detected, e.g. the label group is selected from the group consisting of radioisotopes, fluorescent compounds, enzymes, and enzyme co-factors.

Such probes can also be used as a part of a diagnostic test kit for identifying cells or tissue which misexpress a *hedgehog* protein, such as by measuring a level of a *hedgehog* encoding nucleic acid in a sample of cells from a patient; e.g. detecting *hh* mRNA levels or determining whether a genomic *hh* gene has been mutated or deleted.

To illustrate, nucleotide probes can be generated from the subject *hedgehog* genes which facilitate histological screening of intact tissue and tissue samples for the presence (or absence) of *hedgehog*-encoding transcripts. Similar to the diagnostic uses of anti-*hedgehog* antibodies, the use of probes directed to *hh* messages, or to genomic *hh* sequences, can be used for both predictive and therapeutic evaluation of allelic mutations which might be manifest in, for example, neoplastic or hypoplastic disorders (e.g. unwanted cell growth) or abnormal differentiation of tissue. Used in conjunction with immunoassays as described above, the oligonucleotide probes can help facilitate the determination of the molecular basis for a developmental disorder which may involve some abnormality associated with expression (or lack thereof) of a *hedgehog* protein. For instance, variation in polypeptide synthesis can be differentiated from a mutation in a coding sequence.

Furthermore, by making available purified and recombinant *hedgehog* polypeptides, the present invention facilitates the development of assays which can be used to screen for drugs, including *hedgehog* homologs, which are either agonists or antagonists of the normal cellular function of the subject *hedgehog* polypeptides, or of their role in the pathogenesis of cellular differentiation and/or proliferation and disorders related thereto. In one embodiments, the assay evaluates the ability of a compound to modulate binding between a *hedgehog* polypeptide and a *hedgehog* receptor. A variety of assay formats will suffice and, in light of the present invention, will be comprehended by skilled artisan.

In many drug screening programs which test libraries of compounds and natural extracts, high throughput assays are desirable in order to maximize the number of compounds surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test compound Moreover, the effects of cellular tonicity and/or bioavailability of the test compound can be generally ignored in the *in vitro* system, the assay instead being focused primarily on the effect of the drug on the molecular target as may be manifest in an alteration of binding affinity with receptor proteins. Accordingly, in an exemplary screening assay of the present invention, the compound of interest is contacted with a *hedgehog* receptor polypeptide which is ordinarily capable of binding a *hedgehog* protein. To the mixture of the compound and receptor is then added a composition containing a *hedgehog* polypeptide. Detection and quantification of receptor/*hedgehog* complexes provides a means for determining the compound's efficacy at inhibiting (or potentiating) complex formation between the receptor protein and the *hedgehog* polypeptide. The efficacy of the compound can be assessed by generating dose response curves from data obtained using various concentrations of the test compound. Moreover, a control assay can also be performed to provide a baseline for comparison. In the control assay, isolated and purified *hedgehog* polypeptide is added to a composition containing the receptor protein, and the formation of receptor/*hedgehog* complex is quantified in the absence of the test compound.

The polypeptide utilized as a *hedgehog* receptor can be generated from the drosophila *patched* protein or a vertebrate homolog thereof. In light of the ability of, for example, *Shh* to activate Dros-HH pathways in transgenic drosophila (see Example 4), it may be concluded that vertebrate *hedgehog* proteins are capable of binding to Drosophila HH receptors. Accordingly, an exemplary screening assay includes a suitable portion of the *patched* protein (SEQ ID No. 42), such as one or both of the substantial extracellular domains (e.g. residues Lys-93 to His-426 and Arg-700 to Arg-966). For instance, the *patched* protein can be provided in soluble form, as for example a preparation of one of the extracellular domains, or a preparation of both of the extracellular domains which are covalently connected by an unstructured linker (see, for example, Huston et al. (1988) PNAS 85:4879; and U.S. Patent No. 5,091,513), or can be provided as part of a liposomal preparation or expressed on the surface of a cell.

Complex formation between the *hedgehog* polypeptide and a *hedgehog* receptor may be detected by a variety of techniques. For instance, modulation of the formation of complexes can be quantitated using, for example, detectably labeled proteins such as radiolabelled, fluorescently labeled, or enzymatically labeled *hedgehog* polypeptides, by immunoassay, or by chromatographic detection.

Typically, it will be desirable to immobilize either the *hedgehog* receptor or the *hedgehog* polypeptide to facilitate separation of receptor/*hedgehog* complexes from uncomplexed forms of one of the proteins, as well as to accommodate automation of the assay. In one embodiment, a fusion protein can be provided which adds a domain that allows the protein to be bound to a matrix. For example, glutathione-S-transferase/receptor (GST/receptor) fusion proteins can be adsorbed onto glutathione sepharose beads (Sigma Chemical, St. Louis, MO) or glutathione derivatized microtitre plates, which are then combined with the *hedgehog* polypeptide, e.g. an ³⁵S-labeled *hedgehog* polypeptide, and the test compound and incubated under conditions conducive to complex formation, e.g. at physiological conditions for salt and pH, though slightly more stringent conditions may be desired. Following incubation, the beads are washed to remove any unbound *hedgehog* polypeptide, and the matrix bead-bound radiolabel determined directly (e.g. beads placed in scintillant), or in the supernatant after the receptor/*hedgehog* complexes are dissociated. Alternatively, the complexes can dissociated from the bead, separated by SDS-PAGE gel, and the level of *hedgehog* polypeptide found in the bead fraction quantitated from the gel using standard electrophoretic techniques.

Other techniques for immobilizing proteins on matrices are also available for use in the subject assay. For instance, soluble portions of the *hedgehog* receptor protein can be immobilized utilizing conjugation of biotin and streptavidin. For instance, biotinylated receptor molecules can be prepared from biotin-NHS (N-hydroxy-succinimide) using techniques well known in the art (e.g., biotinylation kit, Pierce Chemicals, Rockford, IL), and immobilized in the wells of streptavidin-coated 96 well plates (Pierce Chemical). Alternatively, antibodies reactive with the *hedgehog* receptor but which do not interfere with *hedgehog* binding can be derivatized to the wells of the plate, and the receptor trapped in the wells by antibody conjugation. As above, preparations of a *hedgehog* polypeptide and a test compound are incubated in the receptor-presenting wells of the plate, and the amount of receptor/*hedgehog* complex trapped in the well can be quantitated. Exemplary methods for detecting such complexes, in addition to those described above for the GST-immobilized complexes, include immunodetection of complexes using antibodies reactive with the *hedgehog* polypeptide, or which are reactive with the receptor proteins and compete for binding with the *hedgehog* polypeptide; as well as enzyme-linked assays which rely on detecting an enzymatic activity associated with the *hedgehog* polypeptide. In the instance of the latter, the enzyme can be chemically conjugated or provided as a fusion protein with the *hedgehog* polypeptide. To illustrate, the *hedgehog* polypeptide can be chemically cross-linked or genetically fused with alkaline phosphatase, and the amount of *hedgehog* polypeptide trapped in the complex can be assessed with a chromogenic substrate of the enzyme, e.g. paranitrophenylphosphate. Likewise, a fusion protein comprising the *hedgehog* polypeptide and glutathione-S-transferase can be provided, and complex formation quantitated by detecting the GST activity using 1-chloro-2,4-dinitrobenzene (Habig et al (1974) J Biol Chem 249:7130).

For processes which rely on immunodetection for quantitating one of the proteins trapped in the complex, antibodies against the protein, such as the anti*-hedgehog* antibodies described herein, can be used. Alternatively, the protein to be detected in the complex can be "epitope tagged" in the form of a fusion protein which includes, in addition to the *hedgehog* polypeptide or *hedgehog* receptor sequence, a second polypeptide for which antibodies are readily available (e.g. from commercial sources). For instance, the GST fusion proteins described above can also be used for quantification of binding using antibodies against the GST moiety. Other useful epitope tags include myc-epitopes (e.g., see Ellison et al. (1991) J Biol Chem 266:21150-21157) which includes a 10-residue sequence from c-myc, as well as the pFLAG system (International Biotechnologies, Inc.) or the pEZZ-protein A system (Pharamacia, NJ).

Where the desired portion of the *hh* receptor (or other *hedgehog* binding molecule) cannot be provided in soluble form, liposomal vesicles can be used to provide manipulatable and isolatable sources of the receptor. For example, both authentic and recombinant forms of the *patched* protein can be reconstituted in artificial lipid vesicles (e.g. phosphatidylcholine liposomes) or in cell membrane-derived vesicles (see, for example, Bear et al. (1992) Cell 68:809-818; Newton et al. (1983) Biochemistry 22:6110-6117; and Reber et al. (1987) J Biol Chem 262:11369-11374).

In addition to cell-free assays, such as described above, the readily available source of vertebrate *hedgehog* proteins provided by the present invention also facilitates the generation of cell-based assays for identifying small molecule agonists/antagonists and the like. Analogous to the cell-based assays described above for screening combinatorial libraries, cells which are sensitive to *hedgehog* induction can be contacted with a *hedgehog* protein and a test agent of interest, with the assay scoring for modulation in *hedgehog* inductive responses by the target cell in the presence and absence of the test agent. As with the cell-free assays, agents which produce a statistically significant change in *hedgehog* activities (either inhibition or potentiation) can be identified. In an illustrative embodiment, motor neuron progenitor cells, such as from neural plate explants, can be used as target cells. Treatment of such explanted cells with, for example, *Shh* causes the cells to differentiate into motor neurons. By detecting the co-expression of the LIM homeodomain protein Islet-1 (Thor et al. (1991) Neuron 7:881-889; Ericson et al. (1992) Science 256:1555-1560) and the immunoglobulin-like protein SC1 (Tanaka et al. (1984) Dev Biol 106:26-37), the ability of a candidate agent to potentiate or inhibit *Shh* induction of motor neuron differentiation can be measured. The *hedgehog* protein can be provided as a purified source, or in the form of cells/tissue which express the protein and which are co-cultured with the target cells.

In yet another embodiment, the method of the present invention can be used to isolate and clone *hedgehog* receptors. For examples, purified *hedgehog* proteins of the present invention can be employed to precipitate *hedgehog* receptor proteins from cell fractions prepared from cells which are responsive to a *hedgehog* protein. For instance, purified *hedgehog* protein can be derivatized with biotin (using, for instance, NHS-Biotin, Pierce Chemical catalog no. 21420G), and the biotinylated protein utilized to saturate membrane bound *hh* receptors. The *hedgehog* bound receptors can subsequently be adsorbed or immobilized on streptavidin. If desired, the *hedgehog*-receptor complex can be cross-linked with a chemical cross-linking agent. In such as manner, *hh* receptors can be purified, preferably to near homogeneity. The isolated *hh* receptor can then be partially digested with, for example, trypsin, and the resulting peptides separated by reverse-phase chromatography. The chromatography fragments are then analyzed by Edman degradation to obtain single sequences for two or more of the proteolytic fragments. From the chemically determined amino acid sequence for each of these tryptic fragments, a set of oligonucleotide primers can be designed for PCR. These primers can be used to screen both genomic and cDNA libraries. Similar strategies for cloning receptors have been employed, for example, to obtain the recombinant gene for somatostatin receptors (Eppler et al. (1992) J Biol Chem 267:15603-15612).

Other techniques for identifying *hedgehog* receptors by expression cloning will be evident in light of the present disclosure. For instance, purified *hh* polypeptides can be immobilized in wells of micro titre plates and contacted with, for example, COS cells transfected with a cDNA library (e.g.. from tissue expected to be responsive to *hedgehog* induction). From this panning assay, cells which express *hedgehog* receptor molecules can be isolated on the basis of binding to the immobilized *hedgehog* protein. Another closing system, described in PCT publications WO 92/06220 of Flanagan and Leder, involves the use of an expression cloning system whereby a *hedgehog* receptor is stored on the basis of binding to a *hedgehog*/alkaline phosphatase fusion protein (see also Cheng et al. (1994) Cell 79:157-168)

Another aspect of the present invention relates to a use for inducing and/or maintaining a differentiated state, enhancing survival, and/or promoting proliferation of a cell responsive to a vertebrate *hedgehog* protein, by contacting the cells with an *hh* agonist or an *hh* antagonist as the circumstances may warrant. For instance, it is contemplated by the invention that, in light of the present finding of an apparently broad involvement of *hedgehog* proteins in the formation of ordered spatial arrangements of differentiated tissues in vertebrates, the subject proteins could be used to generate and/or maintain an array of different vertebrate tissue both *in vitro* and *in vivo*. The *hh* agent, whether inductive or anti-inductive, can be, as appropriate, any of the preparations described above, including isolated polypeptides, gene therapy constructs or agents identified in the drug assays provided herein. Moreover, the source of *hedgehog* protein can be, in addition to purified protein or recombinant cells, cells or tissue explants which naturally produce one or more *hedgehog* proteins. For instance, as described in Example 2, neural tube explants from embryos, particularly floorplate tissue, can provide a source for *Shh* polypeptide, which source can be implanted in a patient or otherwise provided, as appropriate, for induction or maintenance of differentiation.

For example, the present method is applicable to cell culture techniques. *In vitro* neuronal culture systems have proved to be fundamental and indispensable tools for the study of neural development, as well as the identification of neurotrophic factors such as nerve growth factor (NGF), ciliary trophic factors (CNTF), and brain derived neurotrophic factor (BDNF). Once a neuronal cell has become terminally-differentiated it typically will not change to another terminally differentiated cell-type. However, neuronal cells can nevertheless readily lose their differentiated state. This is commonly observed when they are grown in culture from adult tissue, and when they form a blastema during regeneration. The present method provides a means for ensuring an adequately restrictive environment in order to maintain neuronal cells at various stages of differentiation, and can be employed, for instance, in cell cultures designed to test the specific activities of other trophic factors. In such embodiments of the subject method, the cultured cells can be contacted with an *hh* polypeptide, or an agent identified in the assays described above, in order to induce neuronal differentiation (e.g. of a stem cell), or to maintain the integrity of a culture of terminally-differentiated neuronal cells by presenting loss of differentiation. The source of *hedgehog* proteins in the culture can be derived from, for example, a purified or semi-purified protein composition added directly to the cell culture media, or alternatively, supported and/or released from a polymeric device which supports the growth of various neuronal cells and which has been doped with the protein. The source of the *hedgehog* protein can also be a cell that is co-cultured with the intended neuronal cell and which produces a recombinantor wild-type *hedgehog* protein. Alternatively, the source can be the neuronal cell itself which has been engineered to produce a recombinant *hedgehog* protein. In an exemplary embodiment, a naive neuronal cell (e.g. a stem cell) is treated with an *hh* agonist in order to induce differentiation of the cells into, for example, sensory neurons or, alternatively, motorneurons. Such neuronal cultures can be used as convenient assay systems as well as sources of implantable cells for therapeutic treatments. For example, *hh* polypeptides may be useful in establishing and maintaining the olfactory neuron cultures described in U.S. Patent 5,318,907 and the like.

According to the present invention, large numbers of non-tumorigenic neural progenitor cells can be perpetuated *in vitro* and induced to differentiate by contact with *hedgehog* proteins. Generally, a method is provided comprising the steps of isolating neural progenitor cells from a non-human, perpetuating these cells *in vitro* or *in vivo*, preferably in the presence of growth factors, and differentiating these cells into particular neural phenotypes, e.g., neurons and glia, by contacting the cells with a *hedgehog* agonist.

Progenitor cells are thought to be under a tonic inhibitory influence which maintains the progenitors in a suppressed state until their differentiation is required. However, recent techniques have been provided which permit these cells to be proliferated, and unlike neurons which are terminally differentiated and therefore non-dividing, they can be produced in unlimited number and are highly suitable for transplantation into heterologous and autologous hosts with neurodegenerative diseases.

By "progenitor" it is meant an oligopotent or multipotent stem cell which is able to divide without limit and, under specific conditions, can produce daughter cells which terminally differentiate such as into neurons and glia. These cells can be used for transplantation into a heterologous or autologous host. By heterologous is meant a host other than the animal from which the progenitor cells were originally derived. By autologous is meant the identical host from which the cells were originally derived.

Cells can be obtained from embryonic, post-natal, juvenile or adult neural tissue from any non-human animal. By any animal is meant any multicellular non-human animal which contains nervous tissue. More particularly, is meant any fish, reptile, bird, amphibian or mammal and the like. The most preferable donors are non-human mammals, especially mice.

In the case of a heterologous donor non-human animal, the non-human animal may be euthanized, and the brain and specific area of interest removed using a sterile procedure. Brain areas of particular interest include any area from which progenitor cells can be obtained which will serve to restore function to a degenerated area of the host's brain. These regions include areas of the central nervous system (CNS) including the cerebral cortex, cerebellum, midbrain, brainstem, spinal cord and ventricular tissue, and areas of the peripheral nervous system (PNS) including the carotid body and the adrenal medulla. More particularly, these areas include regions in the basal ganglia, preferably the striatum which consists of the caudate and putamen, or various cell groups such as the globus pallidus, the subthalamic nucleus, the nucleus basalis which is found to be degenerated in Alzheimer's Disease patients, or the substantia nigra pars compacta which is found to be degenerated in Parkinson's Disease patients.

Human heterologous neural progenitor cells may be derived from fetal tissue obtained from elective abortion, or from a post-natal, juvenile or adult organ donor. Autologous neural tissue can be obtained by biopsy, or from patients undergoing neurosurgery in which neural tissue is removed, in particular during epilepsy surgery, and more particularly during temporal lobectomies and hippocampalectomies.

Cells can be obtained from donor tissue by dissociation of individual cells from the connecting extracellular matrix of the tissue. Dissociation can be obtained using any known procedure, including treatment with enzymes such as trypsin, collagenase and the like, or by using physical methods of dissociation such as with a blunt instrument. Dissociation of fetal cells can be carried out in tissue culture medium, while a preferable medium for dissociation of juvenile and adult cells is artificial cerebral spinal fluid (aCSF). Regular aCSF contains 124 mM NaCl, 5 mM KCl, 1.3 mM. MgCl₂, 2 mM CaCl₂, 26 mM NaHCO₃, and 10 mM D-glucose. Low Ca²⁺ aCSF contains the same ingredients except for MgCl₂ at a concentration of 3.2 mM and CaCl₂ at a concentration of 0.1 mM.

Dissociated cells can be placed into any known culture medium capable of supporting cell growth, including MEM, DMEM, RPMI, F-12, and the like, containing supplements which are required for cellular metabolism such as glutamine and other amino acids, vitamins, minerals and useful proteins such as transferrin and the like. Medium may also contain antibiotics to prevent contamination with yeast, bacteria and fungi such as penicillin, streptomycin, gentamicin and the like. In some cases, the medium may contain serum derived from bovine, equine, chicken and the like. A particularly preferable medium for cells is a mixture of DMEM and F-12.

Conditions for culturing should be close to physiological conditions. The pH of the culture media should be close to physiological pH, preferably between pH 6-8, more preferably close to pH 7, even more particularly about pH 7.4. Cells should be cultured at a temperature close to physiological temperature, preferably between 30°C-40°C. more preferably between 32°C-38°C, and most preferably between 35°C-37°C.

Cells can be grown in suspension or on a fixed substrate, but proliferation of the progenitors is preferably done in suspension to generate large numbers of cells by formation of "neurospheres" (see, for example, Reynolds et al. (1992) Science 255:1070-1709; and PCT Publications WO93/01275, WO94/09119, WO94/10292, and WO94/16718). In the case of propagating (or splitting) suspension cells, flasks are shaken well and the neurospheres allowed to settle on the bottom corner of the flask. The spheres are then transferred to a 50 ml centrifuge tube and centrifuged at low speed. The medium is aspirated, the cells resuspended in a small amount of medium with growth factor, and the cells mechanically dissociated and resuspended in separate aliquots of media.

Cell suspensions in culture medium are supplemented with any growth factor which allows for the proliferation of progenitor cells and seeded in any receptacle capable of sustaining cells, though as set out above, preferably in culture flasks or roller bottles. Cells typically proliferate within 3-4 days in a 37°C incubator, and proliferation can be reinitiated at any time after that by dissociation of the cells and resuspension in fresh medium containing growth factors.

In the absence of substrate, cells lift off the floor of the flask and continue to proliferate in suspension forming a hollow sphere of undifferentiated cells. After approximately 3-10 days *in vitro,* the proliferating clusters (neurospheres) are fed every 2-7 days, and more particularly every 2-4 days by gentle centrifugation and resuspension in medium containing growth factor.

After 6-7 days *in vitro,* individual cells in the neurospheres can be separated by physical dissociation of the neurospheres with a blunt instrument, more particularly by triturating the neurospheres with a pipette. Single cells from the dissociated neurospheres are suspended in culture medium containing growth factors, and differentiation of the cells can be induced by plating (or resuspending) the cells in the presence of a *hedgehog* agonist, and (optionally) any other factor capable of sustaining differentiation, such as bFGF and the like.

To further illustrate other uses of *hedgehog* agonists and antagonists, it is noted that intracerebral grafting has emerged as an additional approach to central nervous system therapies. For example, one approach to repairing damaged brain tissues involves the transplantation of cells from fetal or neonatal animals into the adult brain (Dunnett et al. (1987) J Exp Biol 123:265-289; and Freund et al. (1985) J Neurosci 5:603-616). Fetal neurons from a variety of brain regions can be successfully incorporated into the adult brain, and such grafts can alleviate behavioral defects. For example, movement disorder induced by lesions of dopaminergic projections to the basal ganglia can be prevented by grafts of embryonic dopaminergic neurons. Complex cognitive functions that are impaired after lesions of the neocortex can also be partially restored by grafts of embryonic cortical cells. The use of *hedgehog* proteins or mimetics, such as *Shh* or *Dhh,* in the culture can prevent loss of differentiation, or where fetal tissue is used, especially neuronal stem cells, can be used to induce differentiation.

Stem cells useful in the present invention are generally known. For example, several neural crest cells have been identified, some of which are multipotent and likely represent uncommitted neural crest cells, and others of which can generate only one type of cell, such as sensory neurons, and likely represent committed progenitor cells. The role of *hedgehog* proteins employed in the present method to culture such stem cells can be to induce differentiation of the uncommitted progenitor and thereby give rise to a committed progenitor cell, or to cause further restriction of the developmental fate of a committed progenitor cell towards becoming a terminally-differentiated neuronal cell. For example, the present method can be used *In vitro* to induce and/or maintain the differentiation of neural crest cells into glial cells, schwann cells, chromaffin, cells, cholinergic sympathetic or parasympathetic neurons, as well as peptidergic and serotonergic neurons. The *hedgehog* protein can be used alone, or can be used in combination with other neurotrophic factors which act to more particularly enhance a particular differentiation fate of the neuronal progenitor cell. In the later instance, an *hh* polypeptide might be viewed as ensuring that the treated cell has achieve a particular phenotypic state such that the cell is poised along a certain developmental pathway so as to be properly induced upon contact with a secondary neurotrophic factor. In similar fashion, even relatively undifferentiated stem cells or primitive neuroblasts can be maintained in culture and caused to differentiate by treatment with *hedgehog* agonists. Exemplary primitive cell cultures comprise cells harvested from the neural plate or neural tube of an embryo even before much overt differentiation has occured.

In addition to the implantation of cells cultured in the presence of a functional *hedgehog* activity and other *in vitro* uses described above, yet another aspect of the present invention concerns pharmaceutical compositions and medicaments comprising a *hedgehog* protein or mimetic to enhance survival of neurons and other neuronal cells in both the central nervous system and the peripheral nervous system. The ability of *hedgehog* protein to regulate neuronal differentiation during development of the nervous system and also presumably in the adult state indicates that certain of the *hedgehog* proteins can be reasonably expected to facilitate control of adult neurons with regard to maintenance, functional performance, and aging of normal cells; repair and regeneration processes in chemically or mechanically lesioned cells; and prevention of degeneration and premature death which result from loss of differentiation in certain pathological conditions. In light of this understanding, the present invention specifically contemplates applications of the pharmaceutical compositions and medicaments to the treatment of (prevention and/or reduction of the severity or neurological conditions deriving from: (i) acute, subacute, or chronic injury to the nervous system, including traumatic injury, chemical injury, vasal injury and deficits (such as the ischemia resulting from stroke), together with infectious/inflammatory and tumor-induced injury; (ii) aging of the nervous system including Alzheimer's disease; (iii) chronic neurodegenerative diseases of the nervous system, including Parkinson's disease, Huntington's chorea, amylotrophic lateral sclerosis and the like, as well as spinocerebellar degeneration; and (iv) chronic immunological diseases of the nervous system or affecting the nervous system, including multiple sclerosis.

Many neurological disorders are associated with degeneration of discrete populations of "neuronal elements and" may be treatable with a medicament which includes a *hedgehog* agonist. For example, Alzheimer's disease is associated with deficits in several neurotransmitter systems, both those that project to the neocortex and those that reside with the cortex. For instances, the nucleus basalis in patients with Alzheimer's disease have been observed to have a profound (75%) loss of neurons compared to age-matched controls. Although Alzheimer's disease is by far the most common form of dementia, several other disorders can produce dementia. Several of these are degenerative diseases charactered by the death of neurons in various parts of the central nervous system, especially the cerebral cortex. However, some forms of dementia are associated with regeneration of the thalmus or the white matter underlying the cerebral cortex. Here, the cognitive dysfunction results from the isolation of cortical areas by the regeneration of efferents and afferents. Huntington's disease involves the degeneration of intrnstraital and cortical cholinergic neurons and GABAergic neurons. Pick's disease is a severe neuronal degeneration in the neocortex of the frontal and anterior temporal lobes, sometimes accompanied by death of neurons in the striatum. Treatment of patients suffering from such degenerative conditions can include the application of *hedgehog* polypeptides, or agents which mimic their effects, in order to control, for examples, differentiation and apoptotic events which give rise to loss of neurons (e.g. to enhance survival of existing neurons) as well as promote differentiation and repopulation by progenitor cells in the area affected. In preferred embodiments, a source ofa *hedgehog* agent is stereotactically provided within or proximate the area of degeneration. In addidon to degenerative-induced dementias, a pharmaceutical preparation of one or more of the subject *hedgehog* proteins can be applied opportunely in the treatment of neurodegenerative disorders which have manifestations of tremors and involuntary movements. Parkinson's disease, for example, primarily affects subcortical structures and is characterized by degeneration of the nigrostriatal pathway, raphe nuclei, locus cereleus, and the motor nucleus of vagus. Ballism is typically associated with damage to the subthalmic nucleus, often due to acute vascular accident. Also included are neurogenic and myopathic diseases which ultimately effect the somatic division of the peripheral nervous system and are manifest as neuromuscular disorders. Example include chronic atrophies such as amyotrophic lateral sclerosis, Guillain-Barre syndrome and chronic peripheral neuropathy, as well as other diseases which can be manifest as progressive bulbar palsies or spinal muscular atrophies. The present method is amenable to the treatment of disorder of the cerebellum which result in hypotonia or ataxia, such as those lesions in the cerebellum which produce disorders in the limbs ipsilateral to the lesion. For instance, a preparation of a *hedgehog* homolog can be used as a medicament to treat a restricted form of cerebellar cortical degeneration involving the anterior lobes (vernis and leg areas) such as is common in alcoholic patients.

In an illustrative embodiment, the medicaments are used to treat amyotrophic lateral sclerosis. ALS is a name given to a complex of disorders that comprise upper and lower motor neurons. Patients may present with progressive spinal muscular atrophy, progressive bulbar palsy, primary lateral sclerosis, or a combination of these conditions. The major pathological abnormality is characterized by a selective and progressive degeneration of the lower motor neurons in the spinal cord and the upper motor neurons in the cerebral cortex. The therapeutic application of a *hedgehog* agonist, particularly *Dhh*, can be used alone, or in conjunction with other neurotrophic factors such as CNTF, BDNF or NGF to prevent and/or reverse motor neuron degeneration in ALS patents.

*Hedgehog* proteins of the present invention can also be used in medicaments for the treatment of autonomic disorders of the peripheral nervous system, which include disorders affecting the innervation of smooth muscle and endocrine tissue (such as glandular tissue). For instance, the subject method can be used to treat tachycardia or atrial cardiac arrythmias which may arise from a degenerative condition of the nerves innervating the striated muscle of the heart.

Furthermore, a potential role for certain of the *hedgehog* proteins, which is apparent from the appended example, mainly the data of respecting *hedgehog* expression in sensory and motor neurons of the head and trunk (including limb buds), concerns the role of *hedghog* proteins in development and maintenance of dendritic processes of axonal neurons. Potential roles for *hedgehog* proteins consequently include guidance for axonal projections and the ability to promote differentiation and/or maintenance of the innervating cells to their axonal processes. Accordingly, compositions comprising *hedgehog* agonists or other *hedgehog* agents described herein, may be employed to support, or alternatively antagonize the survival and reprojection of several types of ganglionic neurons sympathetic and sensory neurons as well as motor neurons. In particular, such therapeutic compositions may be useful in treatments designed to rescue, for example, various neurons from lesion-induced death as well as guiding reprojection of these neurons after such damage. Such diseases include, but are not limited to, CNS trauma infarction, infection (such as viral infection with varicella-zoster), metabolic disease, nutritional deficiency, toxic agents (such as cisplatin treatment). Moreover, certain of the *hedgehog* agents (such as antagonistic form) may be useful in the selective ablation of sensory neurons, for examples, in the treatment of chronic pain syndromes.

As appropriate, *hedgehog* agents can be used in nerve prostheses for the repair of central and peripheral nerve damage. In particular, where a crushed or severed axon is intubulated by use of a prosthetic device, *hedgehog* polypeptides can be added to the prosthetic device to increased the rate of growth and regeneration of the dendridic processes. Exemplary nerve guidance channels are described in U.S. patents 5.092.871 and 4,955,892. Accordingly, a severed axonal process can be directed toward the nerve enduing front which it was severed by a prosthesis nerve guide which contains, e.g. a semi-solid formulation containing *hedgehog* polypeptide or mimetic, or which is derivatized along the inner walls with a *hedgehog* protein.

In another embodiment, the subject medicaments can be used in the treatment of neoplastic or hyperplastic transformations such as may occur in the central nervous system. For instance, certain of the *hedgehog* proteins (or *hh* agonists) which induce differentiation of neuronal cells can be utilized to cause such transformed cells to become either post-mitotic or apoptotic. Treatment with a *hedgehog* agent may facilitate disruption of autocrine loops, such as TGF-β or PDGF autostimulatory loops, which are believed to be involved in the neoplastic transformation of several neuronal tumors. *Hedgehog* agonists may, therefore, be of use in the treatment of, for example, malignant gliomas, medulloblastomas, neuroectodermal tumors, and ependymonas.

Yet another aspect of the present invention concerns the application of the discovery that *hedgehog* proteins are morphogenic signals involved in other vertebrate organogenic pathways in addition to neuronal differentiation as described above, having apparent roles in other endodermal patterning, as well as both mesodermal and endodermal differentiation processes. As described in the Examples below, *Shh* clearly plays a role in proper limb growth and patterning by initiating expression of signaling molecules, including *Bmp-2* in the mesoderm and *Fgf-4* in the ectoderm. Thus, it is contemplated by the invention that compositions comprising *hedgehog* proteins can also be utilized for both cell culture and medicaments for the treatment of diseases involving generation and maintenance of non-neuronal tissue.

In one embodiment, the present invention makes use of the discovery that *hedgehog* proteins, such as *Shh**,*** are apparently involved in controlling the development of stem cells responsible for formation of the digestive tract, liver, lungs, and other organs which derive from the primitive gut. As described in the Examples below, *Shh* serves as an inductive signal from the endoderm to the mesoderm, which is critical to gut morphogenesis. Therefore, for example, *hedgehog* agonists can be employed in the development and maintenance of an artificial liver which, can have multiple metabolic functions of a normal liver. In an exemplary embodiments, *hedgehog* agonists can be used to induce differentiation of digestive tube stem cells to form hepatocyte cultures which can be used to populate extracellular matrices, or which can be encapsulated in biocompatible polymers, to form both implantable and extracorporeal artificial livers.

In another embodiment, therapeutic compositions of *hedgehog* agonists can be utilized in conjunction with transplantation of such artificial livers, as well as embryonic liver structures, to promote intraperitoneal implantation, vascularization, and *in vivo* differentiation and maintenance of the engrafted liver tissue.

In yet another embodiment, *hedgehog* agonists can be employed as medicaments to regulate such organs after physical, chemical or pathological insult. For instance, therapeutic medicaments and pharmaceutical compositions comprising *hedgehog* agonists can be utilized in liver repair subsequent to a partial hepatectomy. Similarly, medicaments containing *hedgehog* agonists can be used to promote regeneration of lung tissue in the treatment of emphysema.

In still another embodiment of the present invention, compositions comprising *hedgehog* agonists can be used in the *in vitro* generation of skeletal tissue, such as from skeletogenic stem cells, as well as the *in vivo* treatment of skeletal tissue deficiencies. The present invention particularly contemplates the use of *hedgehog* agonists which maintain a skeletogenic activity, such as an ability to induce chondrogenesis and/or osteogenesis. By "skeletal tissue deficiency", it is meant a deficiency in bone or other skeletal connective tissue at any site where it is desired to restore the bone or connective tissue, no matter how the deficiency originated, e.g. whether as a result of surgical intervention, removal of tumor, ulceration, implant, fracture, or Other traumatic or degenerative conditions.

For instance, the present invention makes available effective medicaments and compositions for restoring cartilage function to a connective tissue. Such medicaments and compositions are useful in, for example, the repair of defects or lesions in Cartilage tissue which is the result of degenerative wear such as that which results in arthritis, as well as other mechanical derangements which may be caused by trauma to the tissue, such as a displacement of torn meniscus tissue, meniscectomy, a laxation of a joint by a torn ligament, malignment of joints, bone fracture, or by hereditary disease. The present reparative medicaments and compositions are useful for remodeling cartilage matrix, such as in plastic or reconstructive surgery, as well as periodontal surgery. The present medicaments and compositions may also be applied to improving a previous reparative procedures, for example, following surgical repair of a meniscus, ligament, or cartilage. Furthermore, it may prevent the onset or exacerbation of degenerative disease if applied early enough after trauma.

In one embodiment of the present invention, the medicaments and compositions may be used to treat the afflicted connective tissue, to generate a cartilage repair response in the connective tissue by simulating the differentiation and/or proliferation of chondrocytes embedded in the tissue. Induction of chondrocytes by treatment with a *hedgehog* agonist can subsequently result in the synthesis of new cartilage matrix by the created cells. Such connective tissues as particular cartilage, interarticular cartilage (menisci), costal cartilage (connecting the true ribs and the sternum), ligaments, and tendons are particularly amenable to treatment in reconstructive and/or regenerative therapies using the subject method. As used herein, regenerative therapies include treatment of degenerative states which have progressed to the point of which impairment of the tissue is obviously manifest, as well as preventive treatments of tissue where regeneration is in its earliest stages or imminent. The subject method can further be used to prevent the spread of mineralisation into fibrotic tissue by maintaining a constant production of new cartilage.

In an illustrative embodiment, the subject 60 medicaments and compositions can be used to treat cartilage of a diarthroidal joint, such as a knee, an ankle, an elbow, a hip, a wrist, a knuckle of either a finger or toc, or a temperomandibular joint. The treatment can be directed to the meniscus of the joint to the articular cartilage of the joint, or both. To further illustrate, the subject medicaments and pharmaceutical compositions can be used to treat a degenerative disorder of a knee, such as which might be the result of traumatic injury (e.g., a sports injury or excessive wear) or osteoarthritis. An injection of a *hedgehog* agonist into the joint with, for instance, an arthroscopic needle, can be used to treat the afflicted cartilage. In some instances, the injected agent can be in the form of a hydrogel or other slow release vehicle described above in order to permit a more extended and regular contact of the agent with the treated tissue.

The present invention further contemplates the use of the subject medicaments and compositions in the field of cartilage transplantation and prosthetic device therapies. To date, the growth of new cartilage from either transplantation of autologous or allogenic cartilage has been largely unsuccessful. Problems arise, for instance, because the characteristics of cartilage and fibrocartilage varies between different tissue: such as between particular, meniscal cartilage, ligaments, and tendons, between the two ends of the same ligament or tendon, and between the superficial and deep parts of the tissue. The zonal arrangement of these tissues may reflect a gradual change in mechanical properties, and failure occurs when implanted tissue, which has not differentiated under those conditions, lacks the ability to appropriately respond. For instance, when meniscal cartilage is used to repair anterior cruciate ligament, the tissue undergoes a metaplasia to pure fibrous tissue. By promoting chondrogenesis, the subject medicaments and compositions can be used to particularly addresses this problem, by causing the implanted cells to become more adaptive to the new environment and effectively resemble hypertrophic chondrocytes of an earlier developmental stage of the tissue. Thus, the action of chondrogensis in the implanted tissue, as provided by the subject medicaments and compositions, and the mechanical forces on the actively remodeling tissue can synergize to produce an improved implant more suitable for the new function to which it is to be put.

In similar fashion, the subject medicaments and compositions can be applied to enhancing both the generation of prosthetic cartilage devices and to their implantation. The need for improved treatment has motivated research aimed at creating new cartilage that is based on collagen-glycosaminoglycan templates (Stone et al. (1990) Clin Orthop Relat Red 252:129), isolated chondrocytes (Grande et al. (1989) J Orthop Res 7:208; and Takigawa et al. (1997) Bone Miner 2:449), and chondrocytes attached to natural or synthetic polymers (Walitani et al. (1989) J Bone Jt Surg 71B:74; Vacanti et al. (1991) Plast Reconstr Surg 88:753: von Schroeder et aL (1991) J Biomed Mater Res 25:329; Freed et al. (1993) J Biomed Mater Res 27:11; and the Vacanti et al. U.S. Patent No. 5,041,138). For example, chondrocytes can be grown in culture on biodegradable, biocompatible highly porous scaffolds formed from polymers such as polyglycolic acid, polylactic acid, agarose gel, or other polymer which degrade over time as function of hydrolysis of the polymer backbone into innocuous monomers. The matrices are designed to allow adequate nutrient and gas exchange to the cells until engraftment occurs. The cells can be cultured *in vitro* until adequate cell volume and density has developed for the cells to be implanted. One advantage of the matrices is that they can be cast or molded into a desired shape on an individual basis, so that the final product closely resembles the patient's own ear or nose (by way of example), or flexible matrices can be used which allow for manipulation at the time of implantation, as in a joint.

In one embodiment of the subject medicaments and compositions, the implants are contacted with a *hedgehog* agonist during the culturing process, such as an *Ihh* agonist, in order to induce and/or maintain differentiated chondrocytes in the culture in order as to further stimulate cartilage matrix production within the implant. In such a manner, the cultured cells can be caused to maintain a phenotype typical of a chondrogenic cell (i.e. hypertrophic), and hence continue the population of the matrix and production of cartilage tissue.

In another embodiments, the implanted device is treated with a *hedgehog* agonist in order to actively remodel the implanted matrix and to make it more suitable for its intended function. As set out above with respect to tissue transplants, the artificial transplants suffer from the same deficiency of not being derived in a setting which is comparable to the actual mechanical environment in which the matrix is implanted. The activation of the chondrocytes in the matrix by the subject medicaments and compositions can allow the implant to acquire characteristics similar to the tissue for which it is intended to replace.

In yet another embodinient, the subject medicaments and compositions are used to enhance attachment of prosthetic devices. To illustrate, the subject medicaments and compositions, can be used in the implantation of a periodontal prosthesis, wherein the treatment of the surrounding connective tissue stimulates formation of periodontal ligament about the prosthesis, as well as inhibits formation of fibrotic tissue proximate the prosthetic device.

In still further embodiments, the subject medicaments and compositions can be employed for the generation of bone (osteogenesis) at a site in the non-human where such skeletal tissue is deficient. Indian *hedgehog* is particularly associated with the hypertrophic chondrocytes that are ultimately replaced by osteoblasts. For instance, administration of a *hedgehog* agent of the present invention can be employed as part of a method for treating bone loss in a subject, e.g. to prevent and/of reverse osteoporosis and other osteopenic disorders, as well as to regulate bone growth and maturation. For examples, preparations comprising *hedgehog* agonists can be employed, for example, to induce endochondral ossification, at least so far as to facilitate the formation of cartilaginous tissue precursors to form the "model" for ossification. Therapeutic compositions of *hedgehog* agonists can be supplemented, if required, with other osteoinductive factors, such as bone growth factors (e.g. TGF-β factors, such as the bone morphogenetic factors *BMP-2* and *BMP-4*, as well as activin), and may also include, or be administered in combination with, an inhibitor of bone resorption such as estrogen, bisphosphonate, sodium fluoride, calcitonin, or tamoxifen, or related compounds. However, it will be appreciated that *hedgehog* proteins, such as *Ihh* and *Shh* are likely to be upstream of BMPs, e.g. *hh* treatment will have the advantage of initiating endogenous expression of BMPs along with other factors.

The source of *hedgehog* polypeptides, whether for cell culture or for *in vivo* application, can be in the form of a purified protein composition, or can be from a cell expressing either a recombinant or endogenous form of the polypeptide, such as embryonic tissue (e.g., floor plate tissue explants). Moreover, is addition to those forms of the vertebrate *hedgehog* polypeptides described herein, the present invention further contemplates the use of the *drosophila* hedgehog (Dros-HH) protein to induce cells and tissue of vertebrate organisms.

In the instance of protein compositions, the *hedgehog* protein, or a pharmaceutically acceptable salt thereof, may be conveniently formulated for administration with a biologically acceptable medium, such as waster, buffered saline, polyol (for example, glycerol, propylene glycol, liquid polyethylene glycol and the like) or suitable mixtures thereof. The optimum concentration of the active ingredient(s) in the chosen medium can be determined empirically, according to procedures well known to medicinal chemists. As used herein. "biologically acceptable medium" includes any and all solvents, dispersion media, and the like which may be appropriate for the desired route of administration of the pharmaceutical preparation. The use of such media for pharmaceutically active substances is known in the art. Except insofar as any conventional media or agent is incompatible with the activity of the *hedgehog* protein, its use in the pharmaceutical preparation of the invention is contemplated. Suitable vehicles and their formulation inclusive of other proteins are described, for example, in the book Remington's Pharmaceutical Sciences (Remington's Pharmaceutical Sciences. Mack Publishing Company, Easton, Pa, USA 1985). These vehicles include injectable "deposit formulations". Based on the above, such pharmaceutical formulations include, although not exclusively, solutions or freeze-dried powders of a *hedgehog* homolog (such as a *Shh, Dhh* or *Mhh*) in association with one or more pharmaceutically acceptable vehicles or diluents, and contained in buffered media at a suitable pH and isosmotic with physiological fluids. For illustrative purposes only and without being limited by the same, possible compositions or formulations which may be prepared in the form of solutions for the treatment of nervous system disorders with a *hedgehog* protein are given in U.S. Patent No. 5,218,094. In the case of freeze-dried preparations, supporting excipients such as, but not exclusively, mannitol or glycine may be used and appropriate buffered solutions of the desired volume will be provided so as to obtain adequate isotonic buffered solutions of the desired pH. Similar solutions may also be used for the pharmaceutical compositions of *hh* in isotonic solutions of the desired volume and include, but not exclusively, the use of buffered saline solutions with phosphate or citrate at suitable concentrations so as to obtain at all times isotonic pharmaceutical preparations of the desired pH, (for example, neutral pH).

Methods of introduction of exogenous *hh* at the site of treatment include, but are not limited to, intradermal, intramuscular, intraperitoneal, intravenous, subcutaneous, oral, intranasal and topical. In addition, it may be desirable to introduce the pharmaceutical compositions of the invention into the central nervous system by any suitable route, including intraventricular and intrathecal injection. Intraventricular injection may be facilitated by an intraventricular catheter, for example, attached to a reservoir, such as an Ommaya reservoir.

Methods of introduction may also be provided by rechargeable or biodegradable devices. Various slow release polymeric devices have been developed and tested *in vivo* in recent years for the controlled delivery of drugs, including proteinacious biopharmaceuticals. A variety of biocompatible polymers (including hydrogels), including both biodegradable and non-degradable polymers, can be used to form an implant for the sustained release of an *hh* at a particular target site. Such embodiments of the present invention can be used for the delivery of an exogenously purified *hedgehog* protein, which has been incorporated in the polymeric device, or for the delivery of *hedgehog* produced by a cell encapsulated in the polymeric device.

An essential feature of certain embodiments of the implant can be the linear release of the *hh*, which can be achieved through the manipulation of the polymer composition and form. By choice of monomer composition or polymerization technique, the amount of water, porosity and consequent permeability characteristics can be controlled. The selection of the shape, size, polymer, and method for implantation can be determined on an individual basis according to the disorder to be treated and the individual patient response. The generation of such implants is generally known in the art. See, for example, *Concise Encyclopedia of Medical* & *Dental Materials,* ed. by David Williams (MIT Press: Cambridge, MA, 1990); and the Sabel et al. U.S. Patent No. 4,883,666. In another embodiment of an implant, a source of cells producing a *hedgehog* protein, or a solution of hydogel matrix containing purified *hh*, is encapsulated in implantable hollow fibers. Such fibers can be pre-spun and subsequently loaded with the *hedgehog* source (Aebischer et al. U.S. Patent No. 4,892,538; Aebischer et al. U.S. Patent No. 5,106,627: Hoffman et al. (1990) Expt. Neurobiol. 110:39-44; Jaeger et al. (1990) Prog. Brain Res. 82:41-46; and Aebischer et al. (1991) J. Biomech. Eng. 113:178-183), or can be co-extruded with a polymer which acts to form a polymeric coat about the *hh* source (Lim U.S. Patent No. 4,391,909; Sefton U.S. Patent No. 4,353,888; Sugamori et al. (1989) Trans. Am. Artif. Intern. Organs 35:791-799; Sefton et al. (1987) Biotehnol. Bioeng. 29:1135-1143; and Aebischer et al. (1991) Biomaterials 12:50.55).

In yet another embodiment of the present invention, the pharmaceutical *hedgehog* protein can be administered as part of a combinatorial therapy with other agents. For example, the combinatorial therapy can include a *hedgehog* protein with at least one trophic factor. Exemplary trophic factors include nerve growth factor, cilliary neurotrophic growth factor, schwanoma-derived growth factor, glial growth factor, stiatal-derived neuronotrophic factor, platelet-derived growth factor, and scatter factor (HGF-SF). Antimitogenic agents can also be used, for example, when proliferation of surrounding glial cells or astrocytes is undesirable in the regeneration of nerve cells. Examples of such antimitotic agents include cytosine, arabinoside. 5-fluorouracil, hydroxyurea, and methotrexate.

### Exemplification

The invention, now being generally described, will be more readily understood by reference to the following examples, which are included merely for purposes of illustration of certain aspects and embodiments of the present invention and are not intended to limit the invention.

### Example 1

### Cloning and Expression of Chick Sonic Hedgehog

### (i) Experimental Procedures

Using degenerate PCR primers, vHH5O (SEQ ID No:18), vHH3O (SEQ ID No: 19) and vHH3I (SEQ ID No:20) corresponding to a sequence conserved between Drosophila *hedgehog* (Dros-HH)(SEQ ID No:34) (Lee, JJ. et al. (1992) Cell 71: 33-50; Mohler, J. et al., (1992) Development 115: 957-971) and mouse Indian *hedgehog* (*Ihh*) (SEQ ID No:10), a 220 base pair (bp) fragment was amplified from chicken genomic DNA. From 15 isolates, two distinct sequences were cloned, pCHA (SEQ ID No:35) and pCHB (SEQ ID No:36), each highly homologous to mouse *Ihh* (Figure 1). A probe made from isolate pCHA did not detect expression in embryonic tissues. Isolate pCHB, however, detected a 4 kb message in RNA prepared from embryonic head, trunk, or limb bud RNA. This cloned PCR fragment was therefore used as a probe to screen an unamplified cDNA library prepared from Hamburger Hamilton stage 22 (Hamburger, W. et al., (1951) J. Morph. 88: 49-92) limb bud RNA as described below.

A single 1.6 kilobase (kb) cDNA clone, pHH-2, was selected for characterization and was used in all subsequent analyses. The gene encoding for this cDNA was named *Sonic Hedgehog* (after the Sega computer game cartoon character). Sequencing of the entire cDNA confirmed the presence of a single long open reading frame potentially encoding for a protein of 425 amino acids (aa). The clone extends 220 bp upstream of the predicted initiator methionine and approximately 70 bp beyond the stop codon. No consensus polyadenylation signal could be identified in the 3' untranslated region. A second potential initiator methionine occurs at amino acid residue 4. The putative translation initiation signals surrounding both methionines are predicted to be equally efficient (Kozak, M., (1987) Nuc. Acids.Res. 15: 8125-8132). When the pHH-2 *Sonic* cDNA is used to probe a northern blot of stage 24 embryonic chick RNA, a single mRNA species of approximately 4 kb is detected in both limb and trunk tissue. The message size was predicted by comparing it to the position of 18S and 28S ribosomal RNA. Hybridized mRNA was visualized after a two day exposure to a phosphoscreen. Because the *Sonic* cDNA clone pHH-2 is only 1.6 kb, it is likely to be missing approximately 2.4 kb of untranslated sequence.

### PCR Cloning

All standard cloning techniques were performed according to Ausubel et. al. (1989), and all enzymes were obtained from Boehringer Mannheim Biochemicals. Degenerate oligonucleotides corresponding to amino acid residues 161 to 237 of the Drosophila *hedgehog* protein (SEQ ID No:34) (Lee, J.J. et. al., (1992) Cell 71: 33-50) were synthesized. These degenerate oligonucleotides, vHH50 (SEQ ID No: 18), vHH3O (SEQ ID No:19), and vHH3I (SEQ ID No:20) also contained Eco RI, Cla I, and Xba I sites, respectively, on their 5' ends to facilitate subcloning. The nucleotide sequence of these oligos is given below:
vHH5O: 5'-GGAATTCCCAG(CA)GITG(CT)AA(AG)GA(AG)(CA)(AG)I(GCT)IAA-3'
vHH3O: 5'-TCATCGATGGACCCA(GA)TC(GA)AAICCIGC(TC)TC-3'
vHH3I: 5'-GCTCTAGAGCTCIACIGCIA(GA)IC(GT)IGC-3'
where I represents inosine. Nested PCR was performed by first amplifying chicken genomic DNA using the vHH5O and vHH3O primer pair and then further amplifying that product using the vHH50 and vHH3I primer pair. In each case the reaction conditions were: initial denaturation at 93° C for 2.5 min., followed by 30 cycles of 94° C for 45 s, 50° C for 1 min., 72° C for 1, and a final incubation of 72° C for 5 min. The 220 bp PCR product was subcloned into pGEM7zf (Promega). Two unique clones, pCHA (SEQ ID No:35) and pCHB (SEQ ID No:36) were identified.

### DNA Sequence Analysis

Nucleotide sequences were determined by the dideoxy chain termination method (Sanger, F. et al., (1977) Proc. Natl. Acad Sci. USA 74: 5463-5467) using Sequenase v2.0 T7 DNA polymerase (US Biochemicals). 5' and 3' nested deletions of pHH-2 were generated by using the nucleases Exo III and S1 (Erase a Base, Promega) and individual subclones sequenced. DNA and amino acid sequences were analyzed using both GCG (Devereux, J. et al., (1984) Nuc. Acids Res. 12: 387-394) and DNAstar software. Searches for related sequences were done through the BLAST network service (Altschul, S.F. et al., (1990) J. Mol. Biol. 215: 403-410) provided by the National Center for Biotechnology Information.

### Southern Blot Analysis

Five (5) µg of chick genomic DNA was digested with Eco RI and/or Bam HI, fractionated on a 1 % agarose gel, and transferred to a nylon membrane (Genescreen, New England Nuclear). The filters were probed with ³²P-labeled *hh*a or *hh*b at 42°C in hybridization buffer (0.5% BSA, 500 mM NaHPO₄, 7% SDS, 1 mM EDTA, pH 7.2; Church, G.M. et al., (1984) Proc. Natl. Acad Sci. USA 81: 1991-1995). The blots were washed at 63° C once in 0.5% bovine serum albumin, 50 mM NaHPO₄ (pH 7.2), 5% SDS, 1 mM EDTA and twice in 40 mM NaHPO₄ (pH 7.2), 1 % SDS, 1 mM EDTA, and visualized on Kodak XAR-5 film.

### Isolation Of Chicken Sonic cDNA Clones

A stage 22 limb bud cDNA library was constructed in λgt10 using Eco RI/NotI linkers. Unamplified phage plaques (10⁶) were transferred to nylon filters (Colony/Plaque screen, NEN) and screened with α³²P-labelled pooled inserts from PCR clones pCHA (SEQ ID No:35) and pCHB (SEQ ID No:36). Hybridization was performed at 42° C in 50% formamide 2X SSC, 10% dextran sulfate, 1% SDS and washing as described in the Southern Blot procedure. Eight positive plaques were identified, purified and their cDNA inserts excised with EcoRI and subcloned into pBluescript SK+ (Stratagene). All eight had approximately 1.7 kb inserts with identical restriction patterns. One, pHH-2, was chosen for sequencing and used in all further manipulations.

### Preparation Of Digoxigenin-Labeled Riboprobes

Plasmid pHH-2 was linearized with Hind III and transcribed with T3 RNA polymerase (for antisense probes) or with Bam HI and transcribed with T7 RNA polymerase according to the manufacturers instructions for the preparation of non-radioactive digoxigenin transcripts. Following the transcription reaction, RNA was precipitated, and resuspended in RNAse-free water.

### Whole Mount In Situ Hybridization

Whole-mount *in situ* hybridization was performed using protocols modified from Parr, B.A. et al., (1993) Development 119: 247-261; Sasaki, H. et al. (1993) Development 118: 47-59; Rosen, B. et al. (1993) Trends Genet. 9: 162-167. Embryos from incubated fertile White Leghorn eggs (Spafas) were removed from the egg and extra-embryonic membranes dissected in calcium/magnesium-free phosphate-buffered saline (PBS) at room temperature. Unless otherwise noted, all washes are for five minutes at room temperature. Embryos were fixed overnight at 4°C with 4% paraformaldehyde in PBS, washed twice with PBT (PBS with 0.1 % Tween-20) at 4°C, and dehydrated through an ascending methanol series in PBT (25%, 50%, 75%, 2 X 100% methanol). Embryos were stored at -20°C until further use.

Both pre-limb bud and limb bud stage embryos were rehydrated through an descending methanol series followed by two washes in PBT. Limb bud stage embryos were bleached in 6% hydrogen peroxide in PBT, washed three times with PBT, permeabilized with proteinase K (Boehringer, 2 µg/ml) for 15 minutes, washed with 2 mg/ml glycine in PBT for 10 minutes, and twice with PBT. Pre-limb bud stage embryos were permealibized (without prior incubation with hydrogen peroxide) by three 30 minute washes in RIPA buffer (150 mM NaCl, 1 % NP-40, 0.5% Deoxycholate, 0.1 % SDS, 1 mM EDTA, 50 mM Tris-HCl, pH 8.0). In all subsequent steps, pre-limb bud and limb bud stage embryos were treated equivalently. Embryos were fixed with 4% paraformaldehyde/0.2% gluteraldehyde in PBT, washed four times with PBT, once with pre-hybridization buffer (50% formamide, 5 X SSC, 1% SDS, 50 µg/ml total yeast RNA, 50 µg/ml heparin, pH 4.5), and incubated with fresh pre-hybridization buffer for one hour at 70°C. The pre-hybridization buffer was then replaced with hybridization buffer (pre-hybridization buffer with digoxigenin labeled riboprobe at 1 µg/ml) and incubated overnight at 70°C.

Following hybridization, embryos were washed 3 X 30 minutes at 70°C with solution 1 (50% formamide, 5 X SSC, 1% SDS; pH 4.5), 3 X 30 minutes at 70°C with solution 3 (50% formamide, 2 X SSC, pH 4.5), and three times at room temperature with TBS (Tris-buffered saline with 2 mM levamisole) containing 0.1% Tween-20. Non-specific binding of antibody was prevented by preblocking embryos in TBS/0.1% Tween-20 containing 10% heat-inactivated sheep serum for 2.5 hours at room temperature and by pre-incubating anti-digoxigenin Fab alkaline-phosphatase conjugate (Boehringer) in TBS/0.1% Tween-20 containing heat inactivated 1% sheep serum and approximately 0.3% heat inactivated chick embryo powder. After an overnight incubation at 4°C with the pre-adsorbed antibody in TBS/0.1 % Tween-20 containing 1% sheep serum, embryos were washed 3 X 5 minutes at room temperature with TBS/0.1% Tween-20, 5 X 1.5 hour room temperature washes with TBS/1% Tween-20, and overnight with TBS/1% Tween-20 at 4°C. The buffer was exchanged by washing 3 X 10 minutes with NTMT (100mM NaCl, 100 mM Tris-HCl, 50 mM MgCl2, 0.1% Tween-20, 2 mM levamisole). The antibody detection reaction was performed by incubating embryos with detection solution (NTMT with 0.25 mg/ml NBT and 0.13 mg/ml X-Phos). In general, pre-limb bud stage embryos were incubated for 5-15 hours and limb bud stage embryos 1-5 hours. After the detection reaction was deemed complete, embryos were washed twice with NTMT, once with PBT (pH 5.5), postfixed with 4% paraformaldehyde/0.1% gluteraldehyde in PBT, and washed several times with PBT. In some cases embryos were cleared through a series of 30%, 50%, 70%, and 80% glycerol in PBT. Whole embryos were photographed under transmitted light using a Nikon zoom stereo microscope with Kodak Ektar 100 ASA film. Selected embryos were processed for frozen sections by dehydration in 30% sucrose in PBS followed by embedding in gelatin and freezing. 25 µm cryostat sections were collected on superfrost plus slides (Fisher), rehydrated in PBS, and mounted with gelvatol. Sections were photographed with Nomarski optics using a Zeiss Axiophot microscope and Kodak Ektar 25 ASA film.

### (ii) Sequence Homolgy Comparison Between Chicken Sonic hh And Dros-HH And Other Vertebrate Sonic hh Proteins

The deduced *Sonic* amino acid sequence (SEQ ID No:8) is shown and compared to the Drosophila *hedgehog* protein (SEQ ID No:34) in Figure 2. Over the entire open reading frame the two proteins are 48% homologous at the amino acids level. The predicted Drosophila protein extends 62 aa beyond that of *Sonic* at its amino terminus. This N-terminal extension precedes the putative signal peptide (residues 1-26) of the fly protein (SEQ ID No:34), and has been postulated to be removed during processing of the secreted form of Drosophila *hedgehog* (Lee, J.J. et al., (1992) Cell 71: 33-50). The sequence of residues 1-26 of the *Sonic* protein (SEQ ID No:8) matches well with consensus sequences for eukaryotic signal peptides (Landry, S.J. et al., (1993) Trends. Biochem. Sci. 16: 159-163) and is therefore likely to serve that function for *Sonic.* Furthermore, Figure 3 shows a hydropathy plot (Kyte, J. et al., (1982) J. Mol. Biol.157: 133-148) indicating that residues 1-26 of the *Sonic* proteins (SEQ ID No:8) exhibit a high hydrophobic moment in accord with identified eukaryotic signal peptides. Cleavage of the putative signal sequence should occur C-terminal to residue 26 according to the predictive method of von Henjie, G. (1986) Nucl. Acid. Res. 11: 1986. A single potential N-linked glycosylation site is located at amino acid residue 282 of the *Sonic* protein (SEQ ID No:8). The predicted *Sonic* protein does not contain any other strong consensus motifs, and is not homologous to any other proteins outside of the *Hedgehog* family.

The mouse (SEQ ID No:11) and zebrafish (SEQ ID No: 12) homologs of *Sonic* have also been isolated. A comparison of these and the Drosophila sequence is shown schematically in Figure 4. All of the vertebrate proteins have a similar predicted structure: a putative signal peptide at their amino terminus, followed by an extraordinarily similar 182 amino acid region (99% identity in chicken versus mouse and 95% identity in chicken versus zebrafish) and a less well conserved carboxy-terminal region.

### (iii) At Least Three Hedgehog Homologues Are Present In The Chicken Genome

Since two distinct PCR products encoding for chicken *hedgehogs* were amplified from genomic DNA, the total number of genes in the chicken *hedgehog* family needed to be estimated. The two PCR clones pCHA (SEQ ID No:35) and pCHB (SEQ ID No:36) were used to probe a genomic Southern blot under moderately stringent conditions as described in the above Experimental Procedures. The blot was generated by digesting 5 µg of chick chromosomal DNA with EcoRI and BamHI alone and together. Each probe reacted most strongly with a distinct restriction fragment. For example, the blot probed with pCHA, shows three bands in each of the Bam HI lanes, one strong at 6.6 kb and two weak at 3.4 and 2.7 kb. The blot probed with pCHB, shows the 2.7 kb band as the most intense, while the 3.4 and 6.6 kb bands are weaker. A similar variation of intensities can also be seen in the Bam HI/Eco RI and EcoRI lanes. Exposure times were 72 hr. This data indicates that each probe recognizes a distinct chicken *hedgehog* gene, and that a third as yet uncharacterized chicken *hedgehog* homolog exists in the chicken genome.

### (iv) Northern Analysis Defining Sites Of Sonic Transcription

Northern analysis was performed which confirmed that *Sonic* is expressed during chick development. The spatial and temporal expression of *Sonic* in the chick embryo from gastrulation to early organogenesis was determined by whole mount *in situ* hybridization using a riboprobe corresponding to the full-length *Sonic* cDNA (SEQ ID No:1).

20µg total RNA isolated from stage 24 chick leg buds or bodies (without heads or limbs) was fractionated on a 0.8% agarose formaldehyde gel and transferred to a nylon membrane (Hybond N, Amersham). The blot was probed with the 1.6 kb EcoRI insert from pHH-2. Random-primed α³²P-labelled insert was hybridized at 42°C hybridization buffer (1% BSA, 500mM NaHPO₄, 7% SDS, 1 mM EDTA, pH 7.2) and washed at 63° C once in 0.5% bovine serum albumin, 50 mM NaHPO₄ (pH 7.2), 5% SDS, 1 mM EDTA and once in 40 mM NaHPO₄ (pH 7.2), 1% SDS, 1mM EDTA. The image was visualized using a phosphoimager (Molecular Dynamics) and photographed directly from the video monitor.

### (v) Expression Of Sonic During Mid-Gastrulation

*Sonic* message is detected in, the gastrulating blastoderm at early stage 4, the earliest stage analyzed. Staining is localized to the anterior end of the primitive streak in a region corresponding to Hensen's node. As gastrulation proceeds, the primitive streak elongates to its maximal cranial-caudal extent, after which Hensen's node regresses caudally and the primitive streak shortens. At an early point of node regression, *Sonic* mRNA can be detected at the node and in midline cells anterior to the node. By late stage 5, when the node has migrated approximately one-third of the length of the fully elongated primitive streak, prominent *Sonic* expression is seen at the node and in the midline of the embryo, reaching its anterior limit at the developing head process. Sections at a cranial level show that *Sonic* mRNA is confined to invaginated axial mesendoderm, tissue which contributes to foregut and notochord. More caudally, but still anterior to Hensen's node, staining of axial mesoderm is absent and *Sonic* expression is confined to the epiblast. At the node itself, high levels of *Sonic* message are observed in an asymmetric distribution extending to the left of and posterior to the primitive pit. This asymmetric distribution is consistently observed (6/6 embryos from stages 5-7) and is always located to the left of the primitive pit. At the node, and just posterior to the node, *Sonic* expression is restricted to the epiblast and is not observed in either mesoderm or endoderm. The expression of *Sonic* in the dorsal epiblast layer without expression in underlying axial mesoderm contrasts markedly with later stages where *Sonic* expression in underlying mesoderm always precedes midline neural tube expression.

### (vi) Expression Of Sonic During Head Fold Stages

During the formation and differentiation of the head process, *Sonic* mRNA is detected in midline cells of the neural tube, the foregut, and throughout most of the axial mesoderm. At stage 7, *Sonic* message is readily detected asymmetrically at the node and in ventral midline cells anterior to the node. The rostral limit of *Sonic* expression extends to the anterior-most portions of the embryo where it is expressed in the foregut and prechordal mesoderm (Adelmann, H.B., (1932) Am. J. Anat. 31, 55-101). At stage 8, expression of *Sonic* persists along the entire ventral midline anterior to Hensen's node, while the node region itself no longer expresses *Sonic.* Transverse sections at different axial levels reveal that at stage 8 *Sonic* is coexpressed in the notochord and the overlying ventromedial neuroectoderm from anterior to Hensen's node to the posterior foregut. The levels of *Sonic* message are not uniform in the neural tube: highest levels are found at the presumptive mid- and hindbrain regions with progressively lower levels anterior and posterior. The increasing graded expression in the neural tube from Hensen's node to the rostral brain may reflect the developmental age of the neuroectoderm as differentiation proceeds from posterior to anterior. At the anterior-most end of the embryo, expression is observed in midline cells of the dorsal and ventral foregut as well as in prechordal mesoderm. Although the prechordal mesoderm is in intimate contact with the overlying ectoderm, the latter is devoid of *Sonic* expression.

### (vii) Expression Of Sonic During Early CNS Differentiation

At stages 10 through 14, *Sonic* expression is detected in the notochord, ventral neural tube (including the floor plate), and gut precursors. By stage 10, there is a marked expansion of the cephalic neuroectoderm, giving rise to the fore- mid- and hind-brain. At stage 10, *Sonic* mRNA is abundantly expressed in the ventral midline of the hindbrain and posterior midbrain. This expression expands laterally in the anterior midbrain and posterior forebrain. Expression does not extend to the rostral forebrain at this or later stages. Sections reveal that *Sonic* is expressed in the notochord, the prechordal mesoderm, and the anterior midline of the foregut. Expression in the neuroepithelium extends from the forebrain caudally. In the posterior-most regions of the embryo which express *Sonic,* staining is found only in the notochord and not in the overlying neurectoderm. This contrasts with earlier expression in which the posterior domains of *Sonic* expression contain cells are located in the dorsal epiblast, but not in underlying mesoderm or endoderm. Midgut precursors at the level of the anterior intestinal portal also show weak *Sonic* expression.

At stage 14, expression continues in all three germ layers. The epithelium of the closing midgut expresses *Sonic* along with portions of the pharyngeal endoderm and anterior foregut. Ectoderm lateral and posterior to the tail bud also exhibits weak expression. At this stage, *Sonic* is also expressed along entire length of the notochord which now extends rostrally only to the midbrain region and no longer contacts the neuroepithelium at the anterior end of the embryo. Expression in head mesenchyme anterior to the notochord is no longer observed. In the neural tube *Sonic* is found along the ventral midline of the fore- mid- and hindbrain and posteriorly in the spinal cord. In the forebrain, expression is expanded laterally relative to the hindbrain. At midgut levels, expression of *Sonic* in the neural tube appears to extend beyond the floor plate into more lateral regions. As observed at stage 10, *Sonic* at stage 14 is found in the notochord, but not in the ventral neural tube in posterior-most regions of the embryo. When neuroectodermal expression is first observed in the posterior embryo, it is located in midline cells which appear to be in contact with the notochord. At later stages, expression continues in areas which show expression at stage 14, namely the CNS, gut epithelium including the allantoic stalk, and axial mesoderm.

### (viii) Sonic Is Expressed In Posterior Limb Bud Mesenchyme

The limb buds initially form as local thickenings of the lateral plate mesoderm. As distal outgrowth occurs during stage 17, *Sonic* expression becomes apparent in posterior regions of both the forelimb and the hindlimb. Sections through a stage 21 embryo at the level of the forelimbs reveal that expression of *Sonic* in limb buds is limited to mesenchymal tissue. A more detailed expression profile of *Sonic* during limb development is discussed below in Example 3. Briefly, as the limb bud grows out, expression of *Sonic* narrows along the anterior-posterior axis to become a thin stripe along the posterior margin closely apposed to the ectoderm. Expression is not found at more proximal regions of the bud. High levels of *Sonic* expression are maintained until around stage 25/26 when staining becomes weaker. Expression of *Sonic* is no longer observed in wing buds or leg buds after stage 28.

### Example 2

### Mouse Sonic Hedgehog Is Implicated in the Regulation of CNS and Limb Polarity

### (i) Experimental Procedures

### Isolation Of Hedgehog Phage Clones

The initial screen for mammalian *hh* genes was performed, as above, using a 700bp PCR fragment encompassing exons 1 and 2 of the Dros-HH gene. Approximately one million plaques of a 129/Sv Lambda Fix II genomic library (Stratagene) were hybridized with an α ³²P-dATP labeled probe at low stringency (55°C in 6xSSC, 0.5%SDS, 5 x Denhardt's; final wash at 60°C in 0.5 x SSC, 0.1% SDS for 20'). Five cross hybridizing phage plaques corresponding to the *Dhh* gene were purified. Restriction enzyme analysis indicated that all clones were overlapping. Selected restriction enzyme digests were then performed to map and subclone one of these. Subclones in pGEM (Promega) or Bluescript (Stratagene) which cross-hybridized with the Dros-HH fragment where sequenced using an ABI automatic DNA sequencer.

Mouse *Ihh* and *Shh* were identified by low stringency hybridization (as described above) with a chick *Shh* cDNA clone to one million plaques of an 8.5 day λgt10 mouse embryo cDNA library (Fahrner, K. et al., (1987) EMBO J. 6: 1265-1271). Phage plaques containing a 1.8kb. *Ihh* and 0.64 and 2.8kb *Shh* inserts were identified. Inserts were excised and subcloned into Bluescript (Stratagene) for dideoxy chain termination sequencing using modified T7 DNA polymerase (USB). The larger *Shh* clone contained a partially processed cDNA in which intron splicing at the exon 1/2 junction had not occurred.

To screen for additional *Ihh* and *Shh* cDNA clones, an 8.5 day λZAPII cDNA library was probed at high stringency (at 65°C in 6xSSC, 0.5% SDS, 5 x Denhardt's; final wash at 65 °C in 0.1xSSC, 0.1% SDS for 30') with the *Ihh* and *Shh* mouse cDNA clones. No additional *Ihh* clones were identified. However several 2.6kb, apparently full length, *Shh* clones were isolated. The DNA sequence of the additional 5' coding region not present in the original 0.64 and 2.8kb *Shh* clones was obtained by analysis of one of the 2.6kb inserts.

### Northern Blot Analysis

Expression *of Shh* was investigated by RNA blot analysis using 20 µg of total RNA from adult brain, spleen, kidney, liver, lung, 16.5dpc brain, liver and lung; 9.5dpc to 17.5dpc whole embryo; 9.5dpc forebrain, midbrain and 10.5dpc brain. RNA samples were electrophoretically separated on a 1.2% agarose gel, transferred and u.v. crosslinked to Genescreen (DuPont) and probed with 2X10⁶ cpm/ml of an α³²P-dATP labeled mouse *Shh* probe (2.8kb insert from λgt 10 screen). Hybridization was performed at 42°C in 50% formamide 5x Denhardt's, 5xSSPE, 0.1%SDS, 6.5% dextran, 200µg/ml salmon sperm DNA. Final wash was at 55°C in 0.1xSSC. 0.1%SDS. The blot was exposed for 6 days in the presence of an intensifying screen.

### In Situ Hybridization, β-Galactosidase Staining And Histological Analysis

Embryos from 7.25 to 14.5dpc were analyzed for either *Shh* or HNF-3β expression by whole mount *in situ* hybridization to digoxygenin labeled RNA probes as described in Wilkinson, (1992) In situ Hybridization: A Practical Approach. Oxford; Parr et al., (1993) Development 119:247-261. The mouse *Shh* probe was either a 2.8kb or 0.6kb RNA transcript generated by T7 (2.8kb) or T3 (0.6kb) transcription of XbaI and HindIII digests of Bluescript (Stratagene) subclones of the original *Shh* cDNA inserts. The HNF-3β probe was generated by HindIII linearization of a HNF-3β cDNA clone (Sasaki, H. et al., (1993) Development 118: 47-59) and T7 polymerase transcription of 1.6kb transcript. Embryos were photographed on an Olympus-SZH photomicroscope using Kodak Ektachrome EPY 64T color slide film.

Sections through wild type and WEXP2-*CShh* transgenic embryos were prepared and hybridized with ^{3S}S-UIP labeled RNA probes (Wilkinson, D.G. et al., (1987) Development 99: 493-500). Sections were photographed as described in McMahon, A.P. et al., (1992) Cell 69: 581-595.

β Staining of WEXP2-lacZ embryos with βwas performed according to Whiting, J. et al., (1991) Genes & Dev. 5: 2048-2059. General histological analysis of wildtype and WEXP2-*CShh* transgenic embryos was performed on paraffin sections of Bouin's fixed embryos counterstained with hematoxylin and eosin. Histological procedures were as described by Kaufman, M.H. (1992) The Atlas of Mouse Development, London: Academic Press. Sections were photographed on a Leitz Aristoplan compound microscope using Kodak EPY 64T color slide film.

### DAY Constructs For Transgenics

Genomic *Wnt-l* fragments were obtained by screening a λGEM12 (Promega) 129/Sv mouse genomic library with a 375 bp *Mlu*I*-Bgl*II fragment derived from the fourth exon of the murine *Wnt-l* gene. One of the clones (W1-15.1) was used in this study.

As an initial step towards the generation of the pWEXP2 expression vector, W1-15.1 was digested to completion with restriction enzymes *Aat*II and *Cla*I, and a 2774 bp *Aat*II-*Cla*I fragment isolated. This fragment was ligated into *Aat*II and *Cla*I cut pGEM-7Zf vector (Promega), generating pW1-18. This plasmid was digested with *Hind*II and ligated to annealed oligonucleotides *lacl* (SEQ ID No:21) and *lac2* (SEQ ID No:22) generating pW1-18S* which has a modified polylinker downstream of the *Cla*I restriction site. This construct (pW1-18S*) was digested with *Cla*I and *Bgl*II and ligated with both the 2.5 kb 3' *Cla*I*-Bgl*II exon-intron region and 5.5 kb 3' *Bgl*II *-Bgl*II W*nt-1* enhancer, generating pWRES4. This construct contains a 10.5 kb genomic region which starts upstream of the *Wnt-1* translation initiation codon (at an AatII site approximately 1.0kb from the ATG) and extends to a *Bgl*II site 5.5 kb downstream of the *Wnt-l* polyadenylation signal. This plasmid also contains a 250 bp region of the neomycin phosphotransferase (neo) gene inserted in inverse orientation in the 3' transcribed but untranslated region. Finally, to generate the WEXP2 expression vector, a 2 kb *Sfi* I fragment was amplified from pWRES4 using Sf-1 (SEQ ID No:23) and Sf-2 (SEQ ID No:24) oligonucleotides. This amplified fragment was digested with *Sfi* I and inserted into *Sfi* I linearized pWRES4, generating pWEXP2. This destroys the *Wnt-l* translation initiation codon, and replaces it by a polylinker containing *Nru* I, *Eco* RV, *Sac* II, and *Bst* BI restriction sites, which are unique in pWEXP2.

The WEXP2 - *lac*Z construct was obtained by inserting an end-filled *Bgl* II - *Xho* I *lac*Z fragment isolated from the pSDKlacZpA vector in the *Nru* I cut pWEXP2 expression vector. Similarly, the WEXP2 - *CShh* construct was obtained by inserting an end-filled *Xba*I cDNA fragment containing the full Chick *Shh* coding sequence (SEQ ID No:1) into the *Nru* I cut WEXP2 expression vector.
Oligonucleotide sequences are as follows:
lac1: 5'-AGCTGTCGACGCGGCCGCTACGTAGGTTACCGACGTCAAGCTTAGATCTC-3'
lac2: 5'-AGCTGAGATCTAAGCTTGACGTCGGTAACCTACGTAGCGGCCGCGTCGAC-3'
Sf-1: 5'-GATCGGCCAGGCAGGCCTCGCGATATCGTCACCGCGGTATTCGAA-3'
Sf-2: 5'-AGTGCCAGTCGGGGCCCCCAGGGCCGCGCC-3'

### Production And Genotyping Of Transgenic Embryos

Transgenic mouse embryos were generated by microinjection of linear DNA fragments into the male pronucleus of B6CBAF1/J (C57BL/6J X CBA/J) zygotes. CD-1 or B6CBAF1/J females were used as recipients for injected embryos. Go mice embryos were collected at 9.5, 10.5, and 11.5 dpc, photographed using an Olympus SZH stereophoto-microscope on Kodak EPY-64T color slide film, then processed as described earlier.

WEXP2-*lacZ* and WEXP2-*CShh* transgenic embryos were identified by PCR analysis of proteinase-K digests of yolk sacs. Briefly, yolk sacs were carefully dissected free from maternal and embryonic tissues, avoiding cross-contamination between littermates, then washed once in PBS. After overnight incubation at 55°C in 50 µl of PCR proteinase-K digestion buffer (McMahon, A.P. et al., (1990) Cell 62: 1073-1085). I µl of heat-inactivated digest was subjected to polymerase chain reaction (PCR) in a 20 µl volume for 40 cycles as follows: 94°C for 30 seconds, 55°C for 30 seconds, 72°C for 1 minute, with the reaction ingredients described previously (McMahon, A.P. et al., (1990) Cell 62: 1073-1085)). In the case of the WEXP2 - *lac*Z transgenic embryos, oligonucleotides 137 (SEQ ID No:25) and 138 (SEQ ID No:26) amplify a 352 bp *lac*Z specific product. In the case of the WEXP2-*CShh* embryos, oligonucleotides WPR2 (*Wnt-1*-specific) (SEQ ID No:27) and 924 (Chick *Shh*-specific) (SEQ ID No:28) amplify a 345 bp fragment spanning the insertion junction of the Chick-*Shh* cDNA in the WEXP2 expression vector. Table 2 summarizes the results of WEXP2-C-*Shh* transgenic studies.
Oligonucleotide sequences are as follows:
137: 5'-TACCACAGCGGATGGTTCGG-3'
138: 5'-GTGGTGGTTATGCCGATCGC-3'
WPR2: 5'-TAAGAGGCCTATAAGAGGCGG-3'
924: 5'-AAGTCAGCCCAGAGGAGACT-3'

### (ii) Mouse hh Genes

The combined screening of mouse genomic and 8.5 day post coitum (dpc) cDNA libraries identified three mammalian *hh* counterparts (Figure 5A) which herein will be referred to as *Desert, Indian* and *Sonic hedgehog (Dhh, Ihh* and *Shh,* respectively). Sequences encoding *Dhh* (SEQ ID No:2) were determined from analysis of clones identified by low stringency screening of a mouse genomic library. DNA sequencing of one of five overlapping *lambda phage* clones identified three homologous regions encoding a single open reading frame interrupted by introns in identical position to those of the Dros-HH gene (Figure 5A). Splicing across the exon 1/2 boundary was confirmed by polymerase chain reaction (PCR) amplification of first strand cDNA generated from adult testicular RNA. The partial sequence of *Ihh* (SEQ ID No:3) and the complete sequence of *Shh* (SEQ ID No:4) coding regions were determined from the analysis of overlapping cDNA clones isolated from 8.5 dpc cDNA libraries. The longest *Shh* clone, 2.6kb, appears to be full length when compared with the *Shh* transcript present in embryonic RNAs. The 1.8kb partial length *Ihh* cDNA is complete at the 3' end, as evidenced by the presence of a polyadenylation consensus sequence and short poly A tail.

Alignment of the predicted Dros-HH protein sequence (SEQ ID No:34) with those of the mouse *Dhh* (SEQ ID No:9), *Ihh* (SEQ ID No:10) and *Shh* (SEQ ID No: 11), and chick *Shh* (SEQ ID No:8) and zebrafish *Shh* (SEQ ID No: 12), reveals several interesting features of the *hh*-family (Figure 5A). All the vertebrate *hh*-proteins contain an amino terminal hydrophobic region of approximately 20 amino acids immediately downstream of the initiation methionine. Although the properties of these new *hh* proteins have not been investigated, it is likely that this region constitutes a signal peptide and vertebrate *hhs* are secreted proteins. Signal peptide cleavage is predicted to occur (von Heijne, G., (1986) Nucleic Acids Research 14: 4683-4690) just before an absolutely conserved six amino acid stretch, CGPGRG (SEQ ID No:29) (corresponding to residues 85-90)(Figure 5A), in all *hh* proteins. This generates processed mouse *Dhh* (SEQ ID No:9) and *Shh* (SEQ ID No: 11) proteins of 41 and 44 kd, respectively. Interestingly, Dros-HH (SEQ ID No:34) is predicted to contain a substantial amino terminal extension beyond the hydrophobic domain suggesting that the *Drosophila* protein enters the secretory pathway by a type II secretory mechanism. This would generate a transmembrane tethered protein which would require subsequent cleavage to release a 43 kd secreted form of the protein. *In vitro* analysis of Dros-HH is consistent with this interpretation (Lee, J.J. et al., (1992) Cell 71: 33-50). However, there also appears to be transitional initiation at a second methionine (position 51 of SEQ ID No:34) just upstream of the hydrophobic region (Lee, J.J. et al., (1992) Cell 71: 33-50), suggesting that Dros-HH, like its vertebrate counterparts, may also be secreted by recognition of a conventional amino terminal signal peptide sequence.

Data base searches for protein sequences related to vertebrate *hh's* failed to identify any significant homologies, excepting Dros-HH. In addition, searching the "PROSITE" data bank of protein motifs did not reveal any peptide motifs which are conserved in the different *hh* proteins. Thus, the *hhs* represent a novel family of putative cell signaling molecules.

One feature of the amino acid alignment is the high conservation of *hh* sequences. Vertebrate *hhs* share 47 to 51% amino acid identity with Dros-HH throughout the predicted processed polypeptide sequence (Figure 6). *Dhh* has a slightly higher identity than that of *Ihh* and *Shh* suggesting that *Dhh* may be the orthologue of Dros-HH. Conservation is highest in the amino terminal half of the proteins, indeed, from position 85 (immediately after the predicted shared cleavage site) to 249, 62% of the amino acids are completely invariant amongst the *Drosophila* and vertebrate proteins. Comparison of mouse *Dhh, Ihh* and *Shh* where their sequences overlap in this more conserved region, indicates that *Ihh* and *Shh* are more closely related (90% amino acid identity; residues 85 to 266) than with the *Dhh* sequence (80% amino acid identity; residues 85 to 266). Thus, *Ihh* and *Shh* presumably resulted from a more recent gene duplication event.

Comparison of cross species identity amongst *Shh* proteins reveals an even more striking sequence conservation. Throughout the entire predicted processed sequence mouse and chick *Shh* share 84% of amino acid residues (Figure 6). However, in the amino terminal half (positions 85 to 266) mouse and chick are 99% and mouse and zebrafish 94% identical in an 180 amino acid stretch. Conservation falls off rapidly after position 266 (Figure 5A). SEQ ID No:40 shows the consensus sequence in the amino terminal half of all vertebrate *Shh* genes (human, mouse, chicken and zebrafish) identified to date. SEQ ID No:41 shows the consensus sequence in the amino terminal half of vertebrate *hedgehog* genes (*Shh, Ihh,* and *Dhh*) identified to date in different species (mouse, chicken, human and zebrafish).

In summary, *hh* family members are likely secreted proteins consisting of a highly conserved amino terminal and more divergent carboxyl terminal halves. The extreme interspecies conservation of the vertebrate *Shh* protein points to likely conservation of *Shh* function across vertebrate species.

### (iii) Expression of Mouse Shh at the Axial Midline

Expression *of Shh* in the mouse was examined in order to explore the role of mouse *Shh* (SEQ ID No: 11) in vertebrate development. Northern blots of embryonic and adult RNA samples were probed with a radiolabelled mouse *Shh* cDNA probe. An *Shh* transcript of approximately 2.6kb was detected in 9.5dpc whole embryo RNA, and 9.5 and 10.5dpc brain RNA fractions. No expression was detected in total RNA samples from later embryonic stages. Of the late fetal and adult tissue RNAs examined *Shh* expression was only detected in 16.5dpc and adult lung.

To better define the precise temporal and spatial expression of *Shh* an extensive series of whole mount and serial section *in situ* hybridizations were performed using digoxygenin and ³⁵S-radiolabelled RNA probes, respectively, to mouse embryo samples from 7.25dpc (mid streak egg cylinder stage of gastrulation) to 13.5dpc. No *Shh* expression is detected at mid-gastrulation stages (7.25dpc) prior to the appearance of the node, the mouse counterpart of the amphibian organizer and chick Hensen's node. When the primitive streak is fully expended and the midline mesoderm of the head process is emerging from the node (7.5 to 7.75dpc), *Shh* is expressed exclusively in the head process. At late head fold stages, *Shh* is expressed in the node and midline mesoderm of the head process extending anteriorly under the presumptive brain. Just prior to somite formation, *Shh* extends to the anterior limit of then midline mesoderm, underlying the presumptive midbrain. As somites are formed, the embryonic axis extends caudally. The notochord, which represents the caudal extension of the head process, also expresses *Shh,* and expression is maintained in the node.

Interestingly, by 8 somites (8.5dpc) strong *Shh* expression appears in the CNS. Expression is initiated at the ventral midline of the midbrain, above the rostral limit of the head process. By 10 somites CNS expression in the midline extends rostrally in the forebrain and caudally into the hindbrain and rostral spinal cord. Expression is restricted in the hindbrain to the presumptive floorplate, whereas midbrain expression extends ventro-laterally. In the forebrain, there is no morphological floor plate, however ventral *Shh* expression here is continuous with the midbrain. By 15 somites ventral CNS expression is continuous from the rostral limit of the diencephalon to the presumptive spinal cord in somitic regions. Over the next 18 to 24 hrs, to the 25-29 somite stage, CNS expression intensifies and forebrain expression extends rostral to the optic stalks. In contrast to all other CNS regions, in the rostral half of the diencephalon, *Shh* is not expressed at the ventral midline but in two strips immediately lateral to this area which merge again in the floor of the forebrain at its rostral limit. Expression of *Shh* in both the notochord and floorplate is retained until at least 133.5dpc.

Several groups have recently reported the cloning and expression of vertebrate members of a family of transcription factors, related to the *Drosophila forkhead* gene. One of these, *HNF-3*β shows several similarities in expression to *Shh* (Sasaki, H. et al., (1993) Development 118: 47-59) suggesting that *HNF-3*β may be a potential regulator of *Shh.* To investigate this possibility, direct comparison of *HNF-3*β and *Shh* expression was undertaken. *HNF-3*β transcripts are first detected in the node (as previously reported by Sasaki, H. et al., (1993) *supra*), prior to the emergence of the head process and before *Shh* is expressed. From the node, expression proceeds anteriorly in the head process, similar to *Shh* expression. Activation of *HNF-3*β within the CNS is first observed at 2-3 somites, in the presumptive mid and hindbrain, prior to the onset of *Shh* expression. By 5 somites, expression in the midbrain broadens ventro-laterally, extends anteriorly into the forebrain and caudally in the presumptive floor plate down much of the neuraxis in the somitic region. Strong expression is maintained at this time in the node and notochord. However, by 10 somites expression in the head process is lost and by 25-29 somites notochordal expression is only present in the most extreme caudal notochord. In contrast to the transient expression of *HNF-3*β in the midline mesoderm, expression in the floor plate is stably retained until at least 11.5dpc. Thus, there are several spatial similarities between the expression of *HNF-3*β and *Shh* in both the midline mesoderm and ventral CNS and it is likely that both genes are expressed in the same cells. However, in both regions, *HNF-3*β expression precedes that of *Shh.* The main differences are in the transient expression of *HNF-3*β in the head process and notochord and *Shh* expression in the forebrain. Whereas *HNF-3*β and *Shh* share a similar broad ventral and ventral lateral midbrain and caudal diencephalic expression, only *Shh* extends more rostrally into the forebrain. In general, these results are consistent with a model in which initial activation of *Shh* expression may be regulated by *HNF-3*β.

The similarity in *Shh* and *HNF-3*β expression domains is also apparent in the definitive endoderm which also lies at the midline. Broad *HNF-3*β expression in the foregut pocket is apparent at 5 somites as previously reported by Sasaki, H. et al., (1993) *supra. Shh* is also expressed in the endoderm, immediately beneath the forebrain. Both genes are active in the rostral and caudal endoderm from 8 to 11 somites. Whereas *HNF-3*β is uniformly expressed, *Shh* expression is initially restricted to two ventro-lateral strips of cells. Ventral restricted expression of *Shh* is retained in the most caudal region of the presumptive gut until at least 9.5dpc whereas *HNP-3*β is uniformly expressed along the dorso-ventral axis. Both genes are expressed in the pharyngeal ectoderm at 9.5dpc and expression is maintained in the gut until at least 11.5dpc. Moreover, expression of *Shh* in the embryonic and adult lung RNA suggests that endodermal expression of *Shh* may continue in, at least some endoderm derived organs.

### (iv) Expression Of Shh In The Limb

Expression of *Shh* is not confined to midline structures. By 30-35 somites (9.75dpc), expression is detected in a small group of posterior cells in the forelimb bud. The forelimb buds form as mesenchymal outpocketings on the flanks, opposite somites 8 to 12, at approximately the 17 to 20 somite stage. *Shh* expression is not detectable in the forelimbs until about 30-35 somites, over 12 hours after the initial appearance of the limbs. Expression is exclusively posterior and restricted to mesenchymal cells. By 10.5dpc, both the fore and hindlimbs have elongated substantially from the body flank. At this time *Shh* is strongly expressed in the posterior, distal aspect of both limbs in close association with the overlying ectoderm. Analysis of sections at this stage detects *Shh* expression in an approximately six cell wide strip of posterior mesenchymal cells. In the forelimb, *Shh* expression ceases by 11.5dpc. However, posterior, distal expression is still detected in the hindlimb. No limb expression is detected beyond 12.5dpc.

### (v) Ectopic Expression Of Shh

Grafting studies carried out principally in the chick demonstrate that cell signals derived from the notochord and floor plate pattern the ventral aspect of the CNS (as described above). In the limb, a transient signal produced by a group of posterior cells in both limb buds, the zone of polarizing activity (ZPA), is thought to regulate patterning across the anterior-posterior axis. Thus, the sequence of *Shh,* which predicts a secreted protein and the expression profile in midline mesoderm, the floor plate and in the limb, suggest that *Shh* signaling may mediate pattern regulation in the ventral CNS and limb.

To determine whether *Shh* may regulate ventral development in the early mammalian CNS, a *Wnt-l* enhancer was used to alter its normal domain of expression. *Wnt-l* shows a dynamic pattern of expression which is initiated in the presumptive midbrain just prior to somite formation. As the neural folds elevate and fuse to enclose the neural tube, *Wnt-l* expression in the midbrain becomes restricted to a tight circle, just anterior of the midbrain, the ventral midbrain and the dorsal midline of the diencephalon, midbrain, myelencephalon and spinal cord (Wilkinson, D.G. et al., (1987) Cell 50: 79-88; McMahon, A.P. et al., (1992) Cell 69: 581-595; Parr, B.A. et al., (1993) Development 119: 247-261).

It was determined that essentially normal expression of *lacZ* reporter constructs within the *Wnt-l* expression domain is dependent upon a 5.5kb enhancer region which lies downstream of the *Wnt-1* polyadenylation sequence. A construct was generated for ectopic expression of cDNA clones in the *Wnt-l* domain and tested in transgenics using a *lacZ* reporter (pWEXP-lacZ; Figure 9). Two of the four Go transgenic embryos showed readily detectable β-galactosidase activity, and in both expression occurred throughout the normal *Wnt-l* expression domain. More extensive studies with a similar construct also containing the 5.5kb enhancer gave similar frequencies. Some ectopic expression was seen in newly emerging neural crest cells, probably as a result of perdurance of β-galactosidase RNA or protein in the dorsally derived crest. Thus, the *Wnt-l* expression construct allows the efficient ectopic expression of cDNA sequences in the midbrain and in the dorsal aspect of much of the CNS.

An *Shh* ectopic expression construct (pWEXP-CShh) containing two tandem head to tail copies of a chick *Shh* cDNA was generated (Figure 7). By utilizing this approach, ectopic expression of the chick *Shh* is distinguishable from that of the endogenous mouse *Shh* gene. Chick *Shh* shows a high degree of sequence identity and similar expression to the mouse gene. Thus, it is highly likely that *Shh* function is widely conserved amongst vertebrates, a conclusion further supported by studies of the same gene in zebrafish.

Table 2 shows the results of several transgenic experiments in which the Go population was collected at 9.5 to 11.5dpc. Approximately half of the transgenic embryos identified at each stage of development had a clear, consistent CNS phenotype. As we expect, on the basis of control studies using the 5.5kb *Wnt-l* enhancer, that only half the transgenics will express the transgene, it is clear that in most embryos ectopically expressing chick *Shh,* an abnormal phenotype results.

**TABLE 2**

| | | | |
|---|---|---|---|
| Summary of WEXP2-Chick *Shh* transgenic studies | | | |

| Age (dpc) | Number of Embryos | Number of Transgenics | Number of Embryos with CNS phenotype^{a} |
|---|---|---|---|
| | | | |
| 9.5 | 37 | 11 | 6 (54.5%) |
| | | | |
| 10.5 | 59 | 16 | 8 (50%) |
| | | | |
| 11.5 | 33 | 7 | 3 (42.9%) |

| | | | |
|---|---|---|---|
| Figures in parentheses, refer to the percentage of transgenic embryos with a CNS phenotype ^{a} In addition one 9.5pc and two 10.5pc transgenic embryos showed non-specific growth retardation, as occurs at low frequency in transgenic studies. These embryos were excluded from further analysis. | | | |

At 9.5dpc, embryos with a weaker phenotype show an open neural plate from the mid diencephalon to the myelencephalon. In embryos with a stronger phenotype at the same stage, the entire diencephalon is open and telencephalic and optic development is morphologically abnormal. As the most anterior diencephalic expression of *Wnt-l* is lower than that in more caudal regions, the differences in severity may relate to differences in the level of chick *Shh* expression in different Go embryos. At the lateral margins of the open neural folds, where *Wnt-l* is normally expressed, there is a thickening of the neural tissue extending from the diencephalon to myelencephalon. The cranial phenotype is similar at 10.5 and 11.5 dpc. However, there appears to be a retardation in cranial expansion of the CNS at later stages.

In addition to the dorsal cranial phenotype, there is a progressive dorsal phenotype in the spinal cord. At 9.5 dpc, the spinal cord appears morphologically normal, except at extreme rostral levels. However by 10.5dpc, there is a dorsal dysmorphology extending to the fore or hindlimbs. By 11.5 dpc, all transgenic embryos showed a dorsal phenotype along almost the entire spinal cord. Superficially, the spinal cord had a rippled, undulating appearance suggestive of a change in cell properties dorsally. This dorsal phenotype, and the cranial phenotype were examined by histological analysis of transgenic embryos.

Sections through a 9.5dpc embryo with an extreme CNS phenotype show a widespread dorsal perturbation in cranial CNS development The neural/ectodermal junction in the diencephalon is abnormal. Neural tissue, which has a columnar epithelial morphology quite distinct from the squamous epithelium of the surface ectoderm, appears to spread dorsolaterally. The myelencephalon, like the diencephalon and midbrain, is open rostrally. Interestingly, there are discontinuous dorso-lateral regions in the myelencephalon with a morphology distinct from the normal roof plate regions close to the normal site of *Wnt-l* expression. These cells form a tight, polarized epithelium with basely located nuclei, a morphology similar to the floor plate and distinct from other CNS regions. Differentiation of dorsally derived neural crest occurs in transgenic embryos as can be seen from the presence of cranial ganglia. In the rostral spinal cord, the neural tube appeared distended dorsolaterally which may account for the superficial dysmorphology.

By 11.5dpc, CNS development is highly abnormal along the entire dorsal spinal cord to the hindlimb level. The dorsal half of the spinal cord is enlarged and distended. Dorsal sensory innervation occurs, however, the neuronal trajectories are highly disorganized. Most obviously, the morphology of dorsal cells in the spinal cord, which normally are elongated cells with distinct lightly staining nuclei and cytoplasm, is dramatically altered. Most of the dorsal half of the spinal cord consists of small tightly packed cells with darkly staining nuclei and little cytoplasm. Moreover, there appears to be many more of these densely packed cells, leading to abnormal outgrowth of the dorsal CNS. In contrast, ventral development is normal, as are dorsal root ganglia, whose origins lie in neural cells derived from the dorsal spinal cord.

### (vi) Ectopic Shh Expression Activates Floor Plate Gene Expression

To determine whether ectopic expression of chick *Shh* results in inapproppriate activation of a ventral midline development in the dorsal CNS, expression of two floor plate expressed genes, HNF-3β and mouse *Shh,* were examined. Whole mounts of 9.5dpc transgenic embryos show ectopic expression of HNF-3β throughout the cranial *Wnt-l* expression domain. In addition to normal expression at the ventral midline, HNF-3β transcripts are expressed at high levels, in a circle just rostral to the mid/hindbrain junction, along the dorsal (actually lateral in unfused brain folds) aspects of the midbrain and, more weakly, in the roof plate of the myelencephalon. No expression is observed in the metencephalon which does not express *Wnt-l.* Thus, ectopic expression of *Shh* leads to the activation of HNF-3β throughout the cranial *Wnt-l* expression domain.

The relationship between chick *Shh* expression and the expression of HNF-3β in serial sections was also examined. Activation of HNF-3β in the brain at 9.5 and 10.5dpc is localized to the dorsal aspect in good agreement with the observed ectopic expression of chick *Shh.* Interestingly mouse *Shh* is also activated dorsally. Thus, two early floor plate markers are induced in response to chick *Shh.*

From 9.5dpc to 11.5dpc, the spinal cord phenotype becomes more severe. The possibility that activation of a floor plate pathway may play a role in the observed phenotype was investigated. In contrast to the brain, where ectopic HNF-3β and *Shh* transcripts are still present, little or no induction of these floor plate markers is observed. Thus, although the dorsal spinal cord shows a widespread transformation in cellular phenotype, this does not appear to result from the induction of floor plate development.

### Example 3

### Chick Sonic Hedgehog Mediates ZPA Activity

### (i) Experimental Procedures

### Retinoic Acid Bead Implants

Fertilized white Leghorn chicken eggs were incubated to stage 20 and then implanted with AG1-X2 ion exchange beads (Biorad) soaked in 1 mg/ml retinoic acid (RA, Sigma) as described by Tickle, C. et al., (1985) Dev. Biol 109: 82-95. Briefly, the beads were soaked for 15 min in 1mg/ml RA in DMSO, washed twice and implanted under the AER on the anterior margin of the limb bud. After 24 or 36 hours, some of the implanted embryos were harvested and fixed overnight in 4% paraformaldehyde in PBS and then processed for whole mount in situ analysis as previously described. The remainder of the animals were allowed to develop to embryonic day 10 to confirm that the dose of RA used was capable of inducing mirror image duplications. Control animals were implanted with DMSO soaked beads and showed no abnormal phenotype or gene expression.

### Plasmids

Unless otherwise noted, all standard cloning techniques were performed according to Ausubel, F.M. et al., (1989) Current Protocols in Molecular Biology (N.Y.: Greene Publishing Assoc. and Wiley Inerscience), and all enzymes were obtained from Boehringer Mannheim Biochemicals. pHH-2 is a cDNA contain the entire coding region of chicken *Sonic hedgehog* (SEQ ID No:1). RCASBP(A) and RCASBP(E) are replication-competent retroviral vectors which encode viruses with differing host ranges. RCANBP(A) is a variant of RCASBP(A) from which the second splice acceptor has been removed. This results in a virus which can not express the inserted gene and acts as a control for the effects of viral infection (Hughes, S.H. et al., (1987) J. Virol. 61: 3004-3012; Fekete, D. et al., (1993) Mol. Cell. Biol. 13: 2604-2613). RCASBP/AP(E) is version of RCASBP(E) containing a human placental alkaline phosphatase cDNA (Fekete, D. et al., (1993b) Proc. Natl. Acad Sci. USA 90: 2350-2354). SLAX13 is a pBluescript SK+ derived plasmid with a second Cla I restriction site and the 5' untranslated region of v-src (from the adaptor plasmid CLA12-Nco, Hughes, S.H. et al., (1987) J. Virol. 61: 3004-3012) cloned 5' of the EcoRI (and ClaI) site in the pBluescript polylinker. RCASBP plasmids encoding *Sonic* from either the first (M1) or second (M2) methionine (at position 4) were constructed by first shuttling the 1.7kb. *Sonic* fragment of pHH-2 into SLAX-113 using oligonucleotides to modify the 5' end of the cDNA such that either the first or second methionine is in frame with the NcoI site of SLAX-13. The amino acid sequence of *Sonic* is not mutated in these constructs. The M1 and M2 *Sonic* ClaI fragments (v-src 5'UTR:*Sonic*) were each then subcloned into RCASBP(A), RCANBP(A) and RCASBP(E), generating Sonic/RCAS-A1, Sonic/RCAS-A2, Sonic/RCAN-A1, Sonic/RCAN-A2, Sonic/RCAS-E1 and Sonic/RCAS-E2.

### Chick Embryos, Cell Lines And Virus Production

All experimental manipulations were performed on standard specific-pathogen free White Leghorn chick embryos (S-SPF) from closed flocks provided fertilized by SPAFAS (Norwich, Conn). Eggs were incubated at 37.5°C and staged according to Hamburger, V. et al., (1951) J. Exp. Morph 88: 49-92. All chick embryo fibroblasts (CEF) were provided by C. Cepko. S-SPF embryos and CEFs have previously been shown to be susceptible to RCASBP(A) infection but resistant to RCASBP(E) infection (Fekete, D. et al., (1993b) Proc. Natl. Acad Sci. USA 90: 2350-2354). Line 15b CEFs are susceptible to infection by both RCASBP(A) and (E). These viral host ranges were confirmed in control experiments. CEF cultures were grown and transfected with retroviral vector DNA as described (Morgan, B.A. et al., (1993) Nature 358: 236-239; Fekete, D. et al., (1993b) Proc. Natl. Acad Sci. USA 90: 2350-2354). All viruses were harvested and concentrated as previously described (Morgan, B.A. et al., (1993) Nature 358: 236-239; Fekete, D. et al., (1993b) Proc. Natl. Acad Sci. USA 90: 2350-2354) and had titers of approximately 10⁸ cfu/ml.

### Cell Implants

A single 60mm dish containing line 15b CEFs which had been infected with either RCASBP/AP(E), Sonic/RCAS-E1 or Sonic/RCAS-E2 were grown to 50-90% confluence, lightly trypsinized and then spun at 1000 rpm for 5 min in a clinical centrifuge. The pellet was resuspended in 1 ml media, transferred to a microcentrifuge tube and then micro centrifuged for 2 min at 2000 rpm. Following a 30 min incubation at 37° C, the pellet was respun for 2 min at 2000 rpm and then lightly stained in media containing 0.01% nile blue sulfate. Pellet fragments of approximately 300µm x 100µm x 50µm were implanted as a wedge to the anterior region of *hh* stage 19-23 wing buds (as described by Riley, B.B. et al.; (1993) Development 118: 95-104). At embryonic day 10, the embryos were harvested, fixed in 4% paraformaldehyde in PBS, stained with alcian green, and cleared in methyl salicylate (Tickle, C. et al., (1985) Dev. Biol 109: 82-95).

### Viral Infections

Concentrated Sonic/RCAS-A2 or Sonic/RCAN-A2 was injected under the AER on the anterior margin of stage 20-22 wing buds. At 24 or 36 hours post-infection, the embryos were harvested, fixed in 4% paraformaldehyde in PBS and processed for whole mount in situ analysis as previously described.

### (ii) Co-Localization Of Sonic Expression And Zpa Activity

ZPA activity has been carefully mapped both spatially and temporally within the limb bud (Honig, L.S. et al., (1985) J. Embryol. exp. Morph. 87: 163-174). In these experiments small blocks of limb bud tissue from various locations and stages of chick embryogenesis (Hamburger, V et al., (1951) J. Exp. Morph. 88: 49-92) were grafted to the anterior of host limb buds and the strength of ZPA activity was quantified according to degrees of digit duplication. Activity is first weakly detected along the flank prior to limb bud outgrowth. The activity first reaches a maximal strength at stage 19 in the proximal posterior margin of the limb bud. By stage 23 the activity extends the full length of the posterior border of the limb bud. The activity then shifts distally along the posterior margin so that by stage 25 it is no longer detectable at the base of the flank. The activity then fades distally until it is last detected at stage 29.

This detailed map of endogenous polarizing activity provided the opportunity to determine the extent of the correlation between the spatial pattern of ZPA activity and *Sonic* expression over a range of developmental stages. Whole mount *in situ* hybridization was used to assay the spatial and temporal pattern of *Sonic* expression in the limb bud. *Sonic* expression is not detected until stage 17, at the initiation of limb bud formation, at which time it is weakly observed in a punctate pattern reflecting a patchy expression in a few cells. From that point onwards the *Sonic* expression pattern exactly matches the location of the ZPA, as determined by Honig, L.S. et al., (1985) J. Embryol. exp. Morph. 87: 163-174, both in position and in intensity of expression.

### (iii) Induction Of Sonic Expression By Retinoic Acid

A source of retinoic acid placed at the anterior margin of the limb bud will induce ectopic tissue capable causing mirror-image duplications (Summerbell, D. et al., (1983) In Limb Development and Regeneration (N.Y.: Ala R. Liss) pp. 109-118; Wanek, N. et al., (1991) Nature 350: 81-83). The induction of this activity is not an immediate response to retinoic acid but rather takes approximately 18 hours to develop (Wanek, N. et al., (1991) Nature 350: 81-83). When it does develop, the polarizing activity is not found surrounding the implanted retinoic acid source, but rather is found distal to it in the mesenchyme along the margin of the limb bud (Wanek, N. et al., (1991) Nature 350: 81-83).

If *Sonic* expression is truly indicative of ZPA tissue, then it should be induced in the ZPA tissue which is ectopically induced by retinoic acid. To test this, retinoic acid-soaked beads were implanted in the anterior of limb buds and the expression of *Sonic* after various lengths of time using whole-mount *in situ* hybridization was assayed. As the limb bud grows, the bead remains imbedded proximally in tissue which begins to differentiate. Ectopic *Sonic* expression is first detected in the mesenchyme 24 hours after bead implantation. This expression is found a short distance from the distal edge of the bead. By 36 hours *Sonic* is strongly expressed distal to the bead in a stripe just under the anterior ectoderm in a mirror-image pattern relative to the endogenous *Sonic* expression in the posterior of the limb bud.

### (iv) Affects Of Ectopic Expression Of Sonic On Limb Patterning

The normal expression pattern of *Sonic,* as well as that induced by retinoic acid, is consistent with *Sonic* being a signal produced by the ZPA. To determine whether *Sonic* expression is sufficient for ZPA activity, the gene was ectopically expressed within the limb bud. In most of the experiments we have utilized a variant of a replication-competent retroviral vector called RCAS (Hughes, S.H. et al., (1987) J. Virol. 61: 3004-3012)) both as a vehicle to introduce the *Sonic* sequences into chick cells and to drive their expression. The fact that there exists subtypes of avian retroviruses which have host ranges restricted to particular strains of chickens was taken advantage of to control the region infected with the Sonic/RCAS virus (Weiss, R. (et al.) (1984) RNA Tumor Viruses, Vol. 1 Weiss et al. eds., (N.Y.: Cold Spring Harbor Laboratories) pp. 209-260*);* Fekete, D. et al., (1993a) Mol. Cell. Biol. 13: 2604-2613). Thus a vector with a type E envelope protein (RCAS-E, Fekete, D. et al., (1993b) Proc. Nat/. Acad Sci. USA 90: 2350-2354) is unable to infect the cells of the SPAFAS outbred chick embryos routinely used in our lab. However, RCAS-E is able to infect cells from chick embryos of line 15b. In the majority of experiments, primary chick embryo fibroblasts (CEFs) prepared from line 15b embryos *in vitro* were infected. The infected cells were pelleted and implanted into a slit made in the anterior of S-SPF host limb buds. Due to the restricted host range of the vector, the infection was thus restricted to the graft and did not spread through the host limb bud.

To determine the fate of cells implanted and to control for any effect of the implant procedure, a control RCAS-E vector expressing human placental alkaline phosphatase was used. Alkaline phosphatase expression can be easily monitored histochemically and the location of infected cells can thus be conveniently followed at any stage. Within 24 hours following implantation the cells are dispersed proximally and distally within the anterior margin of the limb bud. Subsequently, cells are seen to disperse throughout the anterior portion of the limb and into the flank of the embryo.

Limb buds grafted with alkaline phosphatase expressing cells or uninfected cells give rise to limbs with structures indistinguishable from unoperated wild type limbs. Such limbs have the characteristic anterior-to-posterior digit pattern 2-3-4. ZPA grafts give rise to a variety of patterns of digits depending on the placement of the graft within the bud (Tickle, C. et al., (1975) Nature 254: 199-202) and the amount of tissue engrafted (Tickle, C. (1981) Nature 289: 295-298). In some instances the result can be as weak as the duplication of a single digit 2. However, in optimal cases the ZPA graft evokes the production of a full mirror image duplication of digits 4-3-2-2-3-4 or 4-3-2-3-4 (see Figure 8). A scoring system has been devised which rates the effectiveness of polarizing activity on the basis of the most posterior digit duplicated: any graft which leads to the development of a duplication of digit 4 has been defined as reflecting 100% polarizing activity (Honig, L.S. et al., (1985) J. Embryol. Exp. Morph. 87:163-174).

Grafts of 15b fibroblasts expressing *Sonic* resulted in a range of ZPA-like phenotypes. In some instances the resultant limbs deviate from the wild type solely by the presence of a mirror-image duplication of digit 2. The most common digit phenotype resulting from grafting *Sonic-*infected CEF cells is a mirror-image duplication of digits 4 and 3 with digit 2 missing: 4-3-3-4. In many such cases the two central digits appear fused in a 4-3/3-4 pattern. In a number of the cases the grafts induced full mirror-image duplications of the digits equivalent to optimal ZPA grafts 4-3-2-2-3-4. Besides the digit duplications, the ectopic expression of *Sonic* also gave rise to occasional duplications of proximal elements including the radius or ulna, the humerus and the coracoid. While these proximal phenotypes are not features of ZPA grafts, they are consistent with an anterior-to-posterior respecification of cell fate. In some instances, most commonly when the radius or ulna was duplicated, more complex digit patterns were observed. Typically, an additional digit 3 was formed distal to a duplicated radius.

The mirror-image duplications caused by ZPA grafts are not limited to skeletal elements. For example, feather buds are normally present only along the posterior edge of the limb. Limbs exhibiting mirror-image duplications as a result of ectopic *Sonic* expression have feather buds on both their anterior and posterior edges, similar to those observed in ZPA grafts.

While ZPA grafts have a powerful ability to alter limb pattern when placed at the anterior margin of a limb bud, they have no effect when placed at the posterior margin (Saunders, J.W. et al., (1968) Epithelial-Mesenchymal Interaction, Fleischmayer and Billingham, eds. (Baltimore: Williams and Wilkins) pp. 78-97). Presumably, the lack of posterior effect is a result of polarizing activity already being present in that region of the bud. Consistent with this, grafts of *Sonic* expressing cells placed in the posterior of limb buds never result in changes in the number of digits. Some such grafts did produce distortions in the shape of limb elements, the most common being a slight posterior curvature in the distal tips of digits 3 and 4 when compared to wild type wings.

### (v) Effect Of Ectopic Sonic Expression On Hoxd Gene Activity

The correct expression of *Hoxd* genes is part of the process by which specific skeletal elements are determined (Morgan, B.A. et al., (1993) Nature 358: 236-239). A transplant of a ZPA into the anterior of a chick limb bud ectopically activates sequential transcription of *Hoxd* genes in a pattern which mirrors the normal sequence of *Hoxd* gene expression (Nohno, T. et al., (1991) Cell 64: 1197-1205; Izpisua-Belmonte, J.C. et al., (1991) Nature 350: 585-589). Since ectopic *Sonic* expression leads to the same pattern duplications as a ZPA graft, we reasoned that *Sonic* would also lead to sequential activation of *Hoxd* genes.

To test this hypothesis, anterior buds were injected with Sonic/RCAS-A2, a virus which is capable of directly infecting the host strains of chicken embryos. This approach does not strictly limit the region expressing *Sonic* (being only moderately controlled by the timing, location and titer of viral injection), and thus might be expected to give a more variable result. However, experiments testing the kinetics of viral spread in infected limb buds indicate that infected cells remain localized near the anterior margin of the bud for at least 48 hours.. *Hoxd* gene expression was monitored at various times post infection by whole mount in situ hybridization. As expected, these genes are activated in a mirror-image pattern relative their expression in the posterior of control limbs. For example, after 36 hours *Hoxd-13* is expressed in a mirror-image symmetrical pattern in the broadened distal region of infected limb buds. Similar results were obtained with other *Hoxd* genes (manuscript in preparation).

### Example 4

### A Functionally Conserved Homolog of Drosophila Hedgehog is Expressed in Tissues With Polarizing Activity in Zebrafish Embryos

### (i) Experimental Procedures

### Cloning and Sequencing

Approximately 1.5 x 10⁶ plaques of a 33h zebrafish embryonic λgt11 DNA library were screened by plaque hybridization at low stringency (McGinnis; W. et al., (1984) Nature 308: 428-433) using a mix of two *hh* sequences as a probe: a Drosophila *hh* 400bp EcoRI fragment and a murine *Ihh* 264bp BamHI-EcoRI exon 2 fragment. Four clones were isolated and subcloned into the EcoRI sites of pUC18 T3T7 (Pharmacia). Both strands of clone 8.3 were sequenced using nested deletions (Pharmacia) and internal oligonucleotide primers. DNA sequences and derived amino acid sequences were analyzed using "Geneworks" (Intelligenetics) and the GCG software packages.

### PCR amplification

Degenerate oligonucleotides *hh5.1* (SEQ ID No:30) and *hh*3.3 (SEQ ID No:31) were used to amplify genomic zebrafish DNA
*hh* 5.1: AG(CA)GITG(CT)AA(AG)GA(AG)(CA)(AG)I(GCT)IAA
*hh* 3.3: CTCIACIGCIA(GA)ICK(GT)IGCIA
PCR was performed with an initial denaturation at 94°C followed by 35 cycles of 47°C for 1 min, 72°C for 2min and 94°C for 1 min with a final extension at 72°C. Products were subcloned in ρUC18 (Pharmacia).

### In Situ Hybridization

*In situ* hybridizations of zebrafish embryos were performed as described in Oxtoby, E. et al., (1993) Nuc. Acids REs. 21: 1087-1095 with the following modifications: Embryos were rehydrated in ethanol rather than methanol series; the proteinase K digestion was reduced to 5 min and subsequent washes were done in PBTw without glycine; the antibody was preadsorbed in PBTw, 2mg/ml BSA without sheep serum; and antibody incubation was performed in PBTw, 2mg/ml BSA. Drosophila embryos were processed and hybridized as previously described.

### Histology

Stained embryos were dehydrated through ethanol:butanol series, as previously described (Godsave, S.F. et al., (1988) Development 102: 555-566), and embedded in Fibrowax. 8µm sections were cut on an Anglian rotary microtome

### RNA Probe Synthesis

For analysis of *Shh* expression, two different templates were used with consistent results; (i) *phh[c]* 8.3 linearized with BgI II to transcribe an antisense RNA probe that excludes the conserved region, and (ii) *phh*[c] 8.3 linearized with Hind III to transcribe an antisense RNA that covers the complete cDNA. All *in situ* hybridizations were performed with the latter probe which gives better signal. Other probes were as follows: *Axial* DraI-linearized p6TIN (Strähle, U. et al., (1993) Genes & Dev. 7: 1436-1446) using T3 RNA polymerase. gsc linearized with EcoR1 and transcribed with T7: *pax* 2 Bam HI-linearized pcF16 (Krauss, S. et al., (1991) Development 113: 1193-1206) using T7 RNA polymerase. *In situ* hybridizations were performed using labelled RNA at a concentration of 1 ng/ml final concentration. Antisense RNA probes were transcribed according to the manufacturer's protocol (DIG RNA Labelling Kit, BCL).

### Zebrafish Strains

Wild type fish were bred from a founder population obtained from the Goldfish Bowl, Oxford. The mutant *cyclops* strain b16 and the mutant *notail* strains b160 and b195 were obtained from Eugene, Oregon. Fish were reared at 28°C on a 14h light/10h dark cycle.

### RNA Injections

The open reading frame of *Shh* was amplified by PCR, using oligonucleotides 5'-CTGCAGGGATCCACCATGCGGCTTTTGACGAG-3' (SEQ ID No:32), which contains a consensus Kozak sequence for translation initiation, and 5'-CTGCAGGGATC-CTTATTCCACACGAGGCATT-3' (SEQ ID No:33), and subcloned into the BglII site of pSP64T (Kreig, P.A. et al., (1984) Nuc.Acids Res. 12: 7057-7070). This vector includes 5' and 3' untranslated Xenopus β-Globin sequences for RNA stabilization and is commonly used for RNA injections experiments in Xenopus. *In vitro* transcribed *Shh* RNA at a concentration of approximately 100 µg/ml was injected into a single cell of naturally spawned zebrafish embryos at one-cell to 4-cell stages using a pressure-pulsed Narishige microinjector. The injected volume was within the picolitre range. Embryos were fixed 20 to 27 hrs after injection in BT-Fix (Westerfield, M. (1989) The Zebrafish Book, (Eugene: The University of Oregon Press)) and processed as described above for whole-mount *in situ* hybridizations with the *axial* probe.

### Transgenic Drosophila

An EcoRI fragment, containing the entire *Shh* ORF, was purified from the plasmid *phh*[c]8.3 and ligated with phosphatased EcoR1 digested transformation vector pCaSpeRhs (Thummel, C.S. et al., (1988) Gene 74: 445-56). The recombinant plasmid, pHS *Shh* containing the *Shh* ORF in the correct orientation relative to the heat shock promoter, was selected following restriction enzyme analysis of miniprep DNA from transformed colonies and used to transform Drosophila embryos using standard microinjection procedures (Roberts, D.B. (1986), Drosophila, A Practical Approach, Roberts, D.B., ed., (Oxford: IRL Press) pp. 1-38).

### Ectopic Expression In Drosophila Embryos

Embryos carrying the appropriate transgenes were collected over 2 hr intervals, transferred to thin layers of 1% agarose on glass microscope slides and incubated in a plastic Petri dish floating in a water bath at 37°C for 30 min intervals. Following heat treatment, embryos were returned to 25°C prior to being fixed for *in situ* hybridization with DIG labelled single stranded *Shh, wg* or *ptc* RNA probes as previously described (Ingham et al., (1991) Curr. Opin. Genet. Dev. 1: 261-267).

### (ii) Molecular Cloning Of Zebra fish Hedgehog Homologues

In an initial attempt to isolate sequences homologous to Drosophila *hh*, a zebrafish genomic DNA library was screened at reduced stringency with a partial cDNA, *hh*PCR4.1, corresponding to the first and second exons of the Drosophila gene (Mohler, J. et al., (1992) Development 115: 957-971). This screen proved unsuccessful; however, a similar screen of a mouse genomic library yielded a single clone with significant homology to *hh*., subsequently designated *Ihh.* A 264bp HamHI-EcoRI fragment from this lambda clone containing sequences homologous to the second exon of the Drosophila gene was subcloned and, together with the Drosophila partial cDNA fragment, used to screen a λgt11 zebrafish cDNA library that was prepared from RNA extracted from 33h old embryos. This screen yielded four clones with overlapping inserts the longest of which is 1.6kb in length, herein referred to as *Shh* (SEQ ID No:5).

### (iii) A Family Of Zebrafish Genes Homologous To The Drosophila Segment Polarity Gene, Hedgehog

Alignment of the predicted amino acid sequences of *Shh* (SEQ ID No:12) and *hh* (SEQ ID No:34) revealed an identity of 47%, confirming that *Shh* is a homolog of the Drosophila gene. A striking conservation occurs within exon 2: an 80 amino acid long domain shows 72% identity between *Shh* and Dros-HH. (Figure 9A). This domain is also highly conserved in all *hh*-related genes cloned so far and is therefore likely to be essential to the function of *hh* proteins. A second domain of approximately 30 amino acids close to the carboxy-terminal end, though it shows only 61% amino-acid identity, possesses 83% similarity between *Shh* and *hh* when allowing for conservative substitutions and could also, therefore, be of functional importance (Figure 9B). Although putative sites of posttranslational modification can be noted, their position is not conserved between *Shh* and *hh.*

Lee, J.J. et al., (1992) Cell 71: 33-50, identified a hydrophobic stretch of 21 amino acids flanked downstream by a putative site of signal sequence cleavage (predicted by the algorithm of von Heijne, G. (1986) Nuc. Acids Res. 11) close to the amino-terminal end of *hh.* Both the hydrophobic stretch and the putative signal sequence cleavage sites of *hh,* which suggest it to be a signaling molecule, are conserved in *Shh.* In contrast to *hh, Shh* does not extend N-terminally to the hydrophobic stretch.

Using degenerate oligonucleotides corresponding to amino-acids flanking the domain of high homology between Dros-HH and mouse *Ihh* exons 2 described above, fragments of the expected size were amplified from zebrafish genomic DNA by PCR. After subcloning and sequencing, it appeared that three different sequences were amplified, all of which show high homology to one another and to Dros-HH (Figure 10). One of these corresponds to *Shh* therein referred to as 2-hh(a) (SEQ ID No:16) and 2hh(b) (SEQ ID No:17), while the other two represent additional zebrafish *hh* homologs (SEQ ID No:5). cDNAs corresponding to one of these additional homologs have recently been isolated, confirming that it is transcribed. Therefore, *Shh* represents a member of a new vertebrate gene family.

### (iv) Shh Expression In The Developing Zebrafish Embryo

### Gastrula stages

*Shh* expression is first detected at around the 60% epiboly stage of embryogenesis in the dorsal mesoderm. Transcript is present in a triangular shaped area, corresponding to the embryonic shield, the equivalent of the amphibian organizer, and is restricted to the inner cell layer, the hypoblast. During gastrulation, presumptive mesodermal cells involute to form the hypoblast, and converge towards the future axis of the embryo, reaching the animal pole at approximately 70% epiboly. At this stage, *Shh* -expressing cells extend over the posterior third of the axis, and the signal intensity is not entirely homogeneous, appearing stronger at the base than at the apex of the elongating triangle of cells.

This early spatial distribution of *Shh* transcript is reminiscent of that previously described for *axial,* a *forkhead-related* gene; however, at 80% epiboly, *axial* expression extends further towards the animal pole of the embryo and we do not see *Shh* expression in the head area at these early developmental stages.

By 100% epiboly, at 9.5 hours of development, the posterior tip of the *Shh* expression domain now constitutes a continuous band of cells that extends into the head. To determine the precise anterior boundary of *Shh* expression, embryos were simultaneously hybridized with probes of *Shh* and pax-2 (previously *pax*[*b*]), the early expression domain of which marks the posterior midbrain (Krauss, S. et al. (1991) Development 113: 1193-1206). By this stage, the anterior boundary of the *Shh* expression domain is positioned in the centre of the animal pole and coincides approximately with that of *axial.* At the same stage, prechordal plate cells expressing the homeobox gene *goosecoid* (*gsc*) overlap and underlay the presumptive forebrain (Statchel, S.E. et al., (1993) Development 117: 1261-1274). Whereas *axial* is also thought to be expressed in head mesodermal tissue at this stage, we cannot be certain whether *Shh* is expressed in the same cells. Sections of stained embryos suggest that in the head *Shh* may by this stage be expressed exclusively in neuroectodermal tissue.

### (v) Somitogenesis

By the onset of somitogenesis (approximately 10.5h of development), *Shh* expression in the head is clearly restricted to the ventral floor of the brain, extending from the tip of the diencephalon caudally through the hindbrain. At this stage, expression of *axial* has also disappeared from the head mesoderm and is similarly restricted to the floor of the brain; in contrast to *Shh,* however, it extends only as far as the anterior boundary of the midbrain. At this point, gsc expression has become very weak and is restricted to a ring of cells that appear to be migrating away from the dorsal midline.

As somitogenesis continues, *Shh* expression extends in a rostral-caudal progression throughout the ventral region of the central nervous system (CNS). Along the spinal cord, the expression domain is restricted to a single row of cells, the floor plate, but gradually broadens in the hindbrain and midbrain to become 5-7 cells in diameter, with a triangular shaped lateral extension in the ventral diencephalon and two strongly staining bulges at the tip of the forebrain, presumably in a region fated to become hypothalamus.

As induction of *Shh* in the floor plate occurs, expression in the underlying mesoderm begins to fade away, in a similar manner to *axial* (Strähle, U. et al., (1993) Genes & Dev. 7: 1436-1446). This downregulation also proceeds in a rostral to caudal sequence, coinciding with the changes in cell shape that accompany notochord differentiation. By the 22 somite stage, mesodermal *Shh* expression is restricted to the caudal region of the notochord and in the expanding tail bud where a bulge of undifferentiated cells continue to express *Shh* at relatively high levels. Expression in the midbrain broadens to a rhombic shaped area; cellular rearrangement that lead to the 90° kink of forebrain structures, position hypothalamic tissue underneath the ventral midbrain. These posterior hypothalamic tissues do not express *Shh.* In addition to *Shh* expression in the ventral midbrain, a narrow stripe of expressing cells extends dorsally on either side of the third ventricle from the rostral end of the *Shh* domain in the ventral midbrain to the anterior end of, but not including, the epiphysis. The most rostral *Shh* expressing cells are confined to the hypothalamus. In the telencephalon, additional *Shh* expression is initiated in two 1-2 cell wide stripes.

By 36 hours of development, *Shh* expression in the ventral CNS has undergone further changes. While expression persists in the floor plate of the tailbud, more rostrally located floor plate cells in the spinal cord cease to express the gene. In contrast, in the hindbrain and forebrain *Shh* expression persists and is further modified.

At 26-28h, *Shh* expression appears in the pectoral fin primordia, that are visible as placode like broadenings of cells underneath the epithelial cell layer that covers the yolk. By 33 hrs of development high levels of transcript are present in the posterior margin of the pectoral buds; at the same time, expression is initiated in a narrow stripe at the posterior of the first gill. Expression continues in the pectoral fin buds in lateral cells in the early larva. At this stage, *Shh* transcripts are also detectable in cells adjacent to the lumen of the foregut.

### (vi) Expression Of Shh In Cyclops And Notail Mutants

Two mutations affecting the differentiation of the *Axial* tissues that express *Shh* have been described in zebrafish embryos homozygous for the *cyclops* (*cyc*) mutation lack a differentiated floorplate (Hatta, K. et al., (1991) Nature 350: 339-341). By contrast, homozygous *notail* (*ntl*) embryos are characterized by a failure in notochord maturation and a disruption of normal development of tail structures (Halpern, M.E. et al., (1993) Cell 75: 99-111).

A change in *Shh* expression is apparent in cyc embryos as early as the end of gastrulation; at this stage, the anterior limit of expression coincides precisely with the two *pax-2* stripes in the posterior midbrain. Thus, in contrast to wild-type embryos, no *Shh* expression is detected in midline structures of the midbrain and forebrain. By the 5 somite stage, *Shh* transcripts are present in the notochord which at this stage extends until rhombomere 4; however, no expression is detected in more anterior structures. Furthermore, no *Shh* expression is detected in the ventral neural keel, in particular in the ventral portions of the midbrain and forebrain.

At 24 hours of development, the morphologically visible cyc phenotype consists of a fusion of the eyes at the midline due to the complete absence of the ventral diencephalon. As at earlier developmental stages, *Shh* expression is absent from neural tissue. *Shh* expression in the extending tail bud of wild-type embryos is seen as a single row of floor plate cells throughout the spinal cord. In a cyc mutant, no such *Shh* induction occurs in cells of the ventral spinal cord with the exception of some scattered, cells that show transient expression near the tail. Similarly, no *Shh* expression is seen rostrally in the ventral neural tube. However, a small group of *Shh* expressing cells is detected underneath the epiphysis which presumably correspond to the dorsal-most group of *Shh* expressing cells in the diencephalon of wild-type embryos.

In homozygous *notail* (*ntl*) embryos, no *Shh* staining is seen in mesodermal tissue at 24 hours of development, consistent with the lack of a notochord in these embryos; by contrast, expression throughout the ventral CNS is unaffected. At the tail bud stage, however, just prior to the onset of somitogenesis, *Shh* expression is clearly detectable in notochord precursor cells.

### (vii) Injection Of Synthetic Shh Transcripts Into Zebrafish Embryos Induces Expression Of A Floor Plate Marker

To investigate the activity of *Shh* in the developing embryo, an over-expression strategy, similar to that employed in the analysis of gene function in Xenopus, was adopted. Newly fertilized zebrafish eggs were injected with synthetic *Shh* RNA and were fixed 14 or 24 hours later. As an assay for possible changes in cell fate consequent upon the ectopic activity of *Shh,* we decided to analyze *Axial* expression, since this gene serves as a marker for cells in which *Shh* is normally expressed. A dramatic, though highly localized ectopic expression of *Axial* in a significant proportion (21/80) of the injected embryos fixed after 24 hours of development is observed. Affected embryos show a broadening of the *Axial* expression domain in the diencephalon and ectopic *Axial* expression in the midbrain; however, in no case has ectopic expression in the telencephalon or spinal cord been observed. Many of the injected embryos also showed disturbed forebrain structures, in particular smaller ventricles and poorly developed eyes. Amongst embryos fixed after 14h, a similar proportion (8/42) exhibit the same broadening and dorsal extension of the *Axial* stripe in the diencephalon as well as a dorsal extension of *Axial* staining in the midbrain; again, no changes in *Axial* expression were observed caudal to the hindbrain with the exception of an increased numbers of expressing cells at the tip of the tail.

### (viii) Overexpression Of Shh In Drosophila Embryos Activates The hh-Dependent Pathway

In order to discover whether the high degree of structural homology between the Drosophila and zebrafish *hh* genes also extends to the functional level, an overexpression system was used to test the activity of *Shh* in flies. Expression of Dros-HH driven by the HSP70 promoter results in the ectopic activation of both the normal targets of *hh* activity; the wg transcriptional domain expands to fill between one third to one half of each parasegment whereas ptc is ectopically activated in all cells except those expressing en (Ingham, P.W. (1993) Nature 366:560-562). To compare the activities of the fly and fish genes, flies transgenic for a HS *Shh* construct were generated described above and subjected to the same heat shock regime as H *Shh* transgenic flies. HS *Shh* embryos fixed immediately after the second of two 30 min heat shocks exhibit ubiquitous transcription of the *Shh* cDNA. Similarly treated embryos were fixed 30 or 90 min after the second heat shock and assayed for wg or *ptc* transcription. Both genes were found to be ectopically activated in a similar manner to that seen in heat shocked H *Shh* embryos; thus, the zebrafish *Shh* gene can activate the same pathway as the endogenous *hh* gene.

### Examp/e 5

### Cloning, Expression and Localization of Human Hedgehogs

### (i) Experimental Procedures

### Isolation of human hedgehog cDNA clones.

Degenerate nucleotides used to clone chick *Shh* (Riddle et al., (1993) Cell 75:1401-1416) were used to amplify by nested PCR human genomic DNA. The nucleotide sequence of these oligos is as follows:
vHH5O:5'-GGAATTCCCAG(CA)GITG(CT)AA(AG)GA(AG)(CA)(AG)I(GCI)TIAA-3' (SEQ ID NO: 18);
vHH3O:5'-TCATCGATGGACCCA(GA)TC(GA)AAICCIGC(TC)TC-3' (SEQ ID NO:19);
vHH3I:5'-GCTCTAGAGCTCTACIGCIA(GA)IC(GT)IGGIA-3' (SEQ ID NO:20)

The expected 220 bp PCR product was subcloned into pGEM7zf (Promega) and sequenced using Sequenase v2.0 (U.S. Biochemicals). One clone showed high nucleotide similarity to mouse *Ihh* and mouse *Shh* sequence (Echelard et al., (1993) Cell 75:1417-1430) and it was used for screening a human fetal lung 5'-stretch plus cDNA library (Clontech) in λ gt10 phage. The library was screened following the protocol suggested by the company and two positive plaques were identified, purified, subcloned into pBluescript SK+ (Stratagene) and sequenced, identifying them as the human homologues of *Shh* (SEQ ID NO:6) and *Ihh* (SEQ ID NO:7).

One clone contained the full coding sequence of a human homolog of *Shh* as well as 150 bp of 5' and 36 bp of 3' untranslated sequence. The other clone, which is the human homolog of *Ihh,* extends from 330 bp 3' of the coding sequence to a point close to the predicted boundary between the first and second exon. The identity of these clones was determined by comparison to the murine and chick genes. The protein encoded by human *Shh* has 92.4% overall identity to the mouse *Shh,* including 99% identity in the amino-terminal half. The carboxyl-terminal half is also highly conserved, although it contains short stretches of 16 and 11 amino acids not present in the mouse *Shh.* The human *Ihh* protein is 96.8% identical to the mouse *Ihh*. The two predicted human proteins are also highly related, particularly in their amino-terminal halves where they are 91.4% identical. They diverge significantly in their carboxyl halves, where they show only 45.1% identity. The high level of similarity in the amino portion of all of these proteins implies that this region encodes domains essential to the activity of this class of signaling molecules.

### Northern blotting

Multiple Tissue Northern Blot (Clontech) prepared from poly A+RNA isolated from human adult tissues was hybridized with either full length ³²P-labeled human *Shh* clone or ³²P-labeled human *Ihh* clone following the protocol suggested by the company.

### Digoxigenin in situ hybridization.

Sections: tissues from normal human second trimester gestation abortus specimens were washed in PBS and fixed overnight at 4°C paraformaldehyde in PBS, equilibrated 24 hours at 4°C in 50% sucrose in PBS and then placed in 50% sucrose in oct for one hour before embedding in oct. Cryostat sections (10-25 mm) were collected on superfrost plus slides (Fisher) and frozen at -80°C until used. Following a postfixation in 4% paraformaldehyde the slides were processed as in Riddle et al., (1993) Cell 75:1401-1416 with the following alterations: proteinase K digestion was performed at room temperature from 1-15 minutes (depending on section thickness), prehybridization, hybridization and washes time was decreased to 1/10 of time.

Whole-mounts: tissues from normal second trimester human abortus specimens were washed in PBS, fixed overnight at 4°C in 4% paraformaldehyde in PBS and then processed as in Riddle et al., (1993) Cell 75:1401-1416.

### Isolation of an Shh P1 clone.

The human *Shh* gene was isolated on a P1 clone from a P1 library (Pierce and Sternberg, 1992) by PCR (polymerase chain reaction) screening. Two oligonucleotide primers were derived from the human *Shh* sequence. The two olignucleotide primers used for PCR were:
SHHF5'-ACCGAGGCTGGGACGAAGATGGC-3' (SEQ ID NO:43)
SHR5'-CGCTCGGTCGTACGGCATGAACGAC-3' (SEQ ID NO:44)
The PCR reaction was carried using standard conditions as described previously (Thierfelder *et al..* 1994) except that the annealing temperature was 65°C. These primers amplified a 119 bp fragment from human and P1 clone DNA. The P1 clone was designated SHHP1. After the P1 clone was isolated these oligonucleotides were used as sequencing primers. A 2.5Kb*Eco*RI fragment that encoded a CA repeat was subcloned from this P1 clone using methods described previously (Thierfelder et al. 1994). Oligonucleotide primers that amplified this CA repeat sequence were fashioned from the flanking sequences:
SHHCAF5'-ATGGGGATGTGTGTGGTCAAGTGTA-3' (SEQ ID NO:45)
SHHCAR5'-TTCACAGACTCTCAAAGTGTATTTT-3' (SEQ ID NO:46)

### Mapping the human Ihh and Shh genes.

The human *Ihh* gene was mapped to chromosome 2 using somatic cell hybrids from NIGMS mapping pannel 2 (GM 10826B).

The *Shh* gene was mapped to chromosome 7 using somatic cell hybrids from NIGMS mapping panel 2 (GM10791 and GM 10868).

Linkage between the limb deformity locus on chromosome 7 and the *Shh* gene was demonstrated using standard procedure. Family LD has been described previously (Tkukurov et al., (1994) Nature Genet. 6:282-286). A CA repeat bearing sequence near the *Shh* gene was amplified from the DNA of all members of Family LD by PCR using the SHHCAF and SHHCAR primers. Linkage between the CA repeat and the LD disease gene segregating in Family LD was estimated by the MLINK program (Oct, 1967). Penetrance was set at 100% and the allele frequencies were determined using unrelated spouses in the LD family.

### Interspecific Backcross Mapping.

Interspecific backcross progeny were generated by mating (C57BL/6J x *M. spretus*) F1 females and C57BL/6J males as described (Copeland and Jenkins, (1991) Trends Genet. 7:113-118). A total of 205 N2 mice were used to map the *Ihh* and *Dhh* loci. DNA isolation, restriction enzyme digestions, agarose gel electrophoresis, Southern blot transfer and hybridization were performed essentially as described (Jenkins et al., (1981) J. Virol. 43:26-36). All blots were prepared with Hybond-N+ nylon membrane (Amersham). The probe, an ∼1.8kb *Eco*RI fragment of mouse cDNA, detected a major fragment of 8.5 kb in C57BL/6j (B) DNA and a major fragment 6.0 kb in *M. spretus* (S) DNA following digestion with *BgI*II*.* The *Shh* probe, an ∼ 900 bp *Sma*I fragment of mouse cDNA, detected *Hinc*II fragments of 7.5 and 2.1 kb (B) as well as 4.6 and 2.1 (S). The *Dhh* probe, and ∼ 800 bp *Bam*Hi/*Eco*Ri fragment of mouse genomic DNA, detected major fragments of 4.7 and 1.3 kb (B) and 8.2 and 1.3 kb (S) following digestion with *Sph*I*.* The presence or absence of *M. spretus* specific fragments was followed in backcross mice.

A description of the probes and RFLPs for loci used to position the *Ihh, Shh* and *Dhh* loci in the interspecific backcross has been reported. These include: *Fn1, Vil* and *Acrg,* chromosome 1 (Wilkie et al., (1993) Genomics 18:175-184), *Gnai1, En2, Il6,* chromosomes 5 (Miao et al., (1994) PNAS USA 91:11050-11054) and *Pdgfb, Gdcl* and *Rarg,* chromosome 15 (Brannan et al., (1992) Genomics 13:1075-1081). Recombination distances were calculated as described (Green, (1981) Linkage, recombination and mapping. In "Genetics and Probability in Animal Breeding Experiments", -pp. 77-113, Oxford University Press, NY) using the computer program SPRETUS MADNESS. Gene order was determined by minimizing the number of recombination events required to explain the allele distribution patterns.

### (ii) Expression of Human Shh and Ihh

To investigate the tissue distribution of *Shh* and *Ihh* expression, poly(A)+RNA samples from various adult human tissues were probed with the two cDNA clones. Of the tissues tested, an *Ihh*-specific message of ∼2.7 kb is only detected in liver and kidney. *Shh* transcripts was not detected in the RNA from any of the adult tissues tested. All the samples contained approximately equal amounts of intact RNA, as determined by hybridization with a control probe.

The *hedgehog* family of genes were identified as mediators of embryonic patterning in flies and vertebrates. No adult expression of these genes had previously been reported. These results indicate that *Ihh* additionally plays a role in adult liver and kidney. Since the *hedgehog* genes encode intercellular signals, *Ihh* may function in coordinating the properties of different cell types in these organs. *Shh* may also be used as a signaling molecule in the adult, either in tissues not looked at here, or at levels too low to be detected under these conditions.

*In situ* hybridization was used to investigate the expression of *Shh* in various mid-gestational human fetal organs. *Shh* expression is present predominantly in endoderm derived tissues: the respiratory epithelium, collecting ducts of the kidney, transitional epithelium of the ureter, hepatocytes, and small intestine epithelium. *Shh* was not detectable in fetal heart or placental tissues. The intensity of expression is increased in primitive differentiating tissues (renal blastema, base villi, branching lung buds) and decreased or absent in differentiated tissues (e.g. glomeruli). *Shh* expression is present in the mesenchyme immediately abutting the budding respiratory tubes. The non-uniform pattern of *Shh* expression in hepatocytes is consistent with expression of other genes in adult liver (Dingemanse et al., (1994) Differentiation 56:153-162). The base of villi, the renal blastema, and the lung buds are all regions expressing *Shh* and they are areas of active growth and differentiation, suggesting *Shh* is important in these processes.

### (iii) The Chromosomal Map Location of Human Shh and Ihh.

Since *Shh* is known to mediate patterning during the development of the mouse and chick and the expression *of Shh* and *Ihh* are suggestive of a similar role in humans, mutations in these genes would be expected to lead to embryonic lethality or congenital defects. One way of investigating this possibility is to see whether they are genetically linked to any known inherited disorders.

*Shh-* and *Ihh*-specific primers were designed from their respective sequences and were used in PCR reactions on a panel of rodent-human somatic cell hybrids. Control rodent DNA did not amplify specific bands using these primers. In contrast, DNA from several rodent-human hybrids resulted in PCR products of the appropriate size allowing us to assign *Shh* to chromosome 7q and *Ihh* to chromosome 2.

One of the central roles of chick *Shh* is in regulating the anterior-posterior axis of the limb. A human congenital polysyndactyly has recently been mapped to chromosome 7q36 (Tsukurov et al., (1994) Nature Genet. 6:282-286; Heutink et al., (1994) Nature Genet. 6:287-291). The phenotype of this disease is consistent with defects that might be expected from aberrant expression of *Shh* in the limb. Therefore, the chromosomal location of *Shh* was mapped more precisely, in particular in relation to the polysyndactyly locus.

A P1 phage library was screened using the *Shh* specific primers for PCR amplification and clone SHHP1 was isolated. Clone SHHP1 contained *Shh* sequence. A Southern blot of an *Eco*RI digest of this phage using [CA]/[GT] probe demonstrated that a 2.5 Kb *Eco*RI fragment contained a CA repeat. Nucleotide sequence analysis of this subcloned *EcoRI* fragment demonstrated that the CA repeat lay near the *Eco*RI sites. Primers flanking the CA repeat were designed and used to map the location *of Shh* relative to other markers on 7q in individuals of a large kindred with complex polysyndactyly (Tsukurov et al., (1994) Nature Genet. 6:282-286). *Shh* maps close to D75550 on 7q36, with no recombination events seen in this study. It is also extremely close to, but distinct from, the polysyndactyly locus with once recombination event observed between them (maximum lod score = 4.82, Θ = 0.05). One unaffected individual (pedigree ID V-10 in Tsukurov et al., (1994) Nature Genet. 6:282-286) has the *Shh* linked CA repeat allele found in all affected family members. No recombination was observed between the locus *En2* and the *Shh* gene (maximum lod score = 1.82, Θ = 0.0).

### (iv) Chromosomal mapping of the Murine Ihh, Shh and Dhh genes.

The murine chromosomal location of *Ihh, Shh* and *Dhh* was determined using an interspecific backcross mapping panel derived from crosses of [(C57BL/6J x *M*. *spetrus*)F1 X C57BL/J)] mice. cDNA fragments from each locus were used as probes in Southern blot hybridization analysis of C57BL/6J and *M. spretus* genomic DNA that was separately digested with several different restriction enzymes to identify informative restriction fragment length polymorphisms (RFLPs) useful for gene mapping. The strain distribution pattern of each RFLP in the interspecific backcross was then determined by following the presence or absence of RFLPs specific for *M*. *spretus* in backcross mice.

*Ihh* mapped to the central region of mouse chromosome 1, 2.7 cM distal of *Fn1* and did not recombine with *Vil* in 190 animals typed in common, suggesting that the two loci are within 1.6 cM (upper 95% confidence level) (Fig. 16). *Shh* mapped to the proximal region of mouse chromosome 5, 0.6 cM distal of *En2* and 1.9 cM proximal of *116* in accordance to Chang et al., (1994) Development 120:3339-3353. *Dhh* mapped to the very distal region of mouse chromosome 15, 0.6 cM distal of *Gdcl* and did not recombine with *Rarg* in 160 animals typed in common, suggesting that the two loci are within 1.9 cM of each other (upper 95% confidence level) (Fig. 16).

Interspecific maps of chromosome 1, 5 and 15 were compared with composite mouse linkage maps that report the map location of many uncloned mouse mutations (compiled by M.T. Davisson, T.H. Roderick, A.L. Hillyard and D.P. Doolittle and provided from GBASE, a computerized database maintained at The Jackson Laboratory, Bar Harbor, ME). The *hemimelic extra-toe (Hx)* mouse mutant maps 1.1 cM distal to *En2* on chromosome 5 (Martin et al., (1990) Genomics 6:302-308), a location in close proximity to where *Shh* has been positioned. *Hx* is a dominant mutation which results in preaxial polydactyly and hemimelia affecting all four limbs (Dickie, (1968) Mouse News Lett 38:24; Knudsen and Kochhar, (1981) J. Embryol. Exp. Morph. 65: Suppl. 289-307). *Shh* has previously been shown to be expressed in the limb (Echelard et al., (1993) Cell 75:1417-1430). To determine whether *Shh* and *Hx* are tightly linked we followed their distribution in a backcross panel in which *Hx* was segregating. Two recombinants between *Shh* and *Hx* were identified, thus excluding the possibility that the two loci are allelic and these observations are again consistent with those of Chang et al., (1994) Development 120:3339-3353. While there are several other mutations in the vicinity of *Ihh* and *Dhh,* none is an obvious candidate for an alteration in the corresponding gene.

The central region of mouse chromosome 1 shares homology with human chromosome 2q (summarized in Fig. 16). Placement of *Ihh* in this interval suggests the human homolog of *Ihh* will reside on 2q, as well. Similarly, it is likely that human homolog of *Dhh* will reside on human chromosome 12q.

### Example 6

### Proteolytic Processing Yields Two Secreted Forms of Sonic Hedgehog

### (i) Experimental Procedures

### In vitro Translation and Processing

Mouse and chick *sonic hedgehog* coding sequences were inserted into the vector pSP64T (kindly provided by D. Melton) which contains an SP6 phage promoter and both 5' and 3' untranslated sequences derived from the *Xenopus laevis* β-Globin gene. After restriction endonuclease digestion with *Sal I* to generate linear templates, RNA was transcribed *in vitro* using SP6 RNA polymerase (Promega, Inc.) in the presence of 1 mM cap structure analog (m⁷G(5')ppp(5')Gm; Boehringer-Mannheim, Inc.) Following digestion with RQ1 DNase I (Promega, Inc.) to remove the DNA template, transcripts were purified by phenol:choloroform extraction and ethanol precipitation.

Rabbit reticulocyte lysate (Promega, Inc.) was used according to the manufacturer's instructions. For each reaction, 12.5 µl of lysate was programmed with 0.5-2.0 µg of *in vitro* transcribed RNA. The reactions contained 20 µCi of Express labeling mix (NEN/DuPont, Inc.) were included. To address processing and secretion *in vitro,* 1.0-2.0 µl of canine pancreatic microsomal membranes (Promega, Inc.) were included in the reactions. The final reaction volume of 25 µl was incubated for one hour at 30°C. Aliquots of each reaction (between 0.25 and 3.0 µl) were boiled for 3 minutes in Laemmli sample buffer (LSB: 125 mM Tris-Hcl [pH 6.8]; 2% SDS; 1% 2-mercaptoethanol; 0.25 mg/ml bromophenol blue) before separating on a 15% polyacrylamide gel. Fixed gels were processed for fluorography using EnHance (NEN/DuPont, Inc.) as described by the manufacturer.

Glycosylation was addressed by incubation with Endoglycosidase H (Endo H; New England Biolabs, Inc.) according to the manufacturer's directions. Reactions were carried out for 1-2 hr at 37°C before analyzing reaction products by polyacrylamide gel electrophoresis (PAGE).

### Xenopus Oocyte Injection and Labeling

Oocytes were enzymatically defolliculated and rinsed with OR2 (50 mM HEPES [pH 7.2], 82 mM NaCl, 2.5 mM KCl, 1.5 mM Na2HPO4). Healthy stage six oocytes were injected with 30 ng of *in vitro* transcribed, capped mouse *Shh* RNA (prepared as described above). Following a 2 hr recovery period, healthy injected oocytes and uninjected controls were cultured at room temperature in groups of ten in 96-well dishes containing 0.2 ml of OR2 (supplemented with 0.1 mg/ml Gentamicin and 0.4 mg/ml BSA) per well. The incubation medium was supplemented with 50 µCi of Express labeling mix. Three days after injection, the culture media were collected and expression of *Shh* protein analyzed by immunoprecipitation. Oocytes were rinsed several times in OR2 before lysing in TENT (20 mM Tris-HCl [pH 8.0]; 150 mM NiCl, 2mM EDTA; 1% Triton-X-100; 10 µl/oocyte) supplemented with 1 µg/ml aprotinin, 2 µg/ml leupeptin and 1mM phenylmethylsufonylfluoride (PMSF). After centrifugation at 13000 x g for 10 minutes at 4°C, soluble protein supernatants were recovered and analyzed by immunoprecipitation (see below).

### Cos Cell Transfection and Labeling.

Cos cells were cultured in Dulbecco's Modified Eagle Medium (DMEM; Sigma, Inc.) supplemented with 10% fetal bovine serum (Gibco/BRL), 2 mM L-Glutamine (Gibco/BRL) and 50 mU/ml penicillin and 50 µg/ml streptomycin (Gibco/BRL). Subconfluent cos cells in 35 mm or 60 mm dishes (Falcon, Inc.) were transiently transfected with 2 mg or 6 mg supercoiled plasmid DNA, respectively. Between 42 and 44 hr post-transfection, cells were labeled for 4-6 hr in 0.5 ml (35 mm dishes) or 1.5 ml (60 mm dishes) serum-free DMEM lacking Cysteine and Methionine (Gibco/BRL) and supplemented with 125 µCi/ml each of Express labeling mix and L-35S-Cysteine (NEN/DuPont). After labeling, media were collected and used for immunoprecipitation. Cells were rinsed with cold PBS and lysed in the tissue culture dishes by the addition of 0.5 ml (35 mm dishes) or 1.5 ml (60 mm dishes) TENT (with protease inhibitors as described above) and gentle rocking for 30 minutes at 4°C. Lysates were cleared by centrifugation (13000 x g for 5 min. at 4°C) and the supernatants were analyzed by immunoprecipitation (see below).

### Baculovirus Production and Infection

A recombinant baculovirus expressing mouse sonic *hedgehog* with a myc epitope tag inserted at the carboxy terminus was generated using the Baculogold kit (Pharmingen, Inc.). The initial virus production used Sf 9 cells, followed by two rounds of amplification in High Five cells (Invitrogen, Inc.) in serum-free medium (ExCell 401; Invitrogen, Inc.). A baculovirus lacking *Shh* coding sequences was also constructed as a control. For protein induction, High Five cells were infected at a multiplicity of approximately 15. Three days later, medium and cells were collected by gentle pipetting. Cells were collected by centrifugation (1000 x g) and the medium was recovered for Western blot analysis. Cell pellets were washed twice in cold PBS and lysed in TENT plus protease inhibitors (see above) by rotating for 30 minutes at 4°C in a microcentrifuge tube. The lysate was cleared as described above prior to Western blotting.

### Western Blotting

For Western blotting, 0.25 ml samples of media (1% of the total) were precipitated with TCA and redissolved in 15 µl of LSB. Cell lysate samples (1% of total) were brought to a final volume of 15 µl with water and concentrated (5X) LSB. Samples were boiled S minutes prior to separation on a 15% acrylamide gel. Proteins were transferred to PVDF membrane (Immobilon-P; Millipore, Inc.) and blocked in BLOTTO (5% w/v non-fat dried milk in PBS) containing 0.2% Tween-20. Hybridoma supernatant recognizing the human c-myc epitope (9E10; Evan, G.I. et al., (1985) Mol. Cell. Biol. 5:3610-3616) was added at a dilution of 1:200 for one hour followed by a 1:5000 dilution of Goat anti-Mouse-Alkaline phosphatase conjugate (Promega, Inc.) for 30 minutes. Bands were visualized using the Lumi-Phos 530 reagent (Boehringer-Mannheim) according to the manufacturer's directions.

### Immunoprecipitation

Cell lysates (Xenopus oocytes or cos cells) were brought to 0.5 ml with TENT (plus protease inhibitors as above). Media samples (OR2 or DMEM) were cleared by centrifugation at 13000 x g for 5 min. (4°C) and 10X TENT was added to a final concentration of 1X (final volume: 0.5-1.5 ml). The c-myc monoclonal antibody hybridoma supernatant was added to 1/20 of the final volume. Samples were rotated for 1 hr at 4°C., then 0.1 ml of 10% (v/v) protein A-Sepharose CL-4B (Pharmacia, Inc.) was added. Samples were rotated an additional 14-16 h. Immune complexes were washed 4 times with 1.0 ml TENT. Immunoprecipitated material was eluted and denatured by boiling for 10 minutes in 25 µl 1X LSB. Following centrifugation, samples were separated on 15% acrylamide gels and processed for fluorography as described previously. Samples for Endo H digestion were eluted and denatured by boiling for 10 minutes in the provided denaturation buffer followed by digestion with Endo H for 1-2 hr at 37°C. Concentrated (SX) LSB was added and the samples were processed for electrophoresis as described.

For immunoprecipitation with the anti-mouse *Shh* serum, samples (Cos cell lysates and DMEM) were precleared by incubating 1 hr on ice with 3 µl pre-immune serum, followed by the addition of 0.1 ml 10% (v/v) Protein A-Sepharose. After rotating for 1 hr at 4 C, supernatants were recovered and incubated for 1 hr on ice with 3µl depleted anti-mouse *Shh* serum (see below). Incubation with Protein A-Sepharose, washing, elution and electrophoresis were then performed as described above.

### Immunofluorescent Staining of Cos Cells

Twenty-four hours after transfection, cells were transferred to 8-chamber slides (Lab-Tek, Inc.) and allowed to attach an additional twenty-four hours. Cells were fixed in 2% paraformaldehyde/0.1% glutaraldehyde, washed in PBS and permeabilized in 1% Triton-X-100 (Munro, S. and Pelham, H.R.B., (1987) Cell 48:899-907)*.* After washing in PBS, cells were treated for 10 minutes in 1 mg/ml sodium borohydride. Cells were incubated with the c-myc monoclonal antibody hybridoma supernatant (diluted 1:10) and the affinity purified mouse Sonic *hedgehog* antiserum (diluted 1:4) for 45 minutes followed by incubation in 1:100 Goat-anti Mouse IgG-RITC plus 1:100 Goat anti Rabbit IgG F1TC (Southern Biotechnology Associates, Inc.) for 45 minutes. DAPI (Sigma, Inc.) was included at 0.3 µ g/ml The slides were mounted in Slo-Fade (Molecular Probes, Inc.) and photographed on a Leitz DMR compound microscope.

### Antibody Production and Purification

A PCR fragment encoding amino acids 44-143 of mouse Sonic *hedgehog* was cloned in frame into the *Eco Rl* site of pGEX-2T (Pharmacia, Inc.). Transformed bacteria were induced with IPTG and the fusion protein purified on a Glutathione-Agarose affinity column (Pharmacia, Inc.) according to the manufacturer's instructions. Inoculation of New Zealand White rabbits, as well as test and production bleeding were carried out at Hazelton Research Products, Inc.

To deplete the serum of antibodies against Glutathione-S-transferase (GST) and bacterial proteins, a lysate of E. coli transformed with pGEX-2T and induced with IPTG was coupled to Affi-Gel 10 (Bio-Rad, Inc.) The serum was incubated in batch for two hours with the depletion matrix before centrifugation (1000 x g for 5 min.) and collection of the supernatant. To make an affinity matrix, purified bacterially expressed protein corresponding to the amino terminal two-thirds of mouse Sonic *hedgehog* was coupled to Affi-Gel 10 (Bio-Rad, Inc.). The depleted antiserum was first adsorbed to this matrix in batch, then transferred to a column. The matrix was washed with TBST (25 mM Tris-HCl [pH 7.5], 140 mM NaCl, 5 mM KCl, 0.1% Triton-X-100), and the purified antibodies were eluted with ten bed volumes of 0.15 M Glycine [pH 2.5]. The solution was neutralized with one volume of 1 M Tris-HCl [pH 8.0], and dialyzed against 160 volumes of PBS.

Other antibodies have been generated against *hedgehog* proteins and three polyclonal rabbit antisera obtained to *hh* proteins can be characterized as follows:Ab77 -reacts only with the carboxyl processed chick *Shh* peptide (27 kd); Ab79 -reacts with amino processed chick, mouse and human *Shh* peptide (19 kd). Weakly reacts with 27 kd peptide from chick and mouse. Also reacts with mouse *Ihh*; and Ab80 -reacts with only amino peptide (19kd) of chick, mouse and human.

### (ii) In Vitro Translated Sonic Hedgehog is Proteolytically Processed and Glycosylated

The open reading frames of chick and mouse *Shh* encode primary translation products of 425 and 437 amino acids, respectively, with predicted molecular masses of 46.4 kilodaltons (kDa) and 47.8 kDa (Echelard, Y. et al., (1993) Cell 75:1417-1430; Riddle, R.D. et al., (1993) Cell 75:1401-1416). Further examination of the protein sequences revealed a short stretch of amino terminal residues (26 for chick, 24 for mouse) that are highly hydrophobic and are predicted to encode signal peptides. Removal of these sequences would generate proteins of 43.7 kDa (chick *Shh*) and 45.3 kDa (mouse *Shh*)*.* Also, each protein contains a single consensus site for N-linked glycosylation (Tarentino, A.L. et al., (1989) Methods Cell Biol. 32:111-139) at residue 282 (chick) and 279 (mouse). These features of the *Shh* proteins are summarized in Figure 11.

A rabbit reticulocyte lysate programmed with *in vitro* translated messenger RNA encoding either chick or mouse *Shh* synthesizes proteins with molecular masses of 46 kDa and 47 kDa, respectively. These values are in good agreement with those predicted by examination of the amino acid sequences. To examine posttranslational modifications of *Shh* proteins, a preparation of canine pancreatic microsomal membranes was included in the translation reactions. This preparation allows such processes as signal peptide cleavage and core glycosylation. When the *Shh* proteins are synthesized in the presence of these membranes, two products with apparent molecular masses of approximately 19 and 28 kDa (chick), or 19 and 30 kDa (mouse) are seen in addition to the 46 kDa and 47 kDa forms. When the material synthesized in the presence of the membranes is digested with Endoglycosidase H (Endo H), the mobilities of the two larger proteins are increased. The apparent molecular masses of the Endo H digested forms are 44 kDa and 26 kDa for chick *Shh,* and 45kDa and 27 kDa for mouse *Shh.* The decrease in the molecular masses of the largest proteins synthesized in the presence of the microsomal membranes after Endo H digestion is consistent with removal of the predicted signal peptides. The mobility shift following Endo H treatment indicates that N-linked glycosylation occurs, and that the 26 kDa (chick) and 27 kDa (mouse) proteins contain the glycosylation sites.

The appearance of the two lower molecular weight bands (hereafter referred to as the "processed forms") upon translation in the presence of microsomal membranes suggests that a proteolytic event in addition to signal peptide cleavage takes place. The combined molecular masses of the processed forms (19 kDa and 26 kDa for chick; 19 kDa and 27 kDa for mouse) add up to approximately the predicted masses of the signal peptide cleaved proteins (44 kDa for chick and 45 kDa for mouse) suggesting that only a single additional cleavage occurs.

The mouse *Shh* protein sequence is 12 amino acid residues longer than the chick sequence (437 versus 425 residues). Alignment of the chick and mouse *Shh* protein sequences reveals that these additional amino acids are near the carboxy terminus of the protein (Echelard, Y. et al., (1993) Cell 75:1417-1430). Since the larger of the processed forms differ in molecular mass by approximately 1 kDa between the two species, it appears that these peptides contain the carboxy terminal portions of the *Shh* proteins. The smaller processed forms, whose molecular masses are identical, presumably consist of the amino terminal portions.

### (iii) Secretion of Shh Peptides

To investigate the synthesis of *Shh* proteins in vivo, the mouse protein was expressed in several different eukaryotic cell types. In order to detect synthesized protein, and to facilitate future purification, the carboxy terminus was engineered to contain a twenty-five amino acid sequence containing a recognition site for the thrombin restriction protease followed by a ten amino acid sequence derived from the human c-myc protein and six consecutive histidine residues. The c-myc sequence serves as an epitope tag allowing detection by a monoclonal antibody (9E10; Evan, G.I. et al., (1985) Mol. Cell Biol. 5:3610-3616). The combined molecular mass of the carboxy terminal additions is approximately 3 kDa.

### Xenopus laevis oocytes

Immunoprecipitation with the c-myc antibody detects several proteins in lysates of metabolically labeled Xenopus laevis oocytes injected with *Shh* mRNA. Cell lysates and medium from ³⁵S labeled oocytes injected with RNA encoding mouse *Shh* with the c-myc epitope tag at the at the carboxy terminus, or from control oocytes were analyzed by immunoprecipitation with c-myc monoclonal antibody. A band of approximately 47 kDa is seen, as is a doublet migrating near 30 kDa. Treatment with Endo H increases the mobility of the largest protein, and resolves the doublet into a single species of approximately 30 kDa. These observations parallel the behaviors seen *in vitro.* Allowing for the added mass of the carboxy terminal additions, the largest protein would correspond to the signal peptide cleaved form, while the doublet would represent the glycosylated and unglycosylated larger processed form. Since the epitope tag was placed at the carboxy terminus of the protein, the identity of the 30 kDa peptide as the carboxy terminal portion of *Shh* is confirmed. Failure to detect the 19 kDa species supports its identity as an amino terminal region of the protein.

To test whether *Shh* is secreted by Xenopus oocytes, the medium in which the injected oocytes were incubated was probed by immunoprecipitation with the c-myc antibody. A single band migrating slightly more slowly than the glycosylated larger processed form was observed. This protein is insensitive to Endo H. This result is expected since most secreted glycoproteins lose sensitivity to Endo H as they travel through the Golgi apparatus and are modified by a series of glycosidases (Kornfeld, R. and Kornfeld, S., (1985) Annu. Rev. Biochem. 54:631-664). The enzymatic maturation of the Asn-linked carbohydrate moiety could also explain the slight decrease in mobility of the secreted larger protein versus the intracellular material. Following Endo H digestion, a band with a slightly lower mobility than the signal peptide cleaved protein is also apparent, suggesting that some *Shh* protein is secreted without undergoing proteolytic processing. Failure to detect this protein in the medium without Endo H digestion suggests heterogeneity in the extent of carbohydrate modification in the Golgi preventing the material from migrating as a distinct band. Resolution of this material into a single band following Endo H digestion suggests that the carbohydrate structure does not mature completely in the Golgi apparatus. Structural differences between the unprocessed protein and the larger processed form could account for this observation (Kornfeld, R. and Kornfeld, S., (1985) Annu. Rev. Biochem. 54:631-664).

### Cos cells

The behavior of mouse *Shh* in a mammalian cell type was investigated using transfected cos cells. Synthesis and secretion of the protein was monitored by immunoprecipitation using the c-myc antibody. Transfected cos cells express the same Sonic *hedgehog* species that were detected in the injected Xenopus oocytes, and their behavior following Endo H digestion is also identical. Furthermore, secretion of the 30 kDa glycosylated form is observed in cos cells, as well as the characteristic insensitivity to Endo H after secretion. Most of the secreted protein co-migrates with the intracellular, glycosylated larger processed form, but a small amount of protein with a slightly lower mobility is also detected in the medium. As in the Xenopus oocyte cultures, some *Shh* which has not undergone proteolytic processing is evident in the medium, but only after Endo H digestion.

### Baculovirus infected cells

To examine the behavior of the mouse *Shh* protein in an invertebrate cell type, and to potentially purify *Shh* peptides, a recombinant baculovirus was constructed which placed the *Shh* coding sequence, with the carboxy terminal tag, under the control of the baculoviral Polyhedrin gene promoter. When insect cells were infected with the recombinant baculovirus, *Shh* peptides could be detected in cell lysates and medium by Western blotting with the c-myc antibody.

The *Shh* products detected in this system were similar to those described above. However, virtually no unprocessed protein was seen in cell lysates, nor was any detected in the medium after Endo H digestion. This suggests that the proteolytic processing event occurs more efficiently in these cells than in either of the other two cell types or the *in vitro* translation system. A doublet corresponding to the glycosylated and unglycosylated 30 kDa forms is detected, as well as the secreted, Endo I resistant peptide as seen in the other expression systems. Unlike the other systems, however, all of the secreted larger processed form appears to comigrate with the glycosylated intracellular material.

### (iv) Secretion of a Highly Conserved Amino Terminal Peptide

To determine the behavior of the amino terminal portion of the processed Sonic *hedgehog* protein, the c-myc epitope tag was positioned 32 amino acids after the putative signal peptide cleavage site (Figure 12). Cos cells were transfected with *Shh* expression constructs containing the c-myc tag at the carboxy terminus or near the amino terminus. When this construct was expressed in cos cells, both the full length protein and the smaller processed form (approximately 20 kDa due to addition of the c-myc tag) were detected by immunoprecipitation of extracts from labeled cells. However, the 20 kDa product is barely detected in the medium. In cells transfected in parallel with the carboxy terminal c-myc tagged construct, the full length and 30 kDa products were both precipitated from cell lysates and medium as described earlier.

As the amino terminal c-myc tag may affect the secretion efficiency of the smaller processed form, the expression of this protein was examined in cos cells using an antiserum directed against amino acids 44 through 143 of mouse *Shh* (Figure 12). After transfection with the carboxy-terminal c-myc tagged construct, immunoprecipitation with the anti-Shh serum detected a very low level of the smaller processed form in the medium despite a strong signal in the cell lysate. This recapitulates the results with the myc antibody.

To examine the subcellular localization of *Shh* proteins, cos cells were transfected with the carboxy terminal tagged *Shh* construct and plated on multi-chamber slides, fixed and permeabilized. The cells were incubated simultaneously with the anti-Shh serum and the c-myc antibody followed by FITC conjugated Goat anti-Rabbit-IgG and RITC conjugated Goat anti-Mouse-IgG. DAPI was included to stain nuclei. Strong perinuclear staining characteristic of the Golgi apparatus was observed with the anti-*Shh* serum. The same subcellular region was also stained using the c-myc antibody. The coincidence of staining patterns seen with the two antibody preparations suggest that the low level of the smaller processed form detected in the medium is not due to its retention in the endoplasmic reticulum.

### (v) Hedgehog Processing

In summary, the results discussed above demonstrate that the mouse and chick *Shh* genes encode secreted glycoproteins which undergo additional proteolytic processing. Data indicate that this processing occurs in an apparently similar fashion in a variety of cell types suggesting that it is a general feature of the *Shh* protein, and not unique to any particular expression system. For mouse *Shh,* data indicate that both products of this proteolytic processing are secreted. These observations are summarized in Figure 13.

It was observed that the 19 kDa amino peptide accumulates to a lower level in the medium than the 27 kDa carboxyl peptide. This may reflect inefficient secretion or rapid turnover of this species once secreted. Alternatively, the smaller form may associate with the cell surface or extracellular matrix components making it difficult to detect in the medium. The insensitivity of the secreted, larger form to Endo H is a common feature of secreted glycoproteins. During transit through the Golgi apparatus, the Asn-linked carbohydrate moiety is modified by a series of specific glycosidases (reviewed in Kornfeld, R. and Kornfeld, S., (1985) Annu. Rev. Biochem 54:631-664; Tarentino, A.L. et al., (1989) Methods Cell Biol. 32:111-139). These modifications convert the structure from the immature "high mannose" to the mature "complex" type. At one step in this process, a Golgi enzyme, α-mannosidase II, removes two mannose residues from the complex rendering it insensitive to Endo H (Kornfeld, R. and Kornfeld, S., (1985) Annu. Rev. Biochem 54:631-664).

Based on the observed molecular masses of the processed forms of mouse and chick *Shh,* the predicted secondary proteolytic cleavage site would be located near the border of the sequences encoded by the second and third exons. Interestingly, this region marks the end of the most highly related part of the *hedgehog* proteins. The amino terminal (smaller) form would contain the most highly conserved portion of the protein. In fact, the amino acids encoded by exons one and two (exclusive of sequences upstream of the putative signal peptide cleavage sites) share 69% identity between Dros-HH and mouse *Shh,* and 99% identity between chick and mouse *Shh*. Amino acid identity in the region encoded by the third exon is much lower 30% mouse to Drosophila and 71 % mouse to chick (Echelard, Y. et al., (1993) Cell 75:1417-1430). Therefore, the two processed forms of *Shh* may have conserved as well as divergent signaling activities separated into distinct coding exons in the *Shh* gene. Furthermore, the observation that some unprocessed protein is secreted by Xenopus oocytes and cos cells raises the possibility that it may have a separate function.

The biochemical behavior of mouse *Shh* appears to be quite similar to that described for the Drosophila *Hedgehog* (Dros-HH) protein (Lee, J.L. et al., (1992) Cell 71:33-50; Tabata, T. et al., (1992) Genes & Dev. 6:2635-2645). *In vitro* translation of Dros-HH mRNA, in the presence of microsomes, revealed products with molecular masses corresponding to full length protein, as well as to the product expected after cleavage of the predicted internal (Type II) signal peptide (Lee, J.L. et al., (1992) Cell 71:33-50). Interestingly, no additional, processed forms were observed. However, such forms could have been obscured by breakdown products migrating between 20 and 30 kDa. When an RNA encoding a form of the protein lacking the carboxy-terminal 61 amino acids was translated, no breakdown products were seen, but there is still no evidence of the proteolytic processing observed with mouse *Shh*. A similar phenomenon has been observed in these experiments. A reduction in the extent of proteolytic processing is seen when a mouse *Shh* protein lacking 10 carboxy-terminal amino acids is translated *in vitro* or expressed in cos cells (data not shown). This suggests that sequences at the carboxy termini of Dros-HH proteins act at a distance to influence the efficiency of processing.

*In vivo*, processing of Dros-HH has been demonstrated (Tabata, T. et al., (1992) Genes & Dev. 6:2635-2645). Immunoblots of lysates from Schneider cells transfected with a *hh* expression vector reveal two smaller molecular weight forms similar to those described for mouse *Shh.* These products were also detected in extracts of larvae and imaginal discs derived from flies expressing a heat shock inducible *hh* construct. Thus, it is clear that there are also several distinct forms of Dros-HH proteins.

### (vi) Hedgehog Signaling

In order to satisfy the criteria for intercellular signaling, *hedgehog* proteins must be detected outside of their domains of expression. This has been clearly demonstrated for Dros-HH. Using an antiserum raised against nearly full length Dros-HH protein, Tabata and Komberg (Tabata, T. and Kornberg, T.B., (1992) Cell 76:89-102) detect the protein in stripes that are slightly wider than the RNA expression domains in embryonic segments, and just anterior to the border of the RNA expression domain in wing imaginal discs. Similarly, Taylor, et. al., (1993) Mech. Dev. 42:89-96, detected Dros-HH protein in discrete patches within cells adjacent to those expressing *hh* RNA in embryonic segments using an antiserum directed against an amino-terminal portion of Dros-HH which, based on the proteolytic processing data (Tabata, T. et al., (1992) Genes & Dev. 6:2635-2645), is not likely to recognize the carboxyl cleavage product.

The detection of Dros-HH beyond cells expressing the *hh* gene is consistent with the phenotype of *hh* mutants. In these animals, cellular patterning in each embryonic parasegment in disrupted resulting in an abnormal cuticular pattern reminiscent of that seen in wg mutants. Further analysis has revealed that the loss of *hh* gene function leads to loss of wg expression in a thin stripe of cells just anterior to the *hh* expression domain (Ingham, P.W. and Hidalgo, A., (1993) Development 117:283-291). This suggests that Dros-HH acts to maintain wg expression in neighboring cells. The observation that ubiquitously expressed Dros-HH leads to ectopic activation of wg supports this model (Tabata, T. and Kornberg, T.B., (1992) Cell 76:89-102). In addition to these genetic studies, there is also indirect evidence that Dros-HH acts at a distance from its site of expression to influence patterning of the epidermis (Heemskerk, J. and DiNardo, S., (1994) Cell 76:449-460).

The apparent effect of Dros-HH on neighboring cells, as well as on those located at a distance from the site of *hh* expression is reminiscent of the influence of the notochord and floor plate on the developing vertebrate CNS, and of the ZPA in the limb. The notochord (a site of high level *Shh* expression) induces the formation of the floor plate in a contact dependent manner, while the notochord and floor plate (another area of strong *Shh* expression) are both capable of inducing motomeurons at a distance (Placzek, M. et al., (1993) Development 117:205-218; Yamada, T. et al., (1993) Cell 73:673-686).

Moreover ZPA activity is required not only for patterning cells in the extreme posterior of the limb bud where *Shh* is transcribed, but also a few hundred microns anterior of this zone. Several lines of evidence indicate that *Shh* is able to induce floor plate (Echelard, Y. et al., (1993) Cell 75:1417-1430; Roelink, H. et al., (1994) Cell 76:761-775) and mediate the signaling activity of the ZPA (Riddle, R.D. et al., (1993) Cell 75:1401-1416). Since it has been shown that *Shh* is cleaved, it can be speculated that the processed peptides may have distinct activities. The smaller amino terminal form, which appears to be more poorly secreted, less stable or retained at the cell surface or in the extracellular matrix, may act locally. In contrast, the larger carboxy terminal peptide could possibly function at a distance. In this way, *Shh* peptides may mediate distinct signaling functions in the vertebrate embryo.

### Example 7

### Sonic hedgehog and Fgf-4 act through a signaling cascade and feedback loop to integrate growth and patterning of the developing limb bud

### (i) Experimental Procedures

### Cloning of Chicken Fgf-4 and Bmp-2

A 246 bp fragment of the chicken *Fgf*-*4* gene was cloned by PCR from a stage 22 chicken limb bud library. Degenerate primers were designed against previously cloned *Fgf-4* and *Fgf-6* genes: fgf5' (sense) AAA AGC TTT AYT GYT AYG TIG GIA THG G (SEQ ID No:38) and fgf3' (antisense) AAG AAT TCT AIG CRT TRT ART TRT TIG G (SEQ ID No:39). Denaturation was at 94°C for 2 min, followed by 30 cycles of 94°C for 30 sec, 50°C for 60 sec, and 72°C for 30 sec, with a final extension at 72°C for 5 min. The PCR product was subcloned into the Bluescript SK+ vector. A clone was sequenced and confirmed as *Fgf-4* by comparison with previously published *Fgf-4* genes and a chicken *Fgf-4* gene sequence kindly provided by Lee Niswander.

BMP-related sequences were amplified from a stage 22 posterior limb bud cDNA library prepared in Bluescript using primers and conditions as described by Basler, et al. (1993). Amplified DNAs were cloned and used to screen a stage 22 limb bud library prepared in λ-Zap (Stratagene). Among the cDNAs isolated was chicken *Bump-2.* Its identity was confirmed by sequence comparison to the published clones (Francis, et al., (1994) Development 120:209-218) and by its expression patterns in chick embryos.

### Chick Surgeries and Recombinant Retroviruses

All experimental manipulations were performed on White Leghorn chick embryos (S-SPF) provided by SPAFAS (Norwich, Conn). Eggs were staged according to Hamburger and Hamilton (1951) J. Exp. Morph. 88:49-92.

Viral supernatants of *Sonic*/RCAS-A2 or a variant containing an influenza hemaglutinin epitope tag at the carboxyl terminus of the *hedgehog* protein (*Sonic*7. 1/RCAS-A2, functionally indistinguishable from *Sonic*/RCAS-A2)*,* were prepared as described (Hughes, et al., (1987) J. Viro/. 61:3004-13; Fekete and Cepko, (1993) Mol. & Cell. Biol. 13:2604-13; Riddle, et al., (1993) Cell 75:1401-16). For focal injections the right wings of stage 18-21 embryos were transiently stained with nile blue sulfate (0.01 mg/ml in Ringer's solution) to reveal the AER. A trace amount of concentrated viral supernatant was injected beneath the AER.

The AER was removed using electrolytically sharpened tungsten wire needles. Some embryos had a heparin-acrylic bead soaked in *FGF-4* solution (0.8 mg/ml; a gift from Genetics Institute) or PBS stapled to the limb bud with a piece of 0.025mm platinum wire (Goodfellow, Cambridge UK) essentially as described by Niswander et al, (1993) Cell 75:579-87.

Limbs which were infected with *Sonic*/RCAS virus after AER removal were infected over a large portion of the denuded mesoderm to ensure substantial infection. Those embryos which received both an *Fgf-4* soaked bead and virus were infected only underneath the bead.

### In Situ Hybridizations and Photography

Single color whole mount in situ hybridizations were performed as described (Riddle, et al., (1993) Cell 75:1401-16). Two color whole mount in situ hybridizations were performed essentially as described by Jowett and Lettice (1994) Trends Genet. 10:73-74. The second color detection was developed using 0.125mg/ml magenta-phos (Biosynth) as the substrate. Radioactive in situ hybridizations on 5µm sections was performed essentially as described by Tessarollo, et al. (1992) Development 115:11-20.

The following probes were used for whole mount and section in situ hybridizations: *Sonic:* 1.7kb fragment of pHH2 (Riddle, et al., (1993) Cell 75:1401-16). *Bmp-2:* 1.5 kb fragment encoding the entire open reading frame. *Fgf-4*: 250 bp fragment described above. *Hox d-11:* a 600 bp fragment, *Hoxd*-13*:* 400 bp fragment both including 5' untranslated sequences and coding sequences upstream of the homeobox. RCAS: 900 bp SalI-ClaI fragment of RCAS (Hughes et al., (1987) J Virol. 61:3004-12).

### (ii) Relationship of Sonic to Endogenous Bmp-2 and Hoxd Gene Expression

The best candidates for genes regulated by *Sonic* in vivo are the distal members of the *Hoxd* gene cluster, *Hoxd-9* through *-13,* and *Bmp-2.* Therefore, the relationships of the expression domains of these genes in a staged series of normal chick embryos were analyzed. *Hoxd-9* and *Hoxd-10* are expressed throughout the presumptive wing field at stage 16 (Hamburger and Hamilton, (1951) J. Exp. Morph. 88:49-92), prior to the first detectable expression of *Sonic* at early stage 18. *Hoxd-11* expression is first detectable at early stage 18, the same time as *Sonic,* in a domain coextensive with *Sonic.* Expression of *Hoxd-12* and *Hoxd-13* commence shortly thereafter. These results suggest that *Sonic* might normally induce, directly or indirectly, the expression of only the latter three members of the cluster, even though all five are nested within the early limb bud.

As limb outgrowth proceeds *Sonic* expression remains at the posterior margin of the bud. In contrast the *Hoxd* gene expression domains, which are initially nested posteriorly around the *Sonic* domain, are very dynamic and lose their concentric character. By stage 23 the *Hoxd-11* domain extends anteriorly and distally far beyond that of *Sonic,* while *Hoxd-13* expression becomes biased distally and displaced from *Sonic.*

While it is not clear whether *Bmp-2* is expressed before *Sonic* (see Francis et. al., (1994) Development 120:209-218) *Bump-2* is expressed in a mesodermal domain which apparently overlaps and surrounds that of *Sonic* at the earliest stages of *Sonic* expression. As the limb bud develops, the mesodermal expression of *Bmp-2* remains near the posterior limb margin, centered around that of *Sonic,* but in a larger domain than *Sonic.* This correspondence between *Sonic* and *Bmp-2* expression lasts until around stage 25, much longer than the correspondence between *Sonic* and *Hoxd* gene expression. After stage 25 *Bmp-2* expression shifts distally and is no longer centered on *Sonic.*

### (iii) Relationship of Sonic to Induced Bmp-2 and Hoxd Gene Expression

The fact that the expression domains of the *Hoxd* genes diverge over time from that of *Sonic hedgehog* implies that *Sonic* does not directly regulate their later patterns of expression. This does not preclude the possibility that the later expression domains are genetically downstream of *Sonic.* If this were the case, exogenously expressed *Sonic* would be expected to initiate a program of *Hoxd* gene expression which recapitulates that seen endogenously. Therefore, the spatial distribution of *Hoxd* gene expression at various times following *Sonic* misexpression was compared. The anterior marginal mesoderm of early bud (Stage 18-20) wings was injected at a single point under the AER with a replication competent virus that expresses a chicken *Sonic* cDNA. Ectopic *Sonic* expressed by this protocol leads to both anterior mesodermal outgrowth and anterior extension of the AFR.

The *Sonic* and *Hoxd* gene expression domains in the infected limbs were analyzed in sectioned and intact embryos. Viral *Sonic* message is first detected approximately 18 hours after infection at the anterior margin, at the same time as, and approximately coextensively with, induced *Hoxd-11.* This suggests that *Sonic* can rapidly induce *Hoxd-11* expression and that the lag after injection represents the time required to achieve *Sonic* expression. By 35 hours post infection distal outgrowth of infected cells combined with lateral viral spread within the proliferating cells leads to viral expression in a wedge which is broadest at the distal margin and tapers proximally. By this time, *Hoxd-11* expression has expanded both antero-proximally and distally with respect to the wedge of Sonic-expressing cells, into a domain which appears to mirror the more distal aspects of the endogenous *Hoxd-11* domain. Weak *Hoxd-13* expression is also detected at 35 hours in a subset of the *Sonic* expressing domain at its distal margin. 51 hours after infection the relationship of *Sonic* and *Hoxd-11* expression is similar to that seen at 35 hours, while the induced *Hoxd-13* expression has reached wild type levels restricted to the distal portions of the ectopic growth. Thus the ectopic *Hoxd* expression domains better reflect the endogenous patterns of expression than they do the region expressing *Sonic.* This suggests that there are multiple factors regulating *Hoxd* expression but their actions lie downstream of *sonic.*

Since the endogenous *Bmp-2* expression domain correlates well with that of *Sonic,* and *Bmp-2* is induced by ZPA grafts, it was looked to see if *Bmp-2* is also induced by *Sonic. Bmp-2* is normally expressed in two places in the early limb bud, in the posterior mesoderm and throughout the AER (Francis, et al., (1994) Development 120:209-218). In injected limb buds additional *Bmp-2* expression is seen in both the anterior mesoderm and in the anteriorly extended AER. The domain of *Bmp-2* expression is slightly more restricted than that of viral expression, suggesting a delay in *Bmp-2* induction. *Bmp-2* expression in both the mesoderm and ectoderm is thus a downstream target of *Sonic* activity in the mesoderm. In contrast to the expression domains of the *Hoxd* genes, the endogenous and ectopic *Bmp-2* expression domains correlate well with that of *Sonic.* This suggests that *Bmp-2* expression is regulated more directly by *Sonic* than is expression of the *Hoxd* genes.

### (iv) The AER and Competence to Respond to Sonic

Ectopic activation of *Hoxd* gene expression is biased distally in virally infected regions, suggesting that ectodermal factors, possibly from the AER, are required for *Hoxd* gene induction by *Sonic.* To test this, *Sonic* virus was injected into the proximal, medial mesoderm of stage 21 limb buds, presumably beyond the influence of the AER. Although the level of *Sonic* expression was comparable to that observed in distal injections, proximal misexpression of *Sonic* did not result in ectopic induction of the *Hoxd* genes or *Bmp-2,* nor did it result in any obvious morphological effect (data not shown). The lack of gene induction following proximal misexpression of *Sonic* suggests that exposure to *Sonic* alone is insufficient to induce expression of these genes.

This was tested more rigorously by injection of *Sonic* virus into the anterior marginal mesoderm of stage 20/21 limb buds after the anterior half of the AER had been surgically removed. Embryos were allowed to develop for a further 36 to 48 hours before harvesting. During this time the AER remaining on the posterior half of the limb bud promotes almost wild type outgrowth and patterning of the bud. Gene expression was monitored both in sectioned and intact embryos. In the presence of the AFR, *Sonic* induces both anterior mesodermal proliferation and expression of *Hoxd-11, Hoxd-13* and *Bmp-2.* In the absence of the overlying AER, *Sonic* does not induce either mesodermal proliferation or expression of these genes above background. Signals from the AER are thus required to allow both the proliferative and patterning effects of *Sonic* on the mesoderm.

Since application of FGF protein can rescue other functions of the AER such as promoting PD outgrowth and patterning, it was sought to determine whether FGFs might also promote mesodermal competence to respond to *Sonic.* FGF-4-soaked beads were stapled to AER-denuded anterior mesoderm which was infected with *Sonic* virus. Gene expression and mesodermal outgrowth were monitored as described previously. In the presence of both *Sonic* virus and FGF-4 protein, *Hoxd-11, Hoxd-13* and *Bmp-2* expression are all induced. The expression levels of the induced genes are similar to or greater than the endogenous expression levels, and are equivalent in magnitude to their induction in the presence of the AER. Thus *Fgf-4* can induce the competence of the mesoderm to respond to *Sonic.*

*Sonic* alone is insufficient to induce either gene expression or mesodermal proliferation in the absence of the AER, while the combination of *Sonic* and FGF-4 induces both proliferation and gene expression. It was than asked whether FGF-4 alone has any effect on gene induction or mesodermal proliferation. Application of FGF-4 in the absence of *Sonic* virus does not induce *Hoxd* or *Bmp-2* gene expression above control levels, however FGF-4 alone induces mesodermal outgrowth. These results suggest that mesodermal gene activation requires direct action of *Sonic* on the mesoderm and that proliferative response to *Sonic* is indirect, due to the induction of FGFs.

### (v) Sonic Induces Polarized Fgf-4 Expression in the AER

*Fgf-4* is expressed in a graded fashion in the AER of the mouse limb bud, with maximal expression at the posterior region of the AER tapering to undetectable levels in the anterior ridge (Niswander and Martin, (1992) Development 114:755-68). Therefore, it was appropriate to investigate whether *Fgf-4* is asymmetrically expressed in the chick AER, and whether its expression is induced by *Sonic.* A fragment of the chicken *Fgf-4* gene was cloned from a stage 22 chicken limb library by PCR using degenerate primers designed from mouse *Fgf-4* and Xenopus *e-Fgf* sequence; based on information provided by L. Niswander and G. Martin. Assignment of gene identity was based on primary sequence as well as comparison of expression patterns with that of murine *Fgf-4* (Niswander and Martin, (1992) Development 114:755-68). Whole mount in situ hybridization analysis showed strong limb expression of chick *Fgf-4* in the AER. *Fgf-4,* like *Bmp-2,* is expressed all the way to the posterior border of the AER, but its anterior domain ends before the morphological end of the AER creating a posterior bias that has also been observed by Niswander et al., (1994) Nature (in press). Expression is first detected in the distal AER at about stage 18. As outgrowth proceeds the posterior bias develops. Expression peaks around stage 24/25 and then fades by stage 28/29.

The expression domain of *Fgf-4* becomes posteriorly biased as *Sonic* is expressed in the posterior mesoderm. This observation is consistent with *Sonic* influencing the expression of *Fgf-4* in the posterior AER. To test the effect of *Sonic* on *Fgf-4* expression in the AER, stage 18-20 embryos were infected with *Sonic* virus in a single point at their anterior margin beyond the anterior limit of the AER. The embryos were harvested one to two days later, when an extension of the anterior AER became apparent. The expression, of *Fgf-4* was analyzed by in situ hybridization. *Fgf-4* expression is induced in the anteriormost segment of the AER, in a region which is discontinuous with the endogenous expression domain, and overlies the domain of viral *Sonic* infection. This result contrasts with the *Bmp-2* expression induced in the extended AER, which is always continuous with the endogenous expression domain. The asymmetry of the induced *Fgf-4* expression indicates that *Sonic* polarizes the extended AER, much as a ZPA graft does (Maccabe and Parker, (1979) J. Embryol. Exp. Morph. 53:67-73). Since FGFs by themselves are mitogenic for limb mesoderm, these results are most consistent with *Sonic* inducing distal proliferation indirectly, through the induction of mitogens in the overlying AER.

### (vi) Reciprocal Regulation of Sonic by Fgf-4

*Sonic* thus appears to be upstream of *Fgf-4* expression in the AER. However, since the AER is required to maintain polarizing activity in the posterior mesoderm (Vogel and Tickle, (1993) Development 19:199-206; Niswander et al., (1993) Cell 75:579-87), *Sonic* may also be downstream of the AER. If *Sonic* is regulated by the AER and the AER by *Sonic,* this would imply that they are reinforcing one another through a positive feedback loop.

To test whether the AER dependence of ZPA activity is controlled at the level of transcription of the *Sonic* gene, *Sonic* expression following removal of the AER from the posterior half of the limb bud was assayed. *Sonic* expression is reduced in an operated limb compared to the contralateral control limb within ten hours of AER removal, indicating that *Sonic* expression is indeed AER dependent. The dependence of *Sonic* expression on signals from the AER suggests that one of the functions of the AER is to constrain *Sonic* expression to the more distal regions of the posterior mesoderm.

In addition to their mitogenic and competence-inducing properties, FGFs can also substitute for the AER to maintain the ZPA. In order to test whether FGFs can support the expression of *Sonic,* beads soaked in FGF-4 protein were stapled to the posterior-distal tips of limb buds after posterior AER removal. Embryos were assayed for *Sonic* expression approximately 24 hours later, when *Sonic* expression is greatly reduced in operated limb buds which had not received an FGF-4 bead. Strong *Sonic* expression is detectable in the posterior mesoderm, slightly proximal to the bead implant, and reflecting the normal domain of *Sonic* expression seen in the contralateral limb. With the finding that FGF-4 can maintain *Sonic* expression, the elements required for a positive feedback loop between *Sonic* expression in the posterior mesoderm and *Fgf-4* expression in the posterior AER are established (see also Niswander et al. (1994) Nature (in press)).

The induction of *Bmp-2* expression by *Sonic* requires signals from the AER, and its domain correlates over time with that of *Sonic.* Therefore, it was interesting to learn if the continued expression of *Bmp-2* also requires signals from the AER, and if so, whether they could be replaced by FGF-4. To test this, *Bmp-2* expression following posterior AER removal, and following its substitution with an FGF-4 bead was assayed. *Bmp-2* expression fades within hours of AER removal, and can be rescued by FGF-4. These data indicate that the maintenance of *Bmp-2* expression in the posterior mesoderm, like that of *Sonic,* is dependent on signals from the AER, which are likely to be FGFs.

### (vii) The Mesodermal Response to Sonic

It has been found that only mesoderm underlying the AER is responsive to *Sonic,* apparently because the AER is required to provide competence signals to the limb mesoderm. *Fgf-4,* which is expressed in the AER, can substitute for the AER in this regard, and thus might act in combination with *Sonic* to promote *Hoxd* and *Bmp-2* gene expression in the mesoderm. FGFs may be permissive factors in a number of instructive pathways, as they are also required for activins to pattern Xenopus axial mesoderm (Comell and Kimelman, (1994) Development 120:2187-2198*;* LaBonne and Whitman, (1994) Development 120:463-472).

The induction of *Hoxd* and *Bmp-2* expression in response to *Sonic* and FGF-4 in the absence of an AER suggests that the mesoderm is a direct target tissue of *Sonic* protein. Since *Sonic* can induce *Fgf-4* expression in the AER, it follows that *Sonic* also acts indirectly on the mesoderm through the induction of competence factors in the AER.

### (viii) Downstream Targets and a Cascade of Signals Induced by Sonic

The five AbdB-like *Hoxd* genes, *Hoxd-9* through -*13*, are initially expressed in a nested pattern centered on the posterior of the limb bud, a pattern which suggests they might be controlled by a common mechanism (Dolle, et al., (1989) Cell 75:431-441; Izpisua-Belmonte, et al., (1991) Nature 350:585-9). The analysis of the endogenous and induced domains of *Hoxd* gene expression suggests that *Sonic* normally induces expression of *Hoxd-11,* -*12* and -*13*. In contrast it was found that *Hoxd-9* and *-10* expression initiate before *Sonic* mRNA is detectable. This implies that at least two distinct mechanisms control the initiation of *Hoxd* gene expression in the wing bud, only one of which is dependent on *Sonic.*

Several observations suggest that the elaboration of the *Hoxd* expression domains is not controlled directly by *Sonic,* but rather by signals which are downstream of *Sonic.* The *Hoxd* expression domains rapidly diverge from *Sonic,* and evolve into several distinct subdomains. Moreover these subdomains appear to be separately regulated, as analysis of the murine *Hoxd-11* gene promoter suggests that it contains independent posterior and distal elements (Gerard, et al., (1993) Embo. J. 12:3539-50). In addition, although initiation of *Hoxd-11* through -13 gene expression is dependent on the AER, their expression is maintained following AER removal (Izpisua-Belmonte, et al., (1992) Embo. J. 11:1451-7). As *Sonic* expression fades rapidly under similar conditions, this implies that maintenance of *Hoxd* gene expression is independent of *Sonic.* Since ectopic' *Sonic* can induce a recapitulation of the *Hoxd* expression domains in the limb, it can be concluded that although indirect effectors appear to regulate the proper patterning of the *Hoxd* expression domains, they are downstream of *Sonic.* Potential mediators of these indirect effects include *Bmp-2* in the mesoderm and *Fgf-4* from the AER.

In contrast to the *Hoxd* genes, *Bmp-2* gene expression in the posterior limb mesoderm appears to be continually regulated by *Sonic.* It was found that both endogenous and ectopic *Bmp-2* expression correspond to that of *Sonic.* Furthermore, continued *Bmp-2* expression is dependent on the AER and can be rescued by FGF-4. It is likely that this is an indirect consequence of the fact that *Sonic* expression is also maintained by the AER and can be rescued by FGF-4. In fact, *Bmp-2* expression might be a direct response of cells to secreted *Sonic* protein. The differences between *Bmp-2* and *Hoxd* gene expression suggest that multiple pathways downstream of *Sonic* regulate gene expression in the mesoderm.

*Bmp-2* itself is a candidate for a secondary signaling molecule in the cascade of patterning events induced by *Sonic. Bmp-2* is a secreted molecule of the TGF-β family and its expression can be induced by *Sonic.* This appears to be an evolutionarily conserved pathway, as Dros-HH, the Drosophila homolog of *Sonic,* activates the expression of *dpp,* the homolog of *Bmp-2,* in the eye and wing imaginal discs (Heberlein, et al., (1993) Cell 75:913-26; Ma, et al., (1993) Cell 75:927-38; Tabata and Kornberg, (1994) Cell 76:89-102). Expression of Dros-HH is normally confined to the posterior of the wing disc. Ectopic expression of Dros-HH in the anterior of the disc results in ectopic expression of *dpp* and ultimately in the duplication of wing structure with mirror image symmetry (Bassler and Struhl, (1994) Nature 368:208-214). This effect is strikingly parallel to the phenotypic results of ectopic expression of *Sonic* in the chick limb.

### (ix) Regulation of Sonic Expression

*Sonic* expression is activated in the posterior of the limb bud very early during mesodermal outgrowth (Riddle et al., (1993) Cell 75:1401-16). The factors which initiate this localized expression are not yet identified but ectopic expression of *Hoxb-8* at the anterior margin of the mouse limb bud results in the activation of a second domain of *Sonic* expression under the anterior AER (Charité el al., (1994) Cell 78:589-601). Since retinoic acid is known to be able to induce the expression of *Hoxb-8* and other *Hox* genes in vitro (Mavilio et al., (1988) Differentiation 37:73-79) it is possible that endogenous retinoic acid acts to make cells competent to express *Sonic* by inducing expression of upstream *Hox* genes, either in the very early limb bud or in the flank prior to the limb bud formation.

Several lines of evidence suggest that once induced *Sonic* expression is dependent on signals from the posterior AER. Following its initiation in the posterior limb mesoderm, the *Sonic* expression domain moves distally as the limb bud grows out, always remaining subjacent to the AER. Similarly, *Sonic* expression can also be induced on the anterior margin of the limb bud by implantation of a retinoic acid bead, but the induced ectopic expression is limited to the mesoderm directly underlying the AER (Riddle, et al., (1993) Cell 75:1401-16 In addition, ZPA activity fades rapidly following removal of the AER (Niswander, et al., (1993) Cell 75:579-87*;* Vogel and Tickle, (1993) Development 119:199-206), and ZPA grafts only function when placed in close proximity to the AER (Tabin, (1991) Cell 66:199-217; Tickle, (1991) Development Supp. 1:113-21). The observation that continued *Sonic* expression depends on signals from the posterior AER reveals the mechanism underlying these observations.

The reliance of *Sonic* expression on AER-derived signals suggests an explanation for the distal shift in *Sonic* expression during limb development (Riddle et al., (1993) Cell 75:1401-16). Signals from the AER also promote distal outgrowth of the mesodermal cells of the progress zone, which in turn results in the distal displacement of the AER. Hence, as maintenance of *Sonic* expression requires signals from the AER, its expression domain will be similarly displaced.

It was found that replacement of the AER with *FGF-4* soaked beads results in the maintenance of *Sonic* expression. This result is consistent with the previous findings that ZPA activity can be maintained in vivo and in vitro by members of the FGF family (Anderson, et al., (1993) Development 117:1421-33; Niswander et al., (1993) Cell 75:1401-16 ; Vogel and Tickle, (1993) Development 119:199-206). Since *Fgf-4* is normally expressed in the posterior AER, these results suggest that *Fgf-4* is the signal from the ectoderm involved in maintaining *Sonic* expression.

### (x) Sonic and Regulation and Maintenance of the AER

*Sonic* can induce anterior extensions of the AER which have an inverted polarity relative to the endogenous AER. This polarity is demonstrated by examining the expression of two markers in the AER. In normal limbs *Bmp-2* is expressed throughout the AER, while *Fgf-4* is expressed in the posterior two thirds of the AER. In the extended AER resulting from ectopic *Sonic* expression, *Bmp-2* is again found throughout the AER, while *Fgf-4* expression is biphasic, found at either end of the AER, overlying the anterior and posterior mesodermal domains expressing *Sonic.* These results are consistent with previous observations that antero-posterior polarity of the AER appears to be regulated by the underlying mesoderm, and that ZPA grafts lead to the induction of ectopic, polarized AER tissue (Maccabe and Parker, (1979) J. Embryol. Exp. Morph. 53:67-73). Our results also suggest that the normal AP polarity of the AER is a reflection of endogenous *Sonic* expression. The induced AER is sufficient to promote complete PD outgrowth of the induced structures (Riddle et al., (1993) Cell 75:1401-16). Hence whatever factors are necessary to maintain the AER are also downstream of *Sonic.*

### (xi) A Positive Feedback Loop Between Sonic and Fgf-4

The induction of *Fgf-4* expression by *Sonic* in the ectopic AER, and the maintenance of *Sonic* expression by FGF-4 suggest that *Sonic* and *Fgf-4* expression are normally sustained by a positive feedback loop. Such a feedback loop would allow the coordination of mesodermal outgrowth and patterning. This coordination is possible because *Sonic* patterns mesodermal tissue and regulates *Fgf-4* expression, while *FGF-4* protein induces mesodermal proliferation and maintains *Sonic* expression. Moreover mesodermal tissue can only be patterned by *Sonic* in the context of a competence activity provided by F8f-4. Thus patterning is always coincident with proliferation.

It remains possible that exogenously applied *Fgf-4* might be mimicking the activity of a different member of the FGF family. For example, Fgf-2 is expressed in the limb mesoderm and the AER (Savage et al., (1993) Development Dynamics 198:159-70) and has similar effects on limb tissue as *Fgf-4* (Niswander and Martin, (1993) Nature 361:68-71; Niswander, et al., (1993) Cell 75:579-87; Riley, et al., (1993) Development 118:95-104; Fallon, et al., (1994) Science 264:104-7).

### (xii) Coordinated Regulation of Limb Outgrowth and Patterning

Patterning and outgrowth of the developing limb are known to be regulated by two major signaling centers, the ZPA and AER. The identification of *Sonic* and FGFs as molecular mediators of the activities of the ZPA and AER has allowed for dissociation of the activities of these signaling centers from their regulation, and investigation of the signaling pathways through which they function.

The results presented above suggest that the ability of cells to respond to *Sonic* protein is dependent on FGFs produced by the AER. It was also found that *sonic* induces a cascade of secondary signals involved in regulating mesodermal gene expression patterns. In addition evidence was found for a positive feedback loop initiated by *Sonic,* which maintains expression of *Sonic* in the posterior mesoderm and *Fgf-4* in the AER. The feedback loop described suggests a mechanism whereby outgrowth and patterning along the AP and PD axes of the limb can be coordinately regulated.

The results described above further suggest that *Sonic* acts as a short range signal which triggers a cascade of secondary signals whose interplay determines the resultant pattern of structures. The data suggest a number of inductive pathways that can be combined to generate a model (Figure 14) which describes how *Sonic,* in coordination with the AER, acts to pattern mesodermal tissues along the anterior-posterior limb axis, while simultaneously regulating proximal-distal outgrowth.

Following its induction, *Sonic* signals to both the limb ectoderm and mesoderm. *Sonic* imposes a distinct polarity on the forming AER, including the posteriorly biased expression of *Fgf-4,* and the AER becomes dependent on continued *Sonic* expression. The mesoderm, as long as it is receiving permissive signals from the overlying ectoderm, responds to the *Sonic* signal by expressing secondary signaling molecules such as *Bmp-2* and by activating *Hoxd* genes. *Bmp-2* expression is directly dependent on continued *Sonic* expression, while the continued expression of the *Hoxd* genes, rapidly becomes *Sonic.* independent. In a reciprocal fashion, maintenance of *Sonic hedgehog* expression in the posterior mesoderm becomes dependent on signals from the AER. Since the factors expressed by the AER are not only required for the maintenance of *Sonic* expression and activity, but are also mitogenic, growth and patterning become inextricably linked. Coordination of limb development through interdependent signaling centers forces the AP and PD structures to be induced and patterned in tandem. The pathways elucidated herein thus provide a molecular framework for the controls governing limb patterning

### Example 8

### Sonic, BMP-4, and Hox Gene Expression Suggest a Conserved Pathway in Patterning the Vertebrate and Drosophila Gut

### (i) Experimental Procedure

### In Situ Hybridization and Photography

BMP probes were isolated using primers designed to amplify members of the TGF-and BMP families (Basler, K. et al., (1993) Cell 73:687-702, eight independent 120 bp BMP fragments were amplified from a stage 22 chicken posterior limb bud plasmid cDNA library. These fragments were pooled and used to screen an unamplified stage 22 limb bud lambda zap cDNA library constructed as in Riddle et al., (1993) Cell 75:1401-16. Among the BMP related clones isolated were an approximately 1.9 kb cDNA clone corresponding to chicken *BMP-2* and an approximately 1.5 kb cDNA clone corresponding to chicken *BMP-4.* Both clones contain the entire coding regions. The *Sonic* clone was obtained as described in Riddle et al, (1993) Cell 75:1401-16. Digoxigenin-UTP labeled RNA probes were transcribed as per Riddle et al., (1993) Cell 75:1401-16. Briefly, harvested chick embryos were fixed overnight in 4% paraformaldehyde, washed in PBS then processed for whole mount *in situ* hybridization methods are per Riddle et al., (1993)Cell 75:1401-16. Embryos were photographed from either ventral or dorsal surfaces under transmitted light using a Nikon zoom stereo microscope with Kodak Ektar 100 ASA film. Whole mount *in situ* hybridization embryos and viscera were processed for sectioning as described in Riddle et al., (1993)Cell 75:1401-16. 15-25 µm transverse sections were air dried and photographed with brightfield or numarski optics using a Zeiss Axiophot microscope and Kodak Ektar 25 ASA film.

### Chick Embryos and Recombinant Retroviruses

A retroviral vector engineered to express a full length cDNA of chicken *Sonic,* as in Riddle et al. (1993) Cell 75:1401-16, was injected unilaterally into stage 8-13 chicken embryos targeting the definitive endoderm at the mid-embryo level. At this stage the CIP has not formed and neither *Sonic* nor *BMP-4* are expressed in the region injected. Injections were performed on the ventral surface on embryos cultured with their ventral surface facing up (New, D.A.T. (1955) Embryol. Exp. Morph. 3:320-31. Embryos were harvested 18-28 hours after injection and prepared for whole mount *in situ* hybridization (see above description of *in situ* experiment), hybridized with *Sonic* or *BMP-4* digoxigenin labeled probes.

### In Situ Hybridization with Hox Genes

Cloned cDNA of the chicken homologues of *Hoxa*-9,-10,-11,-13*; b*-9, *c*-9,-10,-11; *d-*9,-10,-11,-12,and -13 were used to transcribe digoxigenen-UTP labeled riboprobes for whole mount *in situ* hybridization. Domestic chick embryos were harvested into PBS and eviscerated. The visceral organ block was fixed in 4% paraformaldehyde overnight and processed for whole mount *in situ* hybridization. Methods and photographic technique as described above.

### (ii) Expression of Sonic and BMP-4 in Stage 13 Chick Embryos Determined by Whole Mount In Situ Hybridization

Chick gut morphogenesis begins at stage 8 (Hamberger and Hamilton, (1987) Nutr. 6:14-23 with a ventral in-folding of the anterior definitive endoderm to form the anterior intestinal portal (AIP) (Romanoff, A.L., (1960) The Avian Embryo, The Macmillan Co., NY. This lengthens posteriorly forming the foregut. A second wave of endodermal invagination is initiated posteriorly at stage 13, creating the caudal intestinal portal (CIP). The CIP extends anteriorly forming the hindgut. *Sonic* expression, previously noted in the endoderm of the vertebrate gut (Riddle et al., (1993) Cell 75:1401-16; Echelard et al., (1993) Cell 75:1417-1430), is expressed early in a restricted pattern in the endodermal lips of the AIP and CIP. *Sonic* expression is detected in the endoderm of the AIP and CIP in pre gut closure stages. At later stages, stage 28 embryos, *Sonic* is expressed in the gut in all levels (fore-, mid-, and hind-gut) restricted to the endoderm. *Sonic* is known to be an important inductive signal in other regions of the embryo including the limb bud (Riddle et al., (1993) Cell 75:1401-16) and neural tube (Echelard et al., (1993) Cell 75:1417-1430; Kraus et al., (1994) Cell 75:1437-1444; Roelink et al., (1994) Cell 76:761-775). Since primitive gut endoderm is known to cause gut-specific mesodermal differentiation when combined with non-gut mesenchyme (Haffen et al., (1987) Nutr. 6:14-23), we speculated that *Sonic* might function as an inductive signal to the visceral mesoderm. A potential target gene for the action of *Sonic* was suggested by analogy to the *Drosophila* imaginal discs where Dros-HH, the homologue of vertebrate *Sonic,* activates the expression of the *TGF-*β related gene *dpp* in adjacent cells (Tabata abd Kornberg, (1994) Cell 76:89-102; Heberlein et al., (1993) Cell 75:913-926; Ma et al., (1993) Cell 75:913-926; Basler et al., (1993) Cell 73:687-702). There are two vertebrate homologues of *dpp, BMP-2* and *BMP-4.* The earliest detectable expression of *BMP-4* occurs simultaneously with the first observable expression of *Sonic* in the developing *gut. BMP-4* is expressed in a domain abutting *Sonic* at the AIP and the CIP, but is restricted to the adjacent ventral mesoderm. *BMP-4* gut expression persists into later stage embryos, stage 33 embryos, in the visceral mesoderm only. The tissue restricted expression of both genes is maintained in all stages studied. *BMP-2* is not expressed in the gut at the AIP or CIP, but is expressed in clusters of cells in the gut mesoderm in later stages, a pattern distinct from that of *BMP-4.*

### (iii). Ectopic Expression of Sonic Induces Ectopic Expression of BMP-4 in Mesodermal Tissues of the Developing Chick

To test whether *Sonic* is capable of inducing *BMP-4* in the mesoderm we an ectopic expression system previously used to study the role of *Sonic* in limb development was utilized (Riddle et al., (1993) Cell 75:1401-16). A replication competent retrovirus engineered to express *Sonic* was injected unilaterally into the presumptive endoderm and visceral mesoderm at mid-embryo positions in stage 8-13 chick embryos *in vitro* (New, D.A.T. (1955) Embryol. Exp. Morph. 3:320-321). When embryos were examined by *in situ* hybridization 18-26 hours later, the normal wild type expression of *Sonic* is detected at the AIP, CIP, and in the midline (neural tube and notochord). Ectopic *Sonic* expression is present unilaterally on the left ventral surface. Also, wild type *Sonic* expression is seen in the floor plate of the neural tube and notochord. Ectopic expression is seen unilaterally in the visceral endoderm, its underlying splanchnic mesoderm, and somatic mesoderm. *BMP-4* expression can be seen induced in the mesoderm at the site of injection, in addition to its normal expression in the mesoderm of the CIP. Wild type *BMP-4* expression is seen in the most dorsal aspects of the neural tube and symmetrical lateral regions adjacent to the neural tube. Induced BMP-4 expression is present unilaterally in the splanchnic mesoderm at the site of *Sonic* viral injection, and not in the visceral endoderm.

Since *BMP-4* is, itself, a secreted protein, it could function as a secondary signal in an inductive cascade, similar to the signal cascades from Dros-HH to *dpp* in *Drosophila* imaginal discs (Tabata abd Kornberg, (1994) Cell 76:89-102; Heberlein et al., (1993) Cell 75:913-926; Ma et al., (1993) Cell 75:913-926; Basler et al., (1993) Cell 73:687-702) and from *Sonic* to *BMP-2* in the limb bud. In the gut, *BMP-4* could act as a secondary signal either as part of a feedback loop to the endoderm or within the visceral mesoderm. This latter possibility is consistent with the finding that in mice homozygous for a deletion in the *BMP-4* gene, the ventral mesoderm fails to close.

### (iv) Expression of Hox Genes in the Developing Chick Gut

There is a striking parallel between the apparent role of *Sonic* as an endoderm-to-mesoderm signal in early vertebrate gut morphogenesis and that of its *Drosophila* homologue, Dros-HH. Dros-HH (like *Sonic*) is expressed in the *Drosophila* gut endoderm from the earliest stages of morphogenesis (Taylor et al., (1993) Mech. Dev. 42:89-96). Its putative receptor, patched, is found in the visceral mesoderm implicating Dros-HH (like *Sonic*) in endodermal-mesodermal inductive interactions. This led to consideration whether other genes known to be involved in regulating *Drosophila* gut development might also play a role in regulating chick gut morphogenesis. Regionally specific pattern in *Drosophila* gut endoderm is regulated by a pathway involving restricted expression of homeotic genes in the mesoderm (McGinnis and Krumlauf, (1992) Cell 68:283-302). Although the basis for patterning the vertebrate gut is poorly understood, in several other regions of the embryo *Hox* genes have been implicated as key regulators of patterns. Vertebrate *Hox* genes are expressed in overlapping anteroposterior domains which correlate with structural boundaries in the developing hindbrain, vertebrae, and limbs (McGinnis and Krumlauf, (1992) Cell 68:283-302). Whole mount *in situ* hybridization was used to test whether these genes are also expressed in the developing vertebrate hindgut and whether their domains of expression correlate with morphologic borders of the chick gut.

Lumenal gut differentiation creates three morphologically and physiologically distinct regions: fore-, mid-, and hind- gut. The fore-gut and hind-gut are the derivatives of the primitive gut tubes initiated at the AIP and CIP respectively. Ultimately these tubes meet and fuse at the yolk stalk around stage 24-28. The midgut is formed from both foregut and hindgut primordia, just anterior and posterior to the yolk stalk.

The most posterior derivative of the hindgut is the cloaca, the common gut-urogenital opening. The rest of the hindgut develops into the large intestine. The midgut/hindgut border is demarcated by a paired tubal structure, the ceca (analogous to the mammalian appendix), which forms as budding expansions at the midgut/hindgut border at stage 19-20. Anterior to the ceca, the midgut forms the small intestine.

The expression pattern of the 5' members of the *Hox* gene clusters in the chick hindgut by whole mount *in situ* hybridization was studied. *Hox* gene expression patterns in the gut are dynamic. They are initially expressed (by stage 10) in broad mesodermal domains extending anteriorly and laterally. Later they become restricted. By stage 25, the Abd-B like genes of the *Hoxa* and *Hoxd* cluster are regionally restricted in their expression in hindgut mesoderm. The most anteriorly expressed gene, *Hoxa*-9*,* has an anterior border of expression within the mesoderm of the distal midgut (to a point approximating the distal third of the midgut length). Each successive gene within the A and D *Hox* clusters has a more posterior domain of expression. *Hoxa*-10, *Hoxd*-9 and *Hoxd*-10 are restricted in their expression to the ceca. *Hoxa*-11 and *Hoxd*-11 have an anterior limit of expression in the mid-ceca at the approximate midgut/hindgut boundary (Romanoff, A.L. (1960) The Avian Embryo, The Macmillan Co. NY). *Hoxd*-12 has an anterior limit at the posterior border of the ceca and extends posteriorly throughout the hindgut to the cloaca. *Hoxa*-13 and *Hoxd*-13 are expressed in the most posteriorly restricted domain, in the ventral mesoderm surrounding the cloaca. *Hoxa*-13 and *Hoxd*-13 are the only Abd-B like genes which are also expressed within the gut endoderm, from the ceca to the cloaca.

The only member of the B or C *Hox* clusters which we found to be expressed in the hindgut is *Hoxc*-9. The expression of *Hoxc*-9 overlaps with its paralogues *Hoxa*-9 and *Hoxd-*9 in the midgut mesoderm, but has a sharp posterior boundary, complementary to *Hoxa*-11 and *Hoxd*-11 in the mid-ceca.

The restricted expression of the *Abd-B* like *Hox* genes appear to demarcate the successive regions of the gut which will form the cloaca, the large intestine, the ceca, the mid-ceca at the midgut/hindgut border, and the lower portion of the midgut (perhaps identifying that portion of the midgut derived from the posterior gut tube3). Moreover, these molecular events presage regional distinctions. Expression of all *Hox* genes could be detected by stage 14, well before the hindgut lumen is closed (by stage 28) and is maintained in subsequent stages studied. Cytodifferentiation of the hindgut mesoderm and epithelium begins later, at stages 29-31 (Romanoff, A.L. (1960) The Avian Embryo, The Macmillan Co. NY).

These results suggest that specific *Hox* genes might be responsible for regulating morphogenesis of the gut. Consistent with this, there is an apparent homeotic alteration in the gut of a transgenic mouse in which the anterior limit of expression of *Hoxc*-8 is shifted rostrally: a portion of foregut epithelium mis-differentiates as midgut (Pollock and Bieberich, (1992) Cell 71:911-923).

### (v) Conservation in the Expression of Regulatory Genes Involved in the Formation of Vertebrate and Drosophila Gut

There is an intriguing parallel between the expression patterns of *Sonic, BMP-4,* and the *Hox* genes in the vertebrate gut and those of their homologues during *Drosophila* gut morphogenesis (Figure 15). This conservation is of particular interest because in *Drosophila* the role played by these genes has been clarified genetically. Dros-HH (like its vertebrate homologue, *Sonic*) is expressed at the earliest stages in the gut endoderm and may be a signal to visceral mesoderm (Taylor et al., (1993) Mech. Dev. 42:89-96). Nothing is known directly of the relationship between Dros-HH expression and activation of expression of other genes in the *Drosophila* gut. However, in *Drosophila* imaginal discs, Dros-HH is known to activate the expression of *dpp* in a signaling cascade (Kraus et al., (1994) Cell 75:1437-1444; Heberlein et al., (1993) Cell 75:913-926; Ma et al., (1993) Cell 75:913-926; Basler et al., (1993) Cell 73:687-702). Later in gut development, the production of *dpp* in the mesoderm contributes to the regulation of the expression of homeotic genes in both the mesoderm and the endoderm (Bienz, M. (1994) TIG 10:22-26). *Drosophila* homeotic genes are expressed in the gut visceral mesoderm and their expression is known to determine the morphologic borders of the midgut. This involves proper induction of gene expression in the adjacent endoderm, one of the mediators of the interaction is *dpp* (Bienz, M. (1994) TIG 10:22-26). If Dros-HH is required for the ultimate activation of the homeotic genes in the *Drosophila* midgut, this would parallel the situation in the vertebrate limb bud where *Sonic* functions as an upstream activator of the *Hox* genes (Riddle et al., (1993) Cell 75:1401-1416), perhaps in a signaling cascade involving *BMP-2.*

The extraordinary conservation in the expression of regulatory genes in the vertebrate and *Drosophila* gut strongly suggests a conservation of patterning mechanisms. Pathways established by genetic studies in *Drosophila* provide direct insights into the molecular basis for the regionalization and morphogenesis of the vertebrate gut.

### Example 9

### Bacterially Expressed Hedgehog Proteins Retain Motorneuron-inducing Activity

Various fragments of the mouse *Shh* gene were cloned into the pET11D vector as fusion proteins with a poly(His) leader sequence to facilitate purification. Briefly, fusion genes encoding the mature M-*Shh* protein (corresponding to Cys-25 through Ser-437 of SEQ ID No. 11) or N-terminal containing fragments, and an N-terminal exogenous leader having the sequence M-G-S-S-H-H-H-H.H-H L-V-R-R-G-S-H-M (SEQ ID No: 47) were cloned in pET11D and introduced into *E. coli.* The *poly*(His)-*Shh* fusion proteins were purified using nickel chelate chromatography according to the vendor's instructions (Qiagen catalog 30210), and the poly(His) leader cleaved from the purified proteins by treatment with thrombin.

Preparations of the purified *Shh* proteins were added to tissue explants (neural tube) obtained from chicken embryos and cultured in a defined media (e.g., no serum). M-*Shh* protein was added to final concentrations of between 0.5pM to 5nM, and differentiation of the embryonic explant tissue to motorneuron phenotype was detected by expression of Islet-1 antigen. The bacterially produced protein was demonstrated to be active in the explant cultures at concentrations as low as 5 to. 50pM. An *Shh* polypeptide containing all 19kd of the amino terminal fragment and approximately 9kd of the carboxyl terminal fragment (see Example 6) displayed both motor neuron inducing activity and weak floor plate inducing activity, indicating that these activities likely reside with the N-terminal fragment.

### Equivalents

Those skilled in the art will recognize, or be able to ascertain using no more than routine experimentation, numerous equivalents to the specific polypeptides, nucleic acids, methods, assays and reagents described herein. Such equivalents are considered to be within the scope of this invention and are covered by the following claims.

## Claims

1. An isolated and/or recombinantly produced vertebrate hedgehog polypeptide comprising an amino acid sequence which is at least 90 percent identical to SEQ ID No: 11, or to a fragment thereof of at least 50 contiguous amino acids, which hedgehog polypeptide can (i) induce proliferation, survival, and/or differentiation of mesodermally-derived tissue, and/or (ii) induce proliferation, survival, and/or differentiation of ectodermally-derived tissue.

2. The isolated and/or recombinantly produced vertebrate hedgehog polypeptide of claim 1, wherein said polypeptide comprises an amino acid sequence which is at least 90 percent identical to SEQ ID NO: 11, or to a fragment thereof of at least 150 contiguous amino acids, which polypeptide can (i) induce proliferation, survival, and/or differentiation of mesodermally-derived tissue, and/or (ii) induce proliferation, survival, and/or differentiation of ectodermally-derived tissue.

3. The polypeptide of claim 1, wherein said polypeptide has an approximate molecular weight of 27 kd.

4. The polypeptide of claim 1, wherein said polypeptide has an approximate molecular weight of 19 kd.

5. The polypeptide of claim 1, wherein said polypeptide comprises an N-terminal portion of SEQ ID NO: 11.

6. The polypeptide of claim 1, wherein (i) the hedgehog amino acid sequence is encoded by a naturally occurring hedgehog gene of a mouse, (ii) the hedgehog amino acid sequence comprises an N-terminal fragment of a hedgehog protein with an approximate molecular weight of 19kd, (iii) the polypeptide induce expression of BMP-2, BMP-4, or Hoxd genes, (iv) the polypeptide is purified to have less than 20% by dry weight of contaminating proteins, and/or (v) the polypeptide is purified to have less than 5% by dry weight of contaminating proteins.

7. The polypeptide of claim 1, wherein the fragment thereof includes at least 100 contiguous amino acids.

8. The polypeptide of claim 2, wherein said polypeptide comprises an amino acid sequence identical to SEQ ID No: 11, or a fragment thereof of at least 150 contiguous amino acids.

9. The polypeptide of any one of claims 1 or 3, wherein the polypeptide comprises an amino acid sequence represented by SEQ ID No: 40.

10. The polypeptide of any one of claims 1 to 9, wherein the amino acid sequence is encoded by a nucleic acid sequence that hybridizes under stringent conditions, including a wash step of 0.2X SSC at 65 °C, with the vertebrate hedgehog nucleic acid sequence of SEQ ID No: 4.

11. The polypeptide of any one of claims 1, 2, 6, and 10, wherein the polypeptide is a fusion protein.

12. The polypeptide of claim 11, wherein the fusion protein further includes
(i) a detectable label for detecting the presence of said fusion protein, or
(ii) a matrix-binding domain for immobilizing said fusion protein.

13. The polypeptide of any one of claims 1, 2, 6, and 10, wherein the polypeptide is post-translationally modified.

14. The polypeptide of claim 13, wherein the polypeptide is glycosylated.

15. The polypeptide of any one of claims 1, 2, 10, and 13, wherein (i) the polypeptide is purified to at least 80% by dry weight or (ii) the polypeptide is purified to at least 95% by dry weight.

16. A preparation including the polypeptide of claim 1 formulated in a pharmaceutically acceptable medium, vehicle or diluent.

17. An antibody preparation specifically reactive with an epitope of the hedgehog polypeptide of claim 1.

18. An antibody preparation that binds to an epitope of the hedgehog polypeptide of claim 1.

19. The antibody preparation of claim 17 or 18, wherein the preparation comprises a monoclonal antibody.

20. The antibody preparation of claim 17 or 18, wherein the preparation comprises polyclonal antibodies.

21. Use of the polypeptide of claim 1 for the preparation of a medicament for the treatment of a degenerative disorder of the nervous system **characterized by** neuronal cell death.

22. The use of claim 21, wherein said degenerative disorder is selected from the group consisting of a neuromuscular disorder, an autonomic disorder, a central nervous system disorder, Alzheimer's disease, Parkinson's disease, amyotrophic lateral sclerosis, Pick's disease, Huntington's disease, multiple sclerosis, neuronal damage resulting from anoxia-ischemia, neuronal damage resulting from trauma, and neuronal degeneration associated with a natural aging process.

23. The use of claim 21, wherein the medicament further comprises a therapeutically effective amount of a growth factor having neurotrophic activity.

24. The use of claim 23, wherein said growth factor is selected from the group consisting of a nerve growth factor, cilliary neurotrophic growth factor, schwannoma derived growth factor, glial growth factor, striatal-derived neuronotrophic factor and platelet-derived growth factor.

25. Use of the polypeptide of claim 1 in the manufacture of a medicament for treating a neurodegenerative disorder.

26. The use of claim 25, wherein said disorder is selected from Parkinson's disease, Alzheimer's Disease, Huntington's Disease, Pick's Disease, Ballism, Guillain-Barre Syndrome, Amyotrophic Lateral Sclerosis, spinoccrcbcllar degenerations, and peripheral neuropathy.

27. The use of claim 25, wherein said neurodegenerative disorder includes loss of neurons cells selected from cholinergic neurons, GABAergic neurons, and striatal neurons.

28. Use of the polypeptide of claim 1 in the manufacture of a medicament for preventing, treating or reducing the severity of an acute, subacute or chronic injury to the nervous system in a subject.

29. The use of claim 28, wherein said injury is selected from traumatic injury, chemical injury, vasal injury, vasal deficit, infectious injury, inflammatory injury, chronic immunological disease, tumor-induced injury, or ischemia resulting from a stroke.

30. The use of claim 29, wherein said immunological disease is multiple sclerosis.

31. The use of claim 23 or 26, wherein said polypeptide is administered in combination with one or more other factors.

32. The use of claim 31, wherein said other neurotrophic factor is CNTF.

33. Use of the polypeptide of claim 1 in the manufacture of a medicament for treating a skeletal tissue deficiency.

34. An assay method for screening test compounds to identify or characterize potentiators of an interaction between a polypeptide according to claim 1 and a hedgehog receptor.

35. An isolated nucleic acid which hybridizes under stringent conditions, including a wash step of 0.2X SSC at 65 °C, to a vertebrate hedgehog nucleic acid sequence selected from SEQ ID NO: 4, which nucleic acid encodes a *hedgehog* amino acid sequence that can (i) promote proliferation, survival, and/or differentiation of mesodermally-derived tissue, and/or (ii) promote proliferation, survival, and/or differentiation of cetodermally-derived tissue.

36. The nucleic acid of claim 35, wherein the *hedgehog* amino acid sequence includes at least 150 contiguous amino acids.

37. The nucleic acid of claim 35, wherein the *hedgehog* amino acid sequence includes at least 100 contiguous amino acids.

38. The nucleic acid of claim 35, wherein the *hedgehog* amino acid sequence includes at least 50 contiguous amino acids.

39. The nucleic acid of claim 35, which nucleic acid hybridizes under stringent conditions, including a wash step of 0.2X SSC at 65 °C, to a nucleic acid probe having a sequence represented by at least 12 consecutive nucleotides of a hedgehog nucleic acid selected from SEQ ID No: 4.

40. The nucleic acid of claim 35 or 36, further comprising a transcriptional regulatory sequence operably linked to said nucleotide sequence so as to render said nucleic acid suitable for use as an expression vector.

41. A recombinant transfection system, comprising
(i) a gene construct including the nucleic acid of claim 35 or 36 and operably linked to a transcriptional regulatory sequence for causing expression of said hedgehog polypeptide in eukaryotic cells, and
(ii) a gene delivery composition for delivering said gene construct to a cell and causing the cell to be transfected with said gene construct.

42. The recombinant transfection system of claim 41, wherein the gene delivery composition is selected from the group consisting of a recombinant viral particle, a liposome and a poly-cationic nucleic acid binding agent.

43. An expression vector, capable of replicating in at least one of a prokaryotic cell and eukaryotic call, comprising the nucleic acid of claim 35 or 36.

44. A host cell transfected with the expression vector of claim 43 and expressing said hedgehog polypeptide.

45. A method of producing a recombinant hedgehog polypeptide comprising culturing the cell of claim 44 in a cell culture medium to express said hedgehog polypeptide and isolating said hedgehog polypeptide from said cell culture.

46. A pharmaceutical composition comprising
(i) at least one hedgehog polypeptide of claim 1 capable of functioning as an agonist of at least one biological activity of a vertebrate hedgehog protein; and
(ii) a pharmaceutically acceptable carrier.

47. The pharmaceutical composition of claim 46, wherein said polypeptide comprises an amino acid sequence selected from SEQ ID No: 11.

## Patentansprüche

1. Isoliertes und/oder rekombinant hergestelltes Hedgehog-Polypeptid von Vertebraten, umfassend eine Aminosäuresequenz, die mindestens zu 90 % mit SEQ ID NO: 11 oder mit einem Fragment davon aus mindestens 50 benachbarten Aminosäuren identisch ist, welches Hedgehog-Polypeptid (i) die Proliferation, das Überleben und/oder die Differenzierung von mesodermal abgeleitetem Gewebe induzieren kann und/oder (ii) die Proliferation, das Überleben und/oder die Differenzierung von ektodermal abgeleitetem Gewebe induzieren kann.

2. Isoliertes und/oder rekombinant hergestelltes Hedgehog-Polypeptid von Vertebraten nach Anspruch 1, worin das Polypeptid eine Aminosäuresequenz umfasst, die mindestens zu 90 % mit SEQ ID NO: 11 oder einem Fragment davon aus mindestens 150 benachbarten Aminosäuren identisch ist, welches Polypeptid (i) die Proliferation, das Überleben und/oder die Differenzierung von mesodermal abgeleitetem Gewebe induzieren kann und/oder (ii) die Proliferation, das Überleben und/oder die Differenzierung von ektodermal abgeleitetem Gewebe induzieren kann.

3. Polypeptid nach Anspruch 1, worin das Polypeptid ein Molekulargewicht von ca. 27 kd aufweist.

4. Polypeptid nach Anspruch 1, worin das Polypeptid ein Molekulargewicht von ca. 19 kd aufweist.

5. Polypeptid nach Anspruch 1, worin das Polypeptid einen N-terminalen Anteil von SEQ ID NO: 11 umfasst.

6. Polypeptid nach Anspruch 1, worin (i) die Hedgehog-Aminosäuresequenz durch ein natürlich vorkommendes Hedgehog-Gen von einer Maus kodiert ist, (ii) die Hedgehog-Aminosäuresequenz ein N-terminales Fragment von einem Hedgehog-Protein mit einem Molekulargewicht von ca. 19 kd umfasst (iii) das Polypeptid die Expression von BMP-2-, BMP-4- oder Hoxd-Genen induziert, (iv) das Polypeptid gereinigt wird, um weniger als 20 % kontaminierende Proteine bezogen auf das Trockengewicht aufzuweisen, und/oder (v) das Polypeptid gereinigt wird, um weniger als 5 % kontaminierende Proteine bezogen auf das Trockengewicht aufzuweisen.

7. Polypeptid nach Anspruch 1, worin das Fragment davon mindestens 100 benachbarte Aminosäuren einschließt.

8. Polypeptid nach Anspruch 2, worin das Polypeptid eine mit der SEQ ID NO: 11 identische Aminosäuresequenz oder ein Fragment davon aus mindestens 150 benachbarten Aminosäuren umfasst.

9. Polypeptid nach einem der Ansprüche 1 oder 3, worin das Polypeptid eine durch SEQ ID NO: 40 repräsentierte Aminosäuresequenz umfasst.

10. Polypeptid nach einem der Ansprüche 1 bis 9, worin die Aminosäuresequenz durch eine Nukleinsäuresequenz kodiert ist, die unter stringenten Bedingungen, einschließlich eines Waschschritts mit 0,2X SSC bei 65 °C, mit der Hedgehog-Nukleinsäuresequenz von Vertebraten von SEQ ID NO: 4 hybridisiert.

11. Polypeptid nach einem der Ansprüche 1, 2, 6 und 10, worin das Polypeptid ein Fusionsprotein ist.

12. Polypeptid nach Anspruch 11, worin das Fusionsprotein ferner Folgendes einschließt:
(i) eine nachweisbare Markierung zum Nachweis der Anwesenheit des Fusionsproteins, oder
(ii) eine Matrix-bindende Domäne zum Immobilisieren des Fusionsproteins.

13. Polypeptid nach einem der Ansprüche 1, 2, 6 und 10, worin das Polypeptid posttranslational modifiziert ist.

14. Polypeptid nach Anspruch 13, worin das Polypeptid glykosyliert ist.

15. Polypeptid nach einem der Anspruch 1, 2, 10 und 13, worin (i) das Polypeptid auf mindestens 80 % bezogen auf das Trockengewicht gereinigt ist oder (ii) das Polypeptid auf mindestens 95 % bezogen auf das Trockengewicht gereinigt ist.

16. Präparat einschließlich des Polypeptids nach Anspruch 1, das in einem pharmazeutisch verträglichen Medium, Vehikel oder Verdünnungsmittel formuliert ist.

17. Antikörperpräparat, das spezifisch reaktiv mit einem Epitop des Hedgehog-Polypeptids nach Anspruch 1 ist.

18. Antikörperpräparat, das an ein Epitop des Hedgehog-Polypeptids nach Anspruch 1 bindet.

19. Antikörperpräparat nach Anspruch 17 oder 18, worin das Präparat einen monoklonalen Antikörper umfasst.

20. Antikörperpräparat nach Anspruch 17 oder 18, worin das Präparat polyklonale Antikörper umfasst.

21. Verwendung des Polypeptids nach Anspruch 1 zur Herstellung eines Arzneimittels zur Behandlung einer degenerativen Erkrankung des Nervensystems, die durch neuronalen Zelltod **gekennzeichnet** ist.

22. Verwendung nach Anspruch 21, worin die degenerative Erkrankung aus der Gruppe ausgewählt ist, bestehend aus einer neuromuskulären Erkrankung, einer autonomen Erkrankung, einer Erkrankung des Zentralnervensystems, der Alzheimer-Krankheit, der Parkinson-Krankheit, der amyotrophen Lateralsklerose, der Pick-Krankheit, der Chorea Huntington, der multiplen Sklerose, einer neuronalen Schädigung aufgrund einer Anoxie-Ischämie, einer neuronalen Schädigung aufgrund eines Traumas und einer den natürlichen Alterungsprozess begleitenden neuronalen Degeneration

23. Verwendung nach Anspruch 21, worin das Arzneimittel ferner eine therapeutisch wirksame Menge eines Wachstumsfaktors mit neurotropher Aktivität umfasst.

24. Verwendung nach Anspruch 23, worin der Wachstumsfaktor aus der Gruppe ausgewählt ist, bestehend aus einem Nervenwachstumsfaktor, einem ziliären neurotrophen Wachstumsfaktor, einem Schwannom-abgeleiteten Wachstumsfaktor, einem Gliawachstumsfaktor, einem Striatum-abgeleiteten neuronotrophen Faktor und einem Thrombozyten-abgeleiteten Wachstumsfaktor.

25. Verwendung des Polypeptids nach Anspruch 1 in der Herstellung eines Arzneimittels zur Behandlung einer neurodegenerativen Erkrankung.

26. Verwendung nach Anspruch 25, worin die Erkrankung ausgewählt ist aus: der Parkinson-Krankheit, der Alzheimer-Krankheit, der Chorea Huntington, der Pick-Krankheit, dem Ballismus, dem Guillain-Barré-Syndrom, der amyotrophen Lateralsklerose, den spinozerebellären Degenerationen und der peripheren Neuropathie.

27. Verwendung nach Anspruch 25, worin die neurodegenerative Erkrankung den Verlust der Neuronenzellen, ausgewählt aus cholinergen Neuronen, GABAergen Neuronen und striatalen Neuronen, einschließt

28. Verwendung des Polypeptids nach Anspruch 1 in der Herstellung eines Arzneimittels zur Prävention, Behandlung oder Reduktion des Schweregrads einer akuten, subakuten oder chronischen Verletzung des Nervensystems eines Patienten.

29. Verwendung nach Anspruch 28, worin die Verletzung ausgewählt ist aus: einer traumatischen Verletzung, einer chemischen Verletzung, einer vasalen Verletzung, einem nasalen Defizit, einer infektiösen Verletzung, einer inflammatorischen Verletzung, einer chronischen immunologischen Erkrankung, einer Tumor-induzierten Verletzung oder einer Ischämie aufgrund eines Schlaganfalls.

30. Verwendung nach Anspruch 29, worin die immunologische Erkrankung multiple Sklerose darstellt

31. Verwendung nach Anspruch 23 oder 26, worin das Polypeptid in Kombination mit einem oder mehr anderen neurotrophen Faktor(en) verabreicht wird.

32. Verwendung nach Anspruch 31, worin der andere neurotrophe Faktor den CNTF (ziliären neurotrophen Faktor) darstellt.

33. Verwendung des Polypeptids nach Anspruch 1 bei der Herstellung eines Arzneimittels zur Behandlung einer Defizienz des Skelettgewebes.

34. Assay-Verfahren zum Screening von Testverbindungen zum Identifizieren oder Charakterisieren von Potenziatoren einer Interaktion zwischen einem Polypeptid nach Anspruch 1 und einem Hedgehog-Rezeptor.

35. Isolierte Nukleinsäure, die unter stringenten Bedingungen, einschließlich eines Waschschritts mit 0,2X SCC bei 65 °C, an eine aus SEQ ID NO: 4 ausgewählte Hedgehog-Nukleinsäuresequenz von Vertebraten hybridisiert, welche Nukleinsäure für eine Hedgehog-Aminosäuresequenz kodiert, die (i) die Proliferation, das Überleben und/oder die Differenzierung von mesodermal abgeleitetem Gewebe fördern kann und/oder (ii) die Proliferation, das Überleben und/oder die Differenzierung von ektodermal abgeleitetem Gewebe fördern kann.

36. Nukleinsäure nach Anspruch 35, worin die Hedgehog-Aminosäuresequenz mindestens 150 benachbarte Aminosäuren einschließt.

37. Nukleinsäure nach Anspruch 35, worin die Hedgehog-Aminosäuresequenz mindestens 100 benachbarte Aminosäuren einschließt.

38. Nukleinsäure nach Anspruch 35, worin die Hedgehog-Aminosäuresequenz mindestens 50 benachbarte Aminosäure einschließt.

39. Nukleinsäure nach Anspruch 35, worin die Nukleinsäure unter stringenten Bedingungen, einschließlich eines Waschschritts mit 0,2X SSC bei 65 °C, an eine Nukleinsäuresonde mit einer Sequenz hybridisiert, die durch mindestens 12 konsekutive Nukleoside von einer aus SEQ ID NO: 4 ausgewähltes Hedgehog-Nukleinsäure repräsentiert ist.

40. Nukleinsäure nach Anspruch 35 oder 36, die ferner eine transkriptionale regulatorische Sequenz umfasst, die operativ mit der Nukleotidsequenz dergestalt verknüpft ist, um die Nukleinsäure zur Verwendung als einen Expressionsvektor geeignet zu machen.

41. Rekombinantes Transfektionssytem umfassend:
(i) ein Genkonstrukt, das die Nukleinsäure nach Anspruch 35 oder 36 einschließt, und operativ mit einer transkriptionalen regulatorischen Sequenz zur Herbeiführung der Expression des Hedgehog-Polypeptids in eukaryoten Zellen verknüpft ist, und
(ii) eine Gen-Abgabezusammensetzung zur Abgabe des Genkonstrukts an eine Zelle und wobei veranlasst wird, dass die Zelle mit dem Genkonstrukt transfiziert wird.

42. Rekombinantes Transfektionssystem nach Anspruch 41, worin die Genabgabezusammensetzung aus der Gruppe ausgewählt ist, die aus einem rekombinanten viruspartikel, einem Liposom und einem polykationischen Nukleinsäure-Bindemittel besteht.

43. Expressionsvektor, der zum Replizieren in mindestens einer von einer prokaryoten Zelle und eukaryoten Zelle fähig ist, umfassend die Nukleinsäure nach Anspruch 35 oder 36.

44. Wirtszelle, die mit dem Expressionsvektor nach Anspruch 43 transfiziert ist, und das Hedgehog-Palypeptid exprimiert.

45. Verfahren zum Herstellen eines rekombinanten Hedgehog-Polypeptids, umfassend das Kultivieren der Zelle nach Anspruch 44 in einem Zellkulturmedium zum Exprimieren des Hedgehog-Polypeptids und Isolieren des Hedgehog-Polypeptids aus der Zellkultur.

46. Pharmazeutische Zusammensetzung, umfassend:
(i) mindestens ein Hedgehog-Polypeptid nach Anspruch 1, das zum Funktionieren als ein Agonist von mindestens einer biologischen Aktivität eines Hedgehog-Proteins von Vertebraten fähig ist; und
(ii) einen pharmazeutisch verträglichen Träger.

47. Pharmazeutische Zusammensetzung nach Anspruch 46, worin das Polypeptid eine aus SEQ ID NO: 11 ausgewählte Aminosäuresequenz umfasst.

## Revendications

1. Polypeptide hedgehog de vertébrés isolé et/ou produit par recombinaison comprenant une séquence d'acides aminés qui est au moins 90 pour-cent identique à la SEQ ID NO: 11, ou à un fragments de celle-ci d'au moins 50 acides aminés contigus, lequel polypeptide hedgehog peut (ï) induire une prolifération, une survie et/ou une différenciation d'un tissu dérivé mésodermiquement et/ou (ii) induire une prolifération, une survie et/ou une différenciation d'un tissu dérivé ectodermiquement.

2. Polypeptide hedgehog de vertébrés isolé et/ou produit par recombinaison selon la revendication 1, où ledit polypeptide comprend une séquence d'acides animés qui est au moins 90 pour-cent identique à la SEQ ID NO:11, ou à un fragment de celle-ci d'au moins 150 acides aminés contigus, lequel polypeptide peut (i) induire une proliferation, une survie et/ou une différentiation d'un tissu dérivé mésodermiquement et/ou (ii) induire une prolifération, une survie et/ou une différenciation d'un tissu dérivé ectodermiquement.

3. Polypeptide selon la revendication 1, où ledit polypeptide possède un poids moléculaire approximatif de 27 kd.

4. Polypeptide selon la revendications 1, où ledit polypeptide possède un poids moléculaire approximatif de 19 kd.

5. Polypeptide selon la revendication 1, où ledit polypeptide comprend une portion N-terminale de la SEQ ID NO:11.

6. Polypeptide selon la revendication 1, dans lequel (i) la séquence d'acides aminés hedgehog est codée par un gène hedgehog d'origine naturelle d'une souris, (ii) la séquence d'acides aminés hedgehog comprend un fragment N-terminal d'une protéine hedgehog avec un poids moléculaire approximatif de 19 kd, (iii) le polypeptide induit l'expression de BMP-2, de BMP-4 ou de gènes ou Hoxd, (iv) le polypeptide est purifié pour avoir moins de 20% en poids sec de protéines de contamination et/ou (v) le polypeptide est purifié pour avoir moins de 5% en poids sec de protéines de contamination.

7. Polypeptide selon la revendication 1, dans lequel le fragment de celui-ci inclut au moins 100 acides aminés contigus.

8. Polypeptide selon la revendication 2, où ledit polypeptide comprend une séquence d'acides aminés identique à la SEQ ID NO:11, ou un fragments de celle-ci d'au moins 150 acides aminés contigus.

9. Polypeptide selon l'une quelconque des revendications 1 et 3, où le polypeptide comprend une séquence d'acides aminés représentée par la SEQ ID NO:40.

10. Polypeptide selon l'une quelconque des revendications 1 à 9. dans lequel la séquence d'acides aminés est codée par une séquence d'acide nucléique qui s'hybride dans des conditions strictes, incluant une étape de lavage de 0,2X SSC à 65°C, avec la séquence d'acide nucléique hedgehog de vertébrés de la SEQ ID NO:4.

11. Polypeptide selon l'une quelconque des revendications 1. 2, 6 et 10, où le polypeptide est une protéine de fusion.

12. Polypeptide selon la revendication 11, dans lequel la protéine de fusion inclut en outre:
(i) un marqueur détectable pour la détection de la présence de ladite protéine de fusion, ou
(ii) un domaine de liaison de matrice pour l'immobilisation de ladite protéine de fusion.

13. Polypeptide selon l'une quelconque des revendications 1, 2, 6 et 10, où le polypeptide est modifié après la traduction.

14. Polypeptide selon la revendication 13, où le polypeptide est glycosylé.

15. Polypeptide selon l'une quelconque des revendications 1, 2, 10 et 13, où (i) le polypeptide est purifié à au moins 80% en poids sec ou (ii) le polypeptide est purifié à au moins 95% en poids sec.

16. Préparation incluant le polypeptide selon la revendication 1, formulé dans un milieu, véhicule ou diluant pharmaceutiquement acceptable.

17. Préparation d'anticorps spécifiquement réactive avec un épitope du polypeptide hedgehog selon la revendication 1.

18. Préparation d'anticorps qui se lie à un épitope du polypeptide hedgehog selon la revendication 1.

19. Préparation d'anticorps selon la revendication 17 ou 18, où la préparation comprend un anticorps monoclonal.

20. Préparation d'anticorps selon la revendication 17 ou 18, où la préparation comprend des anticorps polyclonaux.

21. Utilisation du polypeptide selon la revendication 1, pour la préparation d'un médicament pour le traitement d'un trouble dégénératif du système nerveux **caractérisé par** une mort de cellules neuronales.

22. Utilisation selon la revendication 21, dans laquelle le trouble dégénératif est choisi dans le groupe constitué d'un trouble neuromusculaire, d'un trouble autonome, d'un trouble du système nerveux central, de la maladie d'Alzheimer, de la maladie de Parkinson, d'une sclérose latérale amyotrophique, de la maladie de Pick, de la maladie de Huntington, d'une sclérose en plaques, d'un dommage neuronal résultant d'une anoxie-ischémie, d'un dommage neuronal résultant d'un traumatisme et d'une dégénérescence neuronale associée à un processus de vieillissement naturel.

23. Utilisation selon la revendication 21, dans laquelle le médicament comprend en outre une quantité thérapeutiquement efficace d'un facteur de croissance présentant une activité neurotrophique.

24. Utilisation selon la revendication 23, dans laquelle ledit facteur de croissance est choisi dans le groupe constitué d'un facteur de croissance neuronal, d'un facteur de croissance neurotrophique ciliaire, d'un facteur de croissance dérivé de schwannomes, d'un facteur de croissance glial, d'un facteur neurotrophique dérivé du striatum et d'un facteur de croissance dérivé des plaquettes.

25. Utilisation du polypeptide selon la revendication 1. dans la fabrication d'un médicament pour le traitement d'un trouble neurodégénératif.

26. Utilisation selon la revendication 25, dans laquelle ledit trouble est choisi parmi la maladie de Parkinson, la maladie d'Alzheimer, la maladie de Huntington, la maladie de Pick, le ballisme, le syndrome de Guillain-Barre, une sclérose latérale amyotrophique, des dégénérescences spinocérébelleuses et une neuropathie périphérique.

27. Utilisation selon la revendication 25, dans laquelle ledit trouble neurodégénératif inclut une perte de cellules neuronales choisies parmi des neurones cholinergiques, des neurones GABAergiques et des neurones striataux.

28. Utilisation du polypeptide selon la revendication 1, dans la fabrication d'un médicament pour la prévention, le traitement ou séduction de la sévérité d'une lésion aiguë, subaiguë ou chronique du système nerveux chez un sujet.

29. Utilisation selon la revendication 28, dans laquelle ladite lésion est choisie parmi une lésion traumatique, une lésion chimique, une légion de vaisseau, un déficit de vaisseau, une lésion infectieuse, une lésion inflammatoire, une maladie immunologiqne chronique, une lésion induite par une tumeur ou une ischémie résultant d'une attaque.

30. Utilisation selon la revendication 29, dans laquelle ladite maladie immunologique est une sclérose en plaques.

31. Utilisation selon la revendication 23 ou 26, dans laquelle ledit polypeptide est administré en combinaison avec un ou plusieurs autres facteurs neurotrophiques.

32. Utilisation selon la revendication 31, dans laquelle ledit autre facteur neurotrophique est CNTF.

33. Utilisation du polypeptide selon la revendication 1, dans la fabrication d'un médicament pour le traitement d'une carence en tissu squelettique.

34. Méthode de dosage biologique pour cribler des composés d'essai afin d'identifier ou de caractériser des potentialisateurs d'une interaction entre un polypeptide selon la revendication 1 et un récepteur hedgehog.

35. Acide nucléique isolé qui s'hybride dans des conditions strictes, incluant une étape de lavage de 0,2X SSC à 65°C, à une séquence d'acide nucléique hedgehog de vertébrés choisie parmi la SEQ ID NO:4, lequel acide nucléique code une séquence d'acides aminés hedgehog qui peut (i) activer une prolifération, une survie et/ou une différenciation d'un tissu dérivé mésodermiquement et/ou (ii) activer une prolifération, une survie et/ou une différenciation d'un tissu dérivé ectodermiquement.

36. Acide nucléique selon la revendication 35, dans lequel la séquence d'acides aminés hedgehog inclut au moins 150 acides aminés contigus.

37. Acide nucléique selon la revendication 35, dans lequel la séquence d'acides aminés hedgehog inclut au moins 100 acides aminés contigus.

38. Acide nucléique selon la revendication 35, dans lequel la séquence d'acides aminés hedgehog inclut au moins 50 acides aminés contigus.

39. Acide nucléique selon la revendication 35, lequel acide nucléique s'hybride dans des conditions strictes, incluant une étape de lavage de 0,2X SSC à 65°C, à une sonde d'acide nucléique possédant une séquence représentée par au moins 12 nucléotides consécutifs d'un acide nucléique hedgehog choisi parmi la SEQ ID NO:4.

40. Acide nucléique selon la revendication 35 ou 36, comprenant en outre une séquence régulatrice transcriptionnelle liée fonctionnellement à ladite séquence de nucléotides de manière à rendre ledit acide nucléique approprié pour une utilisation en tant que vecteur d'expression.

41. Système de transfection recombinant comprenant:
(i) une construction de gène incluant l'acide nucléique selon la revendication 35 ou 36 et liée fonctionnellement à une séquence régulatrice transcriptionnelle pour entraîner l'expression dudit polypeptide hedgehog dans des cellules eucaryotes, et
(ii) une composition de distribution de gène pour distribuer ladite construction de gène à une cellule et entraîner la transfection de la cellule avec ladite construction de gène.

42. Système de transfection recombinant selon la revendication 41, dans lequel la composition de distribution de gène est choisie dans le groupe constitué d'une particule virale recombinante, d'un liposome et d'un agent de liaison d'acide nucléique poly-cationique.

43. Vecteur d'expression, capable de réplication dans au moins une parmi une cellule procaryote et une cellule eucaryote, comprenant l'acide nucléique selon la revendication 35 ou 36.

44. Cellule hôte transfectée avec le vecteur d'expression selon la revendication 43 et exprimant ledit polypeptide hedgehog.

45. Méthode pour la production d'un polypeptide hedgehog recombinant comprenant la culture de la cellule selon la revendication 44 dans un milieu de culture de cellules pour exprimer ledit polypeptide hedgehog et l'isolement dudit polypeptide hedgehog à partir de ladite culture de cellules.

46. Composition pharmaceutique comprenant:
(i) au moins un polypeptide hedgehog selon la revendication 1 capable d'agir comme un agoniste d'au moins une activité biologique d'une protéine hedgehog de vertébrés; et
(ii) un support pharmaceutiquement acceptable.

47. Composition pharmaceutique selon la revendication 46, dans laquelle ledit polypeptide comprend une séquence d'acides aminés choisie parmi la SEQ ID NO: 11.
